(19) Europäisches Patentamt
European Patent Office
Office européen des brevets

(11) EP 1 490 398 B1

(12) **EUROPEAN PATENT SPECIFICATION**

(45) Date of publication and mention of the grant of the patent:
**05.10.2011 Bulletin 2011/40**

(21) Application number: **03722712.1**

(22) Date of filing: **27.03.2003**

(51) Int Cl.:
***C07K 14/47*** *(2006.01)*

(86) International application number:
**PCT/GB2003/001378**

(87) International publication number:
**WO 2003/082916 (09.10.2003 Gazette 2003/41)**

(54) **TUMOUR ASSOCIATED ANTIGENS**

TUMOR ASSOZIIERTE ANTIGENE

ANTIGENES ASSOCIES A UNE TUMEUR

(84) Designated Contracting States:
**AT BE BG CH CY CZ DE DK EE ES FI FR GB GR HU IE IT LI LU MC NL PT RO SE SI SK TR**

(30) Priority: **27.03.2002 GB 0207251**

(43) Date of publication of application:
**29.12.2004 Bulletin 2004/53**

(73) Proprietor: **ISIS INNOVATION LIMITED**
**Summertown,**
**Oxford,**
**Oxfordshire OX2 7BZ (GB)**

(72) Inventors:
- **BANHAM, Alison,**
**Nuffield Dept Clinical Lab. Sci.**
**Headington,**
**Oxford OX3 9DU (GB)**
- **PULFORD, Karen,**
**Nuffield Dept Clinical Lab. Sci.**
**Headington,**
**Oxford OX3 9DU (GB)**
- **ANDERSON, Amanda,**
**Nuffield Dept Clinical Lab. Sci.**
**Headington,**
**Oxford OX3 9DU (GB)**
- **GUINN, Barbara,**
**Nuffield Dept Clinical Lab. Sci.**
**Headington,**
**Oxford OX3 9DU (GB)**

(74) Representative: **Baldock, Sharon Claire et al**
**Boult Wade Tennant**
**Verulam Gardens**
**70 Gray's Inn Road**
**London WC1X 8BT (GB)**

(56) References cited:
**WO-A-01/62917 WO-A-03/011902**
**US-A- 5 922 566**

- **TURECI O ET AL: "SEROLOGICAL ANALYSIS OF HUMAN TUMOR ANTIGENS: MOLECULAR DEFINITION AND IMPLICATIONS" MOLECULAR MEDICINE TODAY, ELSEVIER, CAMBRIDGE, GB, vol. 3, no. 8, August 1997 (1997-08), pages 342-349, XP000985587 ISSN: 1357-4310 cited in the application**
- **CHEN Y-T ET AL: "A TESTICULAR ANTIGEN ABERRANTLY EXPRESSED IN HUMAN CANCERS DETECTED BY AUTOLOGOUS ANTIBODY SCREENING" PROCEEDINGS OF THE NATIONAL ACADEMY OF SCIENCES OF USA, NATIONAL ACADEMY OF SCIENCE. WASHINGTON, US, vol. 94, 1997, pages 1914-1918, XP000960590 ISSN: 0027-8424**
- **JAEGER D ET AL: "CANCER-TESTIS ANTIGENS AND ING1 TUMOR SUPPRESSOR GENE PRODUCT ARE BREAST CANCER ANTIGENS: CHARACTERIZATION OF TISSUE-SPECIFIC ING1 TRANSCRIPTS AND A HOMOLOGUE GENE" CANCER RESEARCH, AMERICAN ASSOCIATION FOR CANCER RESEARCH, BALTIMORE, MD, US, vol. 59, 15 December 1999 (1999-12-15), pages 6197-6204, XP000986298 ISSN: 0008-5472**
- **SAHIN U ET AL: "Serological identification of human tumor antigens" CURRENT OPINION IN IMMUNOLOGY, CURRENT BIOLOGY LTD, XX, vol. 9, no. 5, October 1997 (1997-10), pages 709-716, XP004313590 ISSN: 0952-7915**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 490 398 B1

## Description

The present invention is concerned with lymphoma associated antigens and in particular with nucleic molecules encoding such antigens and the polypeptides encoded therefrom. The invention further relates to treating such lymphomas, methods for their diagnosis and using the polypeptides and nucleic acid molecules of the invention.

EP 1580263 is a divisional application derived from EP 1347046. EP 1580263 claims a priority date of 22 March 2002. The filing date is 12 April 2002. EP 1580263 is citable for novelty purposes for the designated contracting states GB, FR and DE only. SEQ ID NO: 4435 of EP 1580263 is 639 amino acids long. The last amino acid is arginine.

The tumour or lymphoma-associated antigens of the present invention and the nucleic acid molecules encoding them are believed to be important markers that can be used for prognostic purposes or therapeutic treatment for lymphomas of various types such as diffuse large B cell lymphomas (DLBCL). Phenotypic changes which distinguish a tumour cell from a normal cell are often the result of one or more chromosomal abnormalities, gene mutations or changes in gene regulation. The genes can therefore be expressed in tumour cells but not the normal counterpart, can be over expressed in the tumour cells compared to normal cells, or may be aberrantly expressed at different stages of the cell maturation. These genes can be described as 'tumour associated' genes and are therefore useful markers for the tumour phenotype. The tumour markers or 'tumour antigens', encoded by these genes can thus be targeted by antigen-specific immune mechanisms which lead to the destruction of the tumour cell. Their expression can also be used to diagnose the occurrence of such tumour cells or as prognostic markers.

Differences between a wild type protein expressed by 'normal' cells and a corresponding tumour antigen protein may, in some instances, lead to the tumour antigen being recognised by an individuals immune system as 'non-self' and thereby eliciting an immune response in the individual against the tumour antigen. This may be a humoral (i.e. B cell mediated) immune response leading to the production of autoantibodies immunologically specific to the tumour antigen.

Autoantibodies are naturally occurring antibodies directed to an antigen which the individual's immune system recognises as foreign even though that antigen originated in the individual. They may be present in the circulation as circulating free antibodies or in the form of circulating immune complexes consisting of the autoantibodies bound to their target tumour antigen.

Diffuse large B-cell lymphoma accounts for 30-40% of all adult non-Hodgkin's lymphomas and is heterogeneous in terms of its morphology and clinical features (Harris et al., 1994). Approximately 50% of patients relapse after treatment (Project., 1997) and their tumours frequently become resistant to therapy. The genetic abnormalities underlying DLBCL remain poorly understood and, in contrast to other lymphoma types (e.g. follicular lymphoma (FL) or Burkitt's lymphoma (BL)), no single characteristic genetic alteration has been found.

Genomic instability promotes tumourigenesis and can occur through various mechanisms, including defective segregation of chromosomes or inactivation of DNA mismatch repair. Although some B-cell lymphomas are associated with chromosomal translocations that deregulate oncogene expression, a mechanism for genome-wide instability during lymphomagenesis has not until recently been described. During B-cell development, the immunoglobulin variable (V) region genes are subject to somatic hypermutation in germinal-centre B cells. It has been reported recently that an aberrant hypermutation activity targets multiple loci, including the proto-oncogenes PIM1, MYC, RhoH/TTF (ARHH) and PAX5, in more than 50% of diffuse large-cell lymphomas (DLCLs), which are tumours derived from germinal centres. The mutations described were distributed in the 5' untranslated or coding sequences, were independent of chromosomal translocations, and shared features typical of V-region-associated somatic hypermutation. In contrast to mutations in V regions, however, these mutations were not detectable in normal germinal-centre B cells nor in other germinal-centre-derived lymphomas, suggesting a DLBCL-associated malfunction of somatic hypermutation. Intriguingly, the four hypermutable genes are susceptible to chromosomal translocations in the same region, consistent with a role for hypermutation in generating translocations by DNA double-strand breaks. By mutating multiple genes, and possibly by favouring chromosomal translocations, aberrant hypermutation may represent the major contributing factor to lymphomagenesis (Pasqualucci et al., 2001). It is this phenomenon which may account for the genetic variability in DLBCL.

Microarray based gene expression profiling has been utilised to identify different subtypes of DLBCL with different clinical outcomes. These include a good prognosis germinal centre-like (GC-like) subtype and poor prognosis type 3 and activated B-cell-like (ABC-like) subtypes (Rosenwald et al., 2002). The routine subtyping of DLBCL patients will enable the identification of those that do not respond to current treatment regimens and will improve treatment management.

An important factor concerning the results from gene-expression profiling studies is that the expression of mRNA does not always accurately reflect the levels of protein expression. Many proteins whose expression levels are regulated by mechanisms such as proteolysis would not necessarily show changes in mRNA levels. In addition other proteins are regulated by post translational modification, such as phosphorylation, or by changes in subcellular localisation. Thus the mRNA expression profile will fail to identify many molecules whose protein expression is relevant to the pathogenesis of DLBCL. One approach to the identification of tumour-associated molecules is to exploit circulating antibodies present in patients. Antibodies to tumour-related antigenic molecules have been identified in several non-haematopoietic cancers (Crawford et al., 1982; Lubin et al., 1995; Disis and Cheever, 1996; Jäger et al., 1998; Bei et al., 1999; Mosolits et al., 1999), while antibodies to antigens such as p53, c-myc and viral proteins have also been identified in a number of B cell lymphomas and leukaemias (Caron de Fromental et al., 1987; Kamihira et al., 1989; LaFond et al., 1992; Jarrett and MacKenzie, 1999). In addition, levels of antibodies to tumour idiotypes in B cell lymphomas and myelomas can be increased, *in vivo,* by immunisation (Kwak et al., 1993; Kwak et al., 1995; Syrengelas et al., 1996; Hsu et al., 1997; King et al., 1998). The prognostic relevance of these antibody responses is still under investigation and may depend on the tumour type under investigation.

A recent study of ALCL by the LRF Immunodiagnostics Unit has, for the first time, demonstrated the presence of circulating antibodies to the tumour-specific protein NPM-ALK in all ALK-positive ALCL patients studied (Pulford et al., 2000). The inventors have also shown, in a more recent investigation, that antibodies to another tumour-associated protein, BCL-2, are present in plasma from patients with follicular Lymphoma (FL) (Pulford et al., 2002). This result is important since it demonstrates that, although BCL-2 is a 'self' protein present in normal cells, it can still be recognised as antigenic in patients in whom it appears to be a tumour-associated molecule.

Current therapy does not cure the majority of patients with non-Hodgkins' lymphoma (NHL). The identification of tumour-associated antigens has, however, opened up the possibility of immunotherapy for patients with many different types of tumours who are refractory to conventional treatment (Rosenberg, 1996; Nestle et al., 1998; Marchand et al., 1999; Jäger et al., 2000). CD8-positive cytotoxic T lymphocytes (CTLs) have been shown to play a major role in the cell-mediated recognition of tumour-associated antigens such as NY-ESO-1 (Jäger et al., 1998), tyrosinase, gp100 and MART-1/Melan-A (Boon and Old, 1997). Tetrameric soluble class I MHC/peptide complexes (tetramers) have also been used with great success to identify and monitor the presence of CTLs in the peripheral blood and tumour infiltrated lymph nodes of melanoma patients (Dunbar et al., 1998; Romero et al., 1998; Jäger et al., 2000). In the case of FL, immunisation studies have demonstrated that an anti-tumour B and T cell response of these patients can be induced following the *in vivo* administration of either DNA vaccines or purified preparations of idiotypic proteins (i.e. patient specific proteins produced by the lymphoma cells) (Hsu et al., 1997; Stevenson, 1999; Stevenson et al., 2001). Other types of immunotherapeutic treatments currently under investigation include a) the administration of autologous antigen loaded dendritic cells (DCs) to patients (Nestle et al., 1998), b) increasing the expression of co-stimulatory molecules, such as CD80 and CD86 and CTLA4, by the autologous tumour cells or DCs increasing their capacity as antigen presenting cells (Tarte et al., 1999; Takayama et al., 2000), and c) the use of toxins linked to monoclonal antibodies specific for tumour-associated antigens (Grillo-Lopez et al., 2001). The discovery of tumour-associated molecules also identified proteins which may be targets for therapies using anti-sense oligonucleotides (Banerjee, 1999). Currently explored and future innovative therapies for B cell lymphomas and other tumours are reviewed by (Schultze, 1997; Jäger et al., 2000; Smith and Cerundolo, 2001; Stevenson et al., 2001).

The SEREX technique (serological analysis of recombinant cDNA expression libraries) was first used by Sahin et al., 1995. This technique has now been used in a number of laboratories to identify a range of tumour associated antigens (Ling et al., 1998; Scanlan et al., 1998; Itoh et al., 1999) reviewed by (Türeci et al., 1999). These have included molecules that were originally identified by cloning cytotoxic T lymphocyte (CTL)-recognised epitopes (van der Bruggen et al., 1991; Brichard et al., 1993), e.g. MAGE-1 and tyrosinase (Sahin et al., 1995). This technique thus enables the detection of tumour antigens that elicit both cellular and humoral immunity. The SEREX approach is characterised by the analysis of antigens that elicit high-titre IgG responses that usually depend on cognate T cell help in the patient *in vivo* (Tureci et al., 1997). As such, it provides a direct route to the analysis of the CD4 T-cell repertoire against tumour antigens (Old and Chen 1998). There is growing evidence that the antitumour response is a process integrating different effectors of the immune system, and studies have shown the capability of SEREX to retrieve tumour-associated antigens (TAAs) initially defined using CTL epitope approaches (van der Bruggen et al., 1994), and the co-existence of cellular and immune responses has been reported against tumour antigens such as NY-ESO-1 (Jager et al., 1998) and HER-2/neu (Disis et al., 1994).

The original SEREX technique relied on the detection of antigens expressed by a tumour derived cDNA library that was screened with autologous serum (Türeci et al., 1997). However, one variant of this technique aims to identify tumour antigens expressed solely in normal testis and germline cells (Türeci et al., 1998). Since testis is protected from the immune system by the blood-testis barrier, these cancer testis antigens can be considered immunologically tumour-specific (Takahashi et al., 1995; Chen and Old, 1999) and, as such, are not expected to trigger tolerance or induce autoimmunity when used in tumour immunotherapy applications (Gilboa, 2001).

Ideal cancer vaccine candidates should be selectively expressed in tumour tissue and be detectable in most patients. The enormous potential of Cancer-Testis Antigens (CTAs) as vaccine targets is based on their restricted pattern of expression and their high frequency of immunogenicity in cancer patients (Scanlan and Jager 2001). However, the majority of SEREX-defined antigens have a much broader expression profile in healthy tissue (Krackhardt et al., 2002), which may hinder their usefulness as direct vaccines. Recent work has shown that the co-presentation of a plasmid encoding a SEREX defined antigen with a tumour specific protein leads to a profound increase in CD8+ T cells specific for the tumour protein not achieved when immunization is only with the tumour protein (Nishikawa et al., 2001). This co-immunisation of SEREX-defined antigens with tumour proteins presents an attractive alternative vaccine strategy for human cancer immunotherapy and one that will be facilitated by the extensive base of information about SEREX-defined human tumour antigens (Nishikawa et al., 2001).

As explained in more detail in the example provided, the present inventors have, by screening a testis cDNA expresion library with serum from a patient suffering from a lymphoid tumour (DLBCL), identified antigens which are candidates for immunotherapeutic applications. Some of the antigens identified represent novel antigens hitherto unidentified while others represent partially characterised antigens for which no function has yet been ascribed.

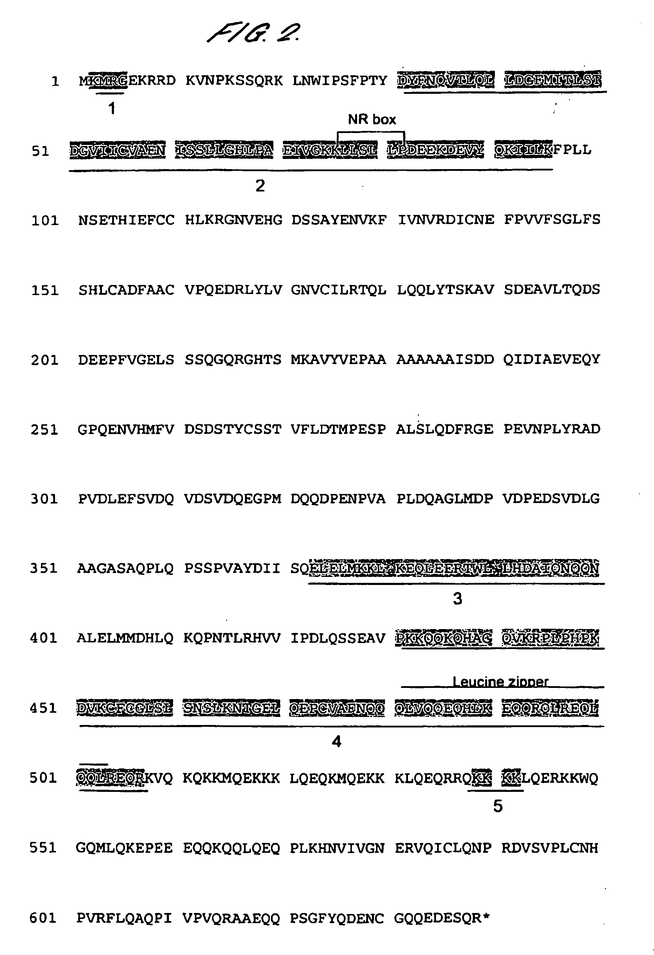

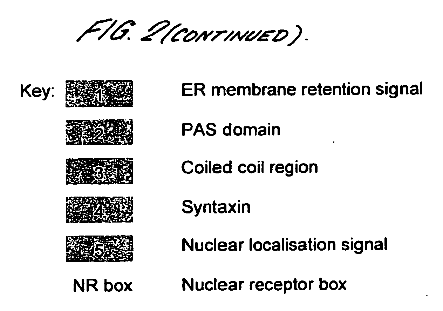

Therefore, according to a first aspect of the invention there is provided a nucleic acid molecule encoding a tumour antigen having the amino acid sequence illustrated in Figure 2. The nucleic acid molecules according to this aspect of the invention encode novel tumour-associated antigens which have not been identified hitherto and which may, advantageously, be utilised to diagnose or treat solid tumours such as kidney, breast, uterus, cervix, colon, lung, stomach, rectum or small intestine. Alternatively, they may be used to diagnose, for example lymphomas, such as DLBCL and therefore represent novel targets for therapy.

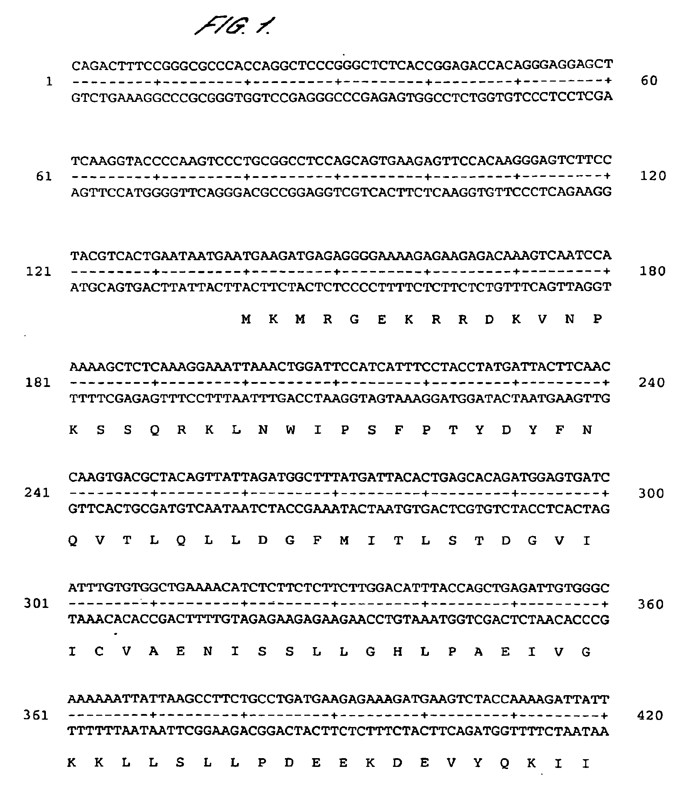

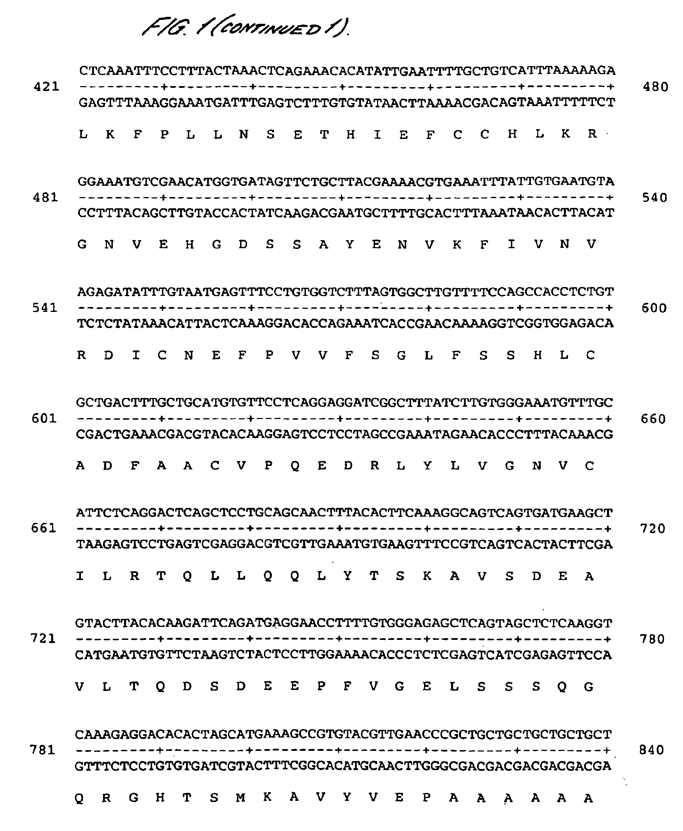

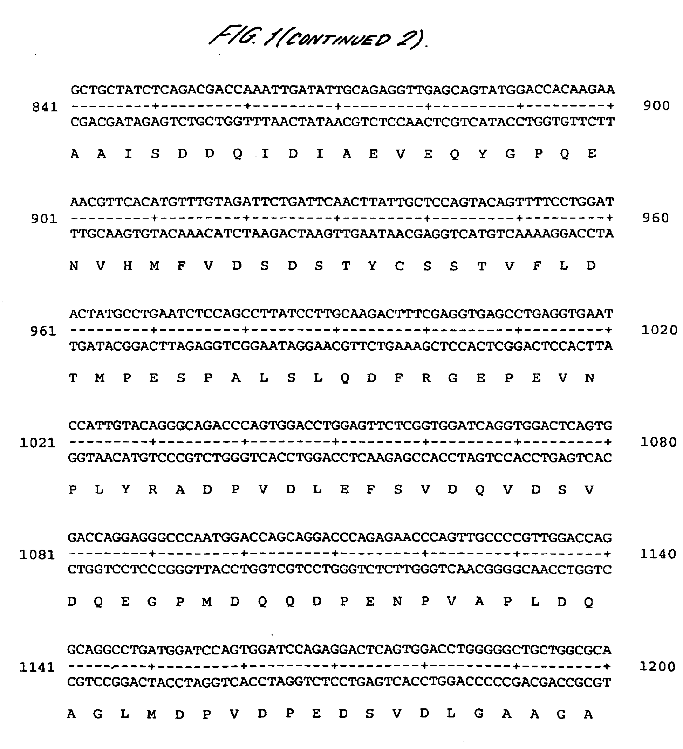

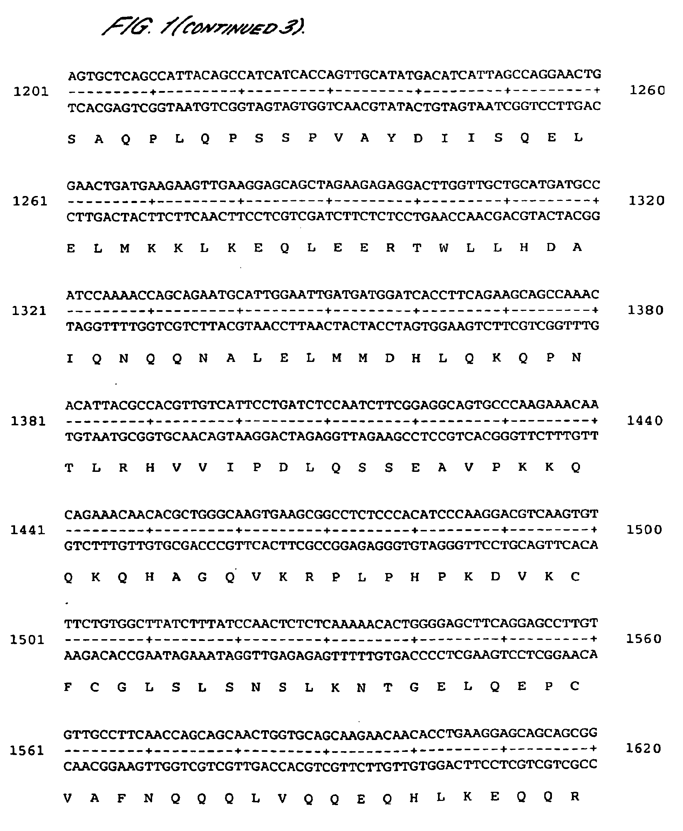

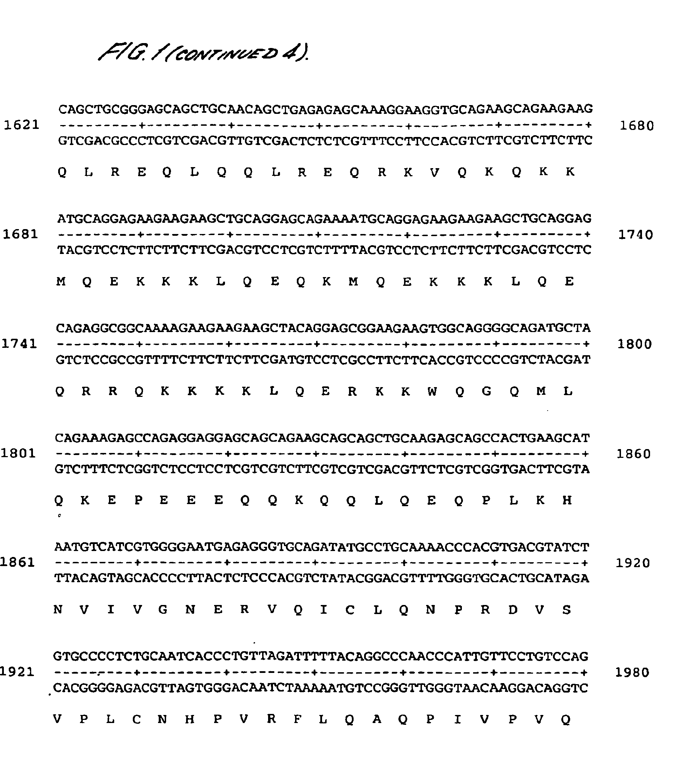

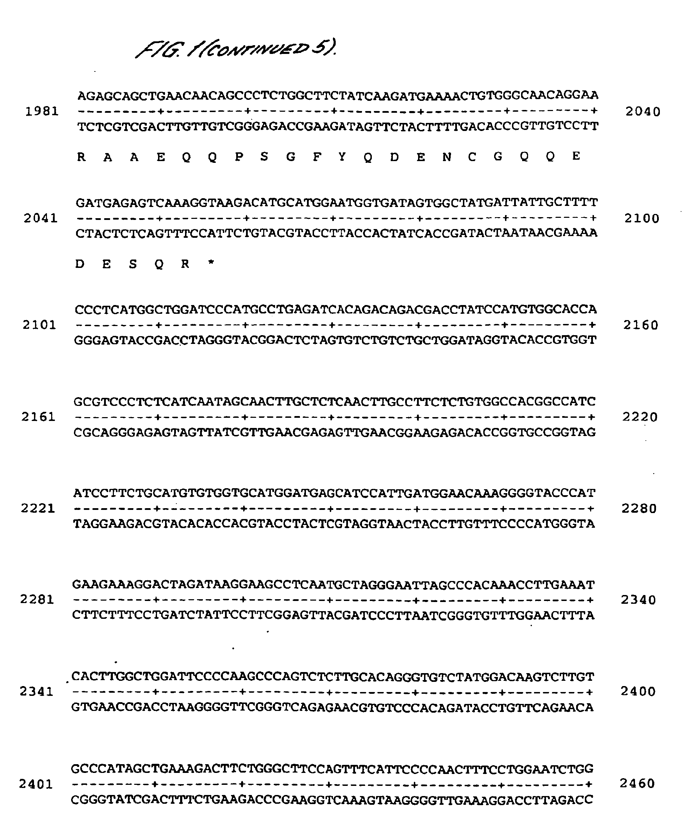

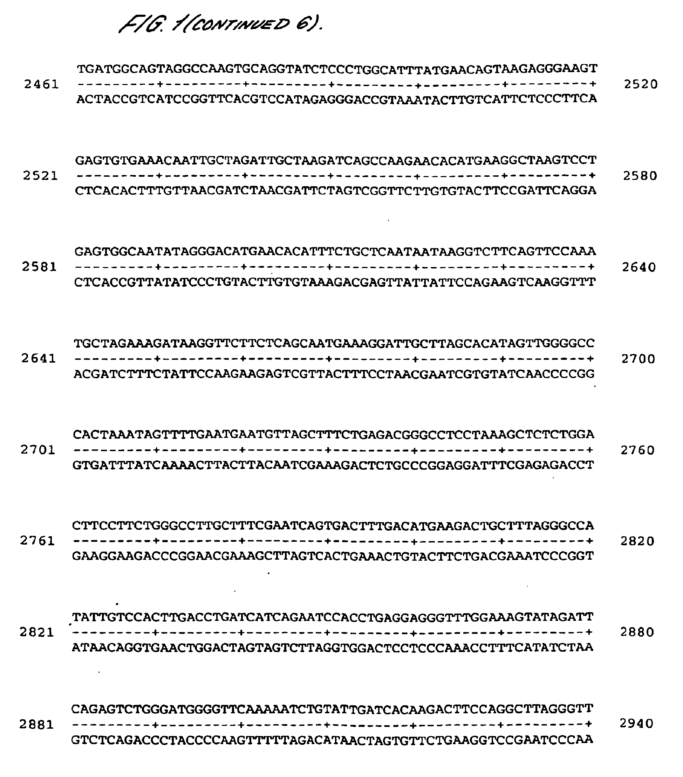

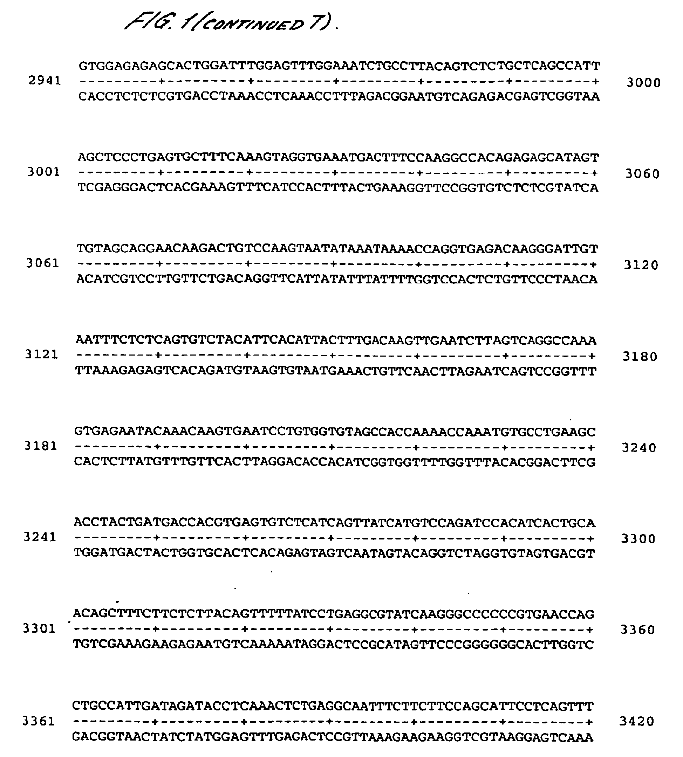

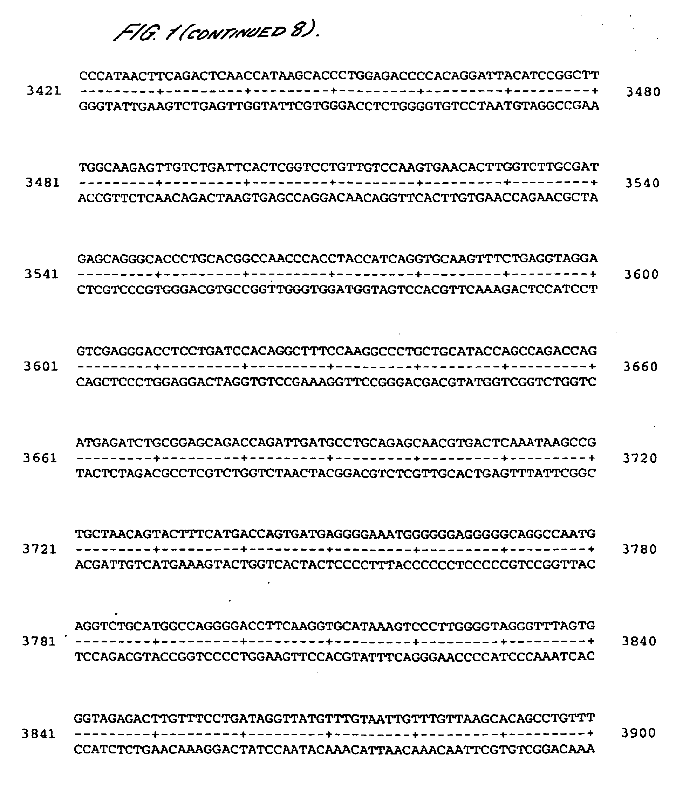

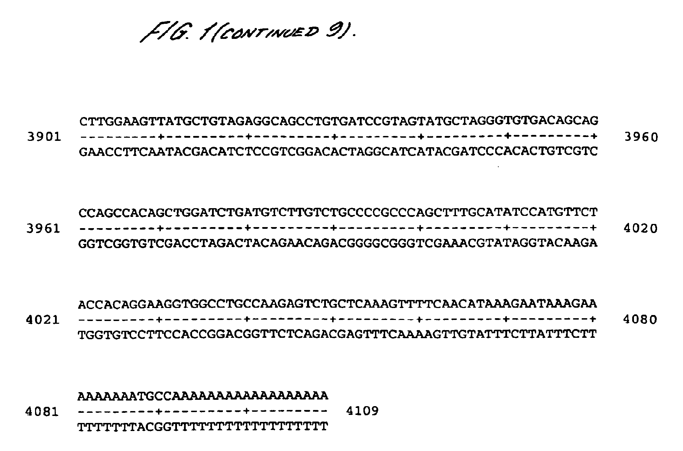

The nucleic acid molecule is preferably DNA and more preferably a cDNA molecules, which is preferably of human origin. In a preferred embodiment, the nucleic acid molecule encoding said antigen comprises the sequence of nucleotides in figure 1.

The antigens themselves encoded by the nucleotides according to the invention also form part of the invention.

A further aspect of the invention comprises nucleic acid molecules capable of hybridising to the nucleic acid molecules of the invention, under conditions of high stringency.

Stringency of hybridisation as used herein refers to conditions under which polynucleic acids are stable. The stability of hybrids is reflected in the melting temperature (Tm) of the hybrids. Tm can be approximated by the formula:

$$81.5°C+16.6(\log_{10}[Na^{+}]+0.41\ (\%G\&C)-600/1$$

wherein 1 is the length of the hybrids in nucleotide. Tm decreases approximately by 1-1.5°C with every 1% decrease in sequence homology.

The term "stringency" refers to the hybridisation conditions wherein a single-stranded nucleic acid joins with a complementary strand when the purine and pyrimidine bases therein pair with their corresponding base by hydrogen bonding. High stringency conditions favour homologous base pairing whereas low stringency conditions favour non-homologous base pairing.

"Low stringency" conditions comprise, for example, a temperature of about 37°C or less, a formamide concentration of less than about 50%, and a moderate to low salt (SSC) concentration; or, alternatively, a temperature of about 50°C or less, and a moderate to high salt (SSPE) concentration, for example 1M NaCl.

"High stringency" conditions comprise, for example, a temperature of about 42°C or less, a formamide concentration of less than about 20%, and a low salt (SSC) concentration; or, alternatively, a temperature of about 65°C, or less, and a low salt (SSPE) concentration. For example, high stringency conditions comprise hybridization in 0.5 M $NaHPO_4$, 7% sodium dodecyl sulfate (SDS), 1 mM EDTA at 65°C (Ausubel, F.M. et al. Current Protocols in Molecular Biology, Vol. I, 1989; Green Inc. New York, at 2.10.3).

"SSC" comprises a hybridization and wash solution. A stock 20X SSC solution contains 3M sodium chloride, 0.3M sodium citrate, pH 7.0.

"SSPE" comprises a hybridization and wash solution. A 1X SSPE solution contains 180 mM NaCl, 9mM $Na_2HPO_4$ and 1 mM EDTA, pH 7.4.

There are other conditions, reagents and so forth which can be used, which result in stringent hybridisation and the skilled practitioner is familiar with such conditions.

The nucleic acid capable of hybridising to nucleic acid molecules according to the invention will generally exhibit at least 70%, preferably at least 80, 85 or 90% and more preferably at least 95% and even more preferably at least 97% similarity or identity to the nucleotide sequences according to the invention.

An antisense molecule capable of hybridising to the nucleic acid according to the invention may be used as a probe or as a medicament or may be included in a pharmaceutical composition with a pharmaceutically acceptable carrier, diluent or excipient therefor to treat the particular lymphomas.

The nucleic acid molecules according to the invention may, advantageously, be included in a suitable expression vector to express the proteins encoded therefrom in a suitable host. Incorporation of cloned DNA into a suitable expression vector for subsequent transformation of said cell and subsequent selection of the transformed cells is well known to those skilled in the art as provided in Sambrook et al. (1989), Molecular cloning: A Laboratory Manual, Cold Spring Harbour Laboratory.

An expression vector, according to the invention, includes a vector having a nucleic acid according to the invention operably linked to regulatory sequences, such as promoter regions, that are capable of effecting expression of said DNA fragments. A vector can include a large number of nucleic acids which can have a desired sequence inserted therein by, for example, using an appropriate restriction enzyme and ligating the sequence in the vector, for transport between cells of different genetic composition. The term "operably linked" refers to a juxtaposition wherein the components described are in a relationship permitting them to function in their intended manner. Such vectors may be transformed into a suitable host cell to provide for expression of a protein according to the invention. The vectors are usually capable of replicating within a host environment and they also comprise one of a number of restriction sites for endonucleases which permits them to be cut in a selective manner at a particular location for insertion of a new nucleic acid molecule or sequence therein. Thus, in a further aspect, the invention provides a process for preparing polypeptides according to the invention, which comprises cultivating a host cell, transformed or transfected with an expression vector as described above under conditions to provide for expression by the vector of a coding sequence encoding the protein, and recovering the expressed protein.

In this regard, the nucleic acid molecule may encode a mature protein or a protein having a prosequence, including encoding a leader sequence on the preprotein which is cleaved by the host cell to form a mature protein.

The vectors may be, for example, plasmid, virus or phagemid vectors provided with an origin of replication, and optionally a promoter for the expression of said nucleotide and optionally a regulator of the promoter. The vectors may contain one or more selectable markers, such as, for example, an antibiotic resistance.

Regulatory elements required for expression include promoter sequences to bind RNA polymerase and to direct an appropriate level of transcription initiation and also translation initiation sequences for ribosome binding. For example, a bacterial expression vector may include a promoter such as the lac promoter and for translation initiation the Shine-Dalgarno sequence and the start codon AUG. Similarly, a eukaryotic expression vector may include a heterologous or homologous promoter for RNA polymerase II, a downstream polyadenylation signal, the start codon AUG, and a termination codon for detachment of the ribosome. However, the precise regulatory elements required for expression of a gene of interest may vary between different cell types but generally include 5' non-transcribing and non-translating regions which are required for initiation of translation and transcription. Such vectors may be obtained commercially or be assembled from the sequences described by methods well known in the art.

Transcription of DNA encoding the polypeptides of the present invention by higher eukaryotes is optimised by including an enhancer sequence in the vector. Enhancers are cis-acting elements of DNA that act on a promoter to increase the level of transcription. Vectors will also generally include origins of replication in addition to the selectable markers.

Nucleic acid molecules according to the invention may be inserted into the vectors described in an antisense orientation in order to provide for the production of antisense RNA. Antisense RNA or other antisense nucleic acids, including, antisense peptide nucleic acid (PNA), may be produced by synthetic means.

In accordance with the present invention, a defined nucleic acid includes not only the identical nucleic acid but also any minor base variations including in particular, substitutions in cases which result in a synonymous codon (a different codon specifying the same amino acid residue) due to the degenerate code in conservative amino acid substitutions. The term "nucleic acid sequence" also includes the complementary sequence to any single stranded sequence given regarding base variations.

As used herein with respect to nucleic acids "isolated" means any of a) amplified *in vitro* by, for example, polymerase chain reaction (PCR), b) recombinantly produced by cloning, c) purified by, for example, gel separation, or d) synthesised, such as by chemical synthesis.

The nucleic acid sequences according to the invention may be produced using recombinant or synthetic techniques, such as for example using PCR which generally involves making a pair of primers, which may be from approximately 10 to 50 nucleotides to a region of the gene which is desired to be cloned, bringing the primers into contact with cDNA, or genomic DNA from a human cell, performing a polymerase chain reaction under conditions which bring about amplification of the desired region, isolating the amplified region or fragment and recovering the amplified DNA. Generally, such techniques are well known in the art, such as described in Sambrook et al. (Molecular Cloning: a Laboratory Manual, 1989).

The nucleic acids according to the invention may carry a revealing label. Suitable labels include radioisotopes such as $^{32}P$ or $^{35}S$, enzyme labels or other protein labels such as biotin or fluorescent markers. Such labels may be added to the nucleic acids of the invention and may be detected using known techniques per se.

Advantageously, human allelic variants or polymorphisms of the nucleic acid according to the invention may be identified by, for example, probing cDNA or genomic libraries from a range of individuals, for example, from different populations. Furthermore, nucleic acids according to the invention may be used to sequence genomic DNA from patients using techniques well known in the art, such as the Sanger Dideoxy chain termination method, which may, advantageously, ascertain any predisposition of a patient to disorders associated with variants of the polypeptides of the invention.

In the very least, the nucleotide sequences can be used as molecular weight markers on Southern gels, as diagnostic probes for the presence of a specific mRNA in a particular cell type, as a probe to "subtract-out" known sequences in the process of discovering novel nucleotide sequences, for selecting and making oligomers for attachment to a "gene chip" or other support, to raise anti-DNA antibodies using DNA immunization techniques, and as an antigen to elicit an immune response.

The nucleotide sequences identified herein according to the invention can be used in numerous ways as a reagent. The following description should be considered exemplary and utilizes known techniques.

There exists an ongoing need to identify new chromosome markers, since few chromosome marking reagents, based on actual sequence data (repeat polymorphisms), are presently available. Other mapping techniques that may be used include in situ hybridization to chromosomal spreads, flow-sorted chromosomal preparations, or artificial chromosome constructions such as yeast artificial chromosomes, bacterial artificial chromosomes, bacterial P1 constructions or single chromosome cDNA libraries as reviewed in Price (Blood Rev. 7 (1993), 127-134) and Trask (Trends Genet. 7 (1991), 149-154). The technique of fluorescent *in situ* hybridization of chromosome spreads has been described, among other places, in Verma, (1988) Human Chromosomes: A Manual of Basic Techniques, Pergamon Press, New York NY. Fluorescent *in situ* hybridization of chromosomal preparations and other physical chromosome mapping techniques may be correlated with additional genetic map data. Examples of genetic map data can be found in the art. Correlation between the location of the gene encoding a polypeptide of the invention on a physical chromosomal map and a specific feature, e.g., a disease related to the dysfunction of the gene may help to delimit the region of DNA associated with this feature. The nucleotide sequences of the subject invention may be used to detect differences in gene sequences between normal, carrier or affected individuals. Furthermore, the means and methods described herein can be used for marker-assisted animal breeding.

*In situ* hybridization of chromosomal preparations and physical mapping techniques such as linkage analysis using established chromosomal markers may be used for extending genetic maps. For example a sequence tagged site based map of the human genome was recently published by the Whitehead-MIT Center for Genomic Research (Hudson, Science 270 (1995), 1945-1954) and is also available on the internet. Often the placement of a gene on the chromosome of another species may reveal associated markers even if the number or arm of a particular chromosome is not known. New sequences can be assigned to chromosomal arms, or parts thereof, by physical mapping. This provides valuable information to investigators searching for interacting genes using positional cloning or other gene discovery techniques. Once such gene has been crudely localized by genetic linkage to a particular genomic region, any sequences mapping to that area may represent associated or regulatory genes for further investigation. The nucleotide sequence of the subject invention may also be used to detect differences in the chromosomal location due to translocation, inversion, etc. among normal, carrier or affected individuals.

Briefly, sequences can be mapped to chromosomes by preparing PCR primers (preferably 15-25 bp) from the sequences of the invention. Primers can be selected using computer analysis so that primers do not span more than one predicted exon in the genomic DNA. These primers are then used for PCR screening of somatic cell hybrids containing individual human chromosomes. Only those hybrids containing the human gene of interest corresponding to the above sequences will yield an amplified fragment.

Similarly, somatic hybrids provide a rapid method of PCR mapping the nucleotide sequences to particular chromosomes. Three or more clones can be assigned per day using a single thermal cycler. Moreover, sublocalization of the nucleotide sequences can be achieved with panels of specific chromosome fragments. Other gene mapping strategies that can be used include in situ hybridization, prescreening with labeled flow-sorted chromosomes, and preselection by hybridization to construct chromosome specific cDNA libraries.

Precise chromosomal location of the nucleotide sequences can also be achieved using fluorescence in situ hybridization (FISH) of a metaphase chromosomal spread. This technique uses nucleotide sequences as short as 300 to 600 bases; however, nucleotide sequences 1,000-4,000 bp are preferred. For a review of this technique, see Verma et al., "Human Chromosomes: a Manual of Basic Techniques," Pergamon Press, New York (1988).

For chromosome mapping, the nucleotide sequences can be used individually (to mark a single chromosome or a single site on that chromosome) or in panels (for marking multiple sites and/or multiple chromosomes).

Once a nucleotide sequence has been mapped to a precise chromosomal location, the physical position of the nucleotide sequence can be used in linkage analysis. Linkage analysis establishes coinheritance between a chromosomal location and presentation of a particular disease. (Disease mapping data are found, for example, in McKusick, Mendelian Inheritance in Man (available on line through Johns Hopkins University Welch Medical Library)). Assuming 1 megabase mapping resolution and one gene per 20 kb, a cDNA precisely localized to a chromosomal region associated with the disease could be one of 50-500 potential causative genes.

Thus, once coinheritance is established, differences in the nucleotide sequences of the invention and the corresponding gene between affected and unaffected individuals can be examined. First, visible structural alterations in the chromosomes, such as deletions or translocations, are examined in chromosome spreads or by PCR. If no structural alterations exist, the presence of point mutations are ascertained. Mutations observed in some or all affected individuals, but not in normal individuals, indicates that the mutation may cause the disease. However, complete sequencing of the polypeptide encoded and the corresponding gene from several normal individuals is required to distinguish the mutation from a polymorphism. If a new polymorphism is identified, this polymorphic polypeptide can be used for further linkage analysis.

According to a further aspect of the invention, there is also provided an isolated polypeptide encoded by the nucleic acid molecules of the invention. Preferably, the polypeptide comprises the sequence of amino acids set forth in Figure 2. According to a further aspect there is also provided a tumour associated antigen encoded by the nucleic acid molecule identified in Figure 1.

The protein according to the invention may be recombinant, synthetic or naturally occurring, but is preferably recombinant.

As used herein with respect to polypeptides, "isolated" means separated from its native environment and present in sufficient quantity to permit its identification or use. Isolated, when referring to a protein or polypeptide, means, for example: (i) selectively produced by expression cloning or (ii) purified as by chromatography or electrophoresis. Isolated proteins or polypeptides may, but need not be, substantially pure. The term "substantially pure" means that the proteins or polypeptides are essentially free of other substances with which they may be found in nature or *in vivo* systems to an extent practical and appropriate for their intended use.

As aforementioned, the polypeptides according to the invention can be used to identify tumour antigens, and constitute antigens that are expressed in either solid tumours or lymphomas and which are recognised by the hosts immune response. Therefore, in some instances down-regulation or inhibition of the tumour-associated antigen may, advantageously, be used as a therapeutic treatment for such tumours. Therefore, the present invention is further directed to inhibiting expression or activity of the polypeptides of the invention by, for example, inhibiting transcription by, for example, the use of antisense technology. However, as would be appreciated by the skilled practitioner any other suitable method may be utilised. Other methods of inhibiting protein expression may utilise antibodies or binding polypeptides or other small molecules which, for example, bind or block the binding region of the polypeptides of the invention. As used herein, the term "antisense nucleotide" or "antisense" describes an oligonucleotide that is an oligoribonucleotide, oligodeoxyribonucleotide, modified oligoribonucleotide, or modified oligodeoxyribonucleotide which hybridises under physiological conditions to DNA encoding the polypeptide of the invention or to an mRNA transcript of the gene and, thereby, inhibits the transcription of that gene and/or translation of mRNA. Antisense technology can be used to control gene expression through triple-helix formation of antisense DNA or RNA, both of which methods are based on binding of a polynucleotide to DNA or RNA. For example, the 5' coding portion or the mature protein sequence, which encodes for the protein of the present invention, is used to design an antisense RNA oligonucleotide of from 10 to 40 base pairs in length. The antisense RNA oligonucleotide hybridises to the mRNA *in vivo* and blocks translation of an mRNA molecule into the protein (antisense - Okano, J. Neurochem., 56:560 (1991); Oligodeoxynucleotides as Antisense Inhibitors of Gene Expression, CRC Press, Boca Raton, FL (1988)). A DNA oligonucleotide is designed to be complementary to a region of the gene involved in transcription (triple-helix - see Lee et al. Nucl. Acids Res., 6:3073 (1979); Cooney et al., Science, 241:456 (1988); and Dervan et al., Science, 251: 1360 (1991), thereby preventing transcription and the production of the polypeptide.

The term "modified oligonucleotide" as used herein describes an oligonucleotide in which (1) at least two of its nucleotides are covalently linked via a synthetic internucleoside linkage (i.e., a linkage other than a phosphodiester linkage between the 5' end of one nucleotide and the 3' end of another nucleotide) and/or (2) a chemical group not normally associated with nucleic acids has been covalently attached to the oligonucleotide. Preferred synthetic internucleoside linkages are phosphorothioates, alkylphosphonates, phosphorodithioates, phosphate esters, alkylphosphonothioates, phosphoramidates, carbamates, carbonates, phosphate triesters, acetamidates, peptides, and carboxymethyl esters.

The term "modified oligonucleotide" also encompasses oligonucleotides with a covalently modified base and/or sugar. For example, modified oligonucleotides include oligonucleotides having backbone sugars which are covalently attached to low molecular weight organic groups other than a hydroxyl group at the 3' position and other than a phosphate group at the 5' position. Thus, modified oligonucleotides may include a 2'-O-alkylated ribose group. In addition, modified oligonucleotides may include sugars such as arabinose instead of ribose. Modified oligonucleotides also can include base analogs such as C-5 propyne modified bases (Wagner et al., Nature Biotechnology 14:840-844, 1996). The present invention, thus, contemplates pharmaceutical preparations containing modified antisense molecules that are complementary to and hybridizable with, under physiological conditions, nucleic acids encoding polypeptides of the invention together with pharmaceutically acceptable carriers.

The hybrid and modified forms include, for example, when certain amino acids have been subjected to some modification or replacement, such as for example, by point mutation and yet which results in a polypeptide or protein which possesses the same function as the polypeptides of the invention.

The antisense oligonucleotide described above can be delivered to cells by procedures in the art such that the anti-sense RNA and DNA may be expressed *in vivo* to inhibit production of the protein in the manner described above.

A further aspect of the invention provides a host cell or organism, transformed or transfected with an expression vector according to the invention. The cell or organism may be transformed or transfected using techniques that are well known in the art, such as, electroporation or by using liposomes. The host cell or organism may advantageously, be used in a method of producing polypeptides, which comprises recovering any expressed polypeptide from the host or organism transformed or transfected with the expression vector.

According to a further aspect of the invention there is also provided a transgenic cell, tissue or non-human organism comprising a transgene capable of expressing a polypeptide according to the invention. The term "transgene capable of expressing" as used herein encompasses any suitable nucleic acid sequence which leads to expression of a polypeptide(s) having the same function and/or activity as the polypeptides of the invention. The transgene, may include, for example, genomic nucleic acid isolated from human cells or synthetic nucleic acid, including DNA integrated into the genome or in an extrachromosomal state. Preferably, the transgene comprises the nucleic acid sequence encoding the polypeptide according to the invention as described herein, or a functional fragment of said nucleic acid. A functional fragment of said nucleic acid should be taken to mean a fragment of the gene comprising said nucleic acid coding for the polypeptides according to the invention or a functional equivalent, derivative or a non-functional derivative such as a dominant negative mutant of said polypeptides.

Transgenic non-human organisms are being utilised as model systems for studying both normal and disease cell processes. In general, to create such transgenic animals an exogenous gene with or without a mutation is transferred to the animal host system and the phenotype resulting from the transferred gene is observed. Other genetic manipulations can be undertaken in the vector or host system to improve the gene expression leading to the observed phenotype (phenotypic expression). The gene may be transferred on a vector under the control of different inducible or constitutive promoters, may be overexpressed or the endogenous homologous gene may be rendered unexpressible, and the like (WO 92/11358). The vector may be introduced by transfection or other suitable techniques such as electroporation, for example, in embryonic stem cells. The cells that have the exogenous DNA incorporated into their genome, for example, by homologous recombination, may subsequently be injected into blastocytes for generation of the transgenic animals with the desired phenotype. Successfully transformed cells containing the vector may be identified by well known techniques such as lysing the cells and examining the DNA, by, for example, Southern blotting or using the polymerase chain reaction.

Knock-out organisms may be generated to further investigate the role of the polypeptide of the invention *in vivo.* By "knock-out" it is meant an animal which has its endogenous gene knocked out or inactivated. Typically, homologous recombination is used to insert a selectable gene into an essential exon of the gene of interest. Furthermore, the gene of interest can be knocked out in favour of a homologous exogenous gene to investigate the role of the exogenous gene (Robbins, J., GENE TARGETING. The Precise Manipulation of the Mammalian Genome Circ. Res. 1993, J.W.; 73; 3-9). Transgenic animals, such as mice or *Drosophila* or the like, may therefore be used to over or under express the proteins according to the invention to further investigate their role *in vivo* and in the progression or treatment of lymphoma.

The polypeptide expressed by said transgenic cell, tissue or organism or a functional equivalent thereof also forms part of the present invention. Recombinant proteins or polypeptides may be recovered and purified from host cell cultures by methods known in the art, including ammonium sulfate or ethanol precipitation, acid extraction, anion or cation exchange chromatography, phosphocellulose, chromatography, hydrophobic interaction chromatography, affinity chromatography, hydroxyapatite chromatography and lectin chromatography.

The polypeptide of the present invention may be a naturally purified product, or a product of chemical synthetic procedures, or produced by recombinant techniques from a prokaryotic or eukaryotic host (for example, by bacteria, yeast, higher plant, insect and mammalian cells in culture). Depending upon the host employed in a recombinant production procedure, the expressed polypeptide may lack the initiating methionine residue as a result of post-translational cleavage. Proteins or polypeptides which have been modified in this way are also included within the scope of the invention.

In a still further aspect the invention provides an antibody which is capable of binding to the polypeptide of the invention or an epitope thereof which form part of this aspect of the present invention.

Such an antibody may be polyclonal, for example, and may be raised according to standard techniques well known to those skilled in the art by using the polypeptide of the invention or a fragment or single epitope thereof as the challenging antigen. Alternatively, the antibody may be monoclonal in nature and may be produced according to the techniques described by Kohler & Milstein (Nature (1975) 256, 995-497).

The present invention includes not only complete antibody molecules but fragments thereof. Antibody fragments which contain the idiotype of the molecule can be generated by known techniques, for example, such fragments include but are not limited to the $F(ab')_2$ fragment which can be produced by pepsin digestion of the antibody molecule; the Fab' fragments which can be generated by reducing the disulfide bridges of the $F(ab')_2$ fragments and the Fab fragments which can be generated by treating the antibody molecule with papain and a reducing agent. Chimeric, humanized and fully humanized monoclonal antibodies can now be made by recombinant engineering. By addition of the human constant chain to $F(ab')_2$ fragments it is possible to create a humanized monoclonal antibody which is useful in immunotherapy applications where patients making antibodies against the mouse Ig would otherwise be at a disadvantage. Breedveld F.C. Therapeutic Monoclonal Antibodies. Lancet 2000 Feb 26; 335, P735-40.

Furthermore, as is well-known in the art, only a small portion of an antibody molecule, the paratope, is involved in the binding of the antibody to its epiptope (see, in general, Clark, W.R. (1986) The Experimental Foundations of Modern Immunology Wiley & Sons, Inc., New York; Roitt, I. (1991) Essential Immunology, 7th Ed., Blackwell Scientific Publications, Oxford). The pFc' and Fc regions, for example, are effectors of the complement cascade but are not involved in antigen binding. An antibody from which the pFc' region has been enzymatically cleaved, or which has been produced without the pFc' region, designated an $F(ab')_2$ fragment, retains both of the antigen binding sites of an intact antibody. Similarly, an antibody from which the Fc region has been enzymatically cleaved, or which has been produced without the Fc region, designated an Fab fragment, retains one of the antigen binding sites of an intact antibody molecule. Proceeding further, Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation.

Polypeptides that bind to the polypeptide of the invention may be identified by Phage Display. In this technique a phage library is prepared, displaying inserts from between about 4 to 80 amino acid residues using techniques which are well known in the art. It is then possible to select those Phage bearing inserts that bind to the polypeptide of the invention. DNA sequence analysis is then performed to identify the nucleic acid sequences encoding the expressed polypeptides.

Antibody fragments of predetermined binding specificity may also be constructed using Phage Display technology, which obviates the need for hybridoma technology and immunization. These antibody fragments are created from repertoires of antibody V genes which are harvested from populations of lymphocytes, or assembled *in vitro*, and cloned for display of associated heavy and light chain variable domains on the surface of filamentous bacteriophage. The process mimics immune selection and antibodies with many different binding specificities have been isolated from the same Phage repertoire. (Winter et al., Annu. Rev. Immunol. 1994; 12 : 433-55). Such antibodies are also embraced within the scope of the binding polypeptides of the present invention.

Other types of binding polypeptides that may be utilised in accordance with the invention are termed immunoadhesins. Immunoadhesins are a class of fusion proteins, which combine the target-binding region of a receptor, an adhesion molecule, a ligand or an enzyme, with the Fc portion or an immunoglobulin. Production of immunoadhesins is described in Byrn et al (1990) Nature 344, pp 667-670.

In a preferred embodiment an antibody according to the invention is an autoantibody isolated from the sera of a patient suffering from a particular tumour type which, for certain of the antigens in Table 1 for which no previous SEREX association has been identified, directed against a solid tumour or lymphoma and particularly DLBCL.

It is also within the knowledge of the skilled artisan to produce immortalised cell lines capable of producing antibodies according to the invention and these are also embraced within the scope of the invention.

The nucleic acid molecules or the polypeptides of the invention may also be included in a pharmaceutical composition together with any suitable pharmaceutically acceptable carrier diluent or excipient therefor. The nucleic acid molecule or polypeptides or antibodies/binding polypeptide may be encapsulated and/or combined with suitable carriers in solid dosage forms for oral administration which would be well known to those of skill in the art or alternatively with suitable carriers for administration in an aerosol spray.

In the pharmaceutical composition of the invention, preferred compositions include pharmaceutically acceptable carriers including, for example, non-toxic salts, sterile water or the like. A suitable buffer may also be present allowing the compositions to be lyophilized and stored in sterile conditions prior to reconstitution by the addition of sterile water for subsequent administration. The carrier can also contain other pharmaceutically acceptable excipients for modifying other conditions such as pH, osmolarity, viscosity, sterility, lipophilicity, somobility or the like. Pharmaceutical compositions which permit sustained or delayed release following administration may also be used.

Furthermore, as would be appreciated by the skilled practitioner, the specific dosage regime may be calculated according to the body surface area of the patient or the volume of body space to be occupied, dependent on the particular route of administration to be used. The amount of the composition actually administered will, however, be determined by a medical practitioner based on the circumstances pertaining to the disorder to be treated, such as the severity of the symptoms, the age, weight and response of the individual.

As aforementioned, polypeptides according to the invention as set out heerein are considered to represent important markers that may be utilised for the prophylactic or therapeutic immunisation against certain tumours or lymphomas because they are specifically expressed or over-expressed in such tumours or lymphomas compared to normal cells and as such they may be targeted by antigen-specific Immune mechanism leading to the destruction of the tumour.

The invention therefore also contemplates a vaccine composition including, not only the polypeptides according to the invention as already described, but immunogenic fragments or epitopes thereof, which are capable of initiating an immune response, either by themselves or coupled to a suitable carrier. Also comprised within the scope of the invention are mimotopes which exhibit the same immune response initiating characteristics as the epitopes. The invention also therefore includes polypeptides incorporating the epitopes or mimotopes described. A mimotope is described as an entity which is sufficiently similar to the epitopes of the polypeptides of the invention so as to be capable of being recognised by antibodies which recognise the native molecule. They may be generated by addition, deletion or substitution of elected amino acids which, advantageously, means that the polypeptides or epitopes thereof of the invention may be modified, for example, for ease of delivery on a carrier.

Carriers which may be used with the immunogens of the present invention will be well known to those of skill in the art. The function of the carrier is to provide cytokine help to facilitate the induction of an immune response following administration of the vaccine composition to an individual. Methods for immunisation, including formulating the vaccine composition and selecting appropriate doses are well known to those of skill in the art.

According to another embodiment, the vaccine compositions described herein will comprise one or more immunostimulants in addition to the immunogenic polynucleotide, polypeptide, antibody, T-cell and/or antigen presenting cell (APC) compositions of this invention. An immunostimulant refers to essentially any substance that enhances or potentiates an immune response (antibody and/or cell-mediated) to an exogenous antigen. One preferred type of immunostimulant comprises an adjuvant. Many adjuvants contain a substance designed to protect the antigen from rapid catabolism, such as aluminum hydroxide or mineral oil, and a stimulator of immune responses, such as a lipid A, *Bortadella pertussis* or *Mycobacterium tuberculosis* derived proteins. Certain adjuvants are commercially available as, for example, Freund's Incomplete Adjuvant and Complete Adjuvant (Difco Laboratories, Detroit, MI); Merck Adjuvant 65 (Merck and Company, Inc., Rahway, NJ) ; AS-2 (SmithKline Beecham, Philadelphia, PA); aluminum salts such as aluminum hydroxide gel (alum) or aluminum phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF, interleukin-2, -7, -12, and other like growth factors, may also be used as adjuvants.

Within certain embodiments of the invention, the adjuvant composition is preferably one that includes an immune response predominantly of the Th1 type. High levels of Th1 type cytokines (e.g., $IFN_{-\gamma}$, TNF$\alpha$, IL-2 and IL-12) tend to favor the induction of cell mediated immune responses to an administered antigen. In contrast, high levels of Th2-type cytokines (e.g., IL-4, IL-5, IL-6 and IL10) tend to favor the induction of humoral immune responses. Following application of a vaccine as provided herein, a patient will support an immune response that includes both Th1- and Th2-type responses. Within a preferred embodiment, in which a response is predominantly Th1-type, the level of Th1-type cytokines will increase to a greater extent than the level of Th2-type cytokines. The levels of these cytokines may be readily assessed using standard assays. For a review of the families of cytokines, see Mosmann and Coffman, Ann. Rev. Immunol. 7:145-173, 1989.

The present invention also provides a polyvalent vaccine composition comprising a vaccine formulation of the invention in combination with other antigens, in particular antigens useful for treating cancers, autoimmune diseases and related conditions. Such a polyvalent vaccine composition may include a Th-1 inducing adjuvant as hereinbefore described.

According to another embodiment of this invention, an immunogenic composition described herein is delivered to a host via antigen presenting cells (APCs), such as dendritic cells, macrophages, B cells, monocytes, and other cells that may be engineered to be efficient APCs. Such cells may, but need not, be genetically modified to increase the capacity for presenting the antigen, to improve activation and/or maintenance of the T cell response, to have anti-tumor effects per se and/or to be immunologically compatible with the receiver (i.e., matched HLA haplotype). Animal models have shown that APCs may generally be isolated from any of a variety of biological fluids and organs, including tumour and peritumoral tissues, and may be autologous, allogeneic, syngeneic or xenogeneic cells.

Certain preferred embodiments of the present invention use dendritic cells or progenitors thereof as antigen-presenting cells. Dendritic cells are highly potent APCs (Banchereau and Steinman, Nature 392:245-251, 1998) and have been shown to be effective as a physiological adjuvant for eliciting prophylactic or therapeutic antitumour immunity (see Timmerman and Levy, Ann. Rev. Med. 50:507-529, 1999). In general, dendritic cells may be identified based on their typical shape (stellate in situ, with marked cytoplasmic processes (dendrites) visible *in vitro*), they possess a characteristic immunophenotype their ability to take up, process and present antigens with high efficiency and their ability to activate native T cell responses. Dendritic cells may, of course, be engineered to express specific cell-surface receptors or ligands that are not commonly found on dendritic cells *in vivo* or *ex vivo*, and such modified dendritic cells are contemplated by the present invention. As an alternative to dendritic cells, secreted vesicles antigen-loaded dendritic cells (caled exosomes) may be used within a vaccine (see Zitvogel et al., Nature Med. 4:594-600, 1998).

Dendritic cells and progenitors may be obtained from peripheral blood, bone marrow, tumour-infiltrating cells, peritumoral tissues-infiltrating cell, lymph nodes, spleen, skin, umbilical cord blood or any other suitable tissue or fluid. For example, dendritic cells may be differentiated *ex vivo* by adding a combination of cytokines such as GM-CSF, IL-4, IL-13 and/or TNF$\alpha$ to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNFα, CD40 ligand, LPS, flt3 ligand and/or other compound(s) that induce differentiation, maturation and proliferation of dendritic cells.

Dendritic cells are conveniently categorized as "immature" and "mature" cells, which allows a simple way to discriminate between two well characterized phenotypes. However, this nomenclature should not be Construed to exclude all possible intermediate stages of differentiation. Immature dendritic cells are characterized as APC with a high capacity for antigen uptake and processing, which correlates with the high expression of Fcγ receptor and mannose receptor. The mature phenotype is typically characterized by a lower expression of these markers, but a high expression of cell surface molecules responsible for T cell activation such as class I and class II MHC, adhesion molecules (e.g., CD54 and Cell) and costimulatory molecules (e.g., CD40, CD80, CD86 and 4-1BB).

APCs may generally be transfected with a polynucleotide of the invention such that the encoded polypeptide, or an immunogenic portion thereof, is expressed on the cell surface. Such transfection may take place *ex vivo*, and a pharmaceutical composition comprising such transfected cells may then be used for therapeutic purposes, as described herein. Alternatively, a gene delivery vehicle that targets a dendritic or other antigen presenting cell may be administered to a patient, resulting in transfection that occurs *in vivo*. *In vivo* and *ex vivo* transfection of dendritic cells, for example, may generally be performed using any methods known in the art, such as those described in WO 97/24441, or the gene gun approach described by Mahvi et al., Immunology and cell Biology 75: 456-460, 1997. Antigen loading of dendritic cells may be achieved by incubating dendritic cells or progenitor cells with the tumour polypeptide, tumour protein DNA (naked or within a plasmid vector) or RNA; or with antigen-expressing recombinant bacterium or viruses (e.g., vaccinia, fowlpox, adenovirus or lentivirus vectors). Prior to loading, the protein or polypeptide may be covalently conjugated to an immunological partner that provides T cell help (e.g., a carrier molecule). Alternatively, a dendritic cell may be pulsed with a non-conjugated immunological partner, separately or in the presence of the antigen.

While any suitable carrier known to those of ordinary skill in the art may be employed in the pharmaceutical compositions of this invention, the type of carrier will typically vary depending on the mode of administration. Compositions of the present invention may be formulated for any appropriate manner of administration, including for example, topical, oral, nasal, mucosal, intravenous, intracranial, intraperitoneal, subcutaneous and intramuscular administration.

Carriers for use within such pharmaceutical compositions are biocompatible, and may also be biodegradable. In certain embodiments, the formulation preferably provides a relatively constant level of active component release. In other embodiments, however, a more rapid rate of release immediately upon administration may be desired. The formulation of such compositions is well within the level of ordinary skill in the art using known techniques. Illustrative carriers useful in this regard include microparticles of poly(lactide-co-glycolide), polyacrylate, latex, starch, cellulose, destran and the like. Other illustrative delayed-release carriers include supramolecular biovectors, which comprise a non-liquid hydrophilic core (e.g., a cross-linked polysaccharide or oligosaccharide) and, optionally, an external layer comprising an amphiphilic compound, such as a phospholipid (see e.g., US Patent No. 5,151,254 and PCT applications WO94/20078 WO/94/23701 and WO96/06638). The amount of active compound contained within a sustained release formulation depends upon the site of implantation, the rate and expected duration of release and the nature of the condition to be treated or prevented.

The identification of antigens in certain tumours and lymphomas renders it possible to detect or identify patients suffering from this form of cancer and will help in determining the appropriate course of treatment. Identification of various expression levels of the antigens will be useful in both diagnosis and in prognosis by grading the nature and advancement of the tumour or lymphoma.

Therefore, in one aspect of the invention there is provided an in vitro method of identifying tumours or malignant cells in a mammal which method comprises identifying in a sample of bodily fluid from said mammal antibodies from said mammal capable of forming complexes with an antigen according to the invention. In one embodiment the antibodies are specific for the antigens as set out in Figure 2. Alternatively, the antibodies are directed to the antigen as designated herein as OX-TES-1. Preferably, the mammal is a human and the antibodies are human autoantibodies. Preferably, the malignant cell or tumour is either a solid tumour or a leukaemia or lymphoma which itself may be a B cell lymphoma such as any of DLBCL, follicular lymphoma, or Burkitt's lymphoma, but preferably DLBCL. In a further aspect of the invention, there is provided a method of detecting lymphoma in a patient which method comprises identifying autoantibodies from a bodily fluid of said patient directed to said lymphoid tumour antigen designated OX-TES-1 illustrated in Table 1.

The invention may be more clearly understood from the following examples with reference to the accompanying Tables and Figures wherein:

Figure 1 is a nucleic acid molecule encoding a novel, PAS domain containing polypeptide which was identified as a DLBCL-associated antigen (OX-TES-1).

Figure 2 is an amino acid sequence of the polypeptide encoded by the nucleic acid molecule of Figure 1 which also identifies the major domains present within the polypeptide.

Figure 3 shows the genomic arrangement of OX-TES-1 (A) and BC040301 (B).

Figure 4 is an alignment of the cDNA sequences of OX-TES-1 and BC040301, showing clearly the retained region in OX-TES-1. Differences at the nucleotide level are shaded.

Figure 5 is an alignment of the predicted protein sequences of OX-TES-1 and BC040301.

Figure 6 illustrates the results obtained from hybridising the *OX-TES-1* cDNA to BD Biosciences' Multiple Tissue Expression Array. The identity and position of tissues on the array is shown in the lower panel. The positive signals are arrowed.

Figure 7 illustrates the results obtained from hybridising the *OX-TES-1* cDNA to BD Biosciences' Matched Tumour/Normal expression array. The identity and position of tissues on the array is shown in the lower panel. K* = kidney. Row P is the human cancer cell lines: 1 HeLa, 2 Daudi (Burkitt's lymphoma), 3 K562 (chromic myeloid leukaemia), 4 HL-60 (promyelocytic leukaemia), 5 G361 (melanoma), 6 A549 (lung carcinoma), 7 MOLT-4 (lymphoblastic leukaemia), 8 SW480 (colorectal adenocarcinoma), and 9 Raji (Burkitt's lymphoma).

Figure 8 shows the products amplified by RT-PCR on DLBCL cell lines for OX-TES-1. A: Primary PCR with primer set A (detects both transcripts); B: Secondary PCR with primer set A; C: Primary PCR with primer set B (OX-TES-1 specific); D: Secondary PCR with primer set B - dT primed cDNA; E: Secondary PCR with primer set B - hexamer primed cDNA; F: Primary PCR with primer set C (flanking primers); G: Secondary PCR with primer set C; H: b-actin control.

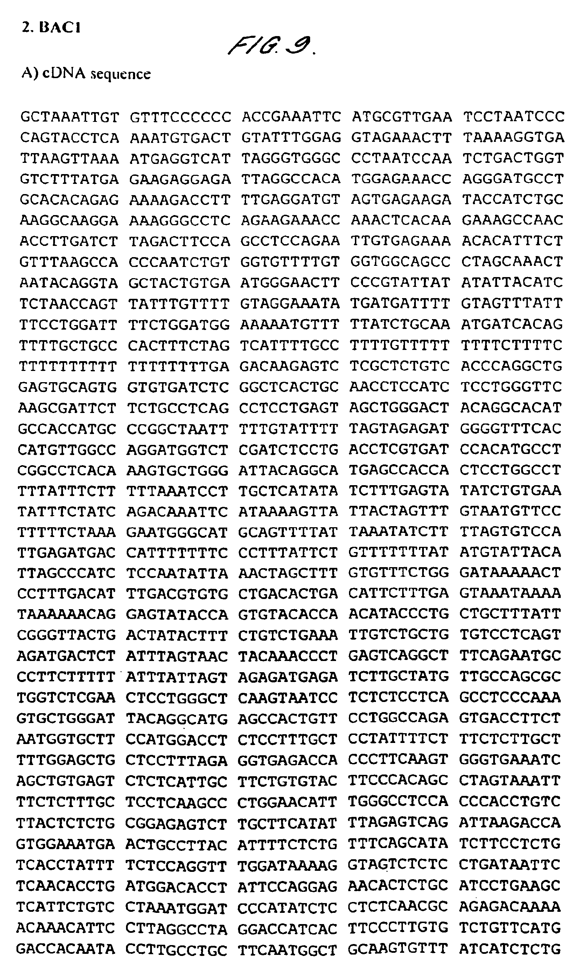

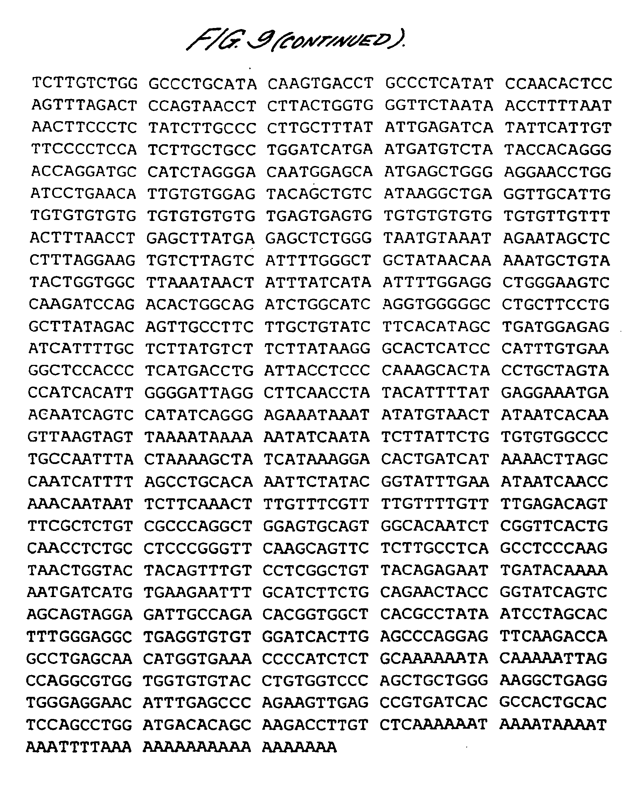

Figure 9 is a nucleic acid molecule encoding a novel DLBCL tumour antigen designated herein as BAC1 (OX-TES-2).

Figure 10 is an amino acid sequence of the longest polypeptide encoded by the nucleic acid molecule of Figure 9.

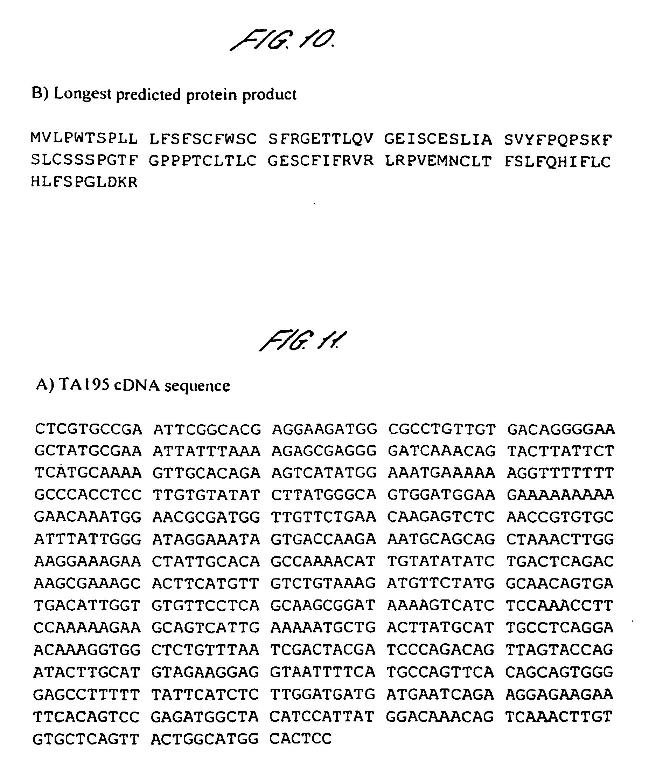

Figure 11 is a nucleic acid molecule encoding a novel DLBCL tumour antigen described as 'similar to recombining binding protein suppressor of hairless (Drosophila). The cDNA sequence was not a perfect match for anything in the database but may represent a variant of the RBP-J Kappa gene. The closest matches were to BC 020780, an uncharacterised gene and *H. sapiens* recombination signal binding protein (LO78721). The cDNA sequence in BG 614689 matched some of the 5' end of the present (TA195) sequence that differed from the BC 020780 sequence suggesting that there are a number of 5' variants of the RBP-J Kappa DNA. All the genes map to chromosome 4 (close NT_006324.7).

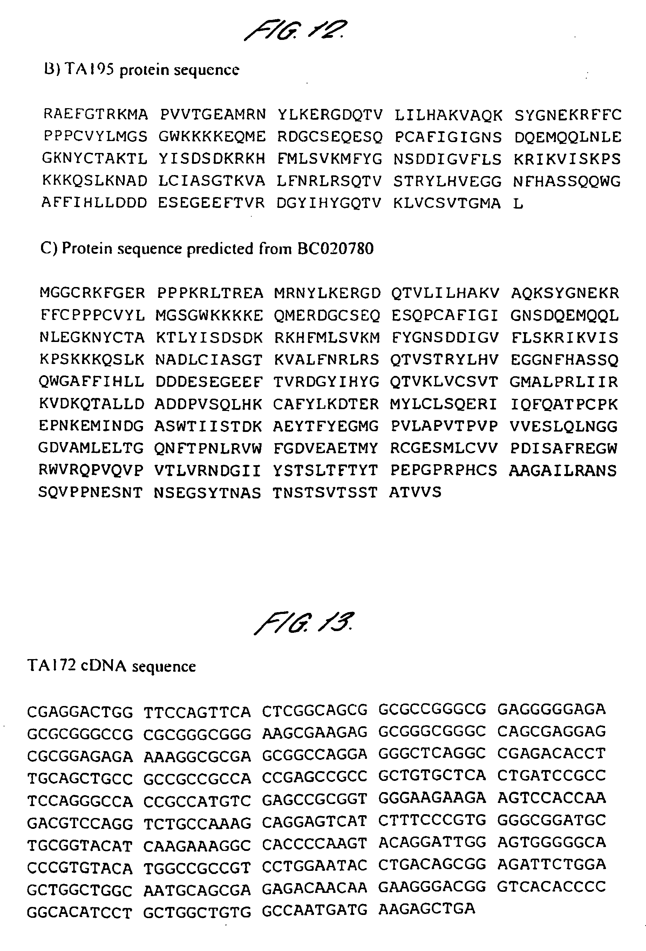

Figure 12 is an amino acid sequence of the polypeptide encoded by the nucleic acid molecule of Figure 11. Figure 6C is an amino acid sequence of the polypeptide encoded by the nucleic acid of an EST sequence with the accession number BC020780.

Figure 13 is a nucleotide sequence encoding a lymphoma-associated antigen encoded by the cDNA sequence identified as Histone Macro H2A1. The first 86 nucleotides in this cDNA do not correspond to that of publicly disclosed sequences and this clone therefore has a longer 5' untranslated region than other cDNA clones encoding this molecule.

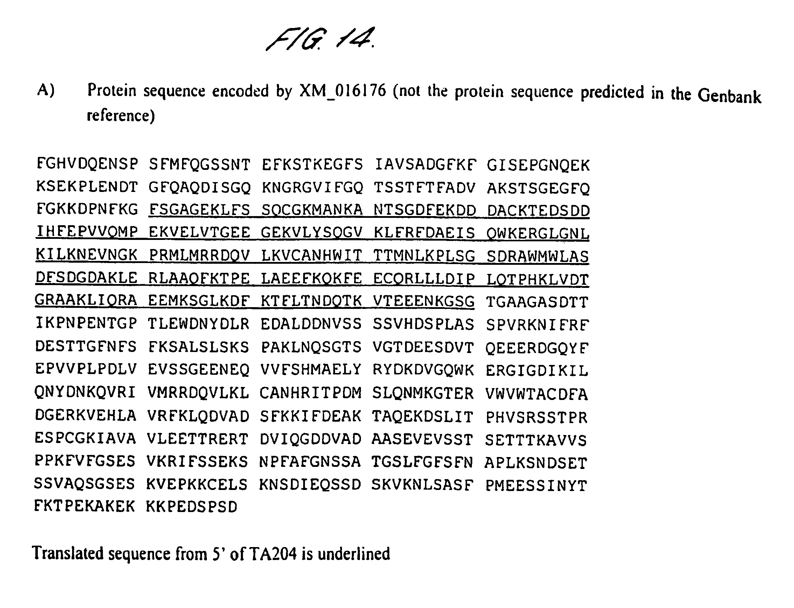

Figure 14 is an amino acid sequence encoded by a cDNA clone designated TA204 which is most similar to a hypothetical gene (XM_016176) both of which are highly related to RanBP2. Both the present sequence and XM_016176 sequence map (and are identical to) the human genome sequence on chromosome 2 (NT_029235). Human RanBP2 also maps to chromosome 2 but is encoded by the sequences in NT_005224 and NT_029895. RanBP2 and the protein produced from TA204 are somewhat different and represent highly-related proteins raising the probability that this protein and/or RanBP2 may be recognised by the serum from patients with DLBCL.

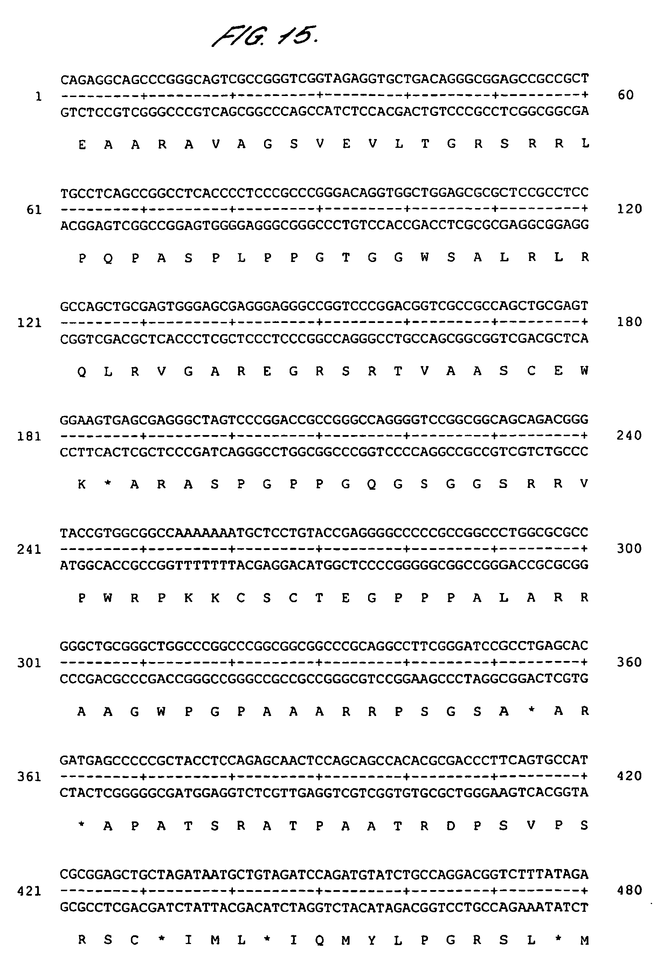

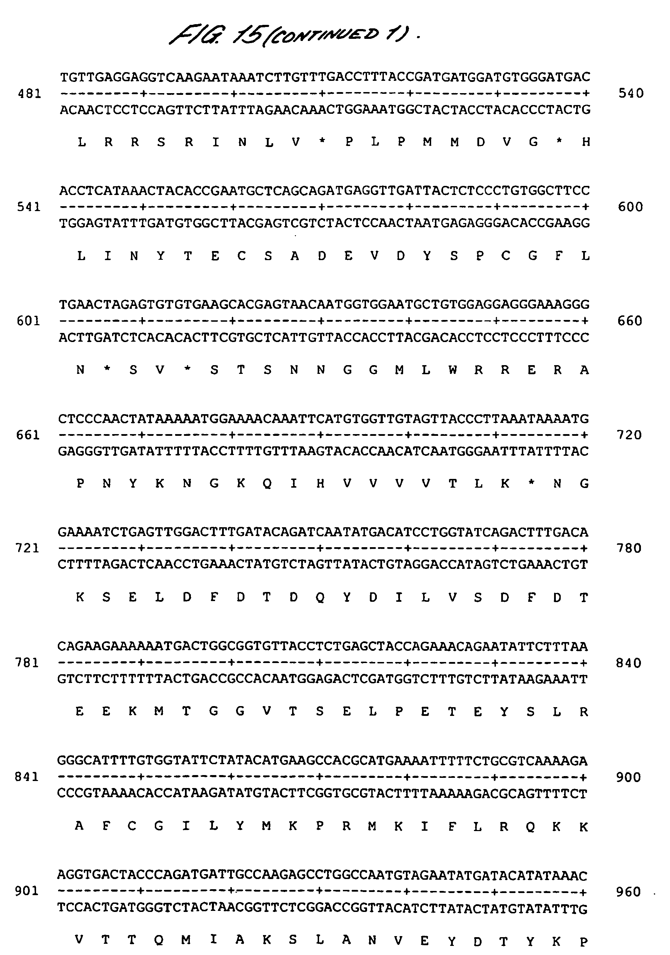

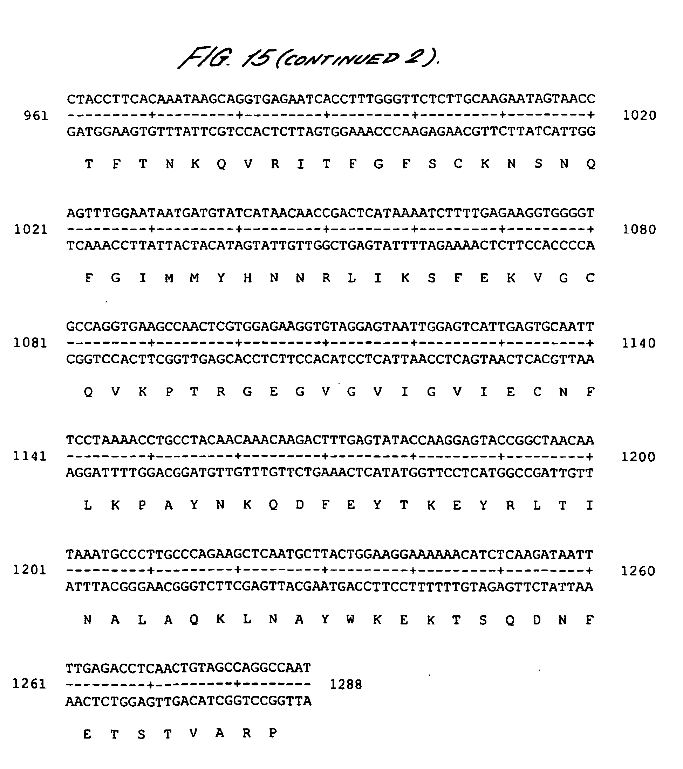

Figure 15 illustrates the cDNA sequence of OX-TES-4 and the predicted encoded protein.

Figure 16 shows the cDNA sequence of (OX-TES-4)FLJ31673 and the predicted encoded protein. Domains present with the protein sequence are annotated.

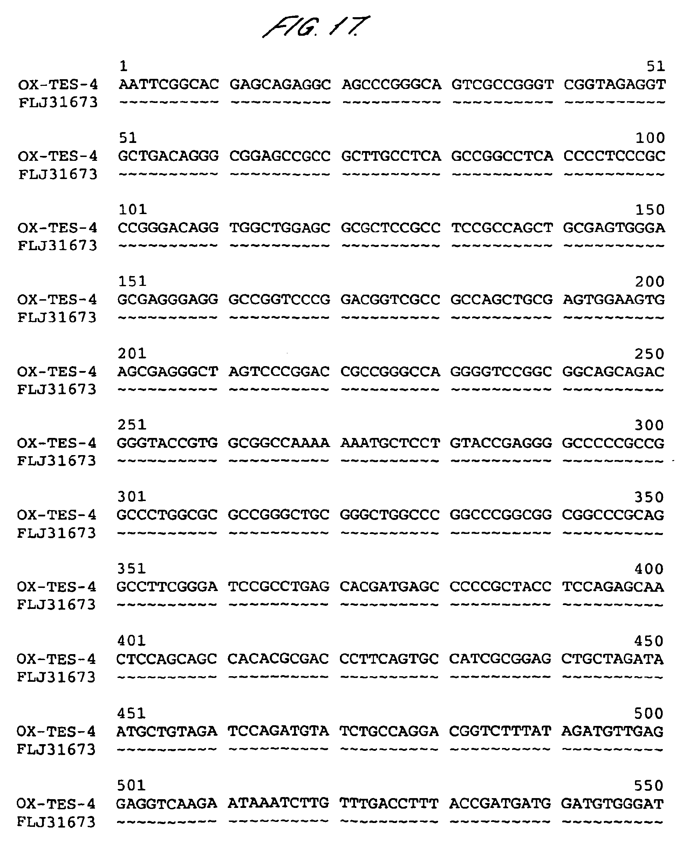

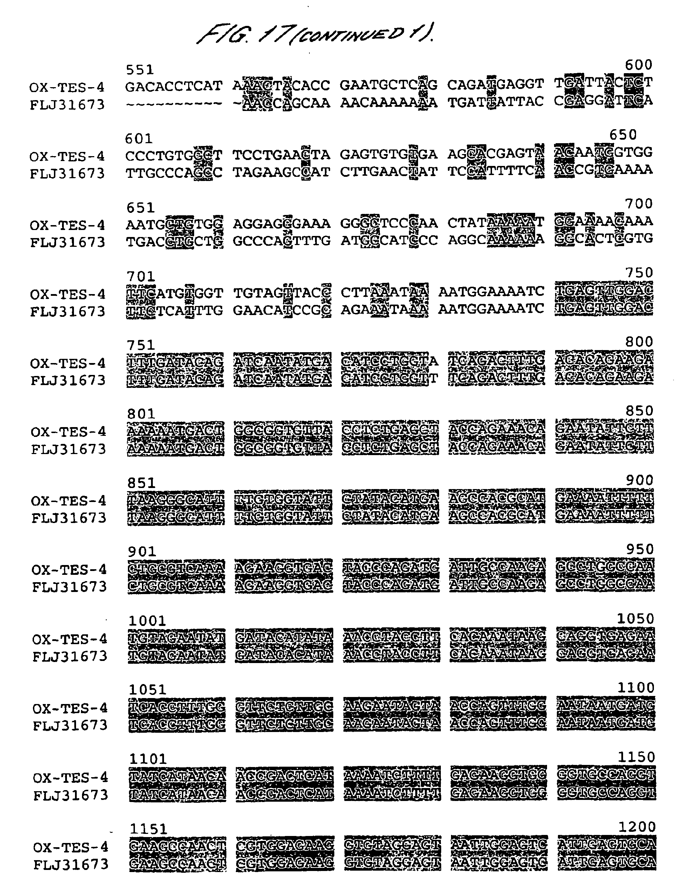

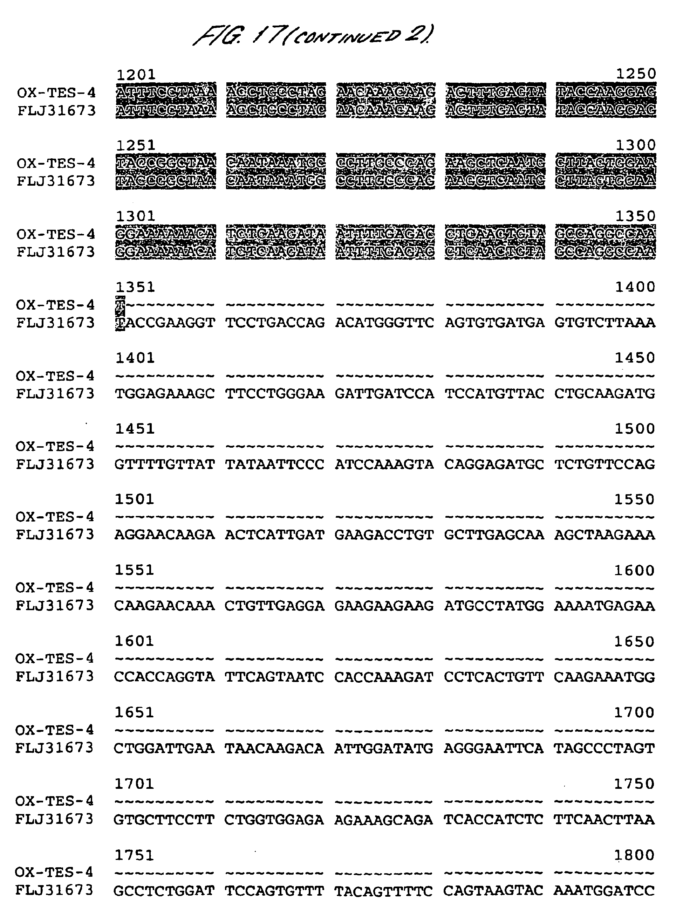

Figure 17 is an alignment of the cDNA sequences of OX-TES-4 and FLJ31673. Similarities at the nucleotide level are shaded.

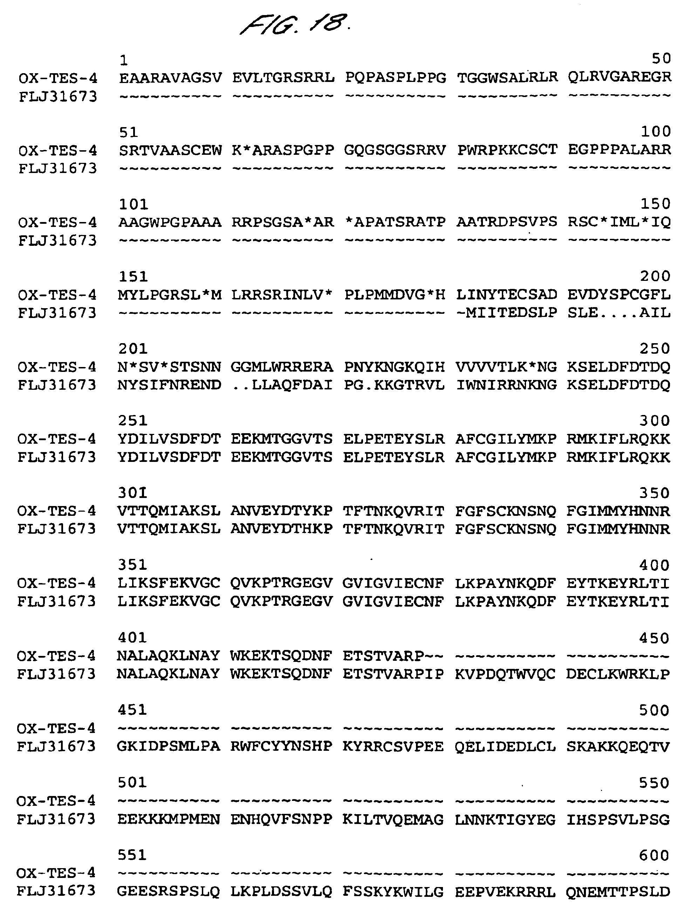

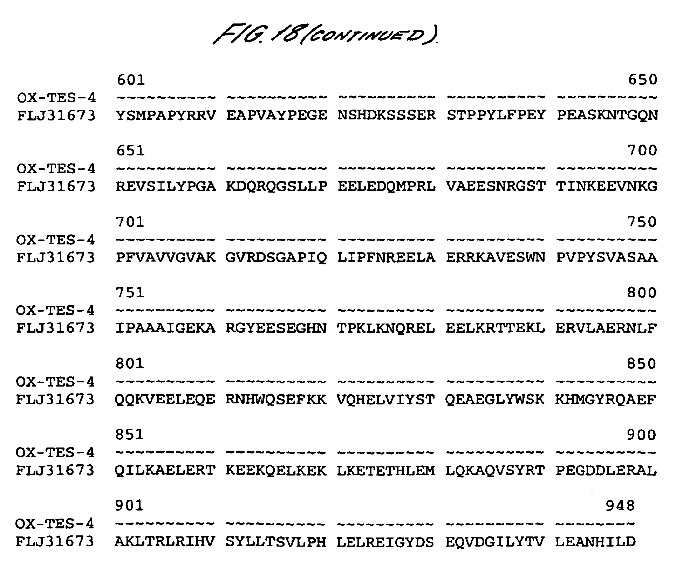

Figure 18 is an alignment of the predicted protein sequences of OX-TES-4 and FLJ31673.

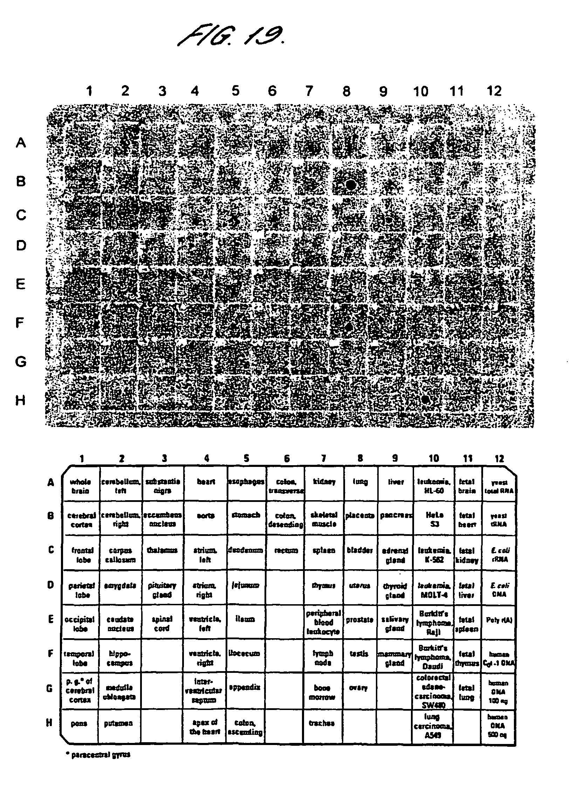

Figure 19 illustrates the results obtained from hybridising the *OX-TES-4* cDNA to BD Biosciences' Multiple Tissue Expression Array. The identities and positions of tissues on the array are shown in the lower panel.

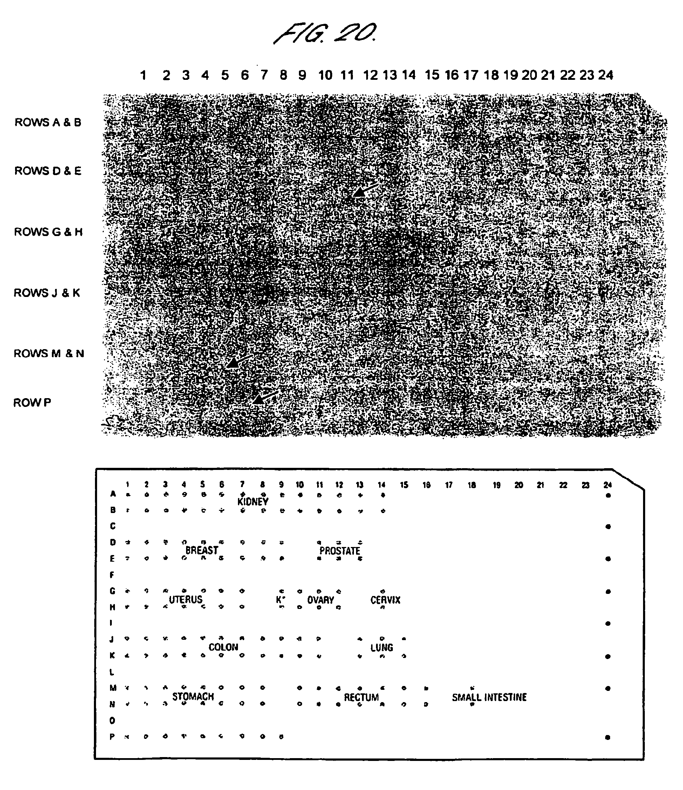

Figure 20 illustrates the results obtained from hybridising the *OX-TES-4* cDNA to BD Biosciences' Matched Tumour/Normal expression array. The identities and positions of tissues on the array are again shown in the lower panel. K* = kidney. Row P is the human cancer cell lines: 1 HeLa, 2 Daudi (Burkitt's lymphoma), 3 K562 (chromic myeloid leukaemia), 4 HL-60 (promyelocytic leukaemia), 5 G361 (melanoma), 6 A549 (lung carcinoma), 7 MOLT-4 (lymphoblastic leukaemia), 8 SW480 (colorectal adenocarcinoma), and 9 Raji (Burkitt's lymphoma).

Figure 21a illustrates the results of RT-PCR on DLBCL cell lines for OX-TES 4 . A:Primary PCR with OX-TES-4 primers; B: Secondary PCR with OX-TES-4 primers; C: B actin control.

Figure 21b illustrates the results of RT-PCR analysis of OX_TES_4 expression in germinal centre, activated and subtype unknown DLBCL cell lines.

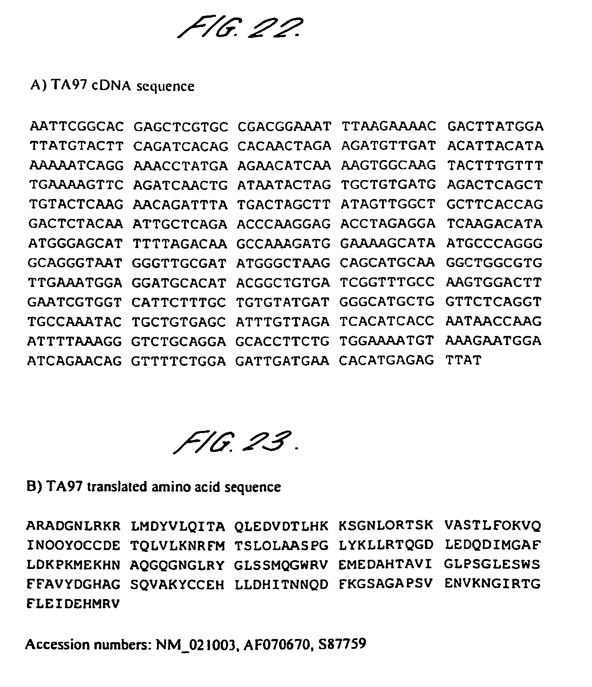

Figure 22 is a cDNA sequence encoding a protein PPM1A. This cDNA clone is a 5' splice variant of the other PPM1A cDNA clones in the public databases. The first 278 bp do not match the PPM1A sequences although the PPM1A sequences do have an additional 337 bp before the homologous region. Searches against the human genome sequence confirm that this 5' sequence comes from an adjacent region on chromosome 14, and that nt 279 is the beginning of the 4$^{th}$ exon. These changes are not predicted to change the protein sequence but may affect gene regulation.

Figure 23 is an amino acid sequence encoded by the cDNA sequence of Figure 22.

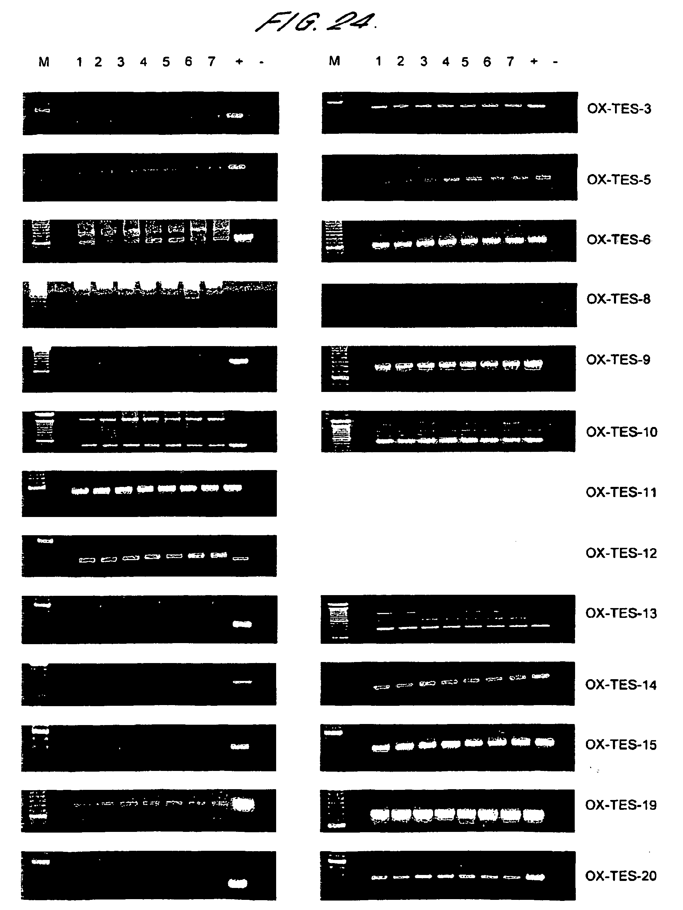

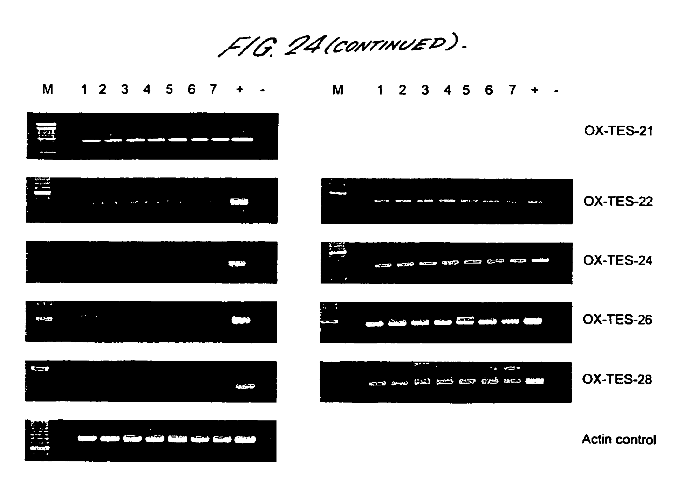

Figure 24 is an illustration of the results of RT-PCR on DLBCL cell lines for a selection of antigens. Primary PCR results are shown in the left hand column and secondary PCR results (where appropriate) are shown in the right hand column.

Figure 25 is an illustration of the results obtained from immunostaining of DLBCL cell lines with the DLBCL patients serum used to screen the testis cDNA library.

Figure 26 is an illustration of the results obtained from immunostaining of DLBCL cell lines with serum from a DLBCL patient that was subsequently used in tertiary screening of the DLBCL tests antigens.

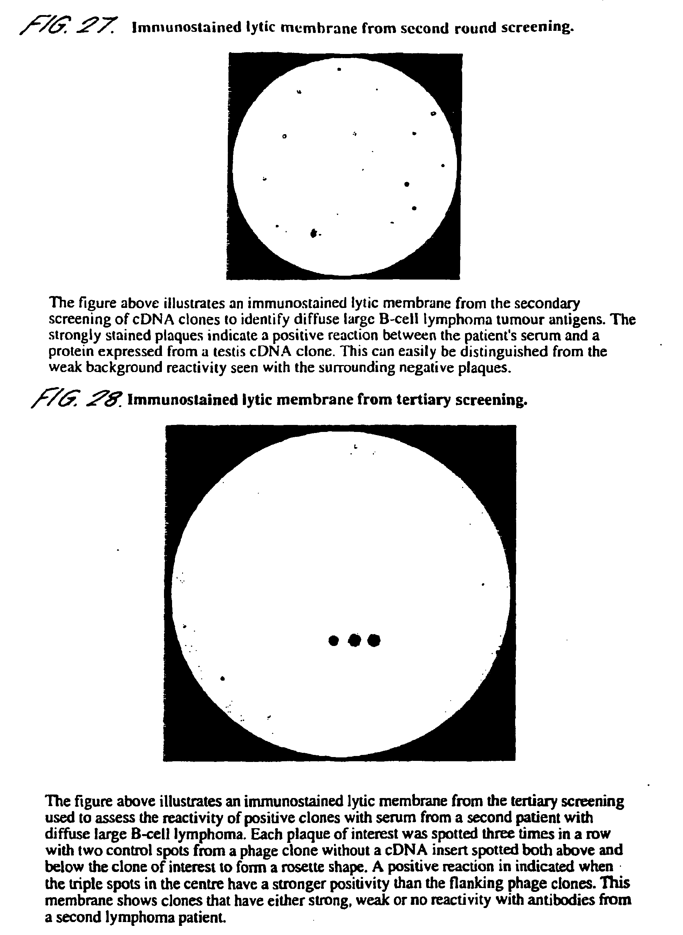

Figure 27 is an illustration of the results obtained from immunostaining lytic membranes from the secondary screening of cDNA clones to identify DLBCL-associated lymphoma tumour antigens. The strongly stained plagues indicate a positive reaction between the patient's serum and a protein expressed from a testis cDNA clone. This can be easily distinguished from the weak background reactivity seen with the surrounding negative plagues.

Figure 28 illustrates an immunostained lytic membrane from the tertiary screening used to assess the reactivity of positive clones with serum from a second patient with DLBCL. Each plaque of interest was spotted three times in a row with two control spots from a phage clone without a cDNA insert spotted both above and below the clone of interst to form a rosette phage. A positive reaction is indicated when the triple spots in the centre have a stronger positivity than the flanking phage clones.

**Experimental Approach**

The inventors have screened a testis cDNA expression library with serum from a single 40 year old female patient with DLBCL. Immunocytochemical screening of a number of DLBCL patients' sera on DLBCL cell lines was used prior to starting the technique to identify patients' sera that contained high titre IgG antibodies. The reasons for screening a testis library are that testis has a uniquely broad transcriptional repertoire, and there would therefore be the opportunity to identify cancer testis antigens which, as mentioned above, are particularly good candidates for immunotherapy applications. The DLBCL patient was seen at the John Radcliffe Hospital in November 2000 with stage IIA progressive disease, despite PMitcebo chemotherapy. Salvage chemotherapy was used to treat progressive disease and she had palliative chemotherapy until death in November 2001. This patient was young to have this disease and had a very aggressive form of DLBCL that was never in remission. The serum was taken post-treatment and therefore the expression of antigens identified in this study-may be related to the failure of this treatment.

**SEREX Protocol**

**Cleaning human serum**

These steps are required before using patients' sera to screen the expression library as it is essential to remove background serum reactivity with either the *E. coli* or phage proteins.

**A. *Lytic column***

1. Grow *E. coli* XL1 Blue MRF' cells in 50m1 LB (supplemented with 0.2% maltose and 10mM $MgSO_4$) overnight at 37˚C and 225rpm.
2. Pellet cells in a 50ml Falcon tube by centrifuging at 3000rpm for 10 minutes. Resuspend the pellet in 2ml LB containing 10mM $MgSO_4$.
3. Introduce 200µl of cells (store remainder at 4˚C) into a 50ml Falcon and add 5ml LB, 50µl 20% maltose, 50µl 1M $MgSO_4$ and 7.5µl tetracycline (12.5µg/ml)
4. Inoculate with the volume of one eluted blue phage (spin phage solution for 1 minute at maximum speed and take supernatant, leaving chloroform behind).
5. Incubate culture at 37˚C and 250rpm for 4 hours.
6. To remainder of culture from step 3, add 5ml LB, 50µl 20% maltose, 50µl 1M $MgSO_4$ and 7.5µl tetracycline (12.5µg/ml). Mix this with the phage-inoculated culture from step 5.
7. Incubate culture at 37˚C and 250rpm for 2 hours.
8. Freeze-thaw the culture three times, then sonicate the culture for eight 5- second pulses.
9. Resuspend 1g CNBr-activated sepharose in 10ml 1mM HC1. Wash beads with 200ml 1mM HCl over a sintered glass filter to remove any additives.
10. Resuspend the beads in 7ml (final volume) of 1mM HCl.
11. Add 5ml coupling buffer to the lysed bacteria from step 8. Add 4ml (out of 7ml final volume) washed sepharose beads to the lysate. Seal neck of falcon with parafilm and rotate mixture overnight at 4˚C to couple bacterial/phage proteins to the sepharose beads.
12. Pellet the matrix (sepharose beads + proteins) by centrifuging at 3000rpm for 10 minutes and pour the supernatant into Virkon. Save the matrix.
13. Wash the matrix in 30ml coupling buffer by resuspending matrix in buffer, centrifuging at 3000rpm for 10 minutes, and discarding supernatant into Virkon.
14. Block any remaining active groups by resuspending matrix in 30ml 0.1M Tris-HC1 pH 8.0 and leaving to stand at room temperature for two hours.
15. Pellet matrix as above and discard supernatant.
16. Wash matrix in 30ml Wash Buffer 1 followed by 30ml Wash Buffer 2.
17. Repeat step 16 twice more.
18. Wash the matrix twice in 50ml 1xTBS/0.1% sodium azide. Matrix can be stored in 1xTBS/0.1% sodium azide at 4˚C until required.
19. If using straight away, resuspend the matrix in a volume of 1xTBS/0.1% sodium azide that will yield a 1:10 dilution of serum (e.g. 18ml 1xTBS/0.1% sodium azide + 2ml serum).
20. Add the serum to the resuspended matrix.
21. Seal the neck of the tube with parafilm and rotate overnight at 4˚C.
22. Pellet the matrix by centrifuging at 3000rpm for 10 minutes. Save the supernatant = serum. Discard the matrix. If not proceeding directly to mechanical column method (B), store the serum at 4˚C with the neck of the tube sealed with parafilm.

***B. Mechanical column.***

1. Grow *E. coli* XL1 Blue MRF' cells in 50ml LB (supplemented with 0.2% maltose and 10mM $MgSO_4$) overnight at 37˚C and 225rpm.
2. Pellet cells in a 50ml Falcon tube by centrifuging at 3000rpm for 10 minutes.
3. Resuspend the pellet in 5ml PBS.
4. Freeze-thaw the cells three times, then sonicate the culture for eight 5-second pulses.
5. Repeat steps 9-18 for lytic column method.
6. Do not resuspend the matrix in 1XTBS/0.1% sodium azide. Instead, add the serum (already at 1:10 from lytic column method) directly to the matrix.
7. Seal the neck of the tube with parafilm and rotate overnight at 4˚C.
8. Pellet the matrix by centrifuging at 3000rpm for 10 minutes. Save the supernatant = serum. Discard the matrix. If not proceeding directly to lytic membrane method (C), store the serum at 4˚C with the neck of the tube sealed with parafilm.

**C. Lytic membrane.**

1. Prepare plates (1 per serum sample to be cleaned): 600μl *E. coli* XL1Blue MRF' cells (OD600 = 0.5) 250μl eluted blue phage
Incubate at 37˚C for 15 minutes to allow phage to infect cells
Add

10ml NZY top agar
40μl 0.5M IPTG
50μl 250mg/ml X-gal (only necessary if you want to see blue color)

2. Plate onto dried NZY agar plates (140mm) and leave to set.
3. Incubate plate(s), inverted, overnight at 37˚C.
4. Next morning, place a nitrocellulose membrane onto the plate and perform a plaque lift at 37˚C for 4 hours.
5. Prepare block solution (see step 8) without tween-20 in a sterile container. Use 20ml per membrane. Boil ~50ml $dH_2O$ in a 500ml bottle to sterilise it, then microwave the appropriate volume of 5% marvel in TBS to ensure sterilisation.
6. Remove membrane carefully, using forceps, and place into TBS. Gently brush off any excess agar that is stuck to the membrane.
7. Wash membrane twice in TBS-T for 5 minutes followed by once in TBS for 5 minutes.
8. Block membrane for 1 hour at room temperature in 20ml 5% marvel in TBS-T.
9. Wash membrane four times in TBS-T then once in TBS for 5 minutes each wash. Following the third wash, change the petri dish.
10. Pour the total volume of serum from step B8 (Mechanical column) onto the membrane and incubate overnight at room temperature on shaker.
11. Remove serum, using a 10ml pipette, into a sterile 50ml Falcon. Store at 4˚C with the neck of the tube sealed with parafilm.
12. Repeat this process twice more. Serum should now be clean. Store in 2ml aliquots at 4˚C for 6 months or -80˚C long term.

***D. Primary screening***

1. Inoculate 50ml LB (supplemented with 0.2% maltose and 10mM $MgSO_4$) with an *E. coli* XL1Blue MRF' colony from a freshly streaked plate and grow overnight at 30˚C and 225rpm.
2. Pellet cells in 50ml Falcon tube by centrifuging at 3000rpm for 10 minutes and resuspend in 25ml 10mM $MgSO_4$. Measure $OD_{600}$ and dilute some of the cells to $OD_{600}$ = 0.5 in $MgSO_4$.
3. Dry large NZY agar plates at 37˚C for approximately 1 hour, and prepare NZY top agar.
4. Into a 15ml Falcon tube introduce 600μl MRF' cells ($OD_{600}$=0.5) and the appropriate volume of phage in SM buffer. The usual density for library screening is 50000pfu/140mm plate but the inventors tend to use considerably lower densities. Mix phage and cells by inverting 2-3 times then incubate at 37˚C for 15 minutes.
5. Quickly add 10ml NZY top agar and 40μl IPTG (0.5M) to the phage/cells and pour onto the NZY agar plate. Leave to set at room temperature for 10 minutes and then incubate plates, inverted, overnight at 37˚C.
6. Perform a 4-hour plaque lift onto nitrocellulose membranes at 37˚C. Write the date and membrane number on the membrane in biro.
7. Prepare block solution. Leave to cool to room temperature; add Tween-20 to a final concentration of 0.05% just prior to use.
8. Pierce the membrane and agar with a sterile needle to orientate plaques at a later stage and remove membrane, using forceps, into TBS. Brush off any excess agar gently and place membranes, protein side down, into clean petri dishes containing TBS-T. Store plates at 4˚C.
9. Wash membrane twice in TBS-T for 5 minutes followed by once in TBS for 5 minutes.
10. Remove TBS and add 20ml block solution onto membrane using a 25ml pipette to ensure full coverage. Incubate for 1 hour at room temperature on the shaker.
11. Remove the block solution and wash the membranes four times for 5 minutes in TBS-T, changing the petri dish after the third wash. Finally, wash once with TBS for 5 minutes.
12. Remove TBS and add 20ml serum to each membrane using a 25ml pipette as before. Incubate at room temperature on the shaker overnight.
13. Remove membranes from serum and place into clean petri dishes containing TBS-T. Pipette the serum into labelled Falcon tubes and store at 4˚C with necks sealed with parafilm.
14. Wash membranes as in 11.
15. Remove TBS and add 20ml antibody solution (Rabbit anti-human IgG, Fc fragment specific, alkaline phosphatase conjugated; 1:5000 dilution in 0.5% marvel in TBS-T). Incubate at room temperature on shaker for 1 hour.
16. Remove antibody solution and wash membranes as in 11.
17. Incubate membranes in colour development reagents.

***E. Colour development***

1. Introduce 20ml AP Colour Development Reagent buffer into a clean 140mm petri dish. Add 100μl BCIP (30mg/ml) and 100μl NBT (60mg/ml). Mix thoroughly and cover with a light-protective box (e.g.cardboard).
2. Remove membrane from TBS using forceps, drain excess TBS off onto tissue and place membrane, protein side up, into development solution.
3. Allow colour to develop for up to 30 minutes, keeping reagents in the dark. Strongly positive plaques will appear within 5 minutes, and background will appear within 10 minutes.
4. At end of development reaction, place membranes into $dH_2O$ to stop the reaction.
5. Check membranes when wet, and circle any positive plaques with biro. Dry membranes on filter paper and check again for any positive plaques.

***F. Positive plaques***

1. Put the plate with positive plaques on, upside down, next to the membrane with the positives spots on. Use the needle marks to orientate the plate and membrane.
2. Using a pair of compasses, locate the positive phage from all three needle holes, and number it with a pen. If there is more than one positive plaque, mark the others in the same way.
3. Turn the plate the right way up and place it on a light box. Using a sterile scalpel, cut out the positive plaque and its surrounding 3-4 negative neighbours as one piece, and place into a 1.5ml eppendorf tube (labelled with the corresponding plaque number) containing 500μl of SM buffer. Repeat for any other plaques.
4. Incubate tubes on an end-over-end rotator overnight at 4˚C to allow phage to elute into SM buffer.
5. Next morning, add 20μl chloroform, vortex, centrifuge at 14000 rpm for 1 minute and store at 4˚C until ready to do secondary screening.

***G. Secondary screening***

1. Dry small NZY agar plates (90mm) at 37˚C for approximately 1 hour, and prepare NZY top agar.
2. Dilute the phage from the primary screen 1:10 in SM buffer.

3. Into a 15ml Falcon tube, introduce 200μl of *E. coli* XL1Blue MRF' cells ($OD_{600}$ = 0.5) and 4μl of diluted phage from step 2. Mix phage and cells by inverting 2-3 times then incubate at 37˚C for 15 minutes.
4. Quickly add 3ml NZY top agar and 18μl IPTG (0.5M) to the phage/cells and pour onto the NZY agar plate. Leave to set at room temperature for 10 minutes and then incubate plates, inverted, overnight at 37˚C.
5. Perform a 4-hour plaque lift onto nitrocellulose membranes at 37˚C.
6. Perform screening as for primary screen with the following exceptions: use 10ml block solution, antibody solution, and serum per membrane.
7. Identify and isolate positive plaques except that in this second round of screening, the positive phage should be isolated alone (with no negatives).

***H. Tertiary screening***

1. Dry large NZY agar plates and prepare NZY top agar.
2. Into a 15ml Falcon tube introduce 600μl XL1Blue MRF' cells ($OD_{600}$ = 0.5). Quickly add 10ml NZY top agar and 40μl IPTG (0.5M) and pour onto NZY agar plates. Leave to set at room temperature for 15 minutes.
3. Spot 1μl of positive phage onto plate following a template and use 0.8μl of blue phage as negative control (see Figures 1 & 2 for examples of templates).
4. Once the phage has dried into the agar, incubate plates, inverted, overnight at 37˚C.
5. Perform a 4-hour plaque lift onto nitrocellulose membranes at 37˚C.
6. Perform tertiary screen as for primary screening.
7. Score membranes to identify which plaques show a positive reaction with which serum. This tertiary screen should be performed three times to be confident of results.

**Results**

Primary screening of the testis library identified 94 clones that were confirmed as positive after second round screening. Phagemids were *in vivo* excised from the lambda phage clones according to the manufacturer's instructions to generate cDNA clones in the vector pBK-CMV. The 5' end of each cDNA clone was then commercially sequenced by MWG-Biotech. In cases where the cDNA clone appeared to be novel (4 clones), the cDNA insert was fully sequenced to publication quality by MWG-Biotech.
These results of sequence analyses indicated that the 94 clones corresponded to 29 different genes, of which 2 are novel and a further 8 are uncharacterised. Two cDNA clones originally thought to encode novel genes are, in fact, longer cDNA sequences for which the additional 5' sequence had not been deposited in the databases.

**Identification of cDNA sequences using publicly-disclosed information**

DNA (and/or protein) sequences were BLAST searched through the National Center for Biotechnology Information (NCBI) website to obtain any publicly available information concerning the sequences:

1. Against the human genome to identify their chromosome localisation.

2. Against the non-redundant database to see if they corresponded to known genes.

3. Against the EST database to identify any homologous clones.

Additional sequence searches were also performed, where possible using either full length sequences from known genes or as much sequence as possible from those that were novel:

4. Against the patent database.

5. Against the Stanford Lymphochip to identify whether their expression in DLBCL had been investigated in this microarray study at http://llmpp.nih.gov/lymphoma/search.shtml.

6. Against the SEREX database to determine whether these molecules had already been identified as tumour antigens in other tumour types.

7. The TIGR Index to identify theoretical cDNA clones.

8. Unigene/Locus link and Online Mendelian inheritance in Man (OMIM) to identify additional gene information.

9. PubMed to investigate published work on known molecules.

10. Cancer Genome Anatomy Project (CGAP) recurrent chromosomal abberations site to look for disease associations with mapped loci (web site: http://www.ncbi.nlm.nih.gov/ncicgap/).

The majority of these data are summarised in Table 1.

**Sequence data analysis of OX-TES-1 and splice variants**

OX-TES-1 is a cDNA of 4109bp that encodes a predicted protein of 639 amino acids (Figure 1). Analysis of this protein using databases on the world wide web predicts a molecular weight of 72.7kDa and a pl of 5.23. This analysis also identified a number of domains that are highlighted in Figure 2. In addition to those shown, there is a proline-rich region between aa 478 and 639, a lysine-rich region between aa 508 and 548, and a second coiled-coil domain between aa 475 and 557. There are no known homologues of this protein - the closest related protein is the CLOCK protein of the Korean rock fish *Sebastes schlegeli* (32% similarity) and neuronal PAS domain protein 2 of the mouse *Mus masculus* (22%). The predicted domains and nuclear localisation signal sequences detected in the OX-TES-1 protein suggest that it may function as a transcription factor.

Analysis of the available human genome sequence shows that OX-TES-1 localises to chromosome X. Searching of the databases revealed a number of ESTs with high similarity to OX-TES-1 at the nucleotide level. This analysis revealed a splice variant of OX-TES-1, also cloned from a testis cDNA library (Accession number BC040301), which is 2850bp in size. BC040301 maps to chromosome Xq28, which is a locus known to have structural aberrations in DLBCL, follicular lymphoma, and mantle cell lymphoma amongst others. The chromosomal band Xq28 has been a focus of interest in human genetics because more than 20 hereditary disease linked genes have been mapped to this region, as have a number of immunogenic tumour antigens with the characteristic cancer/testis expression pattern including, MAGE-A, NY-ESO-1, LAGE-1, TRAG-3, CSAGE and SAGE. In addition Xq28 has been identified as a genetic region that is altered in lymphomas and which may contain lymphoma associated oncogenes (reviewed in (Goyns et al., 1993; Vineis et al., 1999)); one example being the high level amplification of Xq28 identified in some blastic mantle cell lymphoma patients (Bea et al., 1999). Genetic gain within a common region at Xq28 has also been detected in testicular cancer (Skotheim et al., 2001) and two translocation breakpoints in infertile males have also been reported in this region (Olesen et al., 2001).

Thus genes mapped within this region may have additional disease associations.

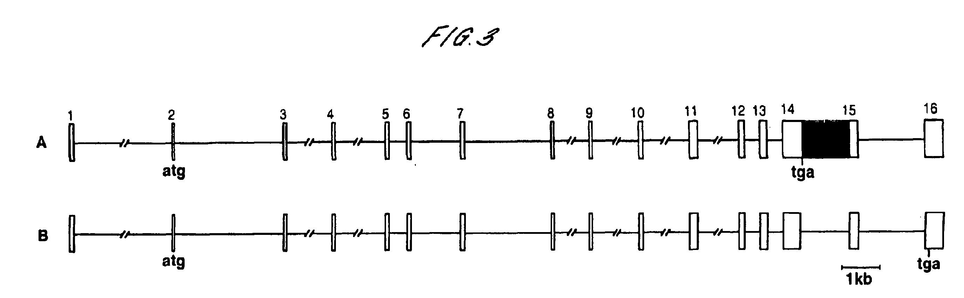

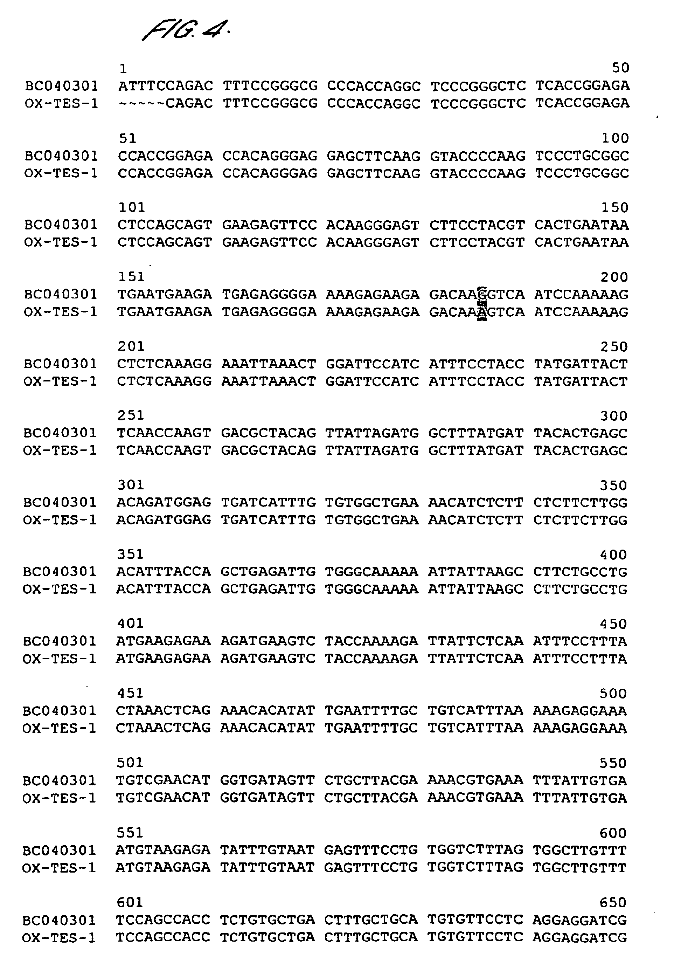

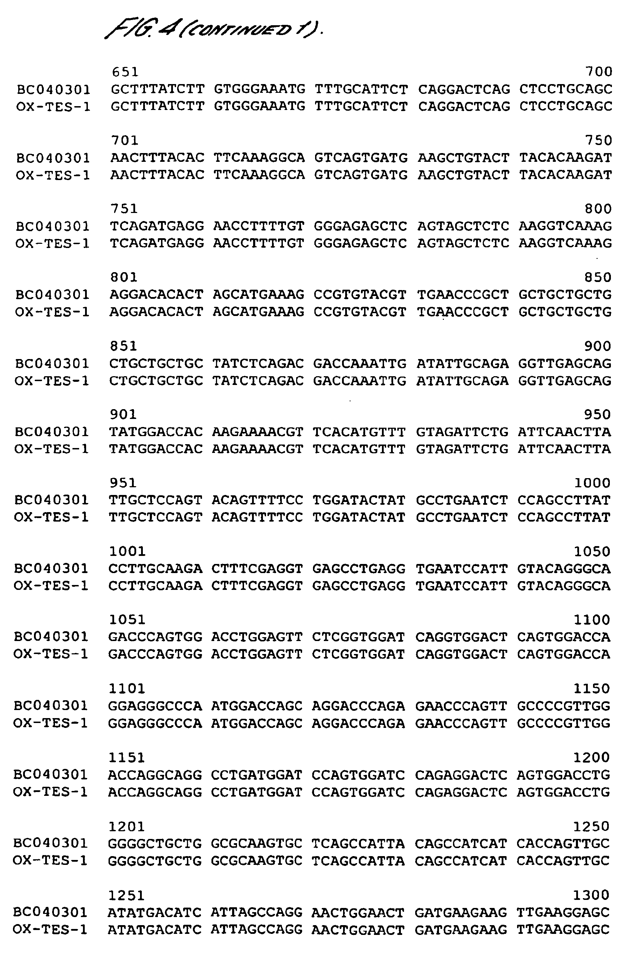

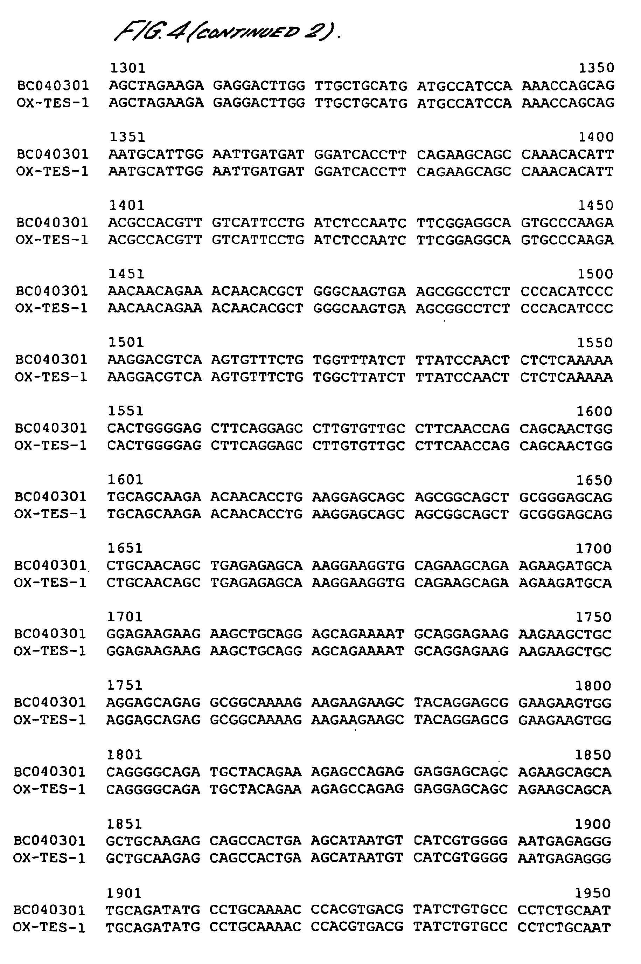

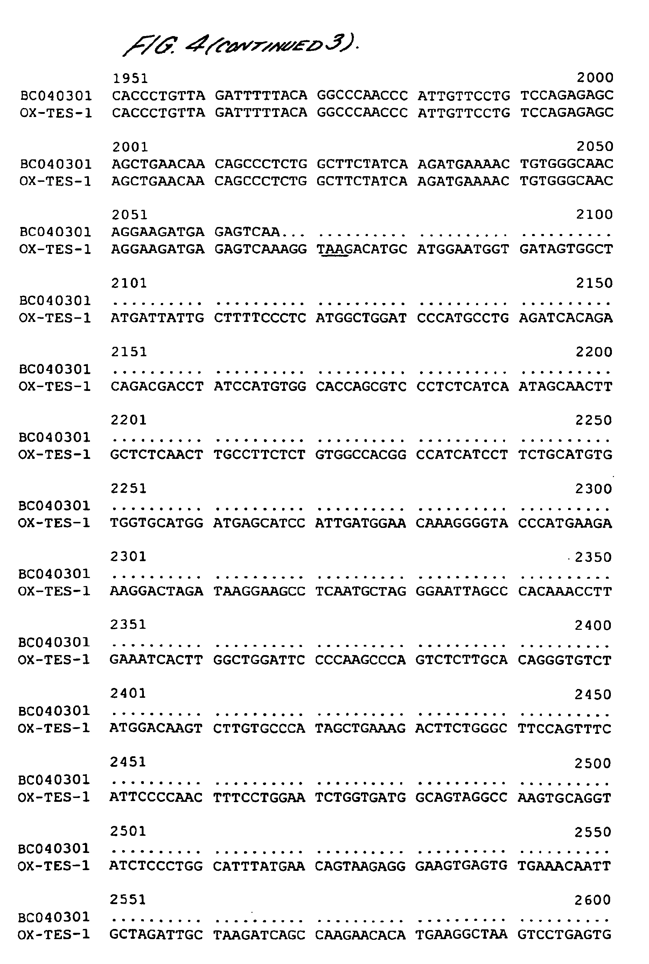

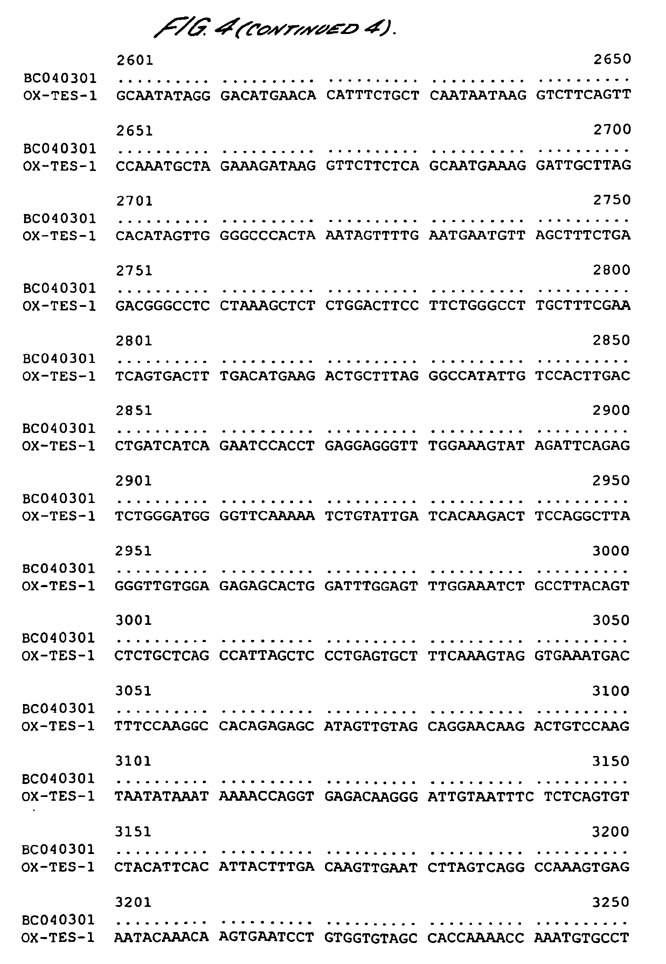

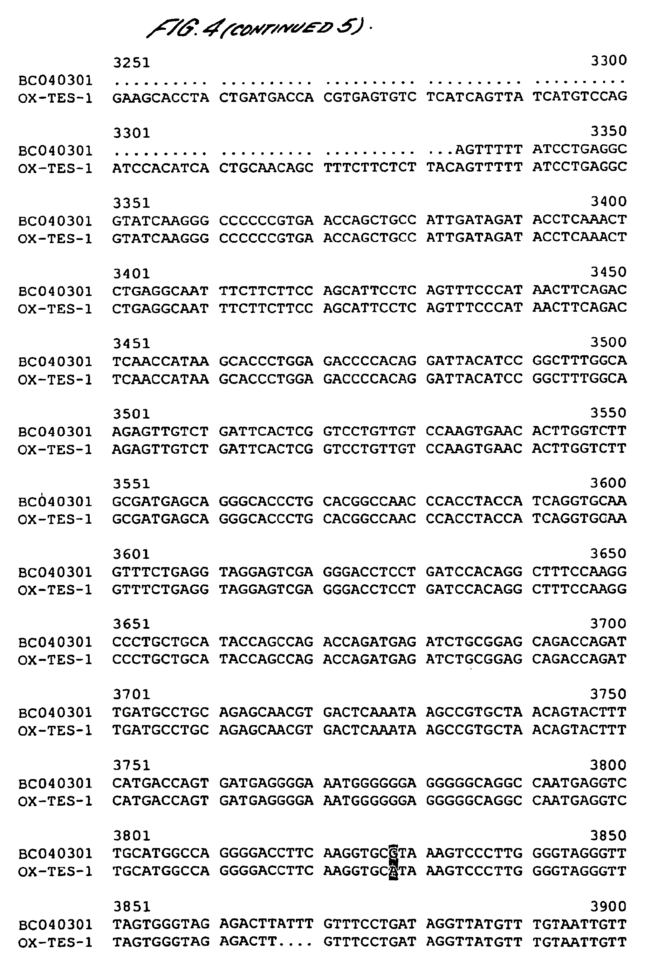

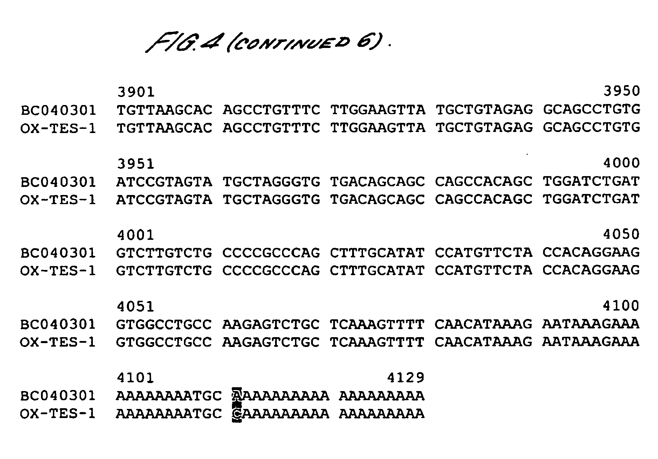

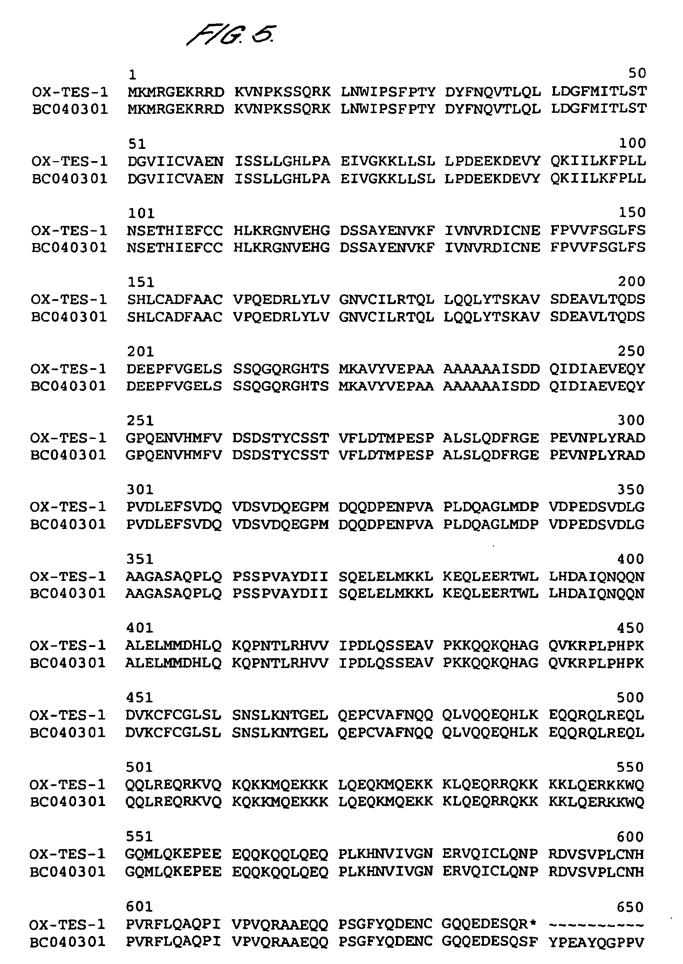

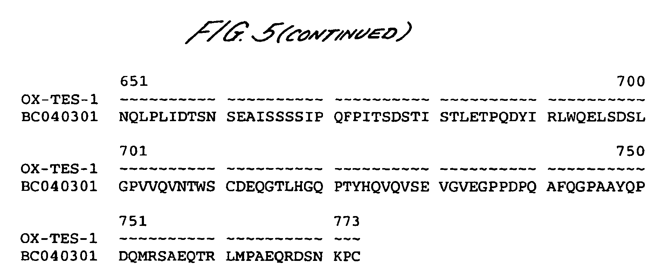

The difference between the sizes of the cDNAs of BC040301 and OX-TES-1 appears to be the result of intron retention, resulting in a deletion of an additional 1.1kb of cDNA (between nucleotides 2062 and 3328) in OX-TES-1. The genomic structure of both OX-TES-1 and BC040301 is shown in Figure 3, whilst Figure 4 shows an alignment of the cDNA sequences of both variants. Since this additional region contains the translational stop codon in OX-TES-1 (underlined in Figure 4), BC040301 encodes a protein that is longer than OX-TES-1; the amino acid sequence is identical to OS-TES-1 from aa 1 to 638, but there is an additional 135 amino acids at the C-terminus (shown in Figure 5). There is only one identifiable domain in this additional region: an ER membrane retention signal.

**PAS domain protein (OX-TES-1)**

Sequence searches using BLAST indicated that the cDNA in TA112 represents a novel gene that has not been deposited in the public databases prior to the present work. Prior to this work, the closest similarity to this sequence was found to be neuronal PAS domain protein 2 and CLOCK. There were two EST sequences corresponding to incomplete regions of our sequence with accession numbers BG723594 and BI458651. Translation of the cDNA sequence in TA112 predicted a 639 amino acid (aa) protein. The poly(A) tail on the cDNA clone and the presence of upstream stop codons in the translated protein sequence indicated that the cDNA encoded a full length protein product. There are two potential methionine start codons for this protein; since the second (aa3) has a slightly better Kozak sequence than the first methionine, it may therefore represent the start of translation.

The protein sequence was analysed using MotifFinder. This programme identified an N-terminal PAS domain (using the Pfam database) between aa 32-96. PAS domains are present in many signalling proteins where they are used as signal sensor domains. Several PAS domain proteins are known to detect their signal using an associated cofactor. Analysis using the PSORT II programme identified an R-2 motif at aa 14 (which is a predicted cleavage site for mitochondrial presequence), a nuclear localisation signal at aa 538, an ER membrane retention signal in the N-terminus, a C-terminal leucine zipper pattern at aa 481-502 and a coiled-coil domain containing the leucine zipper between aa 475-556. These analyses indicate that this molecule is potentially a nuclear DNA binding protein containing a PAS domain.

PAS domains regulate the function of many intracellular signalling pathways in response to both extrinsic and intrinsic stimuli, and regulate circadian rhythmicity in diverse organisms. The PAS domain has been identified as an interface for protein-protein interactions in an evolutionarily related family comprising several hundred proteins.

The PAS domain is found in many archaeal, bacterial, and plant proteins where it is capable of sensing environmental changes in light intensity, oxygen concentration, and redox potentials. The oxygen sensor FixL from *Rhizobium* species contains a heme-bearing PAS domain and a histidine kinase domain that couples sensing to signalling. The 144-kDa PASKIN protein contains a PAS region similar to the FixL PAS domain and a serine/threonine kinase domain which might be involved in signalling. Thus, PASKIN is likely to function as a mammalian PAS sensor protein (Hofer et al., 2001). PAS domain-regulated histidine kinases are common in prokaryotes; in contrast these kinases are rare in eukaryotes and were, for a long time, thought to be completely absent in mammals. However PAS kinase (PASK) is an evolutionarily conserved gene product present in yeast, flies, and mammals. The amino acid sequence of PASK specifies two PAS domains followed by a canonical serine/threonine kinase domain, indicating that it might represent the first mammalian PAS-regulated protein kinase (Rutter et al., 2001).

The key elements of circadian clockwork and oxygen homeostasis are the PAS protein family members PER and CLOCK and hypoxia-inducible factor lalpha (HIF-1 alpha). Alteration of gene expression is a crucial component of adaptive responses to hypoxia and these responses are mediated by hypoxia-inducible transcription factors (HIFs). Hypoxia is also a potent inducer of tumour angiogenesis, the process of which is mostly mediated by induction of vascular endothelial growth factor (VEGF). Endothelial PAS domain protein 1 (EPAS1) is a basic helix-loop-helix (bHLH)/PAS domain transcription factor that is expressed most abundantly in highly vascularized organs. Studies have shown that endogenous VEGF can be up-regulated transcriptionally by EPAS1, and it has been proposed that EPAS1 may be involved in the angiogenesis of renal cell carcinoma (Xia et al., 2001). The effect of hypoxia on the expression of HIF-1 alpha and EPAS1 has been investigated. These two similar but distinct proteins have been postulated to activate VEGF expression in response to hypoxia. Src family kinases have also been shown to mediate the hypoxia-mediated EPAS1 gene expression, which in turn positively autoregulated its own expression (Sato et al., 2002).

Inhibitory PAS domain protein, IPAS, is a bHLH/PAS protein structurally related to HIFs, IPAS contains no endogenous transactivation function but demonstrates dominant negative regulation of HIF-mediated control of gene expression. Application of an IPAS antisense oligonucleotide to the mouse cornea induced angiogenesis under normal oxygen conditions, and demonstrated hypoxia-dependent induction of VEGF gene expression in hypoxic corneal cells. This indicated a previously unknown mechanism for negative regulation of angiogenesis and maintenance of an avascular phenotype (Makino et al., 2001).

Although other PAS domain proteins have been linked to cancer, the novel gene described here has not to our knowledge been described previously. The gene is not on the Lymphochip and has not been identified as a SEREX antigen.

### Expression of the *OX-TES-1* gene in normals and neoplastic human tissues

The inventors have characterised the expression of the *OX-TES-1* mRNA in normal human tissues and in matched normal and tumour tissues from cancer patients to investigate whether differential expression of the *OX-TES-1* mRNA occurs in human cancer.

Multiple Tissue Expression (MTE) and Matched Tumour/Normal (MTN) arrays (BD Biosciences Clontech) were pre-hybridised according to the manufacturer's instructions. A610pb *PvuII* fragment of OX-TES-1 (nt 3360-3970), which is also present in the splice variant BC040301 and represents a portion of the 3'UTR, was radiolabelled using the High Prime DNA Labelling Kit (Roche Diagnostics GmbH, Germany). The probe was denatured and incubated with $C_0$t-1 DNA and sheared salmon sperm DNA as described (MTE/MTN Array User Manual, BD Biosciences), before being added to the arrays and incubated overnight with gentle rotation at 60˚C. The arrays were then washed for 5x 20min in 2x SSC, 1% SDS at 65˚C followed by 2x 20min in 0.1SSC, 0.5% SDS at 55˚C. The washed membranes were exposed to film at -70˚C. Additional information about the tissues and cases on these arrays can be obtained from the BD Biosciences Clontech website (www.clontech.com). Loading of the cDNAs on both arrays is normalised for three housekeeping genes to enable quantitative comparisons between gene expressions in different tissues.

### Normal tissues

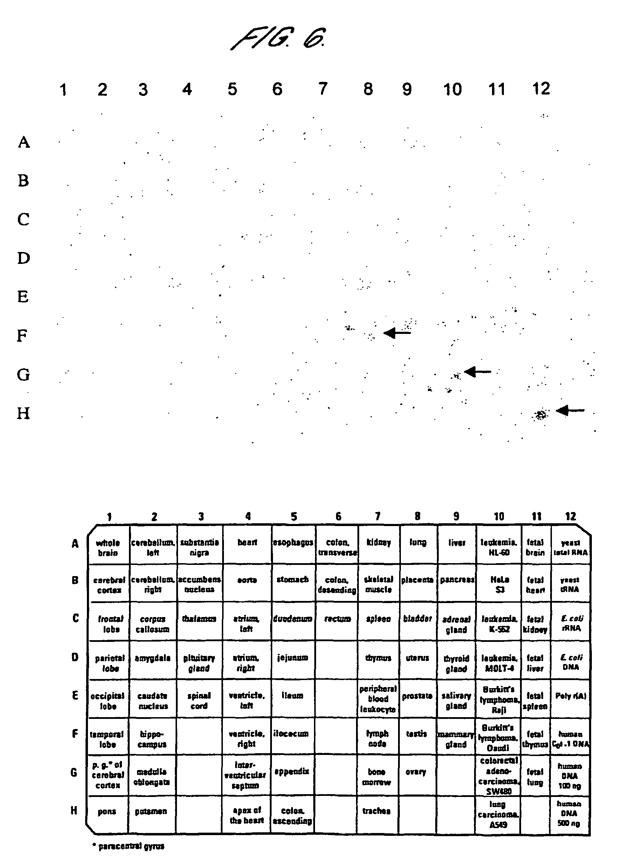

The normal tissues on the MTE array come from non-diseased victims of sudden death/trauma and are pooled from a number of individuals. The expression of the *OX-TES-1* mRNA in normal human tissues was found to be restricted to testis (Figure 6). This is supported by the information in the UniGene folder that contains the splice variant (Hs.

160594), where all of the EST clones have been isolated from testis (or from pooled samples containing testis). The other positive signals sen on this array are with cDNA from a colorectal adenocarcinoma cell line and the human genomic DNA (500ng) control. A positive result with human genomic DNA can indicate the presence of a highly-abundant gene or a gene family. It could also indicate the presence of repetitive sequences in the probe. However, since there was no signal with the $C_0$t-1 DNA, the former is a more likely explanation.

**Neoplastic tissues**

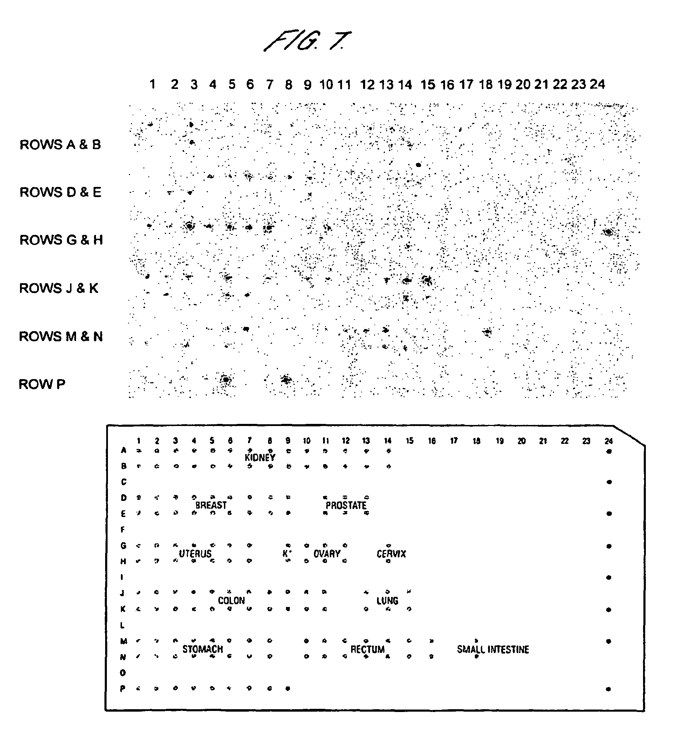

Analysis of *OX-TES-1* mRNA expression in human tumour tissues showed differential expression in all tumours, compared to adjacent histologically-normal tissue, included on the MTN array (Figure 7). It seems likely that this differential expression represents genuine overexpression, as no *OX-TES-1* mRNA expression was observed in those normal tissues on the MTE array (Figure 6). Differential levels of expression were also seen in the human cancer cell lines (Figure 7 Row P), with the melanoma (P5) and colorectal adenocarcinoma (P8) cell lines showing the highest levels. Incidentally, the colorectal adenocarcinoma cell line on the MTN array is the same as that on the MTE array, where it also gave a positive signal (Figure 6). Although the data between the two arrays may seem discordant, an important consideration when interpreting this data is that adjacent histologically-normal tissues from a cancer patient are not necessarily genotypically normal, and early genetic changes, which do not affect the appearance of the tissue, may also be present in the matched normal tissue of cancer patients. A positive signal was also seen with *E. coli* DNA (Figure 7, G24). Since there is no binding to either the yeast samples or the *E. coli* rRNA, this is unlikely to be due to nonspecific binding. More likely it is due to the presence of a bacterial homologue, or to similarity to bacterial DNA in the region of OX-TES-1 selected as the probe, although BLAST searching of the *E. coli* genome found no matches.

In conclusion, from both arrays it can be inferred that OX-TES-1 has a very restricted expression in normal tissues - present only in testis - but that it is widely expressed in a number of tumour and adjacent histologically-normal tissues from cancer patients. As such, this points towards OX-TES-1 being a novel Cancer-Testis Antigen (CTA). CTAs are a novel group of SEREX-identified antigens that are expressed only in normal testis (occasionally placenta and/or uterus) and human cancers, and since testis is an immunologically-privileged site, CTAs are ideal targets for immunotherapeutic vaccines (Chen et al., 1998; Jager et al., 2000). CTAs are reported to be expressed more frequently in T-cell lymphomas than B-cell lymphomas (Xie et al., 2003). Therefore the identification of a novel CTA that is expressed in a B-cell lymphoma furthers the possibility of their use in vaccines for B-cell malignancies. The finding that OX-TES-1 is common to a wide range of different tumours raises the possibility that this antigen may have a diagnostic or therapeutic use in a variety of cancers.

**Expression of the OX-TES-1 gene in DLBCL cell lines**

The inventors have characterised the expression of the OX-TES-1 mRNA in a number of DLBCL cell lines to confirm the expression of OX-TES-1 in lymphomas, and to establish which of the two alternatively-spliced variants might be present and, therefore, relevant to DLBCL.

DLBCL-derived cell lines were maintained in RPMI 1640 medium (Sigma Aldrich, UK) supplemented with 5% foetal calf serum (10% normal human serum for OCILY10) and antibiotics (penicillin (5000U/ml) and streptomycin (5000μg/ml); Invitrogen, UK) in an atmosphere of 5% $CO_2$ at 37˚C. Cells were washed in RNase-free PBS prior to mRNA extraction. The cell lines were representative of the activated (poor prognosis) (OCILY3, OCILY10 and HLY-1) and the germinal centre (good prognosis) (SUDHL-6) subtypes, as well as some of unknown subtype (MIEU, LIB and DEAU).

RT-PCR was carried out as follows: Poly(A)+ mRNA from the seven DLBCL-derived cell lines was extracted using μMACS mRNA Isolation kits (Miltenyi Biotech, Germany). cDNA was reverse transcribed at 42˚C for 50 minutes from 20ng mRNA in a 25μl reaction containing 200U Superscript II™ RNase $H^-$ reverse transcriptase (Invitrogen, UK), 1x First Strand Buffer, 4mM DTT and 100ng of either oligo(dT) primer or random hexamers. The integrity of cDNA templates was assessed using gene-specific primers to b-actin. 2μl cDNA was amplified in a 25μl PCR containing 200μM each dNTP, 10μM each primer, 1x PCR buffer and 1x Advantage 2 Polymerase mix (BD Biosciences Clontech, California, USA). Gene specific primers were designed to amplify fragments from 211bp to 1500bp, and their sequences are shown in Table x. Cycling parameters were as follows: 5 mins at 94˚C (initial denaturation) then 45 seconds at 94˚C, 45 seconds at appropriate annealing temperature and 2 1/2 or 5 minutes at 72˚C for 30 or 35 cycles (see Table x also). Phagemid DNA containing the appropriate cDNA insert was used as a positive control, whilst the negative control was a PCR mixture with no cDNA template. PCR products were visualised after separation in agarose gels by staining with ethidium bromide. Check table name

**Expression of OX-TES-1 in DLBCL cell lines**

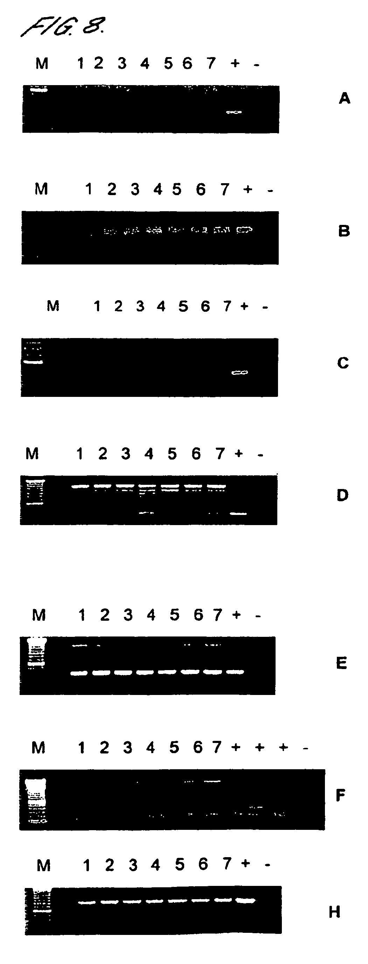

Analysis of OX-TES-1 mRNA with primer set A in DLBCL-derived cell lines successfully amplified fragments, in all cell lines tested, of the same size as the positive control (211bp) (see Figure 8B; 8A shows the results from the primary amplification). No product is observed with the negative control. This suggests that OX-TES-1 is transcribed in DLBCL cell lines. Although the PCR is not quantitative, the fact that all products obtained with the cell line cDNAs are of similar intensity suggests that similar levels of expression occur in all types of DLBCL.

**Analyses of expression of OX-TES-1 splice variants in DLBCL**

Since primer set A is designed to amplify a region of the cDNA that is present in both OX-TES-1 and the splice variant BC040301, RT-PCR was carried out with primers designed to the region that is deleted in the splice variant - i.e. specific to OX-TES-1 (primer set B). Figure 8D (8C shows the results from the primary amplification) shows that a fragment corresponding to the same size as the positive control (360bp) is successfully amplified in all cell lines; no product is observed with the negative control. This suggests that the OX-TES-1 variant is transcribed in DLBCL. Interestingly, when the cDNA is synthesised using oligo(dT) primers, an additional fragment of ~1100bp is observed (seen to a lesser extent with hexamer-primed cDNA (Figure 8E) that is much stronger than the expected product. This fragment is too small to be the 3' RACE product if the transcript in DLBCL is 4109bp, as the original cDNA is, as this would be expected to be 1929bp in size. This larger fragment is currently being analysed to determine its sequence and whether it represents another variant of OX-TES-1 that may be present in DLBCL, or just an artefact of the PCR.

In order to determine whether both variants are transcribed in DLBCL, RT-PCR was carried out with primers designed to flank the region that is deleted in splice variant BC040301 (primer set C). With these primers, a 1505bp fragment indicates the presence of OX-TES-1 whilst a 238bp fragment indicates the presence of the splice variant. Figure 8G shows that a fragment of ~1500bp is amplified in all cell lines, and that there is no ~200bp fragment seen in any cell line, suggesting that only the OX-TES-1 variant is transcribed in DLBCL (8F shows the results from the primary amplification where the 1500bp product is only just visible in some cell lines).

**Expression on the OX-TES-4 gene in DLBCL cell lines**

The inventors have characterised the expression of the OX-TES-4 mRNA in a number of DLBCL cell lines to investigate whether OX-TES-4 expression occurs.

The protocol used was as described for OX-TES-1. Gene specific primers were designed to amplify a fragment of 685bp, and the sequences are shown in Table 2.

**Expression of OX-TES-4 in DLBCL cell lines**

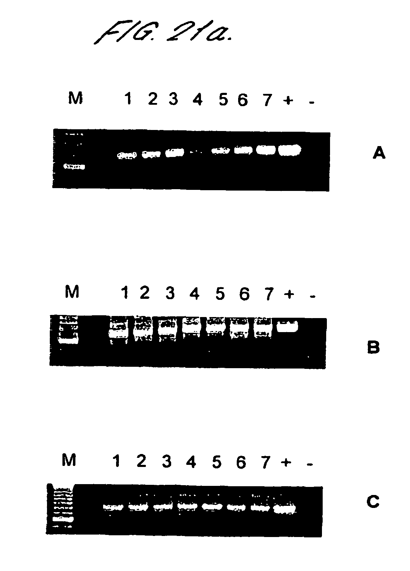

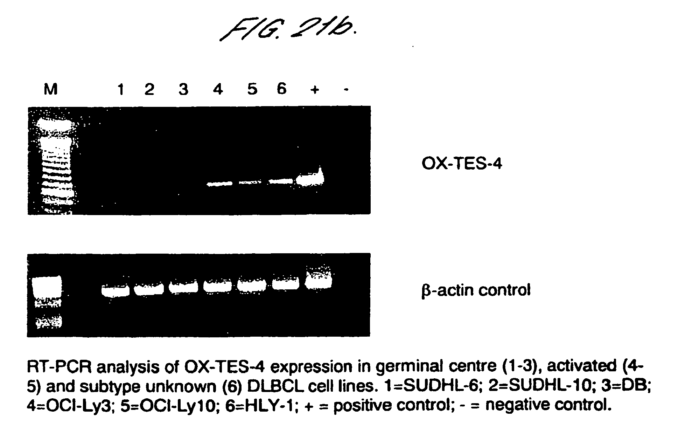

Analysis of OX-TES-4 mRNA in DLBCL-derived cell lines successfully amplified a fragment, in all cell lines tested, of the same size as the positive control (685bp; see Figure 21a). No product is observed with the negative control. This suggests that OX-TES-4 is transcribed in DLBCL cell lines. Interestingly, the primary PCR shows a fragment of fainter intensity for SUDHL6 compared to the remaining cell lines (Figure 21A). Although the PCR is not quantitative, this result suggests that there may be lower levels of expression of OX-TES-4 in germinal centre- compared to activated-DLBCL. After a second round of PCR, the difference in intensities is no longer observed (Figure 21b). This result is reproducible and is independent of whether the cDNA is oligo(dT)- or hexamer-primed. Figure 21C shows the products obtained when primers for β-actin are used on these cDNAs. Strong bands are obtained with all cell lines, which implies that the cDNAs are all suitable for PCR and will give comparable products where expression is equal, adding further support to the suggestion of genuine differential expression of OX-TES-4 mRNA. This result suggests that OX-TES-4 may be a suitable reagent for subtyping DLBCL.

Additional germinal center DLBCL cell lines (SUDHL10 and DB) were analysed for OX-TES-4 mRNA expression using RT-PCR as described above. No PCR product was observed in these two cell lines providing additional support for the utility of OX-TES-1 expression levels in subtyping DLBCL. Further work is underway to quantitate this differential expression.

**Selection of patients' serum for testis library screening using the SEREX technique.**

SEREX screening of a cDNA library requires approximately 20ml of serum from a DLBCL patient. Because this volume of serum required a blood sample to be taken, that was not part of routine monitoring or treatment, ethical permission was required to obtain the serum sample (ethical permission was obtained for this project), as was the informed consent of the subject. Sufficient volumes of serum were obtained from three DLBCL patients attending Dr Chris Hatton's Lymphoma Clinic at the John Radcliffe Hospital in Oxford. All three sera were cleaned using the serum cleaning protocol described in the SEREX methodology.

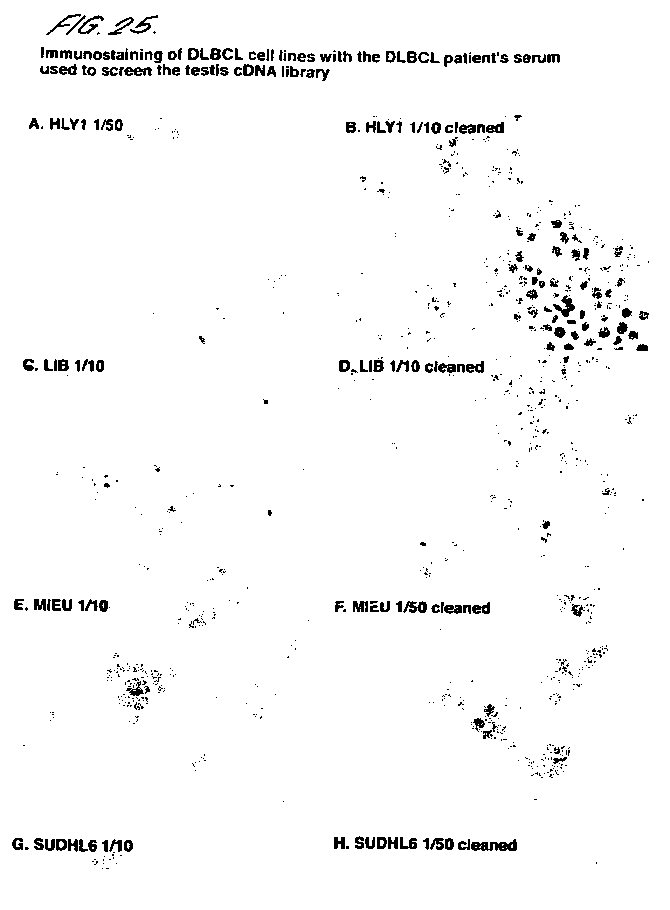

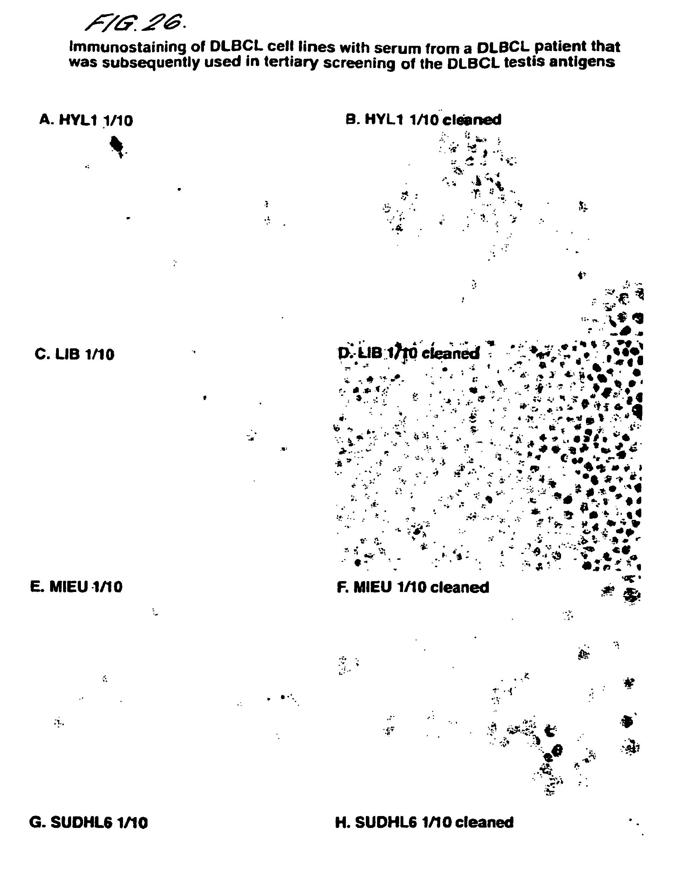

Patients' serum samples, both before and after cleaning, were used to immunostain DLBCL derived cell lines (HLY-1, Mieu, Lib, SUDHL6). Cytocentrifuge preparations of the DLBCL cell lines were stained using an immunoperoxidase technique, as described previously to detect antibodies to NPM-ALK in plasma samples from patients with ALCL (Pulford et al, 2000). The serum sample selected showed immunoreactivity with the DLBCL cell lines, both before and after cleaning, confirming that antibodies to proteins expressed by this lymphoma were present in the serum sample used to screen the testis cDNA library (Figure 25). Each panel in the figure is labelled with the cell line that was immunostained, and the dilution of human serum used ("cleaned" represents staining using cleaned serum). Figure 26 illustrates the immunostaining data of one of the additional DLBCL sera that was used in the tertiary screening process. This demonstrates that this serum also has reactivity on DLBCL cell lines, even after being cleaned.

**Results from screening DLBCL antigens with sera from additional patients.**

The 28 testis antigens identified by the inventors were those proteins that were immunologically recognised by the serum from a single patient with DLBCL. To assess the relevance of these antigens in DLBCL, screening with serum from increased numbers of patients and from control subjects was required. To assess the frequency with which these proteins were recognised by DLBCL patients' all 94 clones were screened with serum from an additional 4 DLBCL patients, and with serum from 5 age- and sex-matched control subjects (provided by the Blood Transfusion Service). The age- and sex-matching is important as the immune response varies with age and sex, for example women over 40-50 naturally have a higher titre of autoantibodies in their serum. This additional information has provided an indication of the tumour specificity of the immune response to these antigens, and a summary of the data is presented below.

**1. Antigens specifically recognised by multiple DLBCL patients' sera**

| | |
|---|---|
| FLJ31673 | 4 patients and no normals |
| PP1Aa | 3 patients and no normals. The mRNA encoding this gene is differentially expressed in DLBCL (Lymphochip data). |
| Histone H3.3B | 3 patients and no normals |
| Novel PAS protein RANBP2 | 3 patients and no normals |
| | 5 patients and no normals. Some low level differential expression of this mRNA was observed in both FL and DLBCL (Lymphochip data). |

**2. Patient specific antigens recognised only by serum used for library screening**

| | |
|---|---|
| Bacl | 1 patient and no normals |
| KIAA03521 patient and no normals | |
| ZHX1 | 1 patient and no normals |
| VAT1 | 1 patient and no normals |

**3. Antigens recognised with a higher frequency by patients' sera**

| | |
|---|---|
| SRPK1 | 4 patients and 1 normal. The mRNA encoding this gene is differentially expressed in DLBCL (Lymphochip data). |
| RNF20 | 3 patients and 1 normal |
| HnRNP A2/B1 | 4 patients and 1 normal |
| STK11 | 5 patients and 2 normals. The mRNA encoding this gene shows increased expression in DLBCL compared to FL and there was some differential expression within DLBCL (Lymphochip data). |
| HIS1 | 5 patients and 2 normals |
| MAPK6 | 3 patients and 1 normal. The mRNA encoding this gene shows higher expression in DLBCL compared to FL. There is some differential expression within DLBCL (Lymphochip data). |

**4. Antigens recognised by both patients' and normal sera**

| | |
|---|---|
| CBF1 | 4 patients and 3 normals. The mRNA expression of this gene is significantly higher in the majority of DLBCL patients compared to FL patients. Within the DLBCL cases on the Lymphochip there is differential expression of this gene. |
| MacroHistone H2A | 1 patient and 1 normal |
| KIAA0643 | 4 patients and 5 normals |
| ERCC3 | 2 patients and 1 normal. The mRNA encoding this gene is expressed in DLBCL but no differential expression was observed (Lymphochip data). |
| GKAP42 | 4 patients and 2 normals |
| Sirtuin | 2 patients and 1 normal |
| PSP1 | 2 patients and 1 normal |
| YB1 | 5 patients and 4 normals |
| TZP | 5 patients and 4 normals |
| FLJ13942 | 5 patients and 3 normals |
| Adducin la | 4 patients and 3 normals |
| ARA267b | 2 patients and 2 normals |
| BAP1 | 2 patients and 2 normals |

The antigens are divided into four groups based on the frequency at which antibodies against the proteins were detected in either DLBCL patients' or control sera. Group one contains 5 antigens that are recognised by serum from more than one patient (in all cases the majority of DLBCL patients contain antibodies to these proteins) and none of the control sera. This is the most significant group at this stage of the analysis. Two of these genes are already known from the Lymphochip data to be expressed in DLBCL, confirming that genes cloned from the testis library are expressed in DLBCL patients. These antigens are potentially immunologically tumour-specific, and may represent good candidates for immunotherapy.

Group 2 contains 4 antigens that are specifically recognised only by the serum that was used to screen the testis library. Further studies are required with larger numbers of patients to assess the overall frequency and relevance of these data. If the clinical characteristics of this patient are significantly different to the additional patients studied, then screening with increased numbers of sera may identify more patients that recognise these antigens. These are possibly of less general interest in DLBCL, but may be valuable for identifying and possibly treating a subset (s) of patients with different clinical characteristics.

Group 3 contains 6 antigens that are recognised by the majority of DLBCL patients' sera and one or two normal sera. Of these, 4 are only recognised by serum from a single normal individual. It is always possible that one of the control individuals has undiagnosed cancer (although no one normal serum is reactive with all these clones) thus larger numbers of patients and control individuals will determine whether this is an infrequent event. The expression of these antigens is still likely to be of interest in DLBCL. Three of these genes have differential mRNA expression within DLBCL patients and 2 of these 3 show increased expression in DLBCL patients when compared to patients with another germinal centre derived B-cell lymphoma, FL (that can transform into DLBCL). Not all clinically relevant lymphoma antigens show tumour specific expression; for example BCL-2 and BCL-6 are both expressed in a subset of normal B cells in addition to B cell lymphomas.

Group 4 contains 13 antigens that are recognised by serum from both DLBCL patients and controls. Immunologically these antigens are the least interesting group. However the Lymphochip mRNA expression data for CBF1 indicates that the mRNA encoding this antigen is expressed at higher levels in the majority of DLBCL patients when compared to those with FL. The expression levels of CBF1 and other members of this group may therefore still have clinical significance in DLBCL that could influence diagnosis or prognosis.

Since a testis library was screened with DLBCL serum, the relevance of the identified antigens to DLBCL needed to be established. For a number of antigens, previous microarray studies had demonstrated that these genes are expressed in DLBCL (Alizadeh et al., 2000). These are OX-TES-6, 10, 13, 15, 16 and 19. The inventors have characterised the expression of a number of the remaining OX-TES antigen mRNAs in a number of DLBCL cell lines to investigate whether expression occurs, alongside some of those already known to be expressed in DLBCL acting as controls. The protocol used was as described for OX-TES-1. Gene specific primers were designed to amplify fragments of 100-800bp, and sequences are shown in Table 2.

**Expression of OX-TES antigens in DLBCL cell lines**

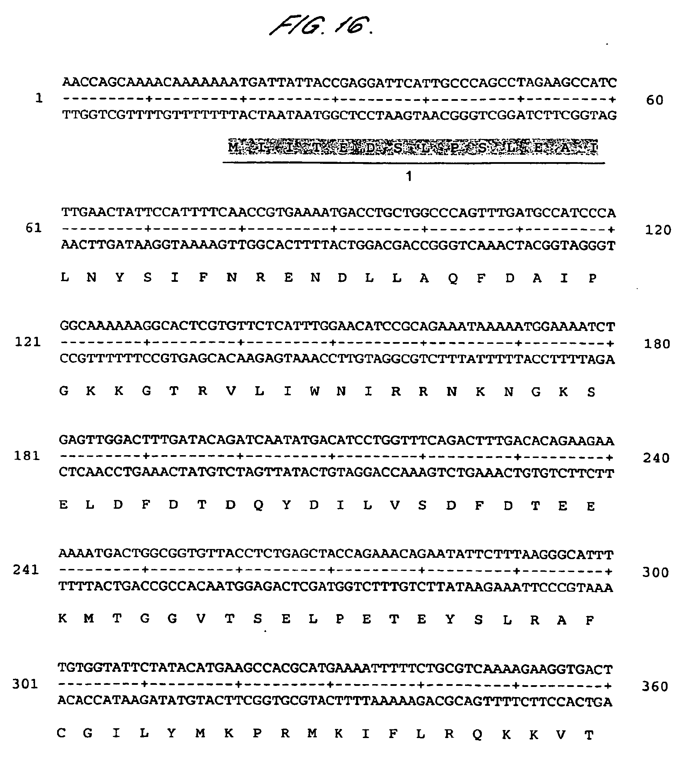

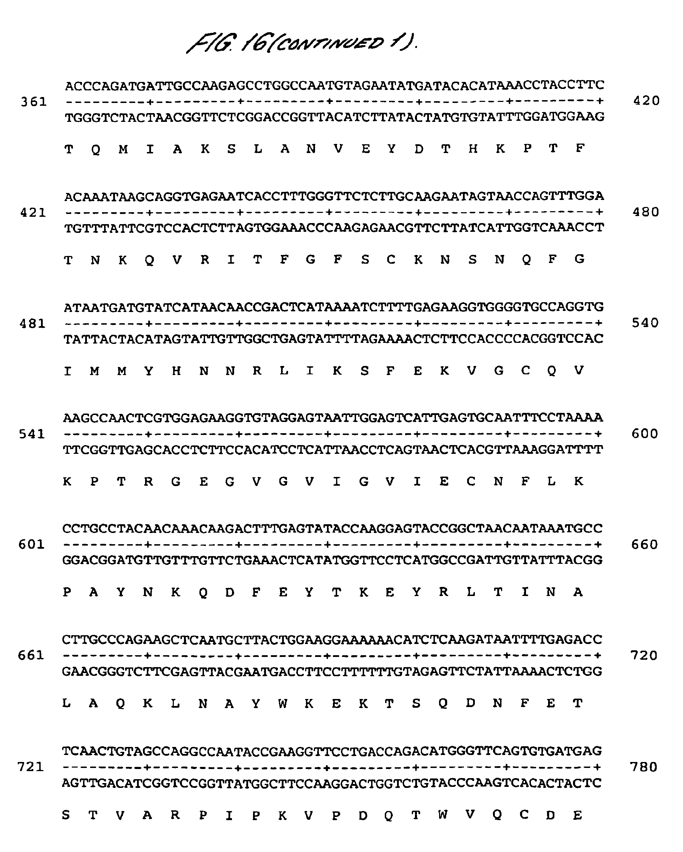

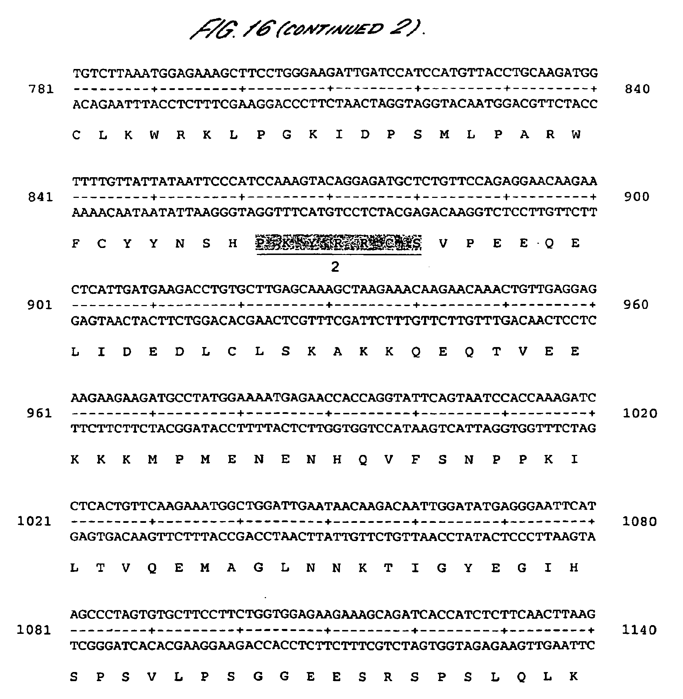

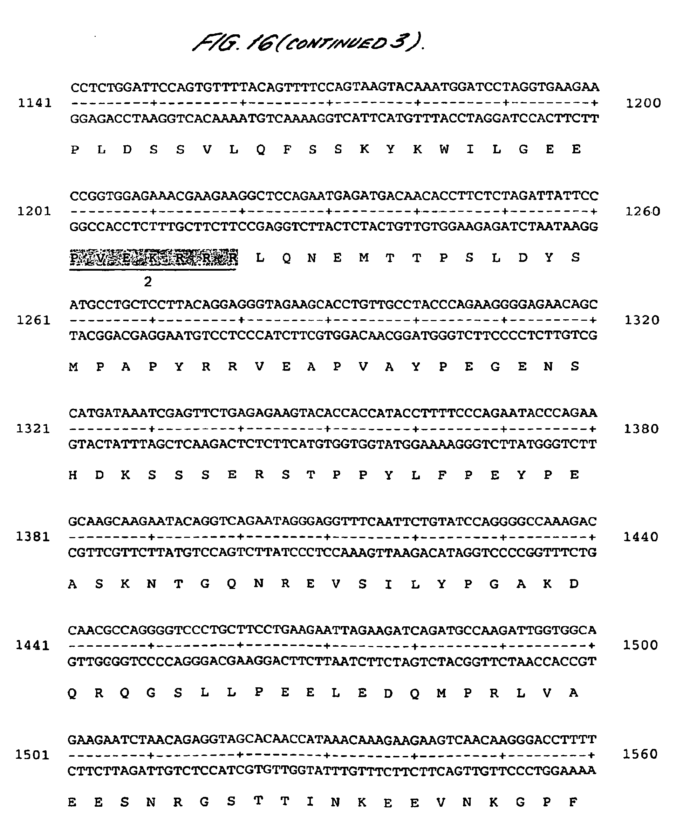

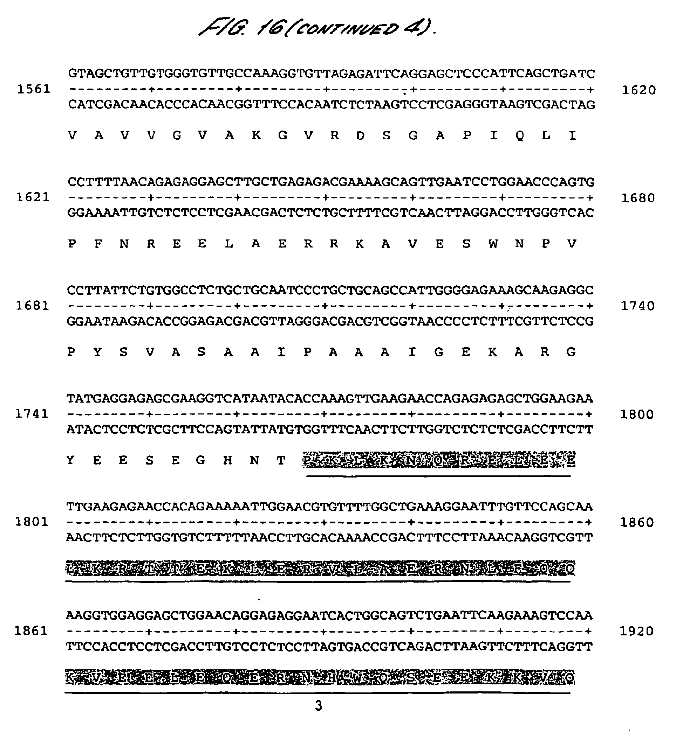

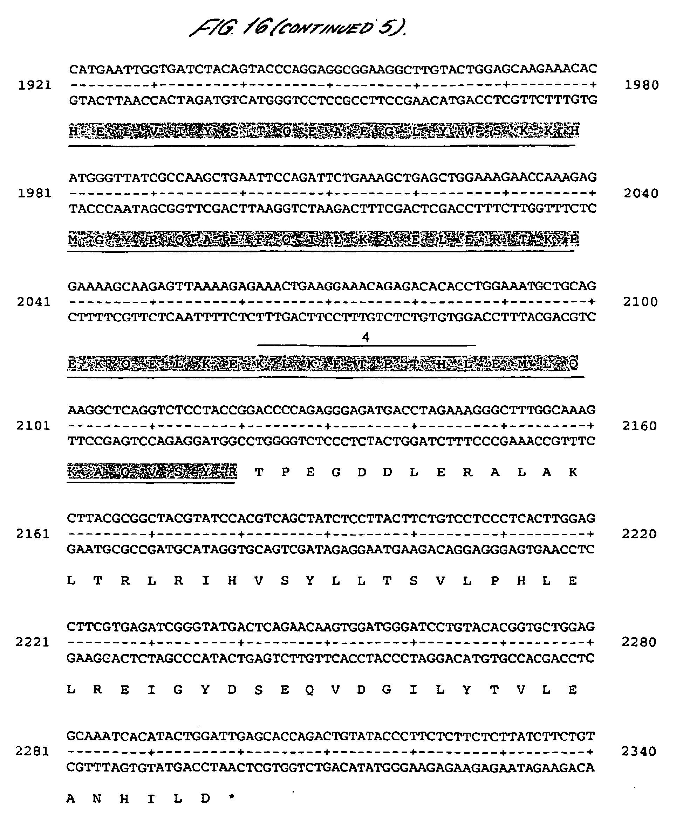

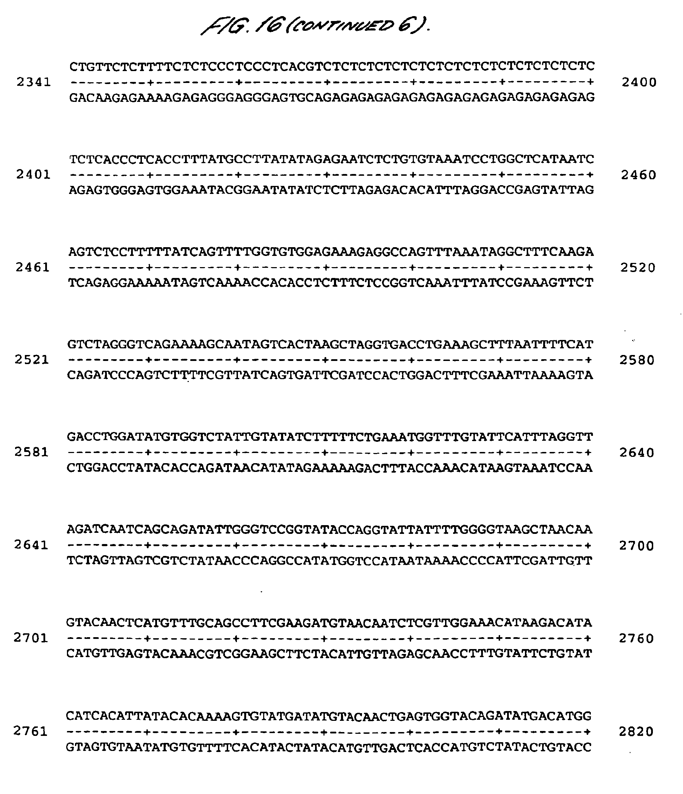

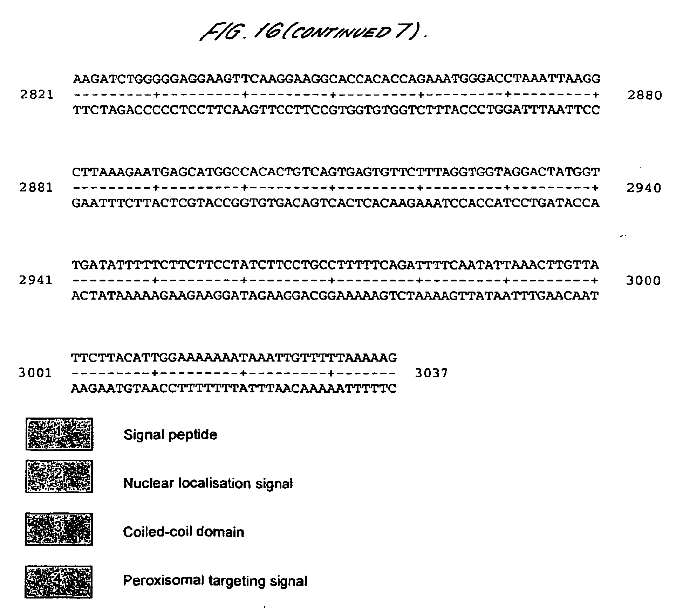

All of the cell lines tested, which included both ABC-DLBCLs and GC-DLBCLs, gave a product of the expected size for the eighteen antigens tested (see Figure 16) with the exception of OX-TES-12. Here, the product was slightly larger than the positive control but sequencing data revealed that the correct fragment had been amplified.

These results indicate that transcripts of these antigens are certainly present in DLBCL. Most of the antigens required two rounds of PCR to

amplify a product of similar intensity of the positive control in each case (after staining with ethidium bromide) due to the small amount of starting material. However, OX-TES-11, 12 and 21 required only one round, which, although the PCR was not quantitative, might suggest a high expression level of these antigens in DLBCL. Since overexpressed antigens are a common group of SEREX-defined antigens, this result was not surprising, although further work is required to determine whether there is genuine overexpression in comparison with normal tissues. Interestingly, only six of the twenty-eight antigens (21%) identified in this study are included on the original microarray study (Alizadeh et al., 2000), which suggests that there are possibly a wide variety of DLBCL-associated antigens as yet unidentified, and that SEREX is a successful approach to use in uncovering them.

**Recognition of SEREX-defined antigens by sera lymphoma/leukaemia patients and healthy individuals**

To determine whether the antigens identified in this study were DLBCL-related, and to assess the frequency and specificity of antibody responses to these antigens in DLBCL, AML and CML patients, the antigens were screened with sera from additional patients and control individuals, resulting in data from ten DLBCL, ten AML and ten CML patients, as well as twenty control individuals in a tertiary screen. The screening data is shown in Tables 3 and 4. With respect to DLBCL only, eight antigens (OX-TES-2 to 9) appear to be disease-specific, whilst twenty-three antigens (all except OX-TES-17, 18, 19, 25 and 28) show a higher reactivity with patient sera over healthy controls. Taking AML and CML into account, of the fifteen antigens tested, three, OX-TES-3, 4 and 6, are now disease-specific, but only one, OX-TES-4, is specific to DLBCL, showing no reactivity with CML, AML or normal control sera, and reacting with 50% of sera from DLBCL patients. Twelve of the antigens (OX-TES-1, 3, 4, 5, 11, 12, 13, 15, 20, 22, 24 and 28) now show a higher frequency of reactivity with sera from patients with disease over normal individuals, but this higher frequency is restricted to DLBCL for OX-TES-1, 4, 12, 15 and 28, i.e. there is no reactivity detected with CML and AML sera for these antigens. None of the antigens have a greater reactivity with any of the leukaemia sera over DLBCL sera, suggesting that these might be more specific to DLBCL, although OX-TES-6, 10 and 21 elicit responses in a similar number of DLBCL and CML patients. Overall, the antigens seem to have a patient reactivity of DLBCL > CML > AML. Only OX-TES-18 and 24 have a greater reactivity with normal control sera over DLBCL, CML or AML sera, whilst OX-TES-19, 25 and 28 show an identical level of reactivity. Four of the antigens (OX-TES-2, 7, 8, and 9) reacted only with the serum used to screen the library. These antigens may therefore be patient- rather than disease-related.

Cancer-related immunogenicity implies a possible biological relevance of the antigens to tumourigenesis and/or disease progression, whilst antigens detected at a higher frequency in patients may also serve as diagnostic markers for disease diagnosis and monitoring (Chen 2000). For antigens to be diagnostically useful, antibodies against the antigen must be found in a considerable proportion of patients, and the antibody response in normal sera must be at a much lower frequency; the diagnostic/ marker potential of the SEREX-defined DLBCL antigens is exemplified by the fact that serum samples from 70% of DLBCL patients detect three or more of these antigens. The lower cross-reactivity with AML and CML sera suggests that these antigens may only be lymphoma specific and not applicable to all haematological malignancies.

On the whole it is unclear as to why some antigens are immunogenic in apparently healthy controls; one suggestion is that the antigens are expressed in normal cells under non-malignant conditions such as viral infection or inflammatory processes (Sahin et al., 1995; Tureci et al., 1997). The fact that some antigens may be ubiquitously expressed but become immunogenic in a cancer patient suggests that the context in which the antigen is presented in is relevant i.e. the presentation of a protein to the immune system in a context of danger is more decisive for its immunogenicity (and breaking of tolerance) rather than its restricted expression in a given tissue (the idea of "contextual immunogenicity" (Ono et al., 2000; Preuss et al., 2002; Schmits et al., 2002)Although it is possible that the antibodies detected in healthy donors may not be directed at the same B-cell epitopes on these proteins, it cannot be ruled out that the normal donor may have the disease unknowingly, or that the epitope is not common tosome other protein, or that the antibody in the control sera is against a wild-type protein and it is cross-reacting with the similar epitope on the mutated cancer-related antigen. Significantly, it remains to be determined if any of the antibody responses identified here arose as a result of treatment; OX-TES-10 (SRPK1), OX-TES-14 (HIS1) and OX-TES-23 (NSEP1) have a known relationship with cisplatin resistance (Ohga et al., 1996; Rifkind et al., 1996; Ise et al., 1999; Schenk et al., 2001; Yahata et al., 2002). In this study we used serum from a patient who was very young to have DLBCL, and had a very aggressive form that was never in remission. The serum was taken post-treatment and therefore the detection of antigens identified in this study may be related to the failure of the treatment, or indicative of a form of DLBCL that will be resistant to current treatment.

**References**

Aasland, R., T. J. Gibson and A. F. Stewart (1995). The PHD finger: implications for chromatin-mediated transcriptional regulation. Trends Biochem. Sci. 20: 56-59.

Aguiar, R. C., Y. Yakushijin, S. Kharbanda, et al. (2000). BAL is a novel risk-related gene in diffuse large B-cell lymphomas that enhances cellular migration. Blood 96: 4328-4334.

Alizadeh, A. A., M. B. Eisen, R. E. Davis, et al. (2000). Distinct types of diffuse large B-cell lymphoma identified by gene expression profiling. Nature 403: 503-511.

Amakawa, R., W. Jing, K. Ozawa, et al. (1993). Human Jk recombination signal binding protein (IGKJRB): comparison with its mouse homologue. Genomics 17: 306-315.

Angrand, P.O., F. Apiou, A. F. Stewart, et al. (2001). NSD3, a new SET domain-containing gene, maps to 8p12 and is amplified in human breast cancer cell lines. Genomics 74: 79-88.

Baars, J. W., D. de Jong, E. M. Willemse, et al. (1999). Diffuse large B-cell non-Hodgkin lymphomas: the clinical relevance of histological subclassification. Br. J. Cancer 79: 1770-1776.

Banerjee, D. (1999). Technology evaluation: G-3139. Curr Opin Mol Ther 1: 404-8.

Bea, S., M. Ribas, J. M. Hernandez, et al. (1999). Increased number of chromosomal imbalances and high-level DNA amplifications in mantle cell lymphoma are associated with blastoid variants. Blood 93: 4365-4374.

Bei, R., L. Masuelli, E. Moriconi, et al. (1999). Immune responses to all ErbB family receptors detectable in serum of cancer patients. 18: 1267-1275.

Benharroch, D., Z. Meguerian-Bedoyan, L. Lamant, et al. (1998). ALK- positive lymphoma: a single disease with a broad spectrum of morphology. Blood 91: 2076-2084.

Biamonti, G., M. Ruggiu, S. Saccone, et al. (1994). Two homologous genes, originated by duplication, encode the human hnRNP proteins A2 and A1. Nucleic Acids Res. 22: 1996-2002.

Bochar, D. A., J. Savard, W. Wang, et al. (2000). A family of chromatin remodeling factors related to Williams syndrome transcription factor. Proc. Natl. Acad. Sci. U.S.A. 97: 1038-1043.

Boon, T. and L. J. Old (1997). Cancer Tumor Antigens. Curr Opin Immunol 9: 681-3.

Brass, N. and e. al. (1997). Translation initiation factor eIF-4g is encoded by an amplified gene and induces an immune response in squamous lung carcinoma. Hum. Mol. Genet. 6: 33-39.

Brichard, V., A. Van Pel, T. Wölfel, et al. (1993). The tyrosinase gene codes for an antigen recognized by autologous cytolytic T lymphocytes on HLA-A2 melanomas. J. Exp. Med. 178: 489-495.

Capowski, E. E., S. Esnault, S. Bharracharya, et al. (2001). Y box-binding factor promotes eosinophil survival by stabilizing granulocyte-macrophage colony-stimulating factor mRNA. J. Immunol. 167: 5970-5976.

Caron de Fromental, C., F. May-Levin, H. Mouriesse, et al. (1987). Presence of circulating antibodies against cellular protein p53 in a notable proportion of children with B cell lymphoma. Int. J. Cancer 39: 185-189.

Castiglia, D., A. Cestelli, M. Scaturro, et al. (1994). H1(0) and H3.3B mRNA levels in developing rat brain. Neurochem. Res. 19: 1531-1537.

Changolkar, L. N. and J. R. Pehrson (2002). Reconstitution of nucleosomes with Histone MacroH2A1.2. Biochem. 41: 179-184.

Chen, C. Y., R. Gherzi, J. S. Anderson, et al. (2000). Nucleolin and YB-1 are required for JNK-mediated interleukin-2 mRNA stabilization during T-cell activation. Genes Dev. 14: 1236-1248.

Chen, Y. T. and L. J. Old (1999). Cancer-testis antigens: targets for cancer immunotherapy. Cancer J. Sci. Am. 5: 16-17.

Chen, Y. T. (2000). "Cancer vaccine: identification of human tumor antigens by SEREX." Cancer J Sci Am 6 Suppl 3: S208-17.

Chen, Y. T., Gure, A. O., Tsang, S., Stockert, E., Jager, E., Knuth, A. and Old, L. J. (1998). "Identification of multiple cancer/testis antigens by allogeneic antibody screening of a melanoma cell line library." Proc Natl Acad Sci U S A 95(12): 6919-23.

Cheng, A., P. Kaldis and M. J. Solomon (2000). Dephosphorylation of human cyclin-dependent kinases by protein phosphatase type 2C alpha and beta 2 isoforms. J. Biol. Chem. 275: 34744-34749.

Cheng, M., T. G. Bouton and M. H. Cobb (1996). ERK3 is a constitutively nuclear protein kinase. J. Biol. Chem. 271: 8951-8958.

Chiou, C. C., C. C. Chan, D. L. Sheu, et al. (2001). Helicobacter pylori infection induced alteration of gene expression in human gastric cells. Gut 48: 598-604.

Cigudosa, J. C., N. Z. Parsa, D. C. Louie, et al. (1999). Cytogenetic analysis of 363 consecutively ascertained diffuse large B-cell lymphomas. Genes Chrm. Cancer 25: 123-133.

Coles, L. S., P. Diamond, F. Occhiodoro, et al. (1996). Cold shock domain proteins repress transcription from the GM-CSF promoter. Nucl. Acids Res. 24: 2311-2317.

Cordell, J. L., K. A. F. Pulford, B. Bigerna, et al. (1999). Detection of normal and chimeric nucleophosmin in human cells. Blood 93: 632-642.

Crawford, L. V., D. C. Pim and R. D. Bulbrook (1982). Detection of antibodies against the cellular protein p53 in sera from patients with breast cancer. Int. J. Cancer 30: 403-408.

Dalla-Favera, R., B. H. Ye, F. Le Coco, et al. (1994). Identification of genetic lesions associated with diffuse large-cell lymphoma. Ann. Oncol. 5 Suppl: S55-S60.

Daniel, J. M. and A. B. Reynolds (1999). The catenin p120(ctn) interacts with Kaiso, a novel BTB/POZ domain zinc finger transcription factor. Mol. Cell Biol. 19: 3614-3623.

Das, A. K., N. R. Helps, P. T. W. Cohen, et al. (1996). Crystal structure of the protein serine/threonine phosphatase 2C at 2.0 angstrom resolution. EMBO J. 15: 6798-6809.

Davis, R. J. (1995). Transcriptional regulation by MAP kinases. Mol. Reprod. Dev. 42: 459-467.

Deltour, S., C. Guerardel and D. Leprince (1999). Recruitment of SMRT/N-CoR-mSin3A-HDAC-repressing complexes is not a general mechanism for BTB/POZ transcriptional repressors: the case of HIC-1 and gammaFBP-B. Proc. Natl. Acad. Sci. U.S.A. 96: 14831-14836.

Didier, D. K., J. Schiffenbauer, S. L. Woulfe, et al. (1988). Characterization of the cDNA encoding a protein binding to the major histocompatibility complex class II Y box. Proc. Natl. Acad. Sci. U.S.A. 85: 7322-7326.

Disis, M. L. and M. A. Cheever (1996). Oncogenic proteins as tumour antigens. Curr. Opin. Immunol. 8: 637-642.

Disis, M. L., Calenoff, E., McLaughlin, G., Murphy, A. E., Chen, W., Groner, B., Jeschke, M., Lydon, N., McGlynn, E., Livingston, R. B. and et al. (1994). "Existent T-cell and antibody immunity to HER-2/neu protein in patients with breast cancer." Cancer Res 54(1): 16-20.

Donoghue, S., H. S. Baden, I. Lauder, et al. (1999). Immunohistochemical localization of caspase-3 correlates with clinical outcome in B-cell diffuse large-cell lymphoma. Cancer Res. 59: 5386-5391.

Dropcho, E. J., Y.-T. Chen, J. B. Posner, et al. (1987). Cloning of a brain protein identified by autoantibodies from a patient with paraneoplastic cerebellar degeneration. Proc. Natl. Acad. Sci. USA 84: 4552-4556.

Dunbar, P., G. Ogg, J. Chen, et al. (1998). Direct isolation, phenotyping and cloning of low-frequency antigen specific cytotoxic T lymphocytes from peripheral blood. Curr. Biol. 8: 413-6.

Evdokimova, V., P. Ruzanov, H. Imataka, et al. (2001). The major mRNA-associated protein YB-1 is a potent 5' cap-dependent mRNA stabilizer. EMBO J. 20: 2491-5502.

Falini, B., Pileri, S., Zinzani, P.L., Carbone, A., Zagonel, V., Wolf-Peeters, C., Verhoef, G., Menestrina, F., Todeschini, G., Paulli, M., Lazzarino, M., Giardini, R., Aiello, A., Foss, HD., Araujo, I., Fizzotti, M., Pelicci, PG., Flenghi, L., Martelli, MF., Santucci, A., 1999. ALK+ lymphoma: clinico-pathological findings and outcome. Blood 93: p2697-706.

Favreau, C., H. J. Worman, R. W. Wozniak, et al. (1996). Cell cycle-dependent phosphorylation of nucleoporins and nuclear pore membrane protein Gp210. Biochem. 35: 8035-8044.

Ferrando-May, E., V. Cordes, I. Biller-Ckovric, et al. (2001). Caspases mediate nucleoporin cleavage, but not early redistribution of nuclear transport factors and modulation of nuclear permeability in apoptosis. Cell Death Differ. 8: 495-505.

Ferreira, P. A., C. Yunfei, D. Schick, et al. (1998). The cyclophilin-like domain mediates the association of Ran-binding protein 2 with subunits of the 19 S regulatory complex of the proteasome. J. Biol. Chem. 273: 24676-24682.

Fleischhacker, M., T. Beinert, M. Ermitsch, et al. (2001). Detection of ampifiable messenger RNA in the serum of patients with lung cancer. Ann. N.Y. Acad. Sci. 945: 179-188.

Fox, A.H., Lam, Y.W., Leung, A.K., Lyon, C.E., Andersen, J., Mann, M., Lamond, A.I., 2002. Paraspeckles. A novel nuclear domain. Curr Biol. 12: p13-25.

Fowler, L., J. Everitt, J. L. Stevens, et al. (1998). Redistribution and enhanced protein kinase C-mediated phosphorylation of alpha- and gamma-adducin during renal tumor progression. Cell Growth Differ. 9: 405-413.

Freemont, P. S. (2000). RING for destruction? Curr. Biol. 10: R84-87.

Friedman, L. S., E. A. Ostermeyer, E. D. Lynch, et al. (1995). 22 genes from chromosome 17q21: cloning, sequencing, and characterization of mutations in breast cancer families and tumors. Genomics 25: 256-263.

Fukata, Y., N. Oshiro, N. Kinoshita, et al. (1999). Phosphorylation of adducin by Rho-kinase plays a crucial role in cell motility. J. Cell Biol. 145: 347-361.

Gascoyne, R.D., Aoun, P., Wu, D., Chhanabhai, M., Skinnider, B.F., Greiner, T.C., Morris, S.W., Connors, J.M., Vose, J.M., Viswanatha, D.S., Coldman, A., Weisenburger, D.D., 1999. Prognostic significance of anaplastic lymphoma kinase (ALK) protein expression in adults with anaplastic large cell lymphoma. Blood 93: p3913-21.

Gilboa, E. (2001). The risk of autoimmunity associated with tumor immunotherapy. Nature Immunol. 2: 789-792.

Goyns, M. H., D. W. Hammond, C. J. Harrison, et al. (1993). Structural abnormalities of the X-chromosome in non-Hodgkins-lymphoma. Leukemia 7: 848-852.

Grillo-Lopez, A. J., B. K. Dallaire, A. McClure, et al. (2001). Monoclonal antibodies: a new era in treatment of Non-Hodgkin's lymphoma. Curr Pharm Biotechnol 2: 301-311.

Gu, C., T. Oyama, T. Osaki, et al. (2001). Expression of Y box-binding protein-1 correlates with DNA topoisomerase IIalpha and proliferating cell nuclear antigen expression in lung cancer. Anticancer Res. 21: 2357-2362.

Gu, Z., G. Kuntz-Simon, J. Rommelaere, et al. (1999). Oncogenic transformation-dependent expression of a transcription factor NF-Y subunit. Mol. Carcinog. 24: 294-299.

Gui, J. F., W. S. Lane and X. D. Fu (1994). A serine kinase regulates intracellular localization of spicing factors in the cell cycle. Nature 369: 678-682.

Halbach, T., N. Scheer and W. Werr (2000). Transcriptional activation by the PHD finger is inhibited through an adjacent leucine zipper that binds 14-3-3 proteins. Nuc. Acids Res. 28: 3542-3550.

Hanada, M., T. Kobayashi, M. Ohnishi, et al. (1998). Selective suppression of stress-activated protein kinase pathway by protein phosphatase 2C in mammalian cells. FEBS Lett. 437: 172-176.

Harris, N. L., E. S. Jaffe, H. Stein, et al. (1994). A revised European-American classification of lymphoid neoplasms - a proposal from the International Lymphoma Study-Group. Blood 84: 1361-1392.

Harris, N. L., E. S. Jaffe, J. Diebold, et al. (1999). The World Health Organization classification of neoplastic diseases of the hematopoietic and lymphoid tissues. Report of the Clinical Advisory Committee meeting, Airlie House, Virginia, November, 1997. Ann Oncol. 10: 1419-1432.

Hassfeld, W., G. Steiner, A. Studnicka-Benke, et al. (1995). Autoimmune response to the spliceosome. An immunologic link between rheumatoid arthritis, mixed connective tissue disease, and systemic lupus erythematosus. Arthritis Rheum. 38: 777-785.

Hayess, K., R. Kraft, J. Sachsinger, et al. (1998). Mammalian protein homologous to VAT-1 of Torpedo californica: isolation from Ehrlich ascites tumor cells, biochemical characterization, and organization of its gene. J. Cell Biochem. 69: 304-315.

Hofer, T., P. Spielmann, P. Stengel, et al. (2001). Mammalian PASKIN, a PAS-serine/threonine kinase related to bacterial oxygen sensors. Biochem. Biophys. Res. Commun. 288: 757-764.

Hraba-Renevey, S. and M. Kress (1989). Expression of a mouse replacement histone H3.3 gene with a highly conserved 3' noncoding region during SV40- and polyoma-induced Go to S-phase transition. Nucleic Acids Res. 17: 2449-2461.

Hsieh, J. J.-D., T. Henkel, P. Salmon, et al. (1996). Truncated mammalian Notch1 activates CBF1/RBPJk-repressed genes by a mechanism resembling that of Epstein=Barr virus EBNA2. Mol. Cell. Biol. 16: 952-959.

Hsu, F. J., C. B. Caspar, C. Czerwinski, et al. (1997). Tumor-specific idiotype vaccines in the treatment of patients with B-cell lymphoma: long term results of a clinical trial. Blood 89: 3129-3135.

Hu, Z., S. Jin and K. W. Scott (2000). Transcriptional activation of the MDR1 gene by UV irradiation. Role of NF-Y and Sp1. J. Biol. Chem. 275: 2979-2985.

Huang, N., E. vom Baur, J. M. Garnier, et al. (1998). Two distinct nuclear receptor interaction domains in NSD1, a novel SET protein that exhibits characteristics of both corepressors and coactivators. EMBO J. 17: 3398-3412.

Ise, T., G. Nagatani, T. Imamura, et al. (1999). Transcription factor Y-box binding protein 1 binds preferentially to cisplatin-modified DNA and interacts with proliferating cell nuclear antigen. Cancer Res. 59: 342-346.

Ise, T., Nagatani, G., Imamura, T., Kato, K., Takano, H., Nomoto, M., Izumi, H., Ohmori, H., Okamoto, T., Ohga, T., Uchiumi, T., Kuwano, M. and Kohno, K. (1999). "Transcription Factor Y-Box Binding Protein 1 Binds Preferentially to Cisplatin-modified DNA and Interacts with Proliferating Cell Nuclear Antigen." Cancer Res 59(2): 342-346.

Itoh, M., M. Watanabe, Y. Yamada, et al. (1999). HUB1 is an autoantigen frequently eliciting humoral immune response in patients with adult T cell leukemia. Int. J. Oncol. 14: 703-708.

Jager, E., Jager, D. and Knuth, A. (2000). "Peptide Vaccination in Clinical Oncology." Onkologie 23(5): 410-415.

Jäger, E., D. Jäger and A. Knuth (2000). Peptide vaccination in clinical oncology. Onkologie 23: 410-415.

Jager, E., Chen, Y. T., Drijfhout, J. W., Karbach, J., Ringhoffer, M., Jager, D., Arand, M., Wada, H., Noguchi, Y., Stockert, E., Old, L. J. and Knuth, A. (1998). "Simultaneous humoral and cellular immune response against cancer-testis antigen NY-ESO-1: definition of human histocompatibility leukocyte antigen (HLA)-A2-binding peptide epitopes." J Exp Med 187(2): 265-70.

Jäger, E., Y.-T. Chen, J. Drijfhout, et al. (1998). Simultaneous humoral and cellular immune response against cancer-testis antigen NY-ESO-1: Definition of human histocompatibility leucocyte antigen (HLA)-A2-binding peptide epitopes. J. Exp. Med. 187: 265-270.

Jäger, E., Y. Nagata, S. Gnjatic, et al. (2000). Monitoring CD8 T cell responses to NY-ESO-1: Correlation of humoral and cellular immune responses. Proc. Natl. Acad. Sci. U.S.A. 97: 4760-4765.

Jaju, R. J., C. Fidler, O. A. Haas, et al. (2001). A novel gene, NSD1, is fused to NUP98 in the t(5;11)(q35;p15.5) in de novo childhood acute myeloid leukemia. Blood 98: 1264-1267.

Janz, M., N. Harbeck, P. Dettmar, et al. (2002§). Y-box factor YB-1 predicts drug resistance and patient outcome in breast cancer independently of clinically relevant tumor biologic factors HER2, uPA and PAI-1. Int. J. Cancer 97: 278-282.

Jarrett, R. F. and J. MacKenzie (1999). Epstein-Barr virus and other candidate viruses in the pathogenesis of Hodgkin's disease. Sem. in Haematol. 36: 260-269.

Jensen, D. E., M. Proctor, S. T. Marquis, et al. (1998). BAP1: a novel ubiquitin hydrolase which binds to the BRCA1 RING finger and enhances BRCA1-mediated cell growth suppression. Oncogene 16: 1097-1112.

Jung, M. S., J. Yun, H. D. Chae, et al. (2001). p53 and its homologues, p63 and p73, induce a replicative senescence through inactivation of NF-Y transcription factor. Oncogene 20: 5818-5825.

Kamihira, S., K. Toriya, T. Amagasaki, et al. (1989). Antibodies against p40tax gene product of human T-lymphotropic virus type-1 (HTLV-1) under various conditions of HTLV-1 infection. Jpn. J. Cancer Res. 80: 1066-1071.

Kamiyama, J., T. Inoue, N. Ohtani-Fujita, et al. (1999). The ubiquitous transcription factor NF-Y positively regulates the transcription of human p27Kip1 through a CCAAT box located in the 5-upstream region of the p27Kip1 gene. FEBS Lett. 455: 281-285.

Kamma, H., M. Fujimoto, M. Fujiwara, et al. (2001). Interaction of hnRNP A2/B1 isoforms with telomeric ssDNA and the in vitro function. Biochem. Biophys. Res. Commun. 280: 625-630.

Kamma, H., H. Satoh, M. Matusi, et al. (2001). Characterization of hnRNP A2 and B1 using monoclonal antibodies: intracellular distribution and metabolism through cell cycle. Immmunol. Lett. 76: 49-54.

Karhumaa, P., S. Parkkila, A. Waheed, et al. (2000). Nuclear NonO/p54(nrb) protein is a nonclassical carbonic anhydrase. J. Biol. Chem. 275: 16044-16049.

Karuman, P., O. Gozani, R. D. Odze, et al. (2001). The Peutz-Jegher gene product LKB1 is a mediator of p53-dependent cell death. Mol. Cell. 7: 1307-1319.

Kiley, S. C., K. J. Clark, S. K. Duddy, et al. (1999). Increased protein kinase C delta in mammary tumor cells: relationship to transformtion and metastatic progression. Oncogene 18: 6748-6757.

King, C. A., M. B. Spellerberg, D. Zhu, et al. (1998). DNA vaccines with single-chain Fv fused to fragment C of tetanus toxin induce induce protective immunity against lymphoma and myeloma. Nature Med. 4: 1281-1286.

Kozu, T., B. Henrich and K. P. Schafer (1995). Structure and expression of the gene (HNRPA2B1) encoding the human hnRNP protein A2/B1. Genomics 25: 365-371.

Krackhardt, A. M., Witzens, M., Harig, S., Hodi, F. S., Zauls, A. J., Chessia, M., Barrett, P. and Gribben, J. G. (2002). "Identification of tumor-associated antigens in chronic lymphocytic leukemia by SEREX." Blood 100(6): 2123-31.

Krimer, D. B., G. Cheng and A. I. Skoultchi (1993). Induction of H3.3 replacement histone mRNAs during the precommitment period of murine erythroleukemia cell differentiation. Nucleic Acids Res. 21: 2873-2879.

Kurotaki, N., N. Harada, K. Yoshiura, et al. (2001). Molecular characterization of NSD1, a human homologue of the mouse Nsd1 gene. Gene 279: 197-204.

Kuroyanagi, N., H. Onogi, T. Wakabayashi, et al. (1998). Novel SR-protein-specific kinase, SRPK2, disassembles nuclear speckles. Biochem. Biophys. Res. Commun. 242: 357-364.

Kwak, L., M. Wilson, L. M. Weiss, et al. (1991). Clinical significance of morphological subdivision in diffuse large B cell lymphoma. Cancer 68: 1988-1993.

Kwak, L. W., D. D. Taub, P. L. Duffey, et al. (1995). Transfer of myeloma idiotype-specific immunity from an actively immunized marrow donor. Lancet 345: 1016-1020.

Kwak, L. W., M. J. Campbell, D. K. Czerwinski, et al. (1993). Induction of immune responses in patients with B-cell lymphoma against the surface-immunoglobulin idiotype expressed by their tumors. New Engl. J. Med. 327: 1209-1215.

LaFond, R. E., R. B. Eaton, R. A. Watt, et al. (1992). Autoantibodies to c-myc protein: elevated levels in patients with African Burkitt's lymphoma and normal Ghanians. Autoimmunity 13: 215-224.

Lee, S. H., X. Wang and J. DeJpng (2000). Functional interactions between an atypical NF-kappaB site from the rat CYP2B1 promoter and the transcriptional repressor RBP-Jkappa/CBF1. Nucleic. Acids Res. 28: 2091-2098.

Lennert, K., N. Mohri, H. Stein, et al. (1975). The histopathology of malignant lymphoma. Br. J. Haematol. 31(Suppl): 193-203.

Lifschitz-Mercer, B., Y. Sheinin, D. Ben-Meir, et al. (2001). Protein phosphatase 2Calpha expression in normal human tissues: an immunohistochemical study. Histochem. Cel Biol. 116: 31-39.

Ling, M., Y.-J. Wen and S. H. Lim (1998). Prevalence of antibodies against proteins derived from leukemia cells in patients with chronic myeloid leukemia. Blood 92: 4764-4770.

Ling, P. D., J. J.-D. Hsieh, I. K. Ruf, et al. (1994). EBNA-2 upregulation of Epstein-Barr virus latency promoters and the cellular CD23 promoter utilizes a common targeting intermediate, CBF1. J. Virol. 68: 5375-5383.

Linial, M. and O. Levius (1993). The protein VAT-1 from Torpedo electric organ exhibits an ATPase activity. Neirosci Lett. 152: 155-157.

Linial, M., K. Miller and R. H. Scheller (1989). VAT-1: an abundant membrane protein from Torpedo cholinergic synaptic vesicles. Neuron 2: 1265-1273.

Liu, J., S. Akoulitchev, A. Weber, et al. (2001). Defective interplay of activators and repressors with TFHIIH in xeroderma pigmentosum. Cell 104: 353-363.

Loewith, R., M. Meijer, S. P. Lees-Miller, et al. (2000). Three yeast proteins related to the human candidate tumor suppressor p33(ING1) are associated with histone acetyltransferase activities. Mol. Cell. Biol. 20: 3807-3816.

Lubin, R., B. Schlichtholz, J. L. Teillaud, et al. (1995). p53 antibodies in patients with various types of cancer: Assay, identification, and characterization. Clin. Can. Res. 1: 1463-1469.

Luo, J., A. Y. Nikolaev, S. Imai, et al. (2001). Negative control of p53 by SIR2alpha promotes cell survival under stress. Cell 107: 137-148.

Makino, Y., R. Cao, K. Svensson, et al. (2001). Inhibitory PAS domain protein is a negative regulator of hypoxia-inducible gene expression. Nature 414: 550-554.

Makino, Y., T. Ohga, S. Toh, et al. (1996). Structural and functional analysis of the human Y-box binding protein (YB-1) gene promoter. Nucleic Acids Res. 24: 1873-1878.

Mann, D. J., D. G. Campbell, C. H. McGown, et al. (1992). Mammalian protein serine/threonine phosphatase 2C: cDNA cloning and comparative analysis of amino acid sequences. Biochim. Biophys. Acta 1130: 100-104.

Marchand, M., N. van Baren, P. Weynants, et al. (1999). Tumor regressions observed in patients with metastatic melanoma treated with an antigenic peptide encoded by gene MAGE-3 and presented by HLA-A1. Int. J. Cancer 80: 912.

Marx, J. (2001). Science 292: 426-429.

Matsuoka, Y., X. Li and V. Bennett (2000). Adducin: structure, function and regulation. Cell. Mol. Life Sci. 57: 884-895.

Matsuoka, Y., X. Li and V. Bennett (1998). Adducin is an in vivo substrate for protein kinase C: phosphorylation in the MARCKS-related domain inhibits activity in promoting spectrin-actin complexes and occurs in many cells, including dendritic spines of neurons. J. Cell. Biol. 142: 485-497.

Mermoud, J. E., A. M. Tassin, J. R. Pehrson, et al. (2001). Centrosomal association of histone macroH2A1.2 in embryonic stem cells and somatic cells. Exp. Cell. Res. 268: 245-251.

Monni, O., H. Joensuu, K. Franssila, et al. (1996). DNA copy number changes in diffuse large B-cell lymphoma comparative genomic hybridization study. Blood 87: 5269-5278.

Mosolits, S., U. Harmenberg, U. Ruden, et al. (1999). Autoantibodies against the tumour-associated antigen GA733-2 in patients with colorectal carcinoma. Cancer Immunol. Immunother. 47: 315-320.

Mushegian, A. R., D. E. J. Bassett, M. S. Boguski, et al. (1997). Positionally cloned human disease genes: patterns of evolutionary conservation and functional motifs. Proc. Natl. Acad. Sci. U.S.A. 20: 5831-5836.

Nestle, F. O., S. Alijagic, M. Gilliet, et al. (1998). Vaccination of melanoma patients with peptide- or tumor lysate pulsed dendritic cells. Nat. Med. 4: 328-.

Nishikawa, H., Tanida, K., Ikeda, H., Sakakura, M., Miyahara, Y., Aota, T., Mukai, K., Watanabe, M., Kuribayashi, K., Old, L. J. and Shiku, H. (2001). "Role of SEREX-defined immunogenic wild-type cellular molecules in the development of tumor-specific immunity." Proc Natl Acad Sci U S A 98(25): 14571-6.

Ohga, T., Koike, K., Ono, M., Makino, Y., Itagaki, Y., Tanimoto, M., Kuwano, M. and Kohno, K. (1996). "Role of the human Y box-binding protein YB-1 in cellular sensitivity to the DNA-damaging agents cisplatin, mitomycin C, and ultraviolet light." Cancer Res 56(18): 4224-8.

Offit, K., S. C. Jhanwar, M. Ladanyi, et al. (1991). Cytogenetic analysis of 434 consecutively ascertained specimens of non-Hodgkin's lymphoma: correlations between recurrent aberations, histology and exposure of cytotoxic treatment. Genes Chrom. Cancer 3: 189-201.

Okabe, S., T. Fukuda, K. Ishibashi, et al. (1998). BAZF, a novel Bcl6 homolog, functions as a transcriptional repressor. Mol. Cell Biol. 18: 4235-4244.

Okamoto, T., H. Izumi, T. Imamura, et al. (2000). Direct interaction of p53 with the Y-box binding protein, YB-1: a mechanism for regulation of human gene expression. Oncogene 19: 6194-6202.

Old, L. J. and Chen, Y.-T. (1998). "New paths in human cancer serology." Journal of Experimental Medicine 187 (8): 1163-1167.

Olesen, C., C. Hansen, E. Bendsen, et al. (2001). Identification of human candidate genes for male infertility by digital differential display. Mol. Hum. Reprod. 7: 11-20.

Ono, T., Sato, S., Kimura, N., Tanaka, M., Shibuya, A., Old, L. J. and Nakayama, E. (2000). "Serological analysis of BALB/C methylcholanthrene sarcoma Meth A by SEREX: identification of a cancer/testis antigen." Int J Cancer 88(6): 845-51.

Pasqualucci, L., P. Neumeister, T. Goossens, et al. (2001). Hypermutation of multiple proto-oncogenes in B-cell diffuse large-cell lymphomas. Nature 412: 341-346.

Pichler, A., A. gast, J. S. Seeler, et al. (2002). The nucleoporin RanBP2 has SUMO1 E3 ligase activity. Cell 108: 109-120.

Ponting, C. P. (1997). Tudor domains in proteins that interact with RNA. Trends Biochem. Sci. 22: 51-52.

Preuss, K. D., Zwick, C., Bormann, C., Neumann, F. and Pfreundschuh, M. (2002). "Analysis of the B-cell repertoire against antigens expressed by human neoplasms." Immunol Rev 188(1): 43-50.

Project., T. N.-H. L. C. (1997). A clinical evaluation of the International Lymphoma Study Group classification of non-Hodgkin's lymphoma. Blood 89: 3909-3918.

Pulford, K., B. Falini, A. H. Banham, et al. (2000). Immune response to the ALK oncogenic tyrosine kinase in patients with anaplastic large-cell lymphoma. Blood 96: 1605-1607.

Pulford, K., H. Roberton, A. H. Banham, et al. (2001). Immunochemical studies of antigenic lymphoma-associated proteins. Brit. J. Haematol. In Press:

Pulford, K., L. Lamant, S. W. Morris, et al. (1997). Detection of anaplastic lymphoma kinase (ALK) and nucleolar protein nucleophosmin (NPM)-ALK proteins in normal and neoplastic cells with the monoclonal antibody ALK1. Blood 89: 1394-1404.

Rao, P. H., J. Houldsworth, K. Dyomina, et al. (1998). Chromosomal and gene amplification in diffuse large B-cell lymphoma. Blood 92: 234.

Rasmussen, T. P., T. Huang, M. A. Mastrangelo, et al. (1999). Messenger RNAs encoding mouse histone macroH2A1 isoforms are expressed at similar levels in male and female cells and result from alternative splicing. Nucleic Acids Res. 27: 3685-3689.

Reeves, R. (2000). Structure and function of the HMGI(Y) family of architectural transcription factors. Environ. Health Perspect. 108: 803-809.

Rifkind, R. A., Richon, V. M. and Marks, P. A. (1996). "Induced differentiation, the cell cycle, and the treatment of cancer." Pharmacol Ther 69(2): 97-102.

Robles, A. I. and C. C. Harris (2001). p53-mediated apoptosis and genomic instability diseases. Acta Oncol. 40: 696-701.

Romero, P., P. Dunbar, D. Valmori, et al. (1998). Ex vivo staining of metastatic lymph nodes by class I major histocompatibility complex tetramer labelling reveals high numbers of antigen-experienced tumour-specific cytolytic T lymphocytes. J. Exp. Med. 188: 1641-50.

Rosenberg, S. A. (1996). The immunotherapy of solid cancers based on cloning the genes encoding tumour-rejection antigens. Annu. Rev. Med. J. Natl. Can. Inst. 88: 1635-1644 : 481-91.

Rutter, J., C. H. Michnoff, S. M. Harper, et al. (2001). PAS kinase: an evolutionarily conserved PAS domain-regulated serine/threonine kinase. Proc. Natl. Acad. Sci. U.S.A. 98: 8991-8996.

Sahin, U., Ö. Türeci, H. Schmitt, et al. (1995). Human neoplasms elicit multiple specific immune responses in the autologous host. Proc. Natl. Acad. Sci. USA 92: 11810-11813.

Sahin, U., Tureci, O., Schmitt, H., Cochlovius, B., Johannes, T., Schmits, R., Stenner, F., Luo, G., Schobert, I. and Pfreundschuh, M. (1995). "Human neoplasms elicit multiple specific immune responses in the autologous host." PNAS USA 92: 11810-11813.

Saitoh, H., M. Delli Pizzi and J. Wang (2001). Perturbation of SUMOlation enzyme Ubc9 by distinct domain within nucleoporin RanBP2/Nup358. J. Biol. Chem. epub ahead of print:

Sampson, E. R., S. Y. Yeh, H. C. Chang, et al. (2001). Identification and characterization of androgen receptor associated coregulators in prostate cancer cells. J. Biol. Regul. Homeost. Agents 15: 123-129.

Sato, M., T. Tanaka, T. Maeno, et al. (2002). Inducible Expression of Endothelial PAS Domain Protein-1 by Hypoxia in Human Lung Adenocarcinoma A549 Cells. Role of src family kinases-dependent pathway. Am. J. Respir. Cell. Mol. Biol. 26: 127-134.

Satoh, H., H. Kamma, H. Ishikawa, et al. (2000). Expression of hnRNP A2/B1 proteins in human cancer cell lines. Int. J. Oncol. 16: 763-767.

Scanlan, M. J. and Jager, D. (2001). "Challenges to the development of antigen-specific breast cancer vaccines." Breast Cancer Res 3(2): 95-8.

Scanlan, M. J., Y.-T. Chen, B. Williamson, et al. (1998). Charaterization of human colon cancer antigens recognized by autologous antibodies. Int. J. Cancer 76: 652-658.

Scaturro, M., A. Cestelli, D. Castiglia, et al. (1995). Posttranscriptional regulation of H1 zero and H3.3B histone genes in differentiating rat cortical neurons. Neurochem. Res. 20: 969-976.

Schaeffer, L., R. Roy, S. Humbert, et al. (1993). DNA repair helicase: a component of BTF2 (THFIIH) basic transcription factor. Science 260: 58-63.

Schenk, P. W., A. W. Boersma, J. A. Brandsma, et al. (2001). SKY1 is involved in cisplatin-induced cell kill in Saccharomyces cerevisiae, and inactivation of its human homologue, SRPK1, induces cisplatin resistance in a human ovarian carcinoma cell line. Cancer Res. 61: 6982-6986.

Schenk, P. W., Boersma, A. W., Brandsma, J. A., den Dulk, H., Burger, H., Stoter, G., Brouwer, J. and Nooter, K. (2001). "SKY1 is involved in cisplatin-induced cell kill in Saccharomyces cerevisiae, and inactivation of its human homologue, SRPK1, induces cisplatin resistance in a human ovarian carcinoma cell line." Cancer Res 61(19): 6982-6.

Schmits, R., Cochlovius, B., Treitz, G., Regitz, E., Ketter, R., Preuss, K. D., Romeike, B. F. and Pfreundschuh, M. (2002). "Analysis of the antibody repertoire of astrocytoma patients against antigens expressed by gliomas." Int J Cancer 98(1): 73-7.

Schillace, R. V., S. F. Andrews, G. A. Liberty, et al. (2002). Identification and characterization of myeloid translocation gene 16b as a novel kinase anchoring protein in T lymphocytes. J. Immunol. 168: 1590-1599.

Schultze, J. L. (1997). Vaccination as immunotherapy for B cell lymphoma. Hematol. Oncol. 15: 129-139.

Schwab, M. and L. C. Amler (1990). Amplification of cellular oncogenes: A predictor of clinical outcome in human cancer. Genes Chrm. Cancer 1: 81-.

Schweisguth, F. and J. W. Posakony (1992). Suppressor of hairless, the drosophila homolog of the mouse recombination signal-binding protein gene, controls sensory organ cell fates. Cell 69: 1199-1212.

Selenko, P., R. Sprangers, G. Stier, et al. (2001). SMN tudor domain structure and its interaction with the Sm proteins. Nat. Struct. Biol. 8: 27-31.

Shibahara, K., K. Sugio, T. Osaki, et al. (2001). Nuclear expression of the Y-box binding protein, YB-1, as a novel marker of disease progression in non-small cell lung cancer. Clin. Cancer Res. 7: 3151-3155.

Shipp, M. A., K. N. Ross, P. Tamayo, et al. (2002). Diffuse large B-cell lymphoma outcome prediction by gene-expression profiling and supervised machine learning. Nature Med. 8: 68-74.

Siegel, D. S., X. Zhang, R. Feinman, et al. (1998). Hexamethylene bisacetamide induces programmed cell death (apoptosis) and down-regulates BCL-2 expression in human myeloma cells. Proc. Natl. Acad. Sci. U.S.A. 95: 162-166.

Skotheim, R. I., S. M. Kraggerud, S. D. Fossa, et al. (2001). Familial/bilateral and sporadic testicular germ line tumors show frequent genetic changes at loci with suggestive linkage evidence. Neoplasia 3: 196-203.

Smith, C. and V. Cerundolo (2001). Immunotherapy of melanoma. Immunology 104: 1-7.

Smith, T. M., M. K. Lee, C. I. Szabo, et al. (1996). Complete genomic sequence and analysis of 117 kb of human DNA containing the gene BRCA1. Genome Res. 6: 1029-1049.

Spitkovsky, D. D., B. Royer-Pokora, H. Delius, et al. (1992). Tissue restricted expression and chromosomal localization of the YB-1 gene encoding a 42 kD nuclear CCAAT binding protein. Nucl. Acids Res. 20: 797-803.

Staudt, L. M., A. L. Dent, A. L. Shaffer, et al. (1999). Regulation of lymphocyte cell fate decisions and lymphomagenesis by BCL-6. Int. Rev. Immunol. 18: 381-403.

Stein, H. and F. Dallenbach (1992). Diffuse large cell lymphomas of B and T cell type. Neoplastic Hematopathology. Baltimore, Williams & Wilkins. 675-714.

Stenina, O. I., K. M. Shaneyfelt and P. E. DiCorleto (2001). Thrombin induces the release of the Y-box protein dbpB from mRNA: a mechnism of trancriptional activation. Proc. Natl. Acad. Sci. U.S.A. 98: 7277-7282.

Stevenson, F. K. (1999). DNA vaccines against cancer: from genes to therapy. Ann Oncol 10: 1913-8.

Stevenson, F. K., D. Zhu and J. Rice (2001). New strategies for vaccination and immunomodulation in NHL. Ann Hematol 80, Suppl 3: B132-4.

Suhasini, M. and R. B. Pilz (1999). Transcriptional elongation of c-myb is regulated by NF-kappaB (p50/RelB). Oncogene 18: 7360-7369.

Sun, W., F. Hou, M. P. Panchenko, et al. (2001). A member of the Y-box family interacts with an upstream element in the alphal(I) collagen gene. Matrix Biol. 20: 527-541.

Syrengelas, A. D., T. T. Chen and R. Levy (1996). DNA immunization induces protective immunity against B-cell lymphoma. Nat. Med. 2: 1038-1041.

Takahashi, K., S. Shichijo, M. Noguchi, et al. (1995). Identification of MAGE-1 and MAGE-4 proteins in spermatogonia and primary spermatocytes of testis. Cancer Res. 55: 3478-3482.

Takayama, T., A. E. Morelli, P. D. Robbins, et al. (2000). Feasibility of CTL4AIg gene delivery and expression in vivo usibg retrovirally transduced myeloid dendritic cells that induce alloantigen-specific T cell anergy in vitro. Gene Ther 7: 1265-73.

Takeda, N., M. Shibuya and Y. Maru (1999). The BCR-ABL oncoprotein potentially interacts with the xeroderma pigmentosum group B protein. Proc. Natl. Acad. Sci. U.S.A 96: 203-207.

Takekawa, M., T. Maeda and H. Saito (1998). Protein phosphatase 2C-alpha inhibits the human stress-responsive p38 and JNK MAPK pathways. EMBO J. 17: 4744-4752.

Tarte, K., Z. G. Zhang, E. Legouffe, et al. (1999). Induced expresion of B7-1 on myeloma cells following retroviral gene transfer results in tumor-specific recognition by cytotoxic T cells. J Immunol 163: 514-24.

Travis, S. M., H. A. Berger and M. J. Welsh (1997). Protein phosphatase 2C dephosphorylates and inactivates cystic fibrosis transmembrane conductance regulator. Proc. Natl. Acad. Sci. U.S.A. 94: 11055-11060.

Tureci, O., Sahin, U. and Pfreundschuh, M. (1997). "Serological analysis of human tumor antigens: molecular definition and implications." Mol Med Today 3(8): 342-9.

Türeci, O., U. Sahin and M. Pfreundschuh (1997). Serological analysis of human tumor antigens: molecular definition and implications. Mol. Med. Today 3: 342-349.

Türeci, Ö., U. Sahin, C. Zwick, et al. (1998). Identification of a meiosis-specific protein as a member of the class of cancer/testis antigens. Proc. Natl. Acad. Sci. U.S.A. 95: 5211-5216.

Türeci, Ö., U. Sahin, C. Zwick, et al. (1999). Exploitation of the antibody repertoire of cancer patients for the identification of human tumor antigens. Hybridoma 18: 23-28.

van der Bruggen, P., C. Traversari, P. Chomez, et al. (1991). A gene encoding an antigen recognized by cytolytic T lymphocytes on a human melanoma. Science 254: 1643-1647.

van der Bruggen, P., Szikora, J. P., Boel, P., Wildmann, C., Somville, M., Sensi, M. and Boon, T. (1994). "Autologous cytolytic T lymphocytes recognize a MAGE-1 nonapeptide on melanomas expressing HLA-Cw*1601." Eur J Immunol 24(9): 2134-40.

van de Water, B., I. B. Tijens, A. Verbrugge, et al. (2000). Cleavage of the actin-capping protein alpha -adducin at Asp-Asp-Ser-Asp633-Ala by caspase-3 is preceded by its phosphorylation on serine 726 in cisplatin-induced apoptosis of renal epithelial cells. J. Biol. Chem. 275: 25805-25813.

Vaziri, H., S. K. Dessain, E. N. Eaton, et al. (2001). hSIR2 (SIRT1) functions as an NAD-dependent p53 deacetylase. Cell 107: 149-159.

Venkitaraman, A. R. (2001). Functions of BRCA1 and BRCA2 in the biological response to DNA damage. J. Cell Sci. 114: 3591-3598.

Vineis, P., G. Masala and A. S. Costantini (1999). Does a gene in the Xq28 region increase the risk of non-Hodgkin's lymphomas? Working group for the Epidemiology of Hematolymphopoietic Malignancies in Italy. Ann. Oncol. 10: 471-473.

Wang, H. Y., W. Lin, J. A. Dyck, et al. (1998). SRPK2: a differentially expressed SR protein-specific kinase involved in mediating the interaction and localization of pre-mRNA splicing factors in mammalian cells. J. Cell Biol. 140: 737-750.

Wang, X., S. Yeh, G. Wu, et al. (2001). Identification and characterization of a novel androgen receptor coregulator ARA267-alpha in prostate cancer cells. J. Biol. Chem. 276: 40417-40423.

Witt, O., W. Albig and D. Doenecke (1997). Transcriptional regulation of the human replacement hitsone gene H3.3B. FEBS Lett. 408: 255-260.

Witt, O., W. Albig and D. Doenecke (1998). cAMP/phorbol ester response element is involved in transcriptional regulation of the human replacement histone gene H3.3B. Biochem J. 329: 609-613.

Wu, J., M. J. Matunis, D. Kraemer, et al. (1995). Nup358, a cytoplasmically exposed nucleoporin with peptide repeats, Ran-GTP binding sites, zinc fingers, a cyclophilin A homologous domain, and a leucine-rich region. J. Biol. Chem. 270: 14209-14213.

Xia, G., Y. Kageyama, T. Hayashi, et al. (2001). Regulation of vascular endothelial growth factor transcription by endothelial PAS domain protein 1 (EPAS1) and possible involvement of EPAS1 in the angiogenesis of renal cell carcinoma. Cancer 91: 1429-1436.

Xie, X., Wacker, H.-H., Huang, S., Regitz, E., Preuss, K.-D., Romeike, B., Parwaresch, R., Tiemann, M. and Pfreundschuh, M. (2003). "Differential Expression of Cancer Testis Genes in Histological Subtypes of Non-Hodgkin's Lymphomas." Clin Cancer Res 9(1): 167-173.

Yahata, H., Kobayashi, H., Kamura, T., Amada, S., Hirakawa, T., Kohno, K., Kuwano, M. and Nakano, H. (2002). "Increased nuclear localization of transcription factor YB-1 in acquired cisplatin-resistant ovarian cancer." J Cancer Res Clin Oncol 128(11): 621-6.

Yamada, K., R. L. Printz, H. Osawa, et al. (1999). Human ZHX1: cloning, chromosomal location, and interaction with transcription factor NF-Y. Biochem. Biophys. Res. Commun. 261: 614-621.

Ylikorkala, A., D. J. Rossi, N. Korsisaari, et al. (2001). Vascular abnormalities and deregulation of VEGF in Lkb1-deficient mice. Science 293: 1323-1326.

Yuasa, K., K. Omori and N. Yanaka (2000). Binding and phosphorylation of a novel male germ cell-specific cGMP-dependent protein kinse-anchoring protein by cGMP-dependent protein kinase Ia. J. Biol. Chem. 275: 4897-4905.

Yun, J., H. D. Chae, H. E. Choy, et al. (1999). p53 negatively regulates cdc2 transcription via the CCAAT-binding NF-Y transcription factor. J. Biol. Chem. 274: 29677-29682.

Zendman, A. J., Ruiter, D. J. and Van Muijen, G. N. (2003). "Cancer/testis-associated genes: Identification, expression profile, and putative function." J Cell Physiol 194(3): 272-88.

Zhang, A., K. Ohshima, K. Sato, et al. (1999). Prognostic clinicopathologic factors, including immunologic expression in diffuse large B-cell lymphomas. Pathol. Int. 49: 1043-1052.

Zhang, W., J. Mi, N. Li, et al. (2001). Identification and characterisation of DPZF, a novel human BTB/POZ zinc finger protein sharing homology to BCL-6. Biochem. Biophys. Res. Commun. 282: 1067-1073.

Zhou, J., D. C. Allred, I. Avis, et al. (2001). Differential expression of the early lung cancer detection marker, heterogeneous nuclear ribonucleoprotein-A2/B1 (hnRNP-A2/B1) in normal breast and neoplastic breast cancer. Breast Cancer Res. Treat. 66: 217-224.

Zhou, J., L. Nong, M. Wloch, et al. (2001). Expression of early lung cancer detection marker: hnRNP-A2/B1 and its relation to microsatellite alteration in non-small cell lung cancer. Lung Cancer 34: 341-350.

| Antigen | Frequency of Identification (/94) | Identity/Unigene name | Chromosomal localisation | Recurrent chromosomal aberrations in DLBCL (CGAP) | Previously-identified SEREX antigen? (Y/N) | mRNA expression data from LLMPP Lymphochip |
|---|---|---|---|---|---|---|
| OX-TES-1 | 1 | Novel PAS domain protein | X | specific locus unknown | N | No data |
| OX-TES-2 | 1 | Novel protein | 2 | Specific locus unknown | N | No data |
| OX-TES-3 | 1 | PPM1A | 14q22.1-q22.3 | Infrequent | N | No data |
| OX-TES-4 | 3 | FLJ11565 | Xq22.3-23 | Infrequent | Y (breast cancer) | No data |
| OX-TES-5 | 1 | H3F3B | 17q25 | Recurrent | N | No data |
| OX-TES-6 | 1 | RANBP2/ RANBP2L1 | 2q11-q13 | None | Y (testis; glioma and breast cancer; giant cell arteritis) | No differential expression in DLBCL |
| OX-TES-7 | 1 | KIAA0352 | 12q13.2 | infrequent | N | No data |
| OX-TES-8 | 1 | ZHX1 | 8p22 | Recurrent | Y (stomach cancer) | No data |
| OX-TES-9 | 1 | VAT1 | 17q21 | Infrequent | N | No data |
| OX-TES-10 | 4 | SRPK1 | 6p21.3-p21.2 | Recurrent | Y (breast cancer) | Some differential expression |
| OX-TES-11 | 2 | RNF20 | 9q22 | Recurrent | Y (stomach and breast cancers) | No data |
| OX-TES-12 | 8 | HNRPA2B1 | 7p15 | Recurrent | Y (testis only) | No data |
| OX-TES-13 | 5 | STK11 | 19p13.3 | Recurrent | Y (renal cancer) | Some differential expression in DLBCL |
| OX-TES-14 | 3 | HIS1 | 17q21.32 | Recurrent | Y (testis; ovarian cancer) | No data |
| OX-TES-15 | 1 | MAPK6 | 15q21 | Recurrent | N | No differential expression in DLBCL |
| OX-TES-16 | 1 | RBPSUH | 4 | Specific locus unknown | Y (breast, renal, stomach & lung cancers; melanoma | Differential expression |
| OX-TES-17 | 1 | H2AFY | 5q31.3-q32 | Infrequent | Y (Systemic lupus erythematosus) | No data |

(continued)

| Antigen | Frequency of Identification (/94) | Identity/Unigene name | Chromosomal localisation | Recurrent chromosomal aberrations in DLBCL (CGAP) | Previously-identified SEREX antigen? (Y/N) | mRNA expression data from LLMPP Lymphochip |
|---|---|---|---|---|---|---|
| OX-TES-18 | 14 | KIAA0643 | 16 | Specific locus unknown | Y (ovarian and colon cancers) | No data |
| OX-TES-19 | 1 | ERCC3 | 2q21 | Infrequent | N | No differential expression |
| OX-TES-20 | 7 | GKAP42 | 9 | Specific locus unknown | N | No data |
| OX-TES-21 | 3 | SIRT1 | 10q21.3 | None | N | No data |
| OX-TES-22 | 2 | PSP1 | 13q11 | None | N | No data |
| OX-TES-23 | 3 | NSEP1 | 1p34 | Infrequent | Y (testis; ovarian cancer; giant cell arteritis) | No data |
| OX-TES-24 | 9 | C20orf104 | 20q11.1-q11.23 | Infrequent | Y (testis; renal and ovarian cancers; glioma; hepatocellular carcinoma) | No data |
| OX-TES-25 | 7 | FLJ13942 | 6 | Specific locus unknown | Y (stomach cancer) | No data |
| OX-TES-26 | 2 | ADD1 | 4p16.3 | Infrequent | N | No data |
| OX-TES-27 | 1 | NSD1 | 5q35 | Infrequent | N | No data |
| OX-TES-28 | 1 | BAP1 | 3p21.31-p21.2 | Infrequent | N | No data |

| Primer sets | Primer sequence 5'-3' | Annealing temperature (˚C) | Extension time (mins) | Cycle number |
|---|---|---|---|---|
| OX-TES-1A | F:TACAGGAGCGGAAGAAGTGG<br>R:ACAGGAACAATGGGTTGGG | 60 | 2 1/2 | 30 |
| OX-TES-1B | F:TCTCATCAATAGCAACTTGCTC<br>R:TCACACTCACTTCCCTCTTAC | 55 | 2 1/2 | 30 |
| OX-TES-1C | F:TCCAGAGAGCAGGCTGAACAA<br>R:AAGCCGGATGTAATCCTGTG | 60 | 5 | 35 |
| OX-TES-3 | F:GGTAATGGGTTGCGATATGG<br>R:GCGGATTACTTGGTTTGTGA | 55 | 2 1/2 | 30 |
| OX-TES-4 | F:ACACCACCATACCTTTTCCC<br>R:TTCTTTCCAGCTCAGCTTTC | 55 | 2 1/2 | 30 |
| OX-TES-5 | F:AGGATTTCAAAACCGACCTG<br>R:CAACTGGATGTCTTTGGGC | 55 | 2 1/2 | 30 |
| OX-TES-6 | F:ACATACCACTTCAAACTCCCC<br>R:ATCTTTATATCACCAATGCCCC | 55 | 2 1/2 | 30 |
| OX-TES-8 | F:ACCAGGAAGAGGATGAAGAAG<br>R:ACGTTTTAGGCAGATTTCAGTC | 55 | 2 1/2 | 30 |
| OX-TES-9 | F:TCCCAATCTCGTCCTCTGTC<br>R:ACCCTCCCTAATCCTAGCTC | 55 | 2 1/2 | 30 |
| OX-TES-10 | F: AAGAGCAAGAACATAACGGAC<br>R: CGACCACTCATACTTCTCCAC | 55 | 2 1/2 | 30 |
| OX-TES-11 | F:GAATAAACGCAAGGCAATGG<br>R: CAAGGAGAGAAGAGGTAGAGG | 55 | 2 1/2 | 30 |
| OX-TES-12 | F:GGCTTTGGCTTTGTTACTTTTG<br>R:TATCCTCCTCTTCCTCCTCC | 55 | 2 1/2 | 30 |
| OX-TES-13 | F:GGAAATTCAACTACTGAGGAGG<br>R:AGGTCGGAGATTTTGAGGG | 55 | 2 1/2 | 30 |
| OX-TES-14 | F:GCTGGGGAAGAAAAAACATAGG<br>R:GATCATCCATGAGGAACTGC | 55 | 2 1/2 | 30 |
| OX-TES-15 | F:ATGGTTTGTTGCCTACCTAGC<br>R:ATTCATGCTTGCCTTGGGATG | 55 | 2 1/2 | 30 |
| OX-TES-19 | F:CACAACCCCAAAATTAACACC<br>R:ACAGAAATGACCCCACTCC | 55 | 2 1/2 | 30 |
| OX-TES-20 | F:TTAGTTCTGTTCCCACCACC<br>R:GCTCATCTCTTTGTCTCCACTC | 60 | 2 1/2 | 30 |
| OX-TES-21 | F:AAACTACTTCGCAACTATACCC<br>R:TCACCACCTAACCTATGACAC | 55 | 2 1/2 | 30 |
| OX-TES-22 | F:AAATCTGCCCACCGACATC<br>R:CCCCATCACCACATCTTTCC | 55 | 2 1/2 | 30 |
| OX-TES-24 | F:AGAAAAGCGACCCAAGCAG<br>R:TGAATCCACAGCAGTAGGAG | 55 | 2 1/2 | 30 |
| OX-TES-26 | F:ACAAAGCCAAGTCCCGTTC<br>R:ATCACTACACCACACAAAACC | 55 | 2 1/2 | 30 |
| OX-TES-28 | F:ATCAGACCAATCCAAGGCAG | 60 | 2 1/2 | 30 |
| β-actin | F:ATCTGGCACCACACCTTCTACAA<br>TGAGCTGCG | 60 | 2 1/2 | 30 |

(continued)

| Primer sets | Primer sequence 5'-3' | Annealing temperature (˚C) | Extension time (mins) | Cycle number |
|---|---|---|---|---|
| | R:CGTCATACTCCTGCTTGCTGATC CACATCTGC | | | |

| Antigen | Immunoreactivity | |
|---|---|---|
| | DLBCL (n=10) | Healthy (n=10) |
| OX-TES-1 | 3 | 1 |
| OX-TES-2 | 1 | 0 |
| OX-TES-3 | 4 | 0 |
| OX-TES-4 | 5 | 0 |
| OX-TES-5 | 4 | 0 |
| OX-TES-6 | 5 | 0 |
| OX-TES-7 | 1 | 0 |
| OX-TES-8 | 1 | 0 |
| OX-TES-9 | 1 | 0 |
| OX-TES-10 | 4 | 1 |
| OX-TES-11 | 3 | 1 |
| OX-TES-12 | 4 | 3 |
| OX-TES-13 | 5 | 3 |
| OX-TES-14 | 5 | 4 |
| OX-TES-15 | 3 | 1 |
| OX-TES-16 | 5 | 4 |
| OX-TES-17 | 1 | 1 |
| OX-TES-18 | 5 | 6 |
| OX-TES-19 | 2 | 2 |
| OX-TES-20 | 7 | 4 |
| OX-TES-21 | 3 | 1 |
| OX-TES-22 | 3 | 1 |
| OX-TES-23 | 7 | 6 |
| OX-TES-24 | 7 | 5 |
| OX-TES-25 | 6 | 6 |
| OX-TES-26 | 4 | 3 |
| OX-TES-27 | 3 | 2 |
| OX-TES-28 | 2 | 2 |

| Antigen | Immunoreactivity | | |
|---|---|---|---|
| | AML(n=10) | CML(n=10) | Healthy (n=10) |
| OX-TES-1 | 0 | 0 | 0 |
| OX-TES-3 | 1 | 0 | 0 |
| OX-TES-4 | 0 | 0 | 0 |
| OX-TES-5 | 1 | 0 | 1 |
| OX-TES-6 | 4 | 2 | 0 |
| OX-TES-10 | 4 | 0 | 0 |
| OX-TES-11 | 1 | 0 | 0 |
| OX-TES-12 | 0 | 0 | 0 |
| OX-TES-13 | 1 | 2 | 1 |

(continued)

| Antigen | Immunoreactivity | | |
|---|---|---|---|
| | AML(n=10) | CML(n=10) | Healthy (n=10) |
| OX-TES-15 | 0 | 0 | 0 |
| OX-TES-20 | 1 | 0 | 0 |
| OX-TES-21 | 3 | 0 | 0 |
| OX-TES-22 | 0 | 1 | 0 |
| OX-TES-24 | 3 | 1 | 4 |
| OX-TES-28 | 0 | 0 | 0 |

## Claims

### Claims for the following Contracting State(s): AT, BE, CH, DK, ES, GR, IT, LI, LU, MC, NL, SE

**1.** A nucleic acid molecule encoding a tumour-associated antigen having the amino acid sequence illustrated in Figure 2.

**2.** A molecule according to claim 1 which is a DNA or RNA molecule.

**3.** A molecule according to claim 1 or 2 which is a cDNA molecule.

**4.** A molecule according to any of claims 1 to 3, wherein said nucleic acid molecule encoding said tumour-associated antigen comprises the sequence in Figure 1.

**5.** A nucleic acid molecule capable of hybridising to a molecule according to any of claims 1 to 4 under conditions of high stringency.

**6.** A nucleic acid molecule according to any of claims 1 to 5, wherein said tumour antigen is one associated with lymphomas.

**7.** A nucleic acid molecule according to claim 6, wherein said lymphoma is a B cell lymphoma.

**8.** A nucleic acid molecule according to any of claims 1 to 5, wherein said tumour is a solid tumour.

**9.** A nucleic acid molecule according to claim 8 wherein said solid tumour is any of, kidney, breast, uterus, cervix, colon, lung, stomach, rectum or small intestine.

**10.** A tumour associated antigen encoded by a nucleic acid molecule according to any of claims 1 to 9.

**11.** A tumour-associated antigen encoded by a nucleic acid molecule having a nucleotide sequence illustrated in Figure 1.

**12.** A tumour associated antigen having the amino acid sequence according to Figure 2.

**13.** An expression vector comprising a nucleic acid molecule according to any of claims 1 to 9.

**14.** A host cell or organism transformed or transfected with an expression vector according to claim 13.

**15.** A transgenic non-human organism comprising a transgene capable of expressing a tumour antigen according to any of claims 10 to 12.

**16.** An antibody or fragment thereof capable of binding to a tumour-associated antigen according to any of claims 10 to 12.

**17.** An antibody according to claim 16 which is any of a polyclonal or monoclonal antibody.

**18.** An antibody according to claim 16 or 17 which is a humanised antibody.

**19.** An antibody according to any of claims 16 to 18 which is an autoantibody.

**20.** An antibody according to any of claims 16 to 19 further comprising a toxic or reporter molecule.

**21.** An antibody according to claim 20 wherein said reporter molecule is a radioisotope.

**22.** An *in vitro* method of identifying alignant cells or tumours in a mammal which method comprises identifying in a sample of bodily fluid from said mammal antibodies from said mammal capable of forming complexes with a tumour-associated antigen according to any of claims 10 to 12 or a tumour-associated antigen having the amino acid sequence illustrated in Figure 5, or an epitope thereof.

**23.** A method according to claim 22 wherein said mammal is a human and the antibodies are human autoantibodies.

**24.** A method according to claim 22 or 23 wherein said malignancy is any of a solid tumour, a leukaemia or a lymphoma.

**25.** A method according to claim 24 wherein said solid tumour is from any of kidney, breast, uterus, cervix, colon, lung, stomach, rectum or small intestine.

**26.** A method according to claim 24 wherein said lymphoma is a B cell lymphoma such as any of diffuse large B-cell lymphoma (DLBCL), follicular lymphoma, Burkitt's lymphoma, but preferably DLBCL.

**27.** A method according to any of claims 22 to 26 wherein said bodily fluid comprises human serum or plasma.

**28.** An *in vitro* method of detecting lymphoma in a patient which method comprises identifying autoantibodies from a bodily fluid of said patient capable of forming complexes with the antigen having an amino acid sequence as set forth in Figure 5, or an epitope thereof.

**29.** A method according to claim 28 wherein said lymphoma is a B cell lymphoma including any of diffuse large B-cell lymphoma, follicular lymphoma or Burkitt's lymphoma.

**30.** A method according to claim 28 or 29, wherein said bodily fluid comprises human serum or plasma.

**31.** An antibody according to any of claims 16 to 21 or an antibody capable of binding to or forming complexes with the tumour-associated antigen having an amino acid sequence as set forth in Figure 5 or an epitope thereof for use in treating malignant cells or tumours.

**32.** An antibody according to claim 31 wherein said tumour is any of a solid tumour, a leukaemia or a lymphoma.

**33.** An antibody according to claim 32 wherein solid tumour is selected from any of kidney, breast, uterus, cervix, colon, lung, stomach, rectum or small intestine.

**34.** An antibody according to claim 32 wherein said lymphoma is a B cell lymphoma such as any of diffuse large B-cell lymphoma (DLBCL), follicular lymphoma, Burkitt's lymphoma, but preferably DLBCL.

**35.** Use of an antibody according to any of claims 16 to 21 or an antibody capable of binding to or forming complexes with a tumour-associated antigen having an amino acid sequence as set forth in Figure 5 or an epitope thereof in the manufacture of a medicament for treating malignant cells or tumours.

**36.** A nucleic acid molecule according to claim 5 for use in treating malignant cells or tumours.

**37.** The nucleic acid molecule according to claim 36 wherein said nucleic acid molecule is an antisense molecule.

**38.** The nucleic acid molecule according to claim 36 wherein said nucleic acid molecule is double stranded RNA.

**39.** Use of a nucleic acid molecule according to claim 5 in the manufacture of a medicament for treating malignant cells or tumours.

**40.** A nucleic acid molecule capable of hybridising to a nucleic acid molecule encoding an antigen having an amino acid sequence illustrated in Figure 5, under conditions of high stringency for use in treating lymphoma.

**41.** Use of a nucleic acid molecule capable of hybridising to the nucleic acid molecule encoding an antigen having the amino acid sequence illustrated in Figure 5 under conditions of high stringency in the manufacture of a medicament for treating lymphoma.

**42.** An assay kit for detecting malignant cells or tumours in a patient said kit comprising one or more tumour-associated antigens according to any of claims 10 to 12, and means for contacting said antigen with a sample of bodily fluid from said patient.

**43.** An assay kit for detecting lymphomas in a patient said kit comprising an antigen having an amino acid sequence as illustrated in Figure 5, and means for contacting said antigen with a sample of bodily fluid from a patient.

**44.** use of the method according to any of claims 22 to 27 in determining a malignant Cell/tumour-associated antigen profile of an individual suffering from a malignancy or tumour.

**45.** Use of the method according to any of claims 28 to 30 in determining a lymphoma-associated antigen profile of an individual suffering from a lymphoma.

**46.** A malignancy or tumour diagnostic reagent comprising mammalian autoantibodies having a specificity for at least one of the tumour-associated antigens according to any of claims 10 to 12.

**47.** Use of a method according to any of claims 22 to 28 to screen for minimal residual disease, recurrence of malignancy or tumour after treatment, or to monitor the progress of the treatment of an individual for a malignancy or tumour.

**48.** Use according to claim 47 wherein said malignancy or tumour is a lymphoma, leukaemia or a solid tumour.

**49.** Use of a method according to any of claims 28 to 30 to screen for recurrence of a lymphoma after treatment, or to monitor the progress of the treatment of an individual for said lymphoma.

**50.** An isolated immortalised cell population capable of producing antibodies against an epitope of tumour-associated antigens in an individual according to any of claims 10 to 12.

**51.** An isolated immortalised cell population capable of producing antibodies against an epitope of lymphoma-associated antigens in an individual according to any of claims 10 to 12, or an antigen having an amino acid sequence as illustrated in Figure 5.

**52.** An isolated immortalised cell population according to claim 50 or 51 wherein said antibodies are directed against an epitope of DLBCL-associated tumour antigens.

**53.** A vaccine composition comprising an effective amount of a polypeptide according to any of claims 10 to 12, or an isolated antigen having an amino acid sequence as illustrated in Figure 5, or an epitope or immunogenic fragment thereof, together with a pharmaceutically acceptable carrier.

**54.** A vaccine composition comprising an effective amount of a nucleic acid molecule according to any of claims 1 to 9, or a nucleotide sequence encoding an antigen having an amino acid sequence as illustrated in Figure 5, or an epitope or immunogenic fragment thereof, together with a pharmaceutically acceptable carrier.

**55.** A vaccine composition comprising an effective amount of antigen presenting cells modified to express any of the antigens according to any of claims 10 to 12 or an antigen having an amino acid sequence as illustrated in Figure 5 or an epitope or immunogenic fragment thereof, together with a pharmaceutically acceptable carrier.

**56.** A vaccine composition according to claim 54 or 55 which further comprises an appropriate adjuvant.

**57.** A nucleic acid molecule according to any of claims 1 to 9, or encoding an antigen having an amino acid sequence as illustrated in Figure 5 for use in treating a human or animal body.

**58.** A tumour or lymphoma associated antigen according to any of claims 10 to 12, or an antigen having an amino acid sequence as illustrated in Figure 5, or an epitope thereof for use in the treatment of a human or animal body.

**59.** An antibody according to any of claims 16 to 21, or an antibody specific for an antigen having an amino acid sequence as illustrated in Figure 5 or an epitope thereof, for use in treatment of a human or animal body.

**60.** An *in vitro* method of monitoring CML responses in a patient to an antigen having an amino acid sequence as illustrated in Figure 5, which comprises contacting a bodily fluid from said patient with a tetramer complex of an antigenic fragment of said antigen and MHC Class I molecules and monitoring the level of CTL responses for said antigens.

**61.** An *in vitro* method of purifying CTL cells from a patient, which comprises contacting a bodily fluid from said patient with a tetramer complex of an antigenic fragment of an antigen having an amino acid sequence as illustrated in Figure 5 and MHC Class I molecules and removing any bound cells that recognise the antigenic peptide (s).

**Claims for the following Contracting State(s): GB, DE, FR**

**1.** A nucleic acid molecule encoding a tumour-associated antigen having the amino acid sequence illustrated in Figure 2.

**2.** A molecule according to claim 1 which is a DNA or RNA molecule.

**3.** A molecule according to claim 1 or 2 which is a cDNA molecule.

**4.** A molecule according to any of claims 1 to 3, wherein said nucleic acid molecule encoding said tumour-associated antigen comprises the sequence in Figure 1.

**5.** A nucleic acid molecule according to any of claims 1 to 4, wherein said tumour antigen is one associated with lymphomas.

**6.** A nucleic acid molecule according to claim 5, wherein said lymphoma is a B cell lymphoma.

**7.** A nucleic acid molecule according to any of claims 1 to 4, wherein said tumour is a solid tumour.

**8.** A nucleic acid molecule according to claim 7 wherein said solid tumour is any of, kidney, breast, uterus, cervix, colon, lung, stomach, rectum or small intestine.

**9.** A tumour associated antigen encoded by a nucleic acid molecule according to any of claims 1 to 8.

**10.** A tumour-associated antigen encoded by a nucleic acid molecule having a nucleotide sequence illustrated in Figure 1.

**11.** A tumour associated antigen having the amino acid sequence according to Figure 2.

**12.** An expression vector comprising a nucleic acid molecule according to any of claims 1 to 8.

**13.** A host cell or organism transformed or transfected with an expression vector according to claim 12.

**14.** A transgenic non-human organism comprising a transgene capable of expressing a tumour antigen according to any of claims 9 to 11.

**15.** An antibody or fragment thereof capable of binding to a tumour-associated antigen according to any of claims 9 to 11.

**16.** An antibody according to claim 15 which is any of a polyclonal or monoclonal antibody.

**17.** An antibody according to claim 15 or 16 which is a humanised antibody.

**18.** An antibody according to any of claims 15 to 17 which is an autoantibody.

**19.** An antibody according to any of claims 15 to 18 further comprising a toxic or reporter molecule.

**20.** An antibody according to claim 19 wherein said reporter molecule is a radioisotope.

**21.** An *in vitro* method of identifying malignant cells or tumours in a mammal which method comprises identifying in a sample of bodily fluid from said mammal antibodies from said mammal capable of forming complexes with a tumour-associated antigen according to any of claims 9 to 11 or a tumour-associated antigen having the amino acid sequence illustrated in Figure 5, or an epitope thereof.

**22.** A method according to claim 21 wherein said mammal is a human and the antibodies are human autoantibodies.

**23.** A method according to claim 21 or 22 wherein said malignancy is any of a solid tumour, a leukaemia or a lymphoma.

**24.** A method according to claim 23 wherein said solid tumour is from any of kidney, breast, uterus, cervix, colon, lung, stomach, rectum or small intestine.

**25.** A method according to claim 23 wherein said lymphoma is a B cell lymphoma such as any of diffuse large B-cell lymphoma (DLBCL), follicular lymphoma, Burkitt's lymphoma, but preferably DLBCL.

**26.** A method according to any of claims 21 to 25 wherein said bodily fluid comprises human serum or plasma.

**27.** An *in vitro* method of detecting lymphoma in a patient which method comprises identifying autoantibodies from a bodily fluid of said patient capable of forming complexes with the antigen having an amino acid sequence as set forth in Figure 5, or an epitope thereof.

**28.** A method according to claim 27 wherein said lymphoma is a B cell lymphoma including any of diffuse large B-cell lymphoma, follicular lymphoma or Burkitt's lymphoma.

**29.** A method according to claim 27 or 28, wherein said bodily fluid comprises human serum or plasma.

**30.** An antibody according to any of claims 15 to 20 or an antibody capable of binding to or forming complexes with the tumour-associated antigen having an amino acid sequence as set forth in Figure 5 or an epitope thereof for use in treating malignant cells or tumours.

**31.** An antibody according to claim 30 wherein said tumour is any of a solid tumour, a leukaemia or a lymphoma.

**32.** An antibody according to claim 31 wherein solid tumour is selected from any of kidney, breast, uterus, cervix, colon, lung, stomach, rectum or small intestine.

**33.** An antibody according to claim 341 wherein said lymphoma is a B cell lymphoma such as any of diffuse large B-cell lymphoma (DLBCL), follicular lymphoma, Burkitt's lymphoma, but preferably DLBCL.

**34.** Use of an antibody according to any of claims 15 to 20 or an antibody capable of binding to or forming complexes with a tumour-associated antigen having an amino acid sequence as set forth in Figure 5 or an epitope thereof in the manufacture of a medicament for treating malignant cells or tumours.

**35.** A nucleic acid molecule capable of hybridising to a molecule according to any of claims 1 to 4 for use in treating malignant cells or tumours.

**36.** The nucleic acid molecule according to claim 35 wherein said nucleic acid molecule is an antisense molecule.

**37.** The nucleic acid molecule according to claim 35 wherein said nucleic acid molecule is double stranded RNA.

**38.** Use of a nucleic acid molecule capable of hybridising to a molecule according to any of claims 1 to 4 in the manufacture of a medicament for treating malignant cells or tumours.

**39.** A nucleic acid molecule capable of hybridising to a nucleic acid molecule encoding an antigen having an amino acid sequence illustrated in Figure 5, under conditions of high stringency for use in treating lymphoma.

**40.** Use of a nucleic acid molecule capable of hybridising to the nucleic acid molecule encoding an antigen having the amino acid sequence illustrated in Figure 5 under conditions of high stringency in the manufacture of a medicament for treating lymphoma.

**41.** An assay kit for detecting malignant cells or tumours in a patient said kit comprising one or more tumour-associated antigens according to any of claims 9 to 11, and means for contacting said antigen with a sample of bodily fluid from said patient.

**42.** An assay kit for detecting lymphomas in a patient said kit comprising an antigen having an amino acid sequence as illustrated in Figure 5, and means for contacting said antigen with a sample of bodily fluid from a patient.

**43.** Use of the method according to any of claims 21 to 26 in determining a malignant cell/tumour-associated antigen profile of an individual suffering from a malignancy or tumour.

**44.** Use of the method according to any of claims 27 to 29 in determining a lymphoma-associated antigen profile of an individual suffering from a lymphoma.

**45.** A malignancy or tumour diagnostic reagent comprising mammalian autoantibodies having a specificity for at least one of the tumour-associated antigens according to any of claims 9 to 11.

**46.** Use of a method according to any of claims 21 to 27 to screen for minimal residual disease, recurrence of malignancy or tumour after treatment, or to monitor the progress of the treatment of an individual for a malignancy or tumour.

**47.** Use according to claim 46 wherein said malignancy or tumour is a lymphoma, leukaemia or a solid tumour.

**48.** Use of a method according to any of claims 27 to 29 to screen for recurrence of a lymphoma after treatment, or to monitor the progress of the treatment of an individual for said lymphoma.

**49.** An isolated immortalised cell population capable of producing antibodies against an epitope of tumour-associated antigens in an individual according to any of claims 9 to 11.

**50.** An isolated immortalised cell population capable of producing antibodies against an epitope of lymphoma-associated antigens in an individual according to any of claims 9 to 11, or an antigen having an amino acid sequence as illustrated in Figure 5.

**51.** An isolated immortalised cell population according to claim 49 or 50 wherein said antibodies are directed against an epitope of DLBCL-associated tumour antigens.

**52.** A vaccine composition comprising an effective amount of a polypeptide according to any of claims 9 to 11, or an isolated antigen having an amino acid sequence as illustrated in Figure 5, or an epitope or immunogenic fragment thereof, together with a pharmaceutically acceptable carrier.

**53.** A vaccine composition comprising an effective amount of a nucleic acid molecule according to any of claims 1 to 8, or a nucleotide sequence encoding an antigen having an amino acid sequence as illustrated in Figure 5, or an epitope or immunogenic fragment thereof, together with a pharmaceutically acceptable carrier.

**54.** A vaccine composition comprising an effective amount of antigen presenting cells modified to express any of the antigens according to any of claims 9 to 11 or an antigen having an amino acid sequence as illustrated in Figure 5 or an epitope or immunogenic fragment thereof, together with a pharmaceutically acceptable carrier.

**55.** A vaccine composition according to claim 53 or 54 which further comprises an appropriate adjuvant.

**56.** A nucleic acid molecule according to any of claims 1 to 8, or encoding an antigen having an amino acid sequence as illustrated in Figure 5 for use in treating a human or animal body.

**57.** A tumour or lymphoma associated antigen according to any of claims 9 to 11, or an antigen having an amino acid sequence as illustrated in Figure 5, or an epitope thereof for use in the treatment of a human or animal body.

**58.** An antibody according to any of claims 15 to 20, or an antibody specific for an antigen having an amino acid sequence as illustrated in Figure 5 or an epitope thereof, for use in treatment of a human or animal body.

**59.** An *in vitro* method of monitoring CTL responses in a patient to an antigen having an amino acid sequence as illustrated in Figure 5, which comprises contacting a bodily fluid from said patient with a tetramer complex of an antigenic fragment of said antigen and MHC Class I molecules and monitoring the level of CTL responses for said antigens.

**60.** An *in vitro* method of purifying CTL cells from a patient, which comprises contacting a bodily fluid from said patient with a tetramer complex of an antigenic fragment of an antigen having an amino acid sequence as illustrated in Figure 5 and MHC Class I molecules and removing any bound cells that recognise the antigenic peptide (s).

## Patentansprüche

### Patentansprüche für folgende(n) Vertragsstaat(en): AT, BE, CH, DK, ES, GR, IT, LI, LU, MC, NL, SE

**1.** Nukleinsäuremolekül, das ein Tumor-assozüertes Antigen mit der Aminosäuresequenz, dargestellt in Figur 2 kodiert.

**2.** Molekül nach Anspruch 1, das ein DNA- oder RNA-Molekül ist.

**3.** Molekül nach Anspruch 1 oder 2, das ein cDNA-Molekül ist.

**4.** Molekül nach einem der Ansprüche 1 bis 3, wobei das Nukleinsäuremolekül, das das Tumor-assoziierte Antigen kodiert, die Sequenz in Figur 1 umfasst.

**5.** Nukleinsäuremolekül, das fähig ist, mit einem Molekül nach einem der Ansprüche 1 bis 4 unter Bedingungen hoher Stringenz zu hybridisieren.

**6.** Nukleinsäuremolekül nach einem der Ansprüche 1 bis 5, wobei das Tumorantigen eines ist, das mit Lymphomen assoziiert ist..

**7.** Nukleinsäuremolekül nach Anspruch 6, wobei das Lymphom ein B-Zell-Lymphom ist.

**8.** Nukleinsäuremolekül nach einem der Ansprüche 1 bis 5, wobei der Tumor ein fester Tumor ist.

**9.** Nukleinsäuremolekül nach Anspruch 8, wobei der feste Tumor ein beliebiger von Niere, Brust, Gebärmutter, Gebärmutterhals, Kolon, Lunge, Magen, Rektum oder Düundarm ist

**10.** Tumor-assoziiertes Antigen, kodiert durch ein Nukleinsäuremolekül nach einem der Ansprüche 1 bis 9.

**11.** Tumor-assoziiertes Antigen, kodiert durch ein Nukleinsäuremolekül mit einer Nukleotidsequenz dargestellt in Figur 1.

**12.** Tumor-assoziiertes Antigen mit der Aminosäuresequenz nach Figur 2.

**13.** Expressionvektor, umfassend ein Nukleinsäuremolekül nach einem der Ansprüche 1 bis 9.

**14.** Wirtszelle oder Organismus, transformiert oder transfiziert mit einem Expressionvektor nach Anspruch 13.

**15.** Transgener nicht-humaner Organismus, umfassend ein Transgen, das fähig ist, ein Tumorantigen nach einem der Ansprüche 10 bis 12 zu exprimieren.

**16.** Antikörper oder Fragment davon, der fähig ist, an ein Tumor-assoziiertes Antigen nach einem der Ansprüche 10 bis 12 zu binden.

**17.** Antikörper nach Anspruch 16, der ein beliebiger von polyklonaler oder monoklonaler Antikörper ist.

**18.** Antikörper nach Anspruch 16 oder 17, der ein humanisierter Antikörper ist.

**19.** Antikörper nach einem Ansprüche 16 bis 18, der ein Autoantikörper ist.

**20.** Antikörper nach einem Ansprüche 16 bis 19, weiterhin umfassend ein toxisches oder ein Reporter-Molekül.

**21.** Antikörper nach Anspruch 20, wobei das Reporter-Molekül ein Radiosotop ist.

**22.** *In vitro* Verfahren zur Identifikation maligner Zellen oder Tumore in einem Säugetier, wobei das Verfahren Identifizieren von Antikörpern aus dem Säugetier in einer Probe aus Körperglüssigkeit aus dem Säugetier, die fähig sind Komplexe mit einem Tumor-assoziierten Antigen nach einem der Ansprüche 10 bis 12 oder einem Tumor-assoziierten Antigen mit der Aminosäuresequenz, dargestellt in Figur 5 oder einem Epitop davon zu bilden, umfasst.

**23.** Verfahren nach Anspruch 22, wobei das Säugetier ein Mensch ist und die Antikörper humane Autoantikörpern sind.

**24.** Verfahren nach Anspruch 22 oder 23, wobei das Malignom ein beliebiges von einem festen Tumor, einer Leukämie oder einem Lymphom ist.

**25.** Verfahren nach Anspruch 24, wobei der feste Tumor ein beliebiges von niere, Brust, Gebärmutter, Gebärmutterhals, Kolon, Lunge, Magen, Rektum oder Dünndarm ist.

**26.** Verfahren nach Anspruch 24, wobei das Lymphom ein B-Zell-Lymphom wie zum Beispiel ein beliebiges von diffus großzelliges B-Zell-Lymphom (DLBCL), follikuläres Lymphom, Burkitt-Lymphom, aber bevorzugt DLBCL ist.

**27.** Verfahren nach einem der Ansprüche 22 bis 26, wobei die Körperflüssigkeit humanes Serum oder Plasma umfasst.

**28.** *In vitro* Verfahren zur Detektion von Lymphom in einem Patienten, wobei das Verfahren Identifizieren von Autoantikörpern aus einer Körperflüssigkeit des Patienten, die fähig sind, Komplexe mit dem Antigen mit der Aminosäuresequenz, die in Figur 5 dargestellt ist oder einem Epitop davon zu bilden, umfasst.

**29.** Verfahren nach Anspruch 28, wobei das Lymphom ein B-Zell-Lymphom einschließlich ein bellebiges von diffus großzelliges B-Zell-Lymphom, follikuläres Lymphom oder Burkitt-Lymphom ist.

**30.** Verfahren nach Anspruch 28 oder 29, wobei die Körperflüssigkeit humanes Serum oder Plasma umfasst.

**31.** Antikörper nach Ansprüche 16 bis 21 oder ein Antikörper, der fähig ist, an das Tumor-assoziierte Antigen mit einer Aminosäuresequenz wie in Figur 5 dargestellt oder ein Epitop davon zu binden oder Komplexe damit zu bilden zur Verwendung bei der Behandlung maligner Zellen oder Tumore.

**32.** Antikörper nach Anspruch 31, wobei der Tumor ein beliebiger von einem festen Tumor, einer Leukämie oder einem Lymphom ist.

**33.** Antikörper nach Anspruch 32, wobei der feste Tumor ausgewählt ist aus einem beliebigen von Niere, Brust, Gebärmutter, Gebärmutterhals, Kolon, Lunge, Magen, Rektum oder Dünndarm.

**34.** Antikörper nach Anspruch 32, wobei das Lymphom ein B-Zell-Lymphom wie zum Beispiel ein beliebiges von diffus großzelliges B-Zell-Lymphom (DLBCL), follikuläres Lymphom, Burkitt-Lymphom, aber bevorzugt DLBCL ist.

**35.** Verwendung eines Antikörpers nach einem der Ansprüche 16 bis 21 oder eines Antokörpers, der fähig ist, an ein Tumor-assoziiertes Antigen mit einer Aminosäuresequenz wir in Figur 5 dargestellt oder an ein Epitop davon zu binden oder Komplexe damit zu bilden bei der Herstellung eines Medikaments zur Behandlung maligner Zellen oder Tumore.

**36.** Nukleinsäuremolekül nach Anspruch 5 zur Verwendung bei der Behandlung maligner Zellen oder Tumore.

**37.** Nukleinsäuremolekül nach Anspruch 36, wobei das Nukleinsäuremolekül ein Antisense-Molekül ist.

**38.** Nukleinsäuremolekül nach Anspruch 36, wobei das Nukleinsäuremolekül doppelsträngige RNA ist.

**39.** Verwendung eines Nukleinsäuremoleküls nach Anspruch 5 bei der Herstellung eines Medikaments für die Behandlung maligner Zellen oder Tumore.

**40.** Nukleinsäuremolekül, das fähig ist mit einem Molekül unter Bedingungen hoher Stringenz zu hybridisieren, das ein Antigen mit einer Aminosäuresequenz, dargestellt in Figur 5 kodiert zur Verwendung bei der Behandlung von Lymphom.

**41.** Verwendung eines Nukleinsäuremoleküls, das fähig ist mit einem Molekül unter Bedingungen hoher Stringenz zu hybridisieren, das ein Antigen mit einer Aminosäuresequenz, dargestellt in Figur 5 kodiert bei der Herstellung eines Medikaments zur Behandlung von Lymphom.

**42.** Assay-Kit zur Detektion maligner Zellen oder Tumore in einem Patienten, wobei das Kit ein oder mehr Tumor-assoziierte Antigene nach einem der Ansprüche 10 bis 12 und Mittel zum in Kontakt bringen des Antigens mit einer Probe aus Körperflüssigkeit aus dem Patienten umfasst.

**43.** Assay-Kit zur Detektion von Lymphomen in einem Patienten, wobei das Kit ein Antigen mit einer Aminosäuresequenz wie in Figur 5 dargestellt und Mittel zum in Kontakt bringen des Antigens mit einer Probe aus Körperflüssigkeit aus dem Patienten umfasst.

**44.** Verwendung des Verfahrens nach einem der Ansprüche 22 bis 27 zur Bestimmung eines maligne Zelle/Tumor-assoziiertes Antigen-Profils eines Individuums, das an einem Malignom oder Tumor leidet.

**45.** Verwendung des Verfahren nach einem der Ansprüche 28 bis 30 zur Bestimmung eines Lymphom-assoziierten Antigen-Profils eines Individuums, das an einem Lymphom leidet.

**46.** Diagnostisches Reagenz für Malignom oder Tumor, umfassend Säugetier-Autoantikörper mit einer Spezifität für mindestens eines der Tumor-assoziierten Antigene nach einem der Ansprüche 10 bis 12.

**47.** Verwendung eines Verfahrens nach einem der Ansprüche 22 bis 28 zum Screening für eine minimale Resterkrankung, Wiederauftreten des Malignoms oder Tumors nach Behandlung oder um das Fortschreiten der Behandlung eines Individuums für ein Malignom oder einen Tumor zu überwachen.

**48.** Verwendung nach Anspruch 47, wobei das Malignom oder der Tumor ein Lymphom, eine Leukämie oder ein fester Tumor ist.

**49.** Verwendung eines Verfahren nach einem der Ansprüche 28 bis 30 zum Screening für Wiederauftreten eines Lymphoms nach Behandlung oder um das Fortschreiten der Behandlung eines Individuums für das Lymphom zu überwachen.

**50.** Isolierte immortalisierte Zellpopulation, die fähig ist, Antikörper gegen ein Epitop von Tumor-assoziierten Antigenen in einem Individuum nach einem der Ansprüche 10 bis 12 herzustellen.

**51.** Isolierte immortalisierte Zellpopulation, die fähig ist, Antikörper gegen ein Epitop von Lymphom-assoziierten Antigenen in einem Individuum nach einem der Ansprüche 9 bis 11 oder einem Antigen mit einer Aminosäuresequenz wie in Figur 5 dargestellt herzustellen.

**52.** Isolierte immortalisierte Zellpopulation nach Anspruch 50 oder 51, wobei die Antikörper gegen ein Epitop von DLBCL-assoziierten Tumor-Antigenen gerichtet sind.

**53.** Vakzin-Zusammensetzung, umfassend eine effektive Menge eines Polypeptids nach einem der Ansprüche 10 bis 12 oder eines isolierten Antigens mit einer Aminosäuresequenz wie in Figur 5 dargestellt oder eines Epitops oder immunogenen Fragments davon, zusammen mit einem pharmazeutisch akzeptablen Träger.

**54.** Vakzin-Zusammensetzung, umfassend eine effektive Menge eines Nukleinsäuremoleküls nach einem der Ansprüche 1 bis 9 oder einer Nukleotidsequenz, die ein Antigen mit einer Aminosäuresequenz wie in Figur 5 dargestellt kodiert oder eines Epitops oder immunogenen Fragments davon, zusammen mit einem pharmazeutisch akzeptablen Träger.

**55.** Vakzin-Zusammensetzung umfassend eine effektive Menge von Antigen-präsentierenden Zellen, die modifiziert sind, um ein beliebiges der Antigene nach einem der Ansprüche 10 bis 12 oder ein Antigen mit einer Aminosäuresequenz wie in Figur 5 dargestellt oder ein Epitop oder immunogenes Fragment davon zu exprimieren, zusammen mit einem pharmazeutisch akzeptablen Träger.

**56.** Vakzin-Zusammensetzung nach einem der Ansprüche 55 oder 55, die weiterhin ein geeignetes Adjuvans umfasst.

**57.** Nukleinsäuremolekül nach einem der Ansprüche 1 bis 9 oder das ein Antigen mit einer Aminosäuresequenz wie in Figur 5 dargestellt kodiert zur Verwendung bei der Behandlung eines menschlichen oder tierischen Körpers.

**58.** Tumor- oder Lymphom-assoziiertes Antigen nach einem der Ansprüche 10 bis 12, oder ein Antigen mit einer Aminosäuresequenz wie in Figur 5 dargestellt oder ein Epitop davon zur Verwendung bei der Behandlung eines menschlichen oder tierischen Körpers.

**59.** Antikörper nach einem der Ansprüche 16 bis 21 oder ein Antikörper, der für ein Antigen mit einer Aminosäuresequenz wie in Figur 5 dargestellt oder ein Epitop davon spezifisch ist zur Verwendung bei der Behandlung eines menschlichen oder tierischen Körpers.

**60.** *In vitro* Verfahren zur Überwachung von CTL-Antworten in einem Patienten auf ein Antigen mit einer Aminosäuresequenz wie in Figur 5 dargestellt, das in Kontakt bringen einer Körperflüssigkeit aus dem Patienten mit einem Tetramer-Komplex eines Antigenfragments des Antigens und MHC Klasse I Moleküle und Überwachen des Levels an CTL-Antworten für die Antigene umfasst.

**61.** *In vitro* Verfahren zur Aufreinigung von CTL-Zellen aus einem Patienten, das in Kontakt bringen einer Körperflüssigkeit aus dem Patienten mit einem Tetramer-Komplex eines Antigenfragments eines Antigens mit einer Aminosäuresequenz wie in Figur 5 dargestellt und MHC Klasse I Moleküle und Entfernen beliebiger gebundener Zellen, die das/die Antigenpeptid(e) erkennen umfasst.

**Patentansprüche für folgende(n) Vertragsstaat(en): GB, DE, FR**

**1.** Nukleinsäuremolekül, das ein Tumor-assozüertes Antigen mit der Aminosäuresequenz, dargestellt in Figur 2 kodiert.

**2.** Molekül nach Anspruch 1, das ein DNA- oder RNA-Molekül ist.

**3.** Molekül nach Anspruch 1 oder 2, das ein cDNA-Molekül ist.

**4.** Molekül nach einem der Ansprüche 1 bis 3, wobei das Nukleinsäuremolekül, das das Tumor-assoziierte Antigen kodiert, die Sequenz in Figur 1 umfasst.

**5.** Nukleinsäuremolekül nach einem der Ansprüche 1 bis 4, wobei das Tumorantigen eines ist, das mit Lymphomen assoziiert ist.

**6.** Nukleinsäuremolekül nach Anspruch 5, wobei das Lymphom ein B-Zell-Lymphom ist.

**7.** Nukleinsäuremolekül nach einem der Ansprüche 1 bis 4, wobei der Tumor ein fester Tumor ist.

**8.** Nukleinsäuremolekül nach Anspruch 7, wobei der feste Tumor ein beliebiger von Niere, Brust, Gebärmutter, Gebärmutterhals, Kolon, Lunge, Magen, Rektum oder Dünndarm ist.

**9.** Tumor-assoziiertes Antigen, kodiert durch ein Nukleinsäuremolekül nach einem der Ansprüche 1 bis 8.

**10.** Tumor-assoziiertes Antigen, kodiert durch ein Nukleinsäuremolekül mit einer Nukleotidsequenz dargestellt in Figur 1.

**11.** Tumor-assoziiertes Antigen mit der Aminosäuresequenz nach Figur 2.

**12.** Expressionsvektor, umfassend ein Nukleinsäuremolekül nach einem der Ansprüche 1 bis 8.

**13.** Wirtszelle oder Organismus, transformiert oder transfiziert mit einem Expressionsvektor nach Anspruch 12.

**14.** Transgener nicht-humaner Organismus, umfassend ein Transgen, das fähig ist, ein Tumorantigen nach einem der Ansprüche 9 bis 11 zu exprimieren.

**15.** Antikörper oder Fragment davon, der fähig ist, an ein Tumor-assoziiertes Antigen nach einem der Ansprüche 9 bis 11 zu binden.

**16.** Antikörper nach Anspruch 15, der ein beliebiger von polyklonaler oder monoklonaler Antikörper ist.

**17.** Antikörper nach Anspruch 15 oder 16, der ein humanisierter Antikörper ist.

**18.** Antikörper nach einem der Ansprüche 15 bis 17, der ein Autoantikörper ist.

**19.** Antikörper nach einem der Ansprüche 15 bis 18, weiterhin umfassend ein toxisches oder ein Reporter-Molekül.

**20.** Antikörper nach Anspruch 19, wobei das Reporter-Molekül ein Radioisotop ist.

**21.** *In vitro* Verfahren zur Identifikation maligner Zellen oder Tumore in einem Säugetier, wobei das Verfahren Identifizieren von Antikörpern aus dem Säugetier in einer Probe aus Körperflüssigkeit aus dem Säugetier, die fähig sind Komplexe mit einem Tumor-assoziierten Antigen nach einem der Ansprüche 9 bis 11 oder einem Tumor-assoziierten Antigen mit der Aminosäuresequenz, dargestellt in Figur 5 oder einem Epitop davon zu bilden, umfasst.

**22.** Verfahren nach Anspruch 21, wobei das Säugetier ein Mensch ist und die Antikörper humane Autoantikörpern sind.

**23.** Verfahren nach Anspruch 21 oder 22, wobei das Malignom ein beliebiges von einem festen Tumor, einer Leukämie oder einem Lymphom ist.

**24.** Verfahren nach Anspruch 23, wobei der feste Tumor ein beliebiges von Niere, Brust, Gebärmutter, Gebärmutterhals, Kolon, Lunge, Magen, Rektum oder Dünndarm ist.

**25.** Verfahren nach Anspruch 23, wobei das Lymphom ein B-Zell-Lymphom wie zum Beispiel ein beliebiges von diffus großzelliges B-Zell-Lymphom (DLBCL), follikuläres Lymphom, Burkitt-Lymphom, aber bevorzugt DLBCL ist.

**26.** Verfahren nach einem der Ansprüche 21 bis 25, wobei die Körperflüssigkeit humanes Serum oder Plasma umfasst.

**27.** *In vitro* Verfahren zur Detektion von Lymphom in einem Patienten, wobei das Verfahren Identifizieren von Autoantikörpern aus einer Körperflüssigkeit des Patienten, die fähig sind, Komplexe mit dem Antigen mit der Aminosäuresequenz, die in Figur 5 dargestellt ist, oder einem Epitop davon zu bilden, umfasst.

**28.** Verfahren nach Anspruch 27, wobei das Lymphom ein B-Zell-Lymphom einschließlich ein beliebiges von diffus großzelliges B-Zell-Lymphom, follikuläres Lymphom oder Burkitt-Lymphom ist.

**29.** Verfahren nach Anspruch 27 oder 28, wobei die Körperflüssigkeit humanes Serum oder Plasma umfasst.

**30.** Antikörper nach einem der Ansprüche 15 bis 20 oder ein Antikörper, der fähig ist, an das Tumor-assoziierte Antigen mit einer Aminosäuresequenz wie in Figur 5 dargestellt oder ein Epitop davon zu binden oder Komplexe damit zu bilden zur Verwendung bei der Behandlung maligner Zellen oder Tumore.

**31.** Antikörper nach Anspruch 30, wobei der Tumor ein beliebiger von einem festen Tumor, einer Leukämie oder einem Lymphom ist.

**32.** Antikörper nach Anspruch 31, wobei der feste Tumor ausgewählt ist aus einem beliebigen von Niere, Brust, Gebärmutter, Gebärmutterhals, Kolon, Lunge, Magen, Rektum oder Dünndarm.

**33.** Antikörper nach Anspruch 31, wobei das Lymphom ein B-Zell-Lymphom wie zum Beispiel ein beliebiges von diffus großzelliges B-Zell-Lymphom (DLBCL), follikuläres Lymphom, Burkitt-Lymphom, aber bevorzugt DLBCL ist.

**34.** Verwendung eines Antikörpers nach einem der Ansprüche 15 bis 20 oder eines Antikörpers, der fähig ist, an ein Tumor-assoziiertes Antigen mit einer Aminosäuresequenz wie in Figur 5 dargestellt oder an ein Epitop davon zu binden oder Komplexe damit zu bilden bei der Herstellung eines Medikaments zur Behandlung maligner Zellen oder Tumore.

**35.** Nukleinsäuremolekül, das fähig ist mit einem Molekül nach einem der Ansprüche 1 bis 4 zu hybridisieren zur Verwendung bei der Behandlung maligner Zellen oder Tumore.

**36.** Nukleinsäuremolekül nach Anspruch 35, wobei das Nukleinsäuremolekül ein Antisense-Molekül ist.

**37.** Nukleinsäuremolekül nach Anspruch 35, wobei das Nukleinsäuremolekül doppelsträngige RNA ist.

**38.** Verwendung eines Nukleinsäuremoleküls, das fähig ist mit einem Molekül nach einem der Ansprüche 1 bis 4 zu hybridisieren bei der Herstellung eines Medikaments für die Behandlung maligner Zellen oder Tumore.

**39.** Nukleinsäuremolekül, das fähig ist mit einem Molekül unter Bedingungen hoher Stringenz zu hybridisieren, das ein Antigen mit einer Aminosäuresequenz, dargestellt in Figur 5 kodiert zur Verwendung bei der Behandlung von Lymphom.

**40.** Verwendung eines Nukleinsäuremoleküls, das fähig ist mit einem Molekül unter Bedingungen hoher Stringenz zu hybridisieren, das ein Antigen mit einer Aminosäuresequenz, dargestellt in Figur 5 kodiert bei der Herstellung eines Medikaments zur Behandlung von Lymphom.

**41.** Assay-Kit zur Detektion maligner Zellen oder Tumore in einem Patienten, wobei das Kit ein oder mehr Tumor-assozüerte Antigene nach einem der Ansprüche 9 bis 11 und Mittel zum in Kontakt bringen des Antigens mit einer Probe aus Körperflüssigkeit aus dem Patienten umfasst.

**42.** Assay-Kit zur Detektion von Lymphomen in einem Patienten, wobei das Kit ein Antigen mit einer Aminosäuresequenz wie in Figur 5 dargestellt und Mittel zum in Kontakt bringen des Antigens mit einer Probe aus Körperflüssigkeit aus dem Patienten umfasst.

**43.** Verwendung des Verfahrens nach einem der Ansprüche 21 bis 26 zur Bestimmung eines maligne Zelle/Tumor-assoziiertes Antigen-Profils eines Individuums, das an einem Malignom oder Tumor leidet.

**44.** Verwendung des Verfahren nach einem der Ansprüche 27 bis 29 zur Bestimmung eines Lymphom-assoziierten Antigen-Profils eines Individuums, das an einem Lymphom leidet.

**45.** Diagnostisches Reagenz für Malignom oder Tumor, umfassend Säugetier-Autoantikörper mit einer Spezifität für mindestens eines der Tumor-assozüerten Antigene nach einem der Ansprüche 9 bis 11.

**46.** Verwendung eines Verfahrens nach einem der Ansprüche 21 bis 27 zum Screening für eine minimale Resterkrankung, Wiederauftreten des Malignoms oder Tumors nach Behandlung oder um das Fortschreiten der Behandlung eines Individuums für ein Malignom oder einen Tumor zu überwachen.

**47.** Verwendung nach Anspruch 46, wobei das Malignom oder der Tumor ein Lymphom, eine Leukämie oder ein fester Tumor ist.

**48.** Verwendung eines Verfahren nach einem der Ansprüche 27 bis 29 zum Screening für Wiederauftreten eines Lymphoms nach Behandlung oder um das Fortschreiten der Behandlung eines Individuums für das Lymphom zu überwachen.

**49.** Isolierte immortalisierte Zellpopulation, die fähig ist, Antikörper gegen ein Epitop von Tumor-assozüerten Antigenen in einem Individuum nach einem der Ansprüche 9 bis 11 herzustellen.

**50.** Isolierte immortalisierte Zellpopulation, die fähig ist, Antikörper gegen ein Epitop von Lymphom-assoziierten Antigenen in einem Individuum nach einem der Ansprüche 9 bis 11 oder einem Antigen mit einer Aminosäuresequenz wie in Figur 5 dargestellt herzustellen.

**51.** Isolierte immortalisierte Zellpopulation nach Anspruch 49 oder 50, wobei die Antikörper gegen ein Epitop von DLBCL-assoziierten Tumor-Antigenen gerichtet sind.

**52.** Vakzin-Zusammensetzung, umfassend eine effektive Menge eines Polypeptids nach einem der Ansprüche 9 bis 11 oder eines isolierten Antigens mit einer Aminosäuresequenz wie in Figur 5 dargestellt oder eines Epitops oder immunogenen Fragments davon, zusammen mit einem pharmazeutisch akzeptablen Träger.

**53.** Vakzin-Zusammensetzung, umfassend eine effektive Menge eines Nukleinsäuremoleküls nach einem der Ansprüche 1 bis 8 oder einer Nukleotidsequenz, die ein Antigen mit einer Aminosäuresequenz wie in Figur 5 dargestellt kodiert oder eines Epitops oder immunogenen Fragments davon, zusammen mit einem pharmazeutisch akzeptablen Träger.

**54.** Vakzin-Zusammensetzung umfassend eine effektive Menge von Antigen-präsentierenden Zellen, die modifiziert sind, um ein beliebiges der Antigene nach einem der Ansprüche 9 bis 11 oder ein Antigen mit einer Aminosäuresequenz wie in Figur 5 dargestellt oder ein Epitop oder immunogenes Fragment davon zu exprimieren, zusammen mit einem pharmazeutisch akzeptablen Träger.

**55.** Vakzin-Zusammensetzung nach einem der Ansprüche 53 oder 54, die weiterhin ein geeignetes Adjuvans umfasst.

**56.** Nukleinsäuremolekül nach einem der Ansprüche 1 bis 8 oder das ein Antigen mit einer Aminosäuresequenz wie in Figur 5 dargestellt kodiert zur Verwendung bei der Behandlung eines menschlichen oder tierischen Körpers.

**57.** Tumor- oder Lymphom-assoziiertes Antigen nach einem der Ansprüche 9 bis 11, oder ein Antigen mit einer Aminosäuresequenz wie in Figur 5 dargestellt oder ein Epitop davon zur Verwendung bei der Behandlung eines menschlichen oder tierischen Körpers.

**58.** Antikörper nach einem der Ansprüche 15 bis 20 oder ein Antikörper, der für ein Antigen mit einer Aminosäuresequenz wie in Figur 5 dargestellt oder ein Epitop davon spezifisch ist zur Verwendung bei der Behandlung eines menschlichen oder tierischen Körpers.

**59.** *In vitro* Verfahren zur Überwachung von CTL-Antworten in einem Patienten auf ein Antigen mit einer Aminosäuresequenz wie in Figur 5 dargestellt, das in Kontakt bringen einer Körperflüssigkeit aus dem Patienten mit einem Tetramer-Komplex eines Antigenfragments des Antigens und MHC Klasse I Moleküle und Überwachen des Levels an CTL-Antworten für die Antigene umfasst.

**60.** *In vitro* Verfahren zur Aufreinigung von CTL-Zellen aus einem Patienten, das in Kontakt bringen einer Körperflüssigkeit aus dem Patienten mit einem Tetramer-Komplex eines Antigenfragments eines Antigens mit einer Aminosäuresequenz wie in Figur 5 dargestellt und MHC Klasse I Moleküle und Entfernen beliebiger gebundener Zellen, die das/die Antigenpeptid(e) erkennen umfasst.

## Revendications

### Revendications pour l'(les) Etat(s) contractant(s) suivant(s): AT, BE, CH, DK, ES, GR, IT, LI, LU, MC, NL, SE

1. Molécule d'acide nucléique codant un antigène associé à une tumeur ayant la séquence d'acides aminés illustrée dans la Figure 2.

2. Molécule selon la revendication 1, qui est une molécule d'ADN ou d'ARN.

3. Molécule selon la revendication 1 ou 2, qui est une molécule d'ADN complémentaire.

4. Molécule selon l'une des revendications 1 à 3, dans laquelle ladite molécule d'acide nucléique codant ledit antigène associé à une tumeur comprend la séquence à la Figure 1.

5. Molécule d'acide nucléique capable de s'hybrider à une molécule selon l'une des revendications 1 à 4 dans des conditions de stringence élevée.

6. Molécule d'acide nucléique selon l'une des revendications 1 à 5, dans laquelle ledit antigène tumoral est un antigène tumoral associé à des lymphomes.

7. Molécule d'acide nucléique selon la revendication 6, dans laquelle ledit lymphome est un lymphome à cellules B.

8. Molécule d'acide nucléique selon l'une des revendications 1 à 5, dans laquelle ladite tumeur est une tumeur solide.

9. Molécule d'acide nucléique selon la revendication 8, dans laquelle ladite tumeur solide est l'une, d'une tumeur solide du rein, du sein, de l'utérus, du col de l'utérus, du côlon, des poumons, de l'estomac, du rectum ou de l'intestin grêle.

10. Antigène associé à une tumeur codé par une molécule d'acide nucléique selon l'une des revendications 1 à 9.

11. Antigène associé à une tumeur codé par une molécule d'acide nucléique ayant une séquence nucléotidique illustrée à la Figure 1.

12. Antigène associé à une tumeur ayant la séquence d'acides aminés selon la Figure 2.

13. Vecteur d'expression comprenant une molécule d'acide nucléique selon l'une des revendications 1 à 9.

14. Cellule hôte ou organisme transformé ou transfecté avec un vecteur d'expression selon la revendication 13.

15. Organisme non humain transgénique comprenant un transgène capable d'exprimer un antigène tumoral selon l'une des revendications 10 à 12.

16. Anticorps ou fragment de celui-ci capable de se lier à un antigène associé à une tumeur selon lune des revendications 10 à 12.

17. Anticorps selon la revendication 16, qui est l'un d'un anticorps polyclonal ou monoclonal.

18. Anticorps selon la revendication 16 ou 17, qui est un anticorps humanisé.

19. Anticorps selon lune des revendications 16 à 18, qui est un autoanticorps.

20. Anticorps selon lune des revendications 16 à 19, comprenant en outre une molécule toxique ou rapporteuse.

21. Anticorps selon la revendication 20, dans lequel ladite molécule rapporteuse est un radio-isotope.

22. Procédé in vitro qui consiste à identifier des cellules malignes ou des tumeurs chez un mammifère, lequel procédé comprend le fait d'identifier dans un échantillon de fluide corporel dudit mammifères des anticorps dudit mammifère capables de former des complexes avec un antigène associé à une tumeur selon lune des revendications 10 à 12 ou un antigène associé à une tumeur ayant la séquence d'acides aminés illustrée à la Figure 5, ou son épitope.

23. Procédé selon la revendication 22, dans lequel ledit mammifère est un humain et les anticorps sont des autoanticorps humains.

24. Procédé selon la revendication 22 ou 23, dans lequel ladite malignité est l'une d'une tumeur solide, d'une leucémie ou d'un lymphome.

25. Procédé selon la revendication 24, dans lequel ladite tumeur solide du rein, du sein, de l'utérus, du col de l'utérus, du côlon, des poumons, de l'estomac, du rectum ou de l'intestin grêle

**26.** Procédé selon la revendication 24, dans lequel ledit lymphome est un lymphome à cellule B tel que l'un d'un lymphome diffus à grandes cellules B (DLBCL), d'un lymphome folliculaire, d'un lymphome de Burkitt, mais de préférence le lymphome DLBCL.

**27.** Procédé selon l'une des revendications 22 à 26, dans lequel ledit fluide corporel comprend du sérum ou du plasma humain.

**28.** Procédé in vitro qui consiste à détecter un lymphome chez un patient, lequel procédé comprend le fait d'identifier des autoanticorps à partir d'un fluide corporel dudit patient capables de former des complexes avec l'antigène ayant une séquence d'acides aminés comme présenté dans la Figure 5, ou son épitope.

**29.** Procédé selon la revendication 28, dans lequel ledit lymphome est un lymphome à cellules B comprenant l'un d'un lymphome diffus à grandes cellules B, d'un lymphome folliculaire et d'un lymphome de Burkitt.

**30.** Procédé selon la revendication 28 ou 29, dans lequel ledit fluide corporel comprend du sérum ou du plasma humain.

**31.** Anticorps selon l'une des revendications 16 à 21, ou un anticorps capable de se lier à ou à former des complexes avec l'antigène associé à une tumeur ayant une séquence d'acides aminés comme présenté à la Figure 5 ou son épitope à utiliser dans un traitement de cellules malignes ou de tumeurs.

**32.** Anticorps selon la revendications 31, dans lequel ladite tumeur est l'une d'une tumeur solide, d'une leucémie ou d'un lymphome.

**33.** Anticorps selon la revendications 32, dans lequel la tumeur solide est choisie parmi l'une d'une tumeur solide du rein, du sein, de l'utérus, du col de l'utérus, du côlon, des poumons, de l'estomac, du rectum ou de l'intestin grêle.

**34.** Anticorps selon la revendication 32, dans lequel ledit lymphome est un lymphome à cellules B, tel que l'un d'un lymphome diffus à grandes cellules B (DLBCL), d'un lymphome folliculaire, d'un lymphome de Burkitt, mais de préférence le lymphome DLBCL.

**35.** Utilisation d'un anticorps selon l'une des revendications 16 à 21, ou d'un anticorps capable de se lier à ou de former des complexes avec un antigène associé à une tumeur ayant une séquence d'acides aminés comme présenté à la Figure 5 ou son épitope pour la fabrication d'un médicament pour traiter des cellules malignes ou des tumeurs.

**36.** Molécule d'acide nucléique selon la revendication 5 à utiliser dans le traitement de cellules malignes ou de tumeurs.

**37.** Molécule d'acide nucléique selon la revendication 36, dans laquelle ladite molécule d'acide nucléique est une molécule antisens.

**38.** Molécule d'acide nucléique selon la revendication 36, dans laquelle ladite molécule d'acide nucléique est un ARN double brin.

**39.** Utilisation d'une molécule d'acide nucléique selon la revendications 5 pour la fabrication d'un médicament pour le traitement de cellules malignes ou de tumeurs.

**40.** Molécule d'acide nucléique capable de s'hybrider à une molécule d'acide nucléique codant un antigène ayant une séquence d'acides aminés illustrée dans la Figure 5, dans des conditions de stringence élevée à utiliser dans le traitement d'un lymphome.

**41.** Utilisation d'une molécule d'acide nucléique capable de s'hybrider à la molécule d'acide nucléique codant un antigène ayant la séquence d'acides aminés illustrée dans la Figure 5 dans des conditions de stringence élevée pour la fabrication d'un médicament pour traiter un lymphome.

**42.** Trousse d'essai destinée à détecter des cellules malignes ou des tumeurs chez un patient, ladite trousse comprenant un ou plusieurs antigènes associés à des tumeurs selon l'une des revendications 10 à 12, et des moyens pour faire mettre en contact ledit antigène avec un échantillon de fluide corporel dudit patient.

**43.** Trousse d'essai destinée à détecter des lymphomes chez un patient ladite trousse comprenant un antigène ayant une séquence d'acides aminés comme illustré dans la Figure 5, et des moyens pour faire mettre en contact ledit antigène avec un échantillon de fluide corporel d'un patient.

**44.** Utilisation du procédé selon l'une des revendications 22 à 27, pour déterminer le profil antigénique associé à une cellule maligne/une tumeur d'un individu souffrant d'une malignité ou d'une tumeur.

**45.** Utilisation du procédé selon l'une des revendications 28 à 30, pour la détermination d'un profil antigénique associé à un lymphome d'un individu souffrant d'un lymphome.

**46.** Réactif de diagnostic de tumeur ou de malignité comprenant des autoanticorps de mammifère ayant une spécificité pour au moins l'un des antigènes associés à des tumeurs selon l'une des revendications 10à 12.

**47.** Utilisation d'un procédé selon l'une des revendications 22 à 28, pour dépister une maladie résiduelle minimale, la récurrence d'une malignité ou d'une tumeur après traitement ou pour surveiller le progrès du traitement d'un individu contre une malignité ou une tumeur.

**48.** Utilisation selon la revendication 47, dans laquelle ladite malignité ou tumeur est un lymphome, une leucémie ou une tumeur solide.

**49.** Utilisation d'un procédé selon l'une des revendications 28 à 30, pour dépister la récurrence d'un lymphome après traitement, ou pour surveiller le progrès du traitement dudit lymphome chez un individu.

**50.** Population de cellules isolées immortalisées capable de produire des anticorps contre un épitope d'antigènes associés à une tumeur chez un individu selon l'une des revendications 10 à 12.

**51.** Population de cellules isolées immortalisées capable de produire des anticorps contre un épitope d'antigènes associés à des lymphomes chez un individu selon l'une des revendications 10 à 12, ou un antigène ayant une séquence d'acides aminés comme illustré dans la Figure 5.

**52.** Population de cellules isolées immortalisées selon la revendication 50 ou 51, dans laquelle lesdits anticorps sont dirigés contre un épitope d'antigènes tumoraux associés à un DLBCL.

**53.** Composition de vaccin comprenant une quantité efficace d'un polypeptide selon l'une des revendications 10 à 12, ou un antigène isolé ayant une séquence d'acides aminés comme illustré dans la Figure 5, ou son fragment épitopique ou immunogène, ainsi qu'un véhicule pharmaceutiquement acceptable.

**54.** Composition de vaccin comprenant une quantité efficace d'une molécule d'acide nucléique selon l'une des revendications 1 à 9, ou une séquence nucléotidique codant un antigène ayant une séquence d'acides aminés comme illustré dans la Figure 5, ou son fragment épitopique ou immunogène, ainsi qu'un véhicule pharmaceutiquement acceptable.

**55.** Composition de vaccin comprenant une quantité efficace de cellules présentant des antigènes modifiés pour exprimer l'un des antigènes selon l'une des revendications 10 à 12 ou un antigène ayant une séquence d'acides aminés comme illustré dans la Figure 5 ou son fragment épitopique ou immunogène, avec un véhicule pharmaceutiquement acceptable.

**56.** Composition de vaccin selon la revendication 53 ou 54, qui comprend en outre un adjuvant approprié.

**57.** Molécule d'acide nucléique selon l'une des revendications 1 à 9, ou codant un antigène ayant une séquence d'acides aminés comme illustré dans la Figure 5 à utiliser dans le traitement d'un corps humain ou animal.

**58.** Antigène associé à une tumeur ou à un lymphome selon l'une des revendications 10 à 12, ou un antigène ayant une séquence d'acides aminés comme illustré dans la Figure 5, ou son épitope à utiliser dans le traitement d'un corps humain ou animal.

**59.** Anticorps selon l'une des revendications 16 à 21, ou un anticorps spécifique pour un antigène ayant une séquence d'acides aminés comme illustré dans la Figure 5 ou son épitope, à utiliser dans un traitement d'un corps humain ou animal.

**60.** Procédé *in vitro* qui consiste à surveiller des réponses CTL chez un patient à un antigène ayant une séquence d'acides aminés comme illustré dans la Figure 5, qui comprend le fait de mettre en contact un fluide corporel dudit patient avec un complexe tétramère d'un fragment antigénique dudit antigène et des molécules MHC de classe I et à surveiller le niveau de réponses CTL pour lesdits antigènes.

**61.** Procédé *in vitro* qui consiste à purifier des cellules CTL d'un patient, qui comprend le fait de mettre en contact un fluide corporel dudit patient avec un complexe tétramère d'un fragment antigénique d'un antigène ayant une séquence d'acides aminés comme illustré dans la Figure 5 et des molécules MHC de classe I et à enlever l'une des cellules liées qui reconnaissent le(s) peptide(s) antigénique(s).

**Revendications pour l'(les) Etat(s) contractant(s) suivant(s): GB, DE, FR**

**1.** Molécule d'acide nucléique codant un antigène associé à une tumeur ayant la séquence d'acides aminés illustrée dans la Figure 2.

**2.** Molécule selon la revendication 1, qui est une molécule d'ADN ou d'ARN.

**3.** Molécule selon la revendication 1 ou 2, qui est une molécule d'ADN complémentaire.

**4.** Molécule selon l'une des revendications 1 à 3, dans laquelle ladite molécule d'acide nucléique codant ledit antigène associé à une tumeur comprend la séquence à la Figure 1.

**5.** Molécule d'acide nucléique selon l'une des revendications 1 à 4, dans laquelle ledit antigène tumoral est un antigène tumoral associé à des lymphomes.

**6.** Molécule d'acide nucléique selon la revendication 5, dans laquelle ledit lymphome est un lymphome à cellules B.

**7.** Molécule d'acide nucléique selon l'une des revendications 1 à 4, dans laquelle ladite tumeur est une tumeur solide.

**8.** Molécule d'acide nucléique selon la revendication 7, dans laquelle ladite tumeur solide est l'une, d'une tumeur solide du rein, du sein, de l'utérus, du col de l'utérus, du côlon, des poumons, de l'estomac, du rectum ou de l'intestin grêle.

**9.** Antigène associé à une tumeur codé par une molécule d'acide nucléique selon l'une des revendications 1 à 8.

**10.** Antigène associé à une tumeur codé par une molécule d'acide nucléique ayant une séquence nucléotidique illustrée à la Figure 1.

**11.** Antigène associé à une tumeur ayant la séquence d'acides aminés selon la Figure 2.

**12.** Vecteur d'expression comprenant une molécule d'acide nucléique selon l'une des revendications 1 à 8.

**13.** Cellule hôte ou organisme transformé ou transfecté avec un vecteur d'expression selon la revendication 12.

**14.** Organisme non humain transgénique comprenant un transgène capable d'exprimer un antigène tumoral selon l'une des revendications 9 à 11.

**15.** Anticorps ou fragment de celui-ci capable de se lier à un antigène associé à une tumeur selon l'une des revendications 9 à 11.

**16.** Anticorps selon la revendication 15, qui est l'un d'un anticorps polyclonal ou monoclonal.

**17.** Anticorps selon la revendication 15 ou 16, qui est un anticorps humanisé.

**18.** Anticorps selon l'une des revendications 15 à 17, qui est un autoanticorps.

**19.** Anticorps selon l'une des revendications 15 à 18, comprenant en outre une molécule toxique ou rapporteuse.

**20.** Anticorps selon la revendication 19, dans lequel ladite molécule rapporteuse est un radio-isotope.

**21.** Procédé in vitro qui consiste à identifier des cellules malignes ou des tumeurs chez un mammifère, lequel procédé comprend le fait d'identifier dans un échantillon de fluide corporel dudit mammifère des anticorps dudit mammifère capables de former des complexes avec un antigène associé à une tumeur selon l'une des revendications 9 à 11 ou un antigène associé à une tumeur ayant la séquence d'acides aminés illustrée à la Figure 5, ou son épitope.

**22.** Procédé selon la revendication 21, dans lequel ledit mammifère est un humain et les anticorps sont des autoanticorps humains.

**23.** Procédé selon la revendication 21 ou 22, dans lequel ladite malignité est l'une d'une tumeur solide, d'une leucémie ou d'un lymphome.

**24.** Procédé selon la revendication 23, dans lequel ladite tumeur solide est l'une d'une tumeur solide du rein, du sein, de l'utérus, du col de l'utérus, du côlon, des poumons, de l'estomac, du rectum ou de l'intestin grêle.

**25.** Procédé selon la revendication 23, dans lequel ledit lymphome est un lymphome à cellules B tel que l'un d'un lymphome diffus à grandes cellules B (DLBCL), d'un lymphome folliculaire, d'un lymphome de Burkitt, mais de préférence le lymphome DLBCL.

**26.** Procédé selon l'une des revendications 21 à 25, dans lequel ledit fluide corporel comprend du sérum ou du plasma humain.

**27.** Procédé in vitro qui consiste à détecter un lymphome chez un patient, lequel procédé comprend le fait d'identifier des autoanticorps à partir d'un fluide corporel dudit patient capables de former des complexes avec l'antigène ayant une séquence d'acides aminés comme présenté dans la Figure 5, ou son épitope.

**28.** Procédé selon la revendication 27, dans lequel ledit lymphome est un lymphome à cellules B comprenant l'un d'un lymphome diffus à grandes cellules B, d'un lymphome folliculaire et d'un lymphome de Burkitt.

**29.** Procédé selon la revendication 27 ou 28, dans lequel ledit fluide corporel comprend du sérum ou du plasma humain.

**30.** Anticorps selon l'une des revendications 15 à 20, ou un anticorps capable de se lier à ou à former des complexes avec l'antigène associé à une tumeur ayant une séquence d'acides aminés comme présenté à la Figure 5 ou son épitope à utiliser dans un traitement de cellules malignes ou de tumeurs.

**31.** Anticorps selon la revendication 30, dans lequel ladite tumeur est l'une d'une tumeur solide, d'une leucémie ou d'un lymphome.

**32.** Anticorps selon la revendication 31, dans lequel la tumeur solide est choisie parmi l'une d'une tumeur solide du rein, du sein, de l'utérus, du col de l'utérus, du côlon, des poumons, de l'estomac, du rectum ou de l'intestin grêle.

**33.** Anticorps selon la revendication 31, dans lequel ledit lymphome est un lymphome à cellules B, tel que l'un d'un lymphome diffus à grandes cellules B (DLBCL), d'un lymphome folliculaire, d'un lymphome de Burkitt, mais de préférence le lymphome DLBCL.

**34.** Utilisation d'un anticorps selon l'une des revendications 15 à 20, ou d'un anticorps capable de se lier à ou de former des complexes avec un antigène associé à une tumeur ayant une séquence d'acides aminés comme présenté à la Figure 5 ou son épitope pour la fabrication d'un médicament pour traiter des cellules malignes ou des tumeurs.

**35.** Molécule d'acide nucléique capable de s'hybrider à une molécule selon l'une des revendications 1 à 4, à utiliser dans le traitement de cellules malignes ou de tumeurs.

**36.** Molécule d'acide nucléique selon la revendication 35, dans laquelle ladite molécule d'acide nucléique est une molécule antisens.

**37.** Molécule d'acide nucléique selon la revendication 35, dans laquelle ladite molécule d'acide nucléique est un ARN double brin.

**38.** Utilisation d'une molécule d'acide nucléique capable de s'hybrider à une molécule selon l'une des revendications 1 à 4 pour la fabrication d'un médicament pour le traitement de cellules malignes ou de tumeurs.

**39.** Molécule d'acide nucléique capable de s'hybrider à une molécule d'acide nucléique codant un antigène ayant une séquence d'acides aminés illustrée dans la Figure 5, dans des conditions de stringence élevée à utiliser dans le traitement d'un lymphome.

**40.** Utilisation d'une molécule d'acide nucléique capable de s'hybrider à la molécule d'acide nucléique codant un antigène ayant la séquence d'acides aminés illustrée dans la Figure 5 dans des conditions de stringence élevée pour la fabrication d'un médicament pour traiter un lymphome.

**41.** Trousse d'essai destinée à détecter des cellules malignes ou des tumeurs chez un patient, ladite trousse comprenant un ou plusieurs antigènes associés à des tumeurs selon l'une des revendications 9 à 11, et des moyens pour faire mettre en contact ledit antigène avec un échantillon de fluide corporel dudit patient.

**42.** Trousse d'essai destinée à détecter des lymphomes chez un patient ladite trousse comprenant un antigène ayant une séquence d'acides aminés comme illustré dans la Figure 5, et des moyens pour faire mettre en contact ledit antigène avec un échantillon de fluide corporel d'un patient.

**43.** Utilisation du procédé selon l'une des revendications 21 à 26, pour déterminer le profil antigénique associé à une cellule maligne/une tumeur d'un individu souffrant d'une malignité ou d'une tumeur.

**44.** Utilisation du procédé selon l'une des revendications 27 à 29, pour la détermination d'un profil antigénique associé à un lymphome d'un individu souffrant d'un lymphome.

**45.** Réactif de diagnostic de tumeur ou de malignité comprenant des autoanticorps de mammifère ayant une spécificité pour au moins l'un des antigènes associés à des tumeurs selon l'une des revendications 9 à 11.

**46.** Utilisation d'un procédé selon l'une des revendications 21 à 27, pour dépister une maladie résiduelle minimale, la récurrence d'une malignité ou d'une tumeur après traitement ou pour surveiller le progrès du traitement d'un individu contre une malignité ou une tumeur.

**47.** Utilisation selon la revendication 46, dans laquelle ladite malignité ou tumeur est un lymphome, une leucémie ou une tumeur solide.

**48.** Utilisation d'un procédé selon l'une des revendications 27 à 29, pour dépister la récurrence d'un lymphome après traitement, ou pour surveiller le progrès du traitement dudit lymphome chez un individu.

**49.** Population de cellules isolées immortalisées capable de produire des anticorps contre un épitope d'antigènes associés à une tumeur chez un individu selon l'une des revendications 9 à 11.

**50.** Population de cellules isolées immortalisées capable de produire des anticorps contre un épitope d'antigènes associés à des lymphomes chez un individu selon l'une des revendications 9 à 11, ou un antigène ayant une séquence d'acides aminés comme illustré dans la Figure 5.

**51.** Population de cellules isolées immortalisées selon la revendication 49 ou 50, dans laquelle lesdits anticorps sont dirigés contre un épitope d'antigènes tumoraux associés à un DLBCL.

**52.** Composition de vaccin comprenant une quantité efficace d'un polypeptide selon l'une des revendications 9 à 11, ou un antigène isolé ayant une séquence d'acides aminés comme illustré dans la Figure 5, ou son fragment épitopique ou immunogène, ainsi qu'un véhicule pharmaceutiquement acceptable.

**53.** Composition de vaccin comprenant une quantité efficace d'une molécule d'acide nucléique selon l'une des revendications 1 à 8, ou une séquence nucléotidique codant un antigène ayant une séquence d'acides aminés comme illustré dans la Figure 5, ou son fragment épitopique ou immunogène, ainsi qu'un véhicule pharmaceutiquement acceptable.

**54.** Composition de vaccin comprenant une quantité efficace de cellules présentant des antigènes modifiés pour exprimer l'un des antigènes selon l'une des revendications 9 à 11 ou un antigène ayant une séquence d'acides aminés comme illustré dans la Figure 5 ou son fragment épitopique ou immunogène, avec un véhicule pharmaceutiquement acceptable.

**55.** Composition de vaccin selon la revendication 53 ou 54, qui comprend en outre un adjuvant approprié.

**56.** Molécule d'acide nucléique selon l'une des revendications 1 à 8, ou codant un antigène ayant une séquence d'acides aminés comme illustré dans la Figure 5 à utiliser dans le traitement d'un corps humain ou animal.

**57.** Antigène associé à une tumeur ou à un lymphome selon l'une des revendications 9 à 11, ou un antigène ayant une séquence d'acides aminés comme illustré dans la Figure 5, ou son épitope à utiliser dans le traitement d'un corps humain ou animal.

**58.** Anticorps selon l'une des revendications 15 à 20, ou un anticorps spécifique pour un antigène ayant une séquence d'acides aminés comme illustré dans la Figure 5 ou son épitope, à utiliser dans un traitement d'un corps humain ou animal.

**59.** Procédé in vitro qui consiste à surveiller des réponses CTL chez un patient à un antigène ayant une séquence d'acides aminés comme illustré dans la Figure 5, qui comprend le fait de mettre en contact un fluide corporel dudit patient avec un complexe tétramère d'un fragment antigénique dudit antigène et des molécules MHC de classe I et à surveiller le niveau de réponses CTL pour lesdits antigènes.

**60.** Procédé in vitro qui consiste à purifier des cellules CTL d'un patient, qui comprend le fait de mettre en contact un fluide corporel dudit patient avec un complexe tétramère d'un fragment antigénique d'un antigène ayant une séquence d'acides aminés comme illustré dans la Figure 5 et des molécules MHC de classe I et à enlever l'une des cellules liées qui reconnaissent le(s) peptide(s) antigénique(s).

FIG. 1.

```
    CAGACTTTCCGGGCGCCCACCAGGCTCCCGGGCTCTCACCGGAGACCACAGGGAGGAGCT
1   ---------+---------+---------+---------+---------+---------+   60
    GTCTGAAAGGCCCGCGGGTGGTCCGAGGGCCCGAGAGTGGCCTCTGGTGTCCCTCCTCGA

    TCAAGGTACCCCAAGTCCCTGCGGCCTCCAGCAGTGAAGAGTTCCACAAGGGAGTCTTCC
61  ---------+---------+---------+---------+---------+---------+   120
    AGTTCCATGGGGTTCAGGGACGCCGGAGGTCGTCACTTCTCAAGGTGTTCCCTCAGAAGG

    TACGTCACTGAATAATGAATGAAGATGAGAGGGGAAAAGAGAAGAGACAAAGTCAATCCA
121 ---------+---------+---------+---------+---------+---------+   180
    ATGCAGTGACTTATTACTTACTTCTACTCTCCCCTTTTCTCTTCTCTGTTTCAGTTAGGT

                          M  K  M  R  G  E  K  R  R  D  K  V  N  P

    AAAAGCTCTCAAAGGAAATTAAACTGGATTCCATCATTTCCTACCTATGATTACTTCAAC
181 ---------+---------+---------+---------+---------+---------+   240
    TTTTCGAGAGTTTCCTTTAATTTGACCTAAGGTAGTAAAGGATGGATACTAATGAAGTTG

    K  S  S  Q  R  K  L  N  W  I  P  S  F  P  T  Y  D  Y  F  N

    CAAGTGACGCTACAGTTATTAGATGGCTTTATGATTACACTGAGCACAGATGGAGTGATC
241 ---------+---------+---------+---------+---------+---------+   300
    GTTCACTGCGATGTCAATAATCTACCGAAATACTAATGTGACTCGTGTCTACCTCACTAG

    Q  V  T  L  Q  L  L  D  G  F  M  I  T  L  S  T  D  G  V  I

    ATTTGTGTGGCTGAAAACATCTCTTCTCTTCTTGGACATTTACCAGCTGAGATTGTGGGC
301 ---------+---------+---------+---------+---------+---------+   360
    TAAACACACCGACTTTTGTAGAGAAGAGAAGAACCTGTAAATGGTCGACTCTAACACCCG

    I  C  V  A  E  N  I  S  S  L  L  G  H  L  P  A  E  I  V  G

    AAAAAATTATTAAGCCTTCTGCCTGATGAAGAGAAAGATGAAGTCTACCAAAAGATTATT
361 ---------+---------+---------+---------+---------+---------+   420
    TTTTTTAATAATTCGGAAGACGGACTACTTCTCTTTCTACTTCAGATGGTTTTCTAATAA

    K  K  L  L  S  L  L  P  D  E  E  K  D  E  V  Y  Q  K  I  I
```

FIG. 1 (CONTINUED 1).

```
    CTCAAATTTCCTTTACTAAACTCAGAAACACATATTGAATTTTGCTGTCATTTAAAAAGA
421 ---------+---------+---------+---------+---------+---------+ 480
    GAGTTTAAAGGAAATGATTTGAGTCTTTGTGTATAACTTAAAACGACAGTAAATTTTTCT

    L  K  F  P  L  L  N  S  E  T  H  I  E  F  C  C  H  L  K  R

    GGAAATGTCGAACATGGTGATAGTTCTGCTTACGAAAACGTGAAATTTATTGTGAATGTA
481 ---------+---------+---------+---------+---------+---------+ 540
    CCTTTACAGCTTGTACCACTATCAAGACGAATGCTTTTGCACTTTAAATAACACTTACAT

    G  N  V  E  H  G  D  S  S  A  Y  E  N  V  K  F  I  V  N  V

    AGAGATATTTGTAATGAGTTTCCTGTGGTCTTTAGTGGCTTGTTTTCCAGCCACCTCTGT
541 ---------+---------+---------+---------+---------+---------+ 600
    TCTCTATAAACATTACTCAAAGGACACCAGAAATCACCGAACAAAAGGTCGGTGGAGACA

    R  D  I  C  N  E  F  P  V  V  F  S  G  L  F  S  S  H  L  C

    GCTGACTTTGCTGCATGTGTTCCTCAGGAGGATCGGCTTTATCTTGTGGGAAATGTTTGC
601 ---------+---------+---------+---------+---------+---------+ 660
    CGACTGAAACGACGTACACAAGGAGTCCTCCTAGCCGAAATAGAACACCCTTTACAAACG

    A  D  F  A  A  C  V  P  Q  E  D  R  L  Y  L  V  G  N  V  C

    ATTCTCAGGACTCAGCTCCTGCAGCAACTTTACACTTCAAAGGCAGTCAGTGATGAAGCT
661 ---------+---------+---------+---------+---------+---------+ 720
    TAAGAGTCCTGAGTCGAGGACGTCGTTGAAATGTGAAGTTTCCGTCAGTCACTACTTCGA

    I  L  R  T  Q  L  L  Q  Q  L  Y  T  S  K  A  V  S  D  E  A

    GTACTTACACAAGATTCAGATGAGGAACCTTTTGTGGGAGAGCTCAGTAGCTCTCAAGGT
721 ---------+---------+---------+---------+---------+---------+ 780
    CATGAATGTGTTCTAAGTCTACTCCTTGGAAAACACCCTCTCGAGTCATCGAGAGTTCCA

    V  L  T  Q  D  S  D  E  E  P  F  V  G  E  L  S  S  S  Q  G

    CAAAGAGGACACACTAGCATGAAAGCCGTGTACGTTGAACCCGCTGCTGCTGCTGCTGCT
781 ---------+---------+---------+---------+---------+---------+ 840
    GTTTCTCCTGTGTGATCGTACTTTCGGCACATGCAACTTGGGCGACGACGACGACGACGA

    Q  R  G  H  T  S  M  K  A  V  Y  V  E  P  A  A  A  A  A  A
```

FIG. 1(CONTINUED 2).

```
     GCTGCTATCTCAGACGACCAAATTGATATTGCAGAGGTTGAGCAGTATGGACCACAAGAA
 841 ---------+---------+---------+---------+---------+---------+  900
     CGACGATAGAGTCTGCTGGTTTAACTATAACGTCTCCAACTCGTCATACCTGGTGTTCTT

     A  A  I  S  D  D  Q  I  D  I  A  E  V  E  Q  Y  G  P  Q  E

     AACGTTCACATGTTTGTAGATTCTGATTCAACTTATTGCTCCAGTACAGTTTTCCTGGAT
 901 ---------+---------+---------+---------+---------+---------+  960
     TTGCAAGTGTACAAACATCTAAGACTAAGTTGAATAACGAGGTCATGTCAAAAGGACCTA

     N  V  H  M  F  V  D  S  D  S  T  Y  C  S  S  T  V  F  L  D

     ACTATGCCTGAATCTCCAGCCTTATCCTTGCAAGACTTTCGAGGTGAGCCTGAGGTGAAT
 961 ---------+---------+---------+---------+---------+---------+  1020
     TGATACGGACTTAGAGGTCGGAATAGGAACGTTCTGAAAGCTCCACTCGGACTCCACTTA

     T  M  P  E  S  P  A  L  S  L  Q  D  F  R  G  E  P  E  V  N

     CCATTGTACAGGGCAGACCCAGTGGACCTGGAGTTCTCGGTGGATCAGGTGGACTCAGTG
1021 ---------+---------+---------+---------+---------+---------+  1080
     GGTAACATGTCCCGTCTGGGTCACCTGGACCTCAAGAGCCACCTAGTCCACCTGAGTCAC

     P  L  Y  R  A  D  P  V  D  L  E  F  S  V  D  Q  V  D  S  V

     GACCAGGAGGGCCCAATGGACCAGCAGGACCCAGAGAACCCAGTTGCCCCGTTGGACCAG
1081 ---------+---------+---------+---------+---------+---------+  1140
     CTGGTCCTCCCGGGTTACCTGGTCGTCCTGGGTCTCTTGGGTCAACGGGGCAACCTGGTC

     D  Q  E  G  P  M  D  Q  Q  D  P  E  N  P  V  A  P  L  D  Q

     GCAGGCCTGATGGATCCAGTGGATCCAGAGGACTCAGTGGACCTGGGGGCTGCTGGCGCA
1141 ---------+---------+---------+---------+---------+---------+  1200
     CGTCCGGACTACCTAGGTCACCTAGGTCTCCTGAGTCACCTGGACCCCCGACGACCGCGT

     A  G  L  M  D  P  V  D  P  E  D  S  V  D  L  G  A  A  G  A
```

FIG. 1(CONTINUED 3).

```
      AGTGCTCAGCCATTACAGCCATCATCACCAGTTGCATATGACATCATTAGCCAGGAACTG
1201  ---------+---------+---------+---------+---------+---------+  1260
      TCACGAGTCGGTAATGTCGGTAGTAGTGGTCAACGTATACTGTAGTAATCGGTCCTTGAC

      S  A  Q  P  L  Q  P  S  S  P  V  A  Y  D  I  I  S  Q  E  L

      GAACTGATGAAGAAGTTGAAGGAGCAGCTAGAAGAGAGGACTTGGTTGCTGCATGATGCC
1261  ---------+---------+---------+---------+---------+---------+  1320
      CTTGACTACTTCTTCAACTTCCTCGTCGATCTTCTCTCCTGAACCAACGACGTACTACGG

      E  L  M  K  K  L  K  E  Q  L  E  E  R  T  W  L  L  H  D  A

      ATCCAAAACCAGCAGAATGCATTGGAATTGATGATGGATCACCTTCAGAAGCAGCCAAAC
1321  ---------+---------+---------+---------+---------+---------+  1380
      TAGGTTTTGGTCGTCTTACGTAACCTTAACTACTACCTAGTGGAAGTCTTCGTCGGTTTG

      I  Q  N  Q  Q  N  A  L  E  L  M  M  D  H  L  Q  K  Q  P  N

      ACATTACGCCACGTTGTCATTCCTGATCTCCAATCTTCGGAGGCAGTGCCCAAGAAACAA
1381  ---------+---------+---------+---------+---------+---------+  1440
      TGTAATGCGGTGCAACAGTAAGGACTAGAGGTTAGAAGCCTCCGTCACGGGTTCTTTGTT

      T  L  R  H  V  V  I  P  D  L  Q  S  S  E  A  V  P  K  K  Q

      CAGAAACAACACGCTGGGCAAGTGAAGCGGCCTCTCCCACATCCCAAGGACGTCAAGTGT
1441  ---------+---------+---------+---------+---------+---------+  1500
      GTCTTTGTTGTGCGACCCGTTCACTTCGCCGGAGAGGGTGTAGGGTTCCTGCAGTTCACA

      Q  K  Q  H  A  G  Q  V  K  R  P  L  P  H  P  K  D  V  K  C

      TTCTGTGGCTTATCTTTATCCAACTCTCTCAAAAACACTGGGGAGCTTCAGGAGCCTTGT
1501  ---------+---------+---------+---------+---------+---------+  1560
      AAGACACCGAATAGAAATAGGTTGAGAGAGTTTTTGTGACCCCTCGAAGTCCTCGGAACA

      F  C  G  L  S  L  S  N  S  L  K  N  T  G  E  L  Q  E  P  C

      GTTGCCTTCAACCAGCAGCAACTGGTGCAGCAAGAACAACACCTGAAGGAGCAGCAGCGG
1561  ---------+---------+---------+---------+---------+---------+  1620
      CAACGGAAGTTGGTCGTCGTTGACCACGTCGTTCTTGTTGTGGACTTCCTCGTCGTCGCC

      V  A  F  N  Q  Q  Q  L  V  Q  Q  E  Q  H  L  K  E  Q  Q  R
```

FIG. 1 (CONTINUED 4).

```
     CAGCTGCGGGAGCAGCTGCAACAGCTGAGAGAGCAAAGGAAGGTGCAGAAGCAGAAGAAG
1621 ---------+---------+---------+---------+---------+---------+ 1680
     GTCGACGCCCTCGTCGACGTTGTCGACTCTCTCGTTTCCTTCCACGTCTTCGTCTTCTTC

     Q  L  R  E  Q  L  Q  Q  L  R  E  Q  R  K  V  Q  K  Q  K  K

     ATGCAGGAGAAGAAGAAGCTGCAGGAGCAGAAAATGCAGGAGAAGAAGAAGCTGCAGGAG
1681 ---------+---------+---------+---------+---------+---------+ 1740
     TACGTCCTCTTCTTCTTCGACGTCCTCGTCTTTTACGTCCTCTTCTTCTTCGACGTCCTC

     M  Q  E  K  K  K  L  Q  E  Q  K  M  Q  E  K  K  K  L  Q  E

     CAGAGGCGGCAAAAGAAGAAGAAGCTACAGGAGCGGAAGAAGTGGCAGGGGCAGATGCTA
1741 ---------+---------+---------+---------+---------+---------+ 1800
     GTCTCCGCCGTTTTCTTCTTCTTCGATGTCCTCGCCTTCTTCACCGTCCCCGTCTACGAT

     Q  R  R  Q  K  K  K  K  L  Q  E  R  K  K  W  Q  G  Q  M  L

     CAGAAAGAGCCAGAGGAGGAGCAGCAGAAGCAGCAGCTGCAAGAGCAGCCACTGAAGCAT
1801 ---------+---------+---------+---------+---------+---------+ 1860
     GTCTTTCTCGGTCTCCTCCTCGTCGTCTTCGTCGTCGACGTTCTCGTCGGTGACTTCGTA

     Q  K  E  P  E  E  E  Q  Q  K  Q  Q  L  Q  E  Q  P  L  K  H

     AATGTCATCGTGGGGAATGAGAGGGTGCAGATATGCCTGCAAAACCCACGTGACGTATCT
1861 ---------+---------+---------+---------+---------+---------+ 1920
     TTACAGTAGCACCCCTTACTCTCCCACGTCTATACGGACGTTTTGGGTGCACTGCATAGA

     N  V  I  V  G  N  E  R  V  Q  I  C  L  Q  N  P  R  D  V  S

     GTGCCCCTCTGCAATCACCCTGTTAGATTTTTACAGGCCCAACCCATTGTTCCTGTCCAG
1921 ---------+---------+---------+---------+---------+---------+ 1980
     CACGGGGAGACGTTAGTGGGACAATCTAAAAATGTCCGGGTTGGGTAACAAGGACAGGTC

     V  P  L  C  N  H  P  V  R  F  L  Q  A  Q  P  I  V  P  V  Q
```

FIG. 1(CONTINUED 5).

```
      AGAGCAGCTGAACAACAGCCCTCTGGCTTCTATCAAGATGAAAACTGTGGGCAACAGGAA
1981  ---------+---------+---------+---------+---------+---------+  2040
      TCTCGTCGACTTGTTGTCGGGAGACCGAAGATAGTTCTACTTTTGACACCCGTTGTCCTT

      R  A  A  E  Q  Q  P  S  G  F  Y  Q  D  E  N  C  G  Q  Q  E

      GATGAGAGTCAAAGGTAAGACATGCATGGAATGGTGATAGTGGCTATGATTATTGCTTTT
2041  ---------+---------+---------+---------+---------+---------+  2100
      CTACTCTCAGTTTCCATTCTGTACGTACCTTACCACTATCACCGATACTAATAACGAAAA

      D  E  S  Q  R  *

      CCCTCATGGCTGGATCCCATGCCTGAGATCACAGACAGACGACCTATCCATGTGGCACCA
2101  ---------+---------+---------+---------+---------+---------+  2160
      GGGAGTACCGACCTAGGGTACGGACTCTAGTGTCTGTCTGCTGGATAGGTACACCGTGGT

      GCGTCCCTCTCATCAATAGCAACTTGCTCTCAACTTGCCTTCTCTGTGGCCACGGCCATC
2161  ---------+---------+---------+---------+---------+---------+  2220
      CGCAGGGAGAGTAGTTATCGTTGAACGAGAGTTGAACGGAAGAGACACCGGTGCCGGTAG

      ATCCTTCTGCATGTGTGGTGCATGGATGAGCATCCATTGATGGAACAAAGGGGTACCCAT
2221  ---------+---------+---------+---------+---------+---------+  2280
      TAGGAAGACGTACACACCACGTACCTACTCGTAGGTAACTACCTTGTTTCCCCATGGGTA

      GAAGAAAGGACTAGATAAGGAAGCCTCAATGCTAGGGAATTAGCCCACAAACCTTGAAAT
2281  ---------+---------+---------+---------+---------+---------+  2340
      CTTCTTTCCTGATCTATTCCTTCGGAGTTACGATCCCTTAATCGGGTGTTTGGAACTTTA

      CACTTGGCTGGATTCCCCAAGCCCAGTCTCTTGCACAGGGTGTCTATGGACAAGTCTTGT
2341  ---------+---------+---------+---------+---------+---------+  2400
      GTGAACCGACCTAAGGGGTTCGGGTCAGAGAACGTGTCCCACAGATACCTGTTCAGAACA

      GCCCATAGCTGAAAGACTTCTGGGCTTCCAGTTTCATTCCCCAACTTTCCTGGAATCTGG
2401  ---------+---------+---------+---------+---------+---------+  2460
      CGGGTATCGACTTTCTGAAGACCCGAAGGTCAAAGTAAGGGGTTGAAAGGACCTTAGACC
```

FIG. 1 (CONTINUED 6).

```
     TGATGGCAGTAGGCCAAGTGCAGGTATCTCCCTGGCATTTATGAACAGTAAGAGGGAAGT
2461 ---------+---------+---------+---------+---------+---------+ 2520
     ACTACCGTCATCCGGTTCACGTCCATAGAGGGACCGTAAATACTTGTCATTCTCCCTTCA

     GAGTGTGAAACAATTGCTAGATTGCTAAGATCAGCCAAGAACACATGAAGGCTAAGTCCT
2521 ---------+---------+---------+---------+---------+---------+ 2580
     CTCACACTTTGTTAACGATCTAACGATTCTAGTCGGTTCTTGTGTACTTCCGATTCAGGA

     GAGTGGCAATATAGGGACATGAACACATTTCTGCTCAATAATAAGGTCTTCAGTTCCAAA
2581 ---------+---------+---------+---------+---------+---------+ 2640
     CTCACCGTTATATCCCTGTACTTGTGTAAAGACGAGTTATTATTCCAGAAGTCAAGGTTT

     TGCTAGAAAGATAAGGTTCTTCTCAGCAATGAAAGGATTGCTTAGCACATAGTTGGGGCC
2641 ---------+---------+---------+---------+---------+---------+ 2700
     ACGATCTTTCTATTCCAAGAAGAGTCGTTACTTTCCTAACGAATCGTGTATCAACCCCGG

     CACTAAATAGTTTTGAATGAATGTTAGCTTTCTGAGACGGGCCTCCTAAAGCTCTCTGGA
2701 ---------+---------+---------+---------+---------+---------+ 2760
     GTGATTTATCAAAACTTACTTACAATCGAAAGACTCTGCCCGGAGGATTTCGAGAGACCT

     CTTCCTTCTGGGCCTTGCTTTCGAATCAGTGACTTTGACATGAAGACTGCTTTAGGGCCA
2761 ---------+---------+---------+---------+---------+---------+ 2820
     GAAGGAAGACCCGGAACGAAAGCTTAGTCACTGAAACTGTACTTCTGACGAAATCCCGGT

     TATTGTCCACTTGACCTGATCATCAGAATCCACCTGAGGAGGGTTTGGAAAGTATAGATT
2821 ---------+---------+---------+---------+---------+---------+ 2880
     ATAACAGGTGAACTGGACTAGTAGTCTTAGGTGGACTCCTCCCAAACCTTTCATATCTAA

     CAGAGTCTGGGATGGGGTTCAAAAATCTGTATTGATCACAAGACTTCCAGGCTTAGGGTT
2881 ---------+---------+---------+---------+---------+---------+ 2940
     GTCTCAGACCCTACCCCAAGTTTTTAGACATAACTAGTGTTCTGAAGGTCCGAATCCCAA
```

FIG. 1 (CONTINUED 7).

```
      GTGGAGAGAGCACTGGATTTGGAGTTTGGAAATCTGCCTTACAGTCTCTGCTCAGCCATT
2941  ---------+---------+---------+---------+---------+---------+  3000
      CACCTCTCTCGTGACCTAAACCTCAAACCTTTAGACGGAATGTCAGAGACGAGTCGGTAA

      AGCTCCCTGAGTGCTTTCAAAGTAGGTGAAATGACTTTCCAAGGCCACAGAGAGCATAGT
3001  ---------+---------+---------+---------+---------+---------+  3060
      TCGAGGGACTCACGAAAGTTTCATCCACTTTACTGAAAGGTTCCGGTGTCTCTCGTATCA

      TGTAGCAGGAACAAGACTGTCCAAGTAATATAAATAAAACCAGGTGAGACAAGGGATTGT
3061  ---------+---------+---------+---------+---------+---------+  3120
      ACATCGTCCTTGTTCTGACAGGTTCATTATATTTATTTTGGTCCACTCTGTTCCCTAACA

      AATTTCTCTCAGTGTCTACATTCACATTACTTTGACAAGTTGAATCTTAGTCAGGCCAAA
3121  ---------+---------+---------+---------+---------+---------+  3180
      TTAAAGAGAGTCACAGATGTAAGTGTAATGAAACTGTTCAACTTAGAATCAGTCCGGTTT

      GTGAGAATACAAACAAGTGAATCCTGTGGTGTAGCCACCAAAACCAAATGTGCCTGAAGC
3181  ---------+---------+---------+---------+---------+---------+  3240
      CACTCTTATGTTTGTTCACTTAGGACACCACATCGGTGGTTTTGGTTTACACGGACTTCG

      ACCTACTGATGACCACGTGAGTGTCTCATCAGTTATCATGTCCAGATCCACATCACTGCA
3241  ---------+---------+---------+---------+---------+---------+  3300
      TGGATGACTACTGGTGCACTCACAGAGTAGTCAATAGTACAGGTCTAGGTGTAGTGACGT

      ACAGCTTTCTTCTCTTACAGTTTTTATCCTGAGGCGTATCAAGGGCCCCCCGTGAACCAG
3301  ---------+---------+---------+---------+---------+---------+  3360
      TGTCGAAAGAAGAGAATGTCAAAAATAGGACTCCGCATAGTTCCCGGGGGGCACTTGGTC

      CTGCCATTGATAGATACCTCAAACTCTGAGGCAATTTCTTCTTCCAGCATTCCTCAGTTT
3361  ---------+---------+---------+---------+---------+---------+  3420
      GACGGTAACTATCTATGGAGTTTGAGACTCCGTTAAAGAAGAAGGTCGTAAGGAGTCAAA
```

FIG. 1 (CONTINUED 8).

```
      CCCATAACTTCAGACTCAACCATAAGCACCCTGGAGACCCCACAGGATTACATCCGGCTT
3421  ---------+---------+---------+---------+---------+---------+  3480
      GGGTATTGAAGTCTGAGTTGGTATTCGTGGGACCTCTGGGGTGTCCTAATGTAGGCCGAA

      TGGCAAGAGTTGTCTGATTCACTCGGTCCTGTTGTCCAAGTGAACACTTGGTCTTGCGAT
3481  ---------+---------+---------+---------+---------+---------+  3540
      ACCGTTCTCAACAGACTAAGTGAGCCAGGACAACAGGTTCACTTGTGAACCAGAACGCTA

      GAGCAGGGCACCCTGCACGGCCAACCCACCTACCATCAGGTGCAAGTTTCTGAGGTAGGA
3541  ---------+---------+---------+---------+---------+---------+  3600
      CTCGTCCCGTGGGACGTGCCGGTTGGGTGGATGGTAGTCCACGTTCAAAGACTCCATCCT

      GTCGAGGGACCTCCTGATCCACAGGCTTTCCAAGGCCCTGCTGCATACCAGCCAGACCAG
3601  ---------+---------+---------+---------+---------+---------+  3660
      CAGCTCCCTGGAGGACTAGGTGTCCGAAAGGTTCCGGGACGACGTATGGTCGGTCTGGTC

      ATGAGATCTGCGGAGCAGACCAGATTGATGCCTGCAGAGCAACGTGACTCAAATAAGCCG
3661  ---------+---------+---------+---------+---------+---------+  3720
      TACTCTAGACGCCTCGTCTGGTCTAACTACGGACGTCTCGTTGCACTGAGTTTATTCGGC

      TGCTAACAGTACTTTCATGACCAGTGATGAGGGGAAATGGGGGGAGGGGGCAGGCCAATG
3721  ---------+---------+---------+---------+---------+---------+  3780
      ACGATTGTCATGAAAGTACTGGTCACTACTCCCCTTTACCCCCCTCCCCCGTCCGGTTAC

      AGGTCTGCATGGCCAGGGGACCTTCAAGGTGCATAAAGTCCCTTGGGGTAGGGTTTAGTG
3781  ---------+---------+---------+---------+---------+---------+  3840
      TCCAGACGTACCGGTCCCCTGGAAGTTCCACGTATTTCAGGGAACCCCATCCCAAATCAC

      GGTAGAGACTTGTTTCCTGATAGGTTATGTTTGTAATTGTTTGTTAAGCACAGCCTGTTT
3841  ---------+---------+---------+---------+---------+---------+  3900
      CCATCTCTGAACAAAGGACTATCCAATACAAACATTAACAAACAATTCGTGTCGGACAAA
```

FIG. 1(CONTINUED 9).

```
     CTTGGAAGTTATGCTGTAGAGGCAGCCTGTGATCCGTAGTATGCTAGGGTGTGACAGCAG
3901 ---------+---------+---------+---------+---------+---------+ 3960
     GAACCTTCAATACGACATCTCCGTCGGACACTAGGCATCATACGATCCCACACTGTCGTC

     CCAGCCACAGCTGGATCTGATGTCTTGTCTGCCCCGCCCAGCTTTGCATATCCATGTTCT
3961 ---------+---------+---------+---------+---------+---------+ 4020
     GGTCGGTGTCGACCTAGACTACAGAACAGACGGGGCGGGTCGAAACGTATAGGTACAAGA

     ACCACAGGAAGGTGGCCTGCCAAGAGTCTGCTCAAAGTTTTCAACATAAAGAATAAAGAA
4021 ---------+---------+---------+---------+---------+---------+ 4080
     TGGTGTCCTTCCACCGGACGGTTCTCAGACGAGTTTCAAAAGTTGTATTTCTTATTTCTT

     AAAAAAATGCCAAAAAAAAAAAAAAAAAA
4081 ---------+---------+--------- 4109
     TTTTTTTACGGTTTTTTTTTTTTTTTTTT
```

FIG. 2.

```
1    MKMRGEKRRD KVNPKSSQRK LNWIPSFPTY DYFNQVTLQL LDGFMITLST
      1
                                    NR box
51   DGVIICVAEN ISSLLGHLPA EIVGKKLLSL LPDEEKDEVY QKIILKFPLL
                         2
101  NSETHIEFCC HLKRGNVEHG DSSAYENVKF IVNVRDICNE FPVVFSGLFS
151  SHLCADFAAC VPQEDRLYLV GNVCILRTQL LQQLYTSKAV SDEAVLTQDS
201  DEEPFVGELS SSQGQRGHTS MKAVYVEPAA AAAAAAISDD QIDIAEVEQY
251  GPQENVHMFV DSDSTYCSST VFLDTMPESP ALSLQDFRGE PEVNPLYRAD
301  PVDLEFSVDQ VDSVDQEGPM DQQDPENPVA PLDQAGLMDP VDPEDSVDLG
351  AAGASAQPLQ PSSPVAYDII SQELELMKKL KEQLEERTWL LHDAIQNQQN
                                         3
401  ALELMMDHLQ KQPNTLRHVV IPDLQSSEAV PKKQQKQHAG QVKRPLPHPK
                                         Leucine zipper
451  DVKCFCGLSL SNSLKNTGEL QEPCVAENQQ QLVQQEQHLK EQQRQLREQL
                              4
501  QQLREQRKVQ KQKKMQEKKK LQEQKMQEKK KLQEQRRQKK KKLQERKKWQ
                                             5
551  GQMLQKEPEE EQQKQQLQEQ PLKHNVIVGN ERVQICLQNP RDVSVPLCNH
601  PVRFLQAQPI VPVQRAAEQQ PSGFYQDENC GQQEDESQR*
```

FIG. 2 (CONTINUED).

| Key: | | |
|---|---|---|
| | 1 | ER membrane retention signal |
| | 2 | PAS domain |
| | 3 | Coiled coil region |
| | 4 | Syntaxin |
| | 5 | Nuclear localisation signal |
| | NR box | Nuclear receptor box |

FIG.3

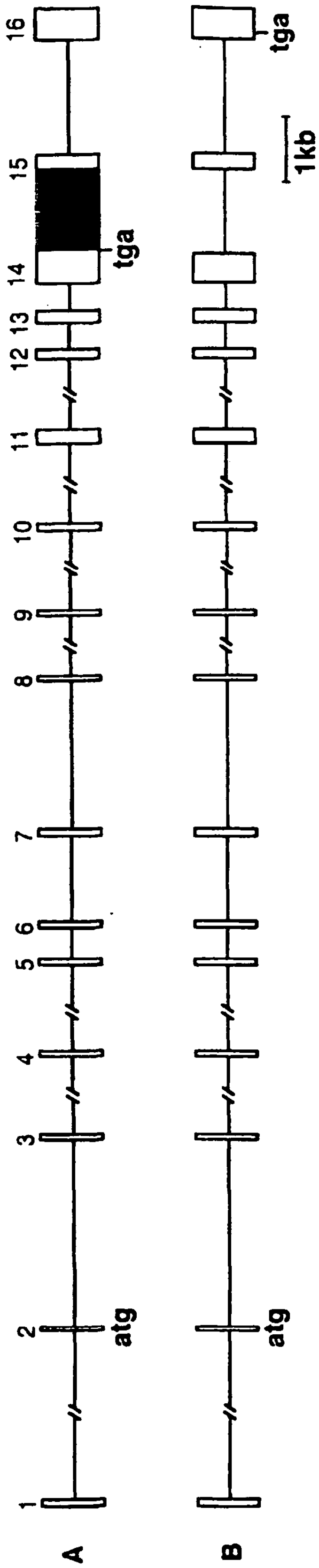

FIG. 4.

```
          1                                                50
BC040301  ATTTCCAGAC TTTCCGGGCG CCCACCAGGC TCCCGGGCTC TCACCGGAGA
OX-TES-1  ~~~~~CAGAC TTTCCGGGCG CCCACCAGGC TCCCGGGCTC TCACCGGAGA

          51                                              100
BC040301  CCACCGGAGA CCACAGGGAG GAGCTTCAAG GTACCCCAAG TCCCTGCGGC
OX-TES-1  CCACCGGAGA CCACAGGGAG GAGCTTCAAG GTACCCCAAG TCCCTGCGGC

          101                                             150
BC040301  CTCCAGCAGT GAAGAGTTCC ACAAGGGAGT CTTCCTACGT CACTGAATAA
OX-TES-1  CTCCAGCAGT GAAGAGTTCC ACAAGGGAGT CTTCCTACGT CACTGAATAA

          151                                             200
BC040301  TGAATGAAGA TGAGAGGGGA AAAGAGAAGA GACAAGGTCA ATCCAAAAAG
OX-TES-1  TGAATGAAGA TGAGAGGGGA AAAGAGAAGA GACAAAGTCA ATCCAAAAAG

          201                                             250
BC040301  CTCTCAAAGG AAATTAAACT GGATTCCATC ATTTCCTACC TATGATTACT
OX-TES-1  CTCTCAAAGG AAATTAAACT GGATTCCATC ATTTCCTACC TATGATTACT

          251                                             300
BC040301  TCAACCAAGT GACGCTACAG TTATTAGATG GCTTTATGAT TACACTGAGC
OX-TES-1  TCAACCAAGT GACGCTACAG TTATTAGATG GCTTTATGAT TACACTGAGC

          301                                             350
BC040301  ACAGATGGAG TGATCATTTG TGTGGCTGAA AACATCTCTT CTCTTCTTGG
OX-TES-1  ACAGATGGAG TGATCATTTG TGTGGCTGAA AACATCTCTT CTCTTCTTGG

          351                                             400
BC040301  ACATTTACCA GCTGAGATTG TGGGCAAAAA ATTATTAAGC CTTCTGCCTG
OX-TES-1  ACATTTACCA GCTGAGATTG TGGGCAAAAA ATTATTAAGC CTTCTGCCTG

          401                                             450
BC040301  ATGAAGAGAA AGATGAAGTC TACCAAAAGA TTATTCTCAA ATTTCCTTTA
OX-TES-1  ATGAAGAGAA AGATGAAGTC TACCAAAAGA TTATTCTCAA ATTTCCTTTA

          451                                             500
BC040301  CTAAACTCAG AAACACATAT TGAATTTTGC TGTCATTTAA AAAGAGGAAA
OX-TES-1  CTAAACTCAG AAACACATAT TGAATTTTGC TGTCATTTAA AAAGAGGAAA

          501                                             550
BC040301  TGTCGAACAT GGTGATAGTT CTGCTTACGA AAACGTGAAA TTTATTGTGA
OX-TES-1  TGTCGAACAT GGTGATAGTT CTGCTTACGA AAACGTGAAA TTTATTGTGA

          551                                             600
BC040301  ATGTAAGAGA TATTTGTAAT GAGTTTCCTG TGGTCTTTAG TGGCTTGTTT
OX-TES-1  ATGTAAGAGA TATTTGTAAT GAGTTTCCTG TGGTCTTTAG TGGCTTGTTT

          601                                             650
BC040301  TCCAGCCACC TCTGTGCTGA CTTTGCTGCA TGTGTTCCTC AGGAGGATCG
OX-TES-1  TCCAGCCACC TCTGTGCTGA CTTTGCTGCA TGTGTTCCTC AGGAGGATCG
```

FIG. 4 (CONTINUED 1).

```
          651                                                700
BC040301  GCTTTATCTT GTGGGAAATG TTTGCATTCT CAGGACTCAG CTCCTGCAGC
OX-TES-1  GCTTTATCTT GTGGGAAATG TTTGCATTCT CAGGACTCAG CTCCTGCAGC

          701                                                750
BC040301  AACTTTACAC TTCAAAGGCA GTCAGTGATG AAGCTGTACT TACACAAGAT
OX-TES-1  AACTTTACAC TTCAAAGGCA GTCAGTGATG AAGCTGTACT TACACAAGAT

          751                                                800
BC040301  TCAGATGAGG AACCTTTTGT GGGAGAGCTC AGTAGCTCTC AAGGTCAAAG
OX-TES-1  TCAGATGAGG AACCTTTTGT GGGAGAGCTC AGTAGCTCTC AAGGTCAAAG

          801                                                850
BC040301  AGGACACACT AGCATGAAAG CCGTGTACGT TGAACCCGCT GCTGCTGCTG
OX-TES-1  AGGACACACT AGCATGAAAG CCGTGTACGT TGAACCCGCT GCTGCTGCTG

          851                                                900
BC040301  CTGCTGCTGC TATCTCAGAC GACCAAATTG ATATTGCAGA GGTTGAGCAG
OX-TES-1  CTGCTGCTGC TATCTCAGAC GACCAAATTG ATATTGCAGA GGTTGAGCAG

          901                                                950
BC040301  TATGGACCAC AAGAAAACGT TCACATGTTT GTAGATTCTG ATTCAACTTA
OX-TES-1  TATGGACCAC AAGAAAACGT TCACATGTTT GTAGATTCTG ATTCAACTTA

          951                                               1000
BC040301  TTGCTCCAGT ACAGTTTTCC TGGATACTAT GCCTGAATCT CCAGCCTTAT
OX-TES-1  TTGCTCCAGT ACAGTTTTCC TGGATACTAT GCCTGAATCT CCAGCCTTAT

          1001                                              1050
BC040301  CCTTGCAAGA CTTTCGAGGT GAGCCTGAGG TGAATCCATT GTACAGGGCA
OX-TES-1  CCTTGCAAGA CTTTCGAGGT GAGCCTGAGG TGAATCCATT GTACAGGGCA

          1051                                              1100
BC040301  GACCCAGTGG ACCTGGAGTT CTCGGTGGAT CAGGTGGACT CAGTGGACCA
OX-TES-1  GACCCAGTGG ACCTGGAGTT CTCGGTGGAT CAGGTGGACT CAGTGGACCA

          1101                                              1150
BC040301  GGAGGGCCCA ATGGACCAGC AGGACCCAGA GAACCCAGTT GCCCCGTTGG
OX-TES-1  GGAGGGCCCA ATGGACCAGC AGGACCCAGA GAACCCAGTT GCCCCGTTGG

          1151                                              1200
BC040301  ACCAGGCAGG CCTGATGGAT CCAGTGGATC CAGAGGACTC AGTGGACCTG
OX-TES-1  ACCAGGCAGG CCTGATGGAT CCAGTGGATC CAGAGGACTC AGTGGACCTG

          1201                                              1250
BC040301  GGGGCTGCTG GCGCAAGTGC TCAGCCATTA CAGCCATCAT CACCAGTTGC
OX-TES-1  GGGGCTGCTG GCGCAAGTGC TCAGCCATTA CAGCCATCAT CACCAGTTGC

          1251                                              1300
BC040301  ATATGACATC ATTAGCCAGG AACTGGAACT GATGAAGAAG TTGAAGGAGC
OX-TES-1  ATATGACATC ATTAGCCAGG AACTGGAACT GATGAAGAAG TTGAAGGAGC
```

FIG. 4 (CONTINUED 2).

```
          1301                                              1350
BC040301  AGCTAGAAGA GAGGACTTGG TTGCTGCATG ATGCCATCCA AAACCAGCAG
OX-TES-1  AGCTAGAAGA GAGGACTTGG TTGCTGCATG ATGCCATCCA AAACCAGCAG

          1351                                              1400
BC040301  AATGCATTGG AATTGATGAT GGATCACCTT CAGAAGCAGC CAAACACATT
OX-TES-1  AATGCATTGG AATTGATGAT GGATCACCTT CAGAAGCAGC CAAACACATT

          1401                                              1450
BC040301  ACGCCACGTT GTCATTCCTG ATCTCCAATC TTCGGAGGCA GTGCCCAAGA
OX-TES-1  ACGCCACGTT GTCATTCCTG ATCTCCAATC TTCGGAGGCA GTGCCCAAGA

          1451                                              1500
BC040301  AACAACAGAA ACAACACGCT GGGCAAGTGA AGCGGCCTCT CCCACATCCC
OX-TES-1  AACAACAGAA ACAACACGCT GGGCAAGTGA AGCGGCCTCT CCCACATCCC

          1501                                              1550
BC040301  AAGGACGTCA AGTGTTTCTG TGGTTTATCT TTATCCAACT CTCTCAAAAA
OX-TES-1  AAGGACGTCA AGTGTTTCTG TGGCTTATCT TTATCCAACT CTCTCAAAAA

          1551                                              1600
BC040301  CACTGGGGAG CTTCAGGAGC CTTGTGTTGC CTTCAACCAG CAGCAACTGG
OX-TES-1  CACTGGGGAG CTTCAGGAGC CTTGTGTTGC CTTCAACCAG CAGCAACTGG

          1601                                              1650
BC040301  TGCAGCAAGA ACAACACCTG AAGGAGCAGC AGCGGCAGCT GCGGGAGCAG
OX-TES-1  TGCAGCAAGA ACAACACCTG AAGGAGCAGC AGCGGCAGCT GCGGGAGCAG

          1651                                              1700
BC040301  CTGCAACAGC TGAGAGAGCA AAGGAAGGTG CAGAAGCAGA AGAAGATGCA
OX-TES-1  CTGCAACAGC TGAGAGAGCA AAGGAAGGTG CAGAAGCAGA AGAAGATGCA

          1701                                              1750
BC040301  GGAGAAGAAG AAGCTGCAGG AGCAGAAAAT GCAGGAGAAG AAGAAGCTGC
OX-TES-1  GGAGAAGAAG AAGCTGCAGG AGCAGAAAAT GCAGGAGAAG AAGAAGCTGC

          1751                                              1800
BC040301  AGGAGCAGAG GCGGCAAAAG AAGAAGAAGC TACAGGAGCG GAAGAAGTGG
OX-TES-1  AGGAGCAGAG GCGGCAAAAG AAGAAGAAGC TACAGGAGCG GAAGAAGTGG

          1801                                              1850
BC040301  CAGGGGCAGA TGCTACAGAA AGAGCCAGAG GAGGAGCAGC AGAAGCAGCA
OX-TES-1  CAGGGGCAGA TGCTACAGAA AGAGCCAGAG GAGGAGCAGC AGAAGCAGCA

          1851                                              1900
BC040301  GCTGCAAGAG CAGCCACTGA AGCATAATGT CATCGTGGGG AATGAGAGGG
OX-TES-1  GCTGCAAGAG CAGCCACTGA AGCATAATGT CATCGTGGGG AATGAGAGGG

          1901                                              1950
BC040301  TGCAGATATG CCTGCAAAAC CCACGTGACG TATCTGTGCC CCTCTGCAAT
OX-TES-1  TGCAGATATG CCTGCAAAAC CCACGTGACG TATCTGTGCC CCTCTGCAAT
```

FIG. 4(CONTINUED 3).

```
            1951                                               2000
BC040301    CACCCTGTTA GATTTTTACA GGCCCAACCC ATTGTTCCTG TCCAGAGAGC
OX-TES-1    CACCCTGTTA GATTTTTACA GGCCCAACCC ATTGTTCCTG TCCAGAGAGC

            2001                                               2050
BC040301    AGCTGAACAA CAGCCCTCTG GCTTCTATCA AGATGAAAAC TGTGGGCAAC
OX-TES-1    AGCTGAACAA CAGCCCTCTG GCTTCTATCA AGATGAAAAC TGTGGGCAAC

            2051                                               2100
BC040301    AGGAAGATGA GAGTCAA... .......... .......... ..........
OX-TES-1    AGGAAGATGA GAGTCAAAGG TAAGACATGC ATGGAATGGT GATAGTGGCT

            2101                                               2150
BC040301    .......... .......... .......... .......... ..........
OX-TES-1    ATGATTATTG CTTTTCCCTC ATGGCTGGAT CCCATGCCTG AGATCACAGA

            2151                                               2200
BC040301    .......... .......... .......... .......... ..........
OX-TES-1    CAGACGACCT ATCCATGTGG CACCAGCGTC CCTCTCATCA ATAGCAACTT

            2201                                               2250
BC040301    .......... .......... .......... .......... ..........
OX-TES-1    GCTCTCAACT TGCCTTCTCT GTGGCCACGG CCATCATCCT TCTGCATGTG

            2251                                               2300
BC040301    .......... .......... .......... .......... ..........
OX-TES-1    TGGTGCATGG ATGAGCATCC ATTGATGGAA CAAAGGGGTA CCCATGAAGA

            2301                                               2350
BC040301    .......... .......... .......... .......... ..........
OX-TES-1    AAGGACTAGA TAAGGAAGCC TCAATGCTAG GGAATTAGCC CACAAACCTT

            2351                                               2400
BC040301    .......... .......... .......... .......... ..........
OX-TES-1    GAAATCACTT GGCTGGATTC CCCAAGCCCA GTCTCTTGCA CAGGGTGTCT

            2401                                               2450
BC040301    .......... .......... .......... .......... ..........
OX-TES-1    ATGGACAAGT CTTGTGCCCA TAGCTGAAAG ACTTCTGGGC TTCCAGTTTC

            2451                                               2500
BC040301    .......... .......... .......... .......... ..........
OX-TES-1    ATTCCCCAAC TTTCCTGGAA TCTGGTGATG GCAGTAGGCC AAGTGCAGGT

            2501                                               2550
BC040301    .......... .......... .......... .......... ..........
OX-TES-1    ATCTCCCTGG CATTTATGAA CAGTAAGAGG GAAGTGAGTG TGAAACAATT

            2551                                               2600
BC040301    .......... .......... .......... .......... ..........
OX-TES-1    GCTAGATTGC TAAGATCAGC CAAGAACACA TGAAGGCTAA GTCCTGAGTG
```

FIG. 4 (CONTINUED 4).

```
          2601                                               2650
BC040301  .......... .......... .......... .......... ..........
OX-TES-1  GCAATATAGG GACATGAACA CATTTCTGCT CAATAATAAG GTCTTCAGTT

          2651                                               2700
BC040301  .......... .......... .......... .......... ..........
OX-TES-1  CCAAATGCTA GAAAGATAAG GTTCTTCTCA GCAATGAAAG GATTGCTTAG

          2701                                               2750
BC040301  .......... .......... .......... .......... ..........
OX-TES-1  CACATAGTTG GGGCCCACTA AATAGTTTTG AATGAATGTT AGCTTTCTGA

          2751                                               2800
BC040301  .......... .......... .......... .......... ..........
OX-TES-1  GACGGGCCTC CTAAAGCTCT CTGGACTTCC TTCTGGGCCT TGCTTTCGAA

          2801                                               2850
BC040301  .......... .......... .......... .......... ..........
OX-TES-1  TCAGTGACTT TGACATGAAG ACTGCTTTAG GGCCATATTG TCCACTTGAC

          2851                                               2900
BC040301  .......... .......... .......... .......... ..........
OX-TES-1  CTGATCATCA GAATCCACCT GAGGAGGGTT TGGAAAGTAT AGATTCAGAG

          2901                                               2950
BC040301  .......... .......... .......... .......... ..........
OX-TES-1  TCTGGGATGG GGTTCAAAAA TCTGTATTGA TCACAAGACT TCCAGGCTTA

          2951                                               3000
BC040301  .......... .......... .......... .......... ..........
OX-TES-1  GGGTTGTGGA GAGAGCACTG GATTTGGAGT TTGGAAATCT GCCTTACAGT

          3001                                               3050
BC040301  .......... .......... .......... .......... ..........
OX-TES-1  CTCTGCTCAG CCATTAGCTC CCTGAGTGCT TTCAAAGTAG GTGAAATGAC

          3051                                               3100
BC040301  .......... .......... .......... .......... ..........
OX-TES-1  TTTCCAAGGC CACAGAGAGC ATAGTTGTAG CAGGAACAAG ACTGTCCAAG

          3101                                               3150
BC040301  .......... .......... .......... .......... ..........
OX-TES-1  TAATATAAAT AAAACCAGGT GAGACAAGGG ATTGTAATTTC TCTCAGTGT

          3151                                               3200
BC040301  .......... .......... .......... .......... ..........
OX-TES-1  CTACATTCAC ATTACTTTGA CAAGTTGAAT CTTAGTCAGG CCAAAGTGAG

          3201                                               3250
BC040301  .......... .......... .......... .......... ..........
OX-TES-1  AATACAAACA AGTGAATCCT GTGGTGTAGC CACCAAAACC AAATGTGCCT
```

FIG. 4 (CONTINUED 5).

```
          3251                                               3300
BC040301  .......... .......... .......... .......... ..........
OX-TES-1  GAAGCACCTA CTGATGACCA CGTGAGTGTC TCATCAGTTA TCATGTCCAG

          3301                                               3350
BC040301  .......... .......... .......... ...AGTTTTT ATCCTGAGGC
OX-TES-1  ATCCACATCA CTGCAACAGC TTTCTTCTCT TACAGTTTTT ATCCTGAGGC

          3351                                               3400
BC040301  GTATCAAGGG CCCCCCGTGA ACCAGCTGCC ATTGATAGAT ACCTCAAACT
OX-TES-1  GTATCAAGGG CCCCCCGTGA ACCAGCTGCC ATTGATAGAT ACCTCAAACT

          3401                                               3450
BC040301  CTGAGGCAAT TTCTTCTTCC AGCATTCCTC AGTTTCCCAT AACTTCAGAC
OX-TES-1  CTGAGGCAAT TTCTTCTTCC AGCATTCCTC AGTTTCCCAT AACTTCAGAC

          3451                                               3500
BC040301  TCAACCATAA GCACCCTGGA GACCCCACAG GATTACATCC GGCTTTGGCA
OX-TES-1  TCAACCATAA GCACCCTGGA GACCCCACAG GATTACATCC GGCTTTGGCA

          3501                                               3550
BC040301  AGAGTTGTCT GATTCACTCG GTCCTGTTGT CCAAGTGAAC ACTTGGTCTT
OX-TES-1  AGAGTTGTCT GATTCACTCG GTCCTGTTGT CCAAGTGAAC ACTTGGTCTT

          3551                                               3600
BC040301  GCGATGAGCA GGGCACCCTG CACGGCCAAC CCACCTACCA TCAGGTGCAA
OX-TES-1  GCGATGAGCA GGGCACCCTG CACGGCCAAC CCACCTACCA TCAGGTGCAA

          3601                                               3650
BC040301  GTTTCTGAGG TAGGAGTCGA GGGACCTCCT GATCCACAGG CTTTCCAAGG
OX-TES-1  GTTTCTGAGG TAGGAGTCGA GGGACCTCCT GATCCACAGG CTTTCCAAGG

          3651                                               3700
BC040301  CCCTGCTGCA TACCAGCCAG ACCAGATGAG ATCTGCGGAG CAGACCAGAT
OX-TES-1  CCCTGCTGCA TACCAGCCAG ACCAGATGAG ATCTGCGGAG CAGACCAGAT

          3701                                               3750
BC040301  TGATGCCTGC AGAGCAACGT GACTCAAATA AGCCGTGCTA ACAGTACTTT
OX-TES-1  TGATGCCTGC AGAGCAACGT GACTCAAATA AGCCGTGCTA ACAGTACTTT

          3751                                               3800
BC040301  CATGACCAGT GATGAGGGGA AATGGGGGGA GGGGGCAGGC CAATGAGGTC
OX-TES-1  CATGACCAGT GATGAGGGGA AATGGGGGGA GGGGGCAGGC CAATGAGGTC

          3801                                               3850
BC040301  TGCATGGCCA GGGGACCTTC AAGGTGCGTA AAGTCCCTTG GGGTAGGGTT
OX-TES-1  TGCATGGCCA GGGGACCTTC AAGGTGCATA AAGTCCCTTG GGGTAGGGTT

          3851                                               3900
BC040301  TAGTGGGTAG AGACTTATTT GTTTCCTGAT AGGTTATGTT TGTAATTGTT
OX-TES-1  TAGTGGGTAG AGACTT.... GTTTCCTGAT AGGTTATGTT TGTAATTGTT
```

FIG. 4 (CONTINUED 6).

```
          3901                                               3950
BC040301  TGTTAAGCAC AGCCTGTTTC TTGGAAGTTA TGCTGTAGAG GCAGCCTGTG
OX-TES-1  TGTTAAGCAC AGCCTGTTTC TTGGAAGTTA TGCTGTAGAG GCAGCCTGTG

          3951                                               4000
BC040301  ATCCGTAGTA TGCTAGGGTG TGACAGCAGC CAGCCACAGC TGGATCTGAT
OX-TES-1  ATCCGTAGTA TGCTAGGGTG TGACAGCAGC CAGCCACAGC TGGATCTGAT

          4001                                               4050
BC040301  GTCTTGTCTG CCCCGCCCAG CTTTGCATAT CCATGTTCTA CCACAGGAAG
OX-TES-1  GTCTTGTCTG CCCCGCCCAG CTTTGCATAT CCATGTTCTA CCACAGGAAG

          4051                                               4100
BC040301  GTGGCCTGCC AAGAGTCTGC TCAAAGTTTT CAACATAAAG AATAAAGAAA
OX-TES-1  GTGGCCTGCC AAGAGTCTGC TCAAAGTTTT CAACATAAAG AATAAAGAAA

          4101                          4129
BC040301  AAAAAAAATGC AAAAAAAAAA AAAAAAAAA
OX-TES-1  AAAAAAAATGC CAAAAAAAAA AAAAAAAAA
```

FIG. 5.

| | 1 | | | | 50 |
|---|---|---|---|---|---|
| OX-TES-1 | MKMRGEKRRD | KVNPKSSQRK | LNWIPSFPTY | DYFNQVTLQL | LDGFMITLST |
| BC040301 | MKMRGEKRRD | KVNPKSSQRK | LNWIPSFPTY | DYFNQVTLQL | LDGFMITLST |
| | 51 | | | | 100 |
| OX-TES-1 | DGVIICVAEN | ISSLLGHLPA | EIVGKKLLSL | LPDEEKDEVY | QKIILKFPLL |
| BC040301 | DGVIICVAEN | ISSLLGHLPA | EIVGKKLLSL | LPDEEKDEVY | QKIILKFPLL |
| | 101 | | | | 150 |
| OX-TES-1 | NSETHIEFCC | HLKRGNVEHG | DSSAYENVKF | IVNVRDICNE | FPVVFSGLFS |
| BC040301 | NSETHIEFCC | HLKRGNVEHG | DSSAYENVKF | IVNVRDICNE | FPVVFSGLFS |
| | 151 | | | | 200 |
| OX-TES-1 | SHLCADFAAC | VPQEDRLYLV | GNVCILRTQL | LQQLYTSKAV | SDEAVLTQDS |
| BC040301 | SHLCADFAAC | VPQEDRLYLV | GNVCILRTQL | LQQLYTSKAV | SDEAVLTQDS |
| | 201 | | | | 250 |
| OX-TES-1 | DEEPFVGELS | SSQGQRGHTS | MKAVYVEPAA | AAAAAAISDD | QIDIAEVEQY |
| BC040301 | DEEPFVGELS | SSQGQRGHTS | MKAVYVEPAA | AAAAAAISDD | QIDIAEVEQY |
| | 251 | | | | 300 |
| OX-TES-1 | GPQENVHMFV | DSDSTYCSST | VFLDTMPESP | ALSLQDFRGE | PEVNPLYRAD |
| BC040301 | GPQENVHMFV | DSDSTYCSST | VFLDTMPESP | ALSLQDFRGE | PEVNPLYRAD |
| | 301 | | | | 350 |
| OX-TES-1 | PVDLEFSVDQ | VDSVDQEGPM | DQQDPENPVA | PLDQAGLMDP | VDPEDSVDLG |
| BC040301 | PVDLEFSVDQ | VDSVDQEGPM | DQQDPENPVA | PLDQAGLMDP | VDPEDSVDLG |
| | 351 | | | | 400 |
| OX-TES-1 | AAGASAQPLQ | PSSPVAYDII | SQELELMKKL | KEQLEERTWL | LHDAIQNQQN |
| BC040301 | AAGASAQPLQ | PSSPVAYDII | SQELELMKKL | KEQLEERTWL | LHDAIQNQQN |
| | 401 | | | | 450 |
| OX-TES-1 | ALELMMDHLQ | KQPNTLRHVV | IPDLQSSEAV | PKKQQKQHAG | QVKRPLPHPK |
| BC040301 | ALELMMDHLQ | KQPNTLRHVV | IPDLQSSEAV | PKKQQKQHAG | QVKRPLPHPK |
| | 451 | | | | 500 |
| OX-TES-1 | DVKCFCGLSL | SNSLKNTGEL | QEPCVAFNQQ | QLVQQEQHLK | EQQRQLREQL |
| BC040301 | DVKCFCGLSL | SNSLKNTGEL | QEPCVAFNQQ | QLVQQEQHLK | EQQRQLREQL |
| | 501 | | | | 550 |
| OX-TES-1 | QQLREQRKVQ | KQKKMQEKKK | LQEQKMQEKK | KLQEQRRQKK | KKLQERKKWQ |
| BC040301 | QQLREQRKVQ | KQKKMQEKKK | LQEQKMQEKK | KLQEQRRQKK | KKLQERKKWQ |
| | 551 | | | | 600 |
| OX-TES-1 | GQMLQKEPEE | EQQKQQLQEQ | PLKHNVIVGN | ERVQICLQNP | RDVSVPLCNH |
| BC040301 | GQMLQKEPEE | EQQKQQLQEQ | PLKHNVIVGN | ERVQICLQNP | RDVSVPLCNH |
| | 601 | | | | 650 |
| OX-TES-1 | PVRFLQAQPI | VPVQRAAEQQ | PSGFYQDENC | GQQEDESQR* | ~~~~~~~~~~ |
| BC040301 | PVRFLQAQPI | VPVQRAAEQQ | PSGFYQDENC | GQQEDESQSF | YPEAYQGPPV |

FIG. 5(CONTINUED)

```
          651                                                  700
OX-TES-1  ~~~~~~~~~~ ~~~~~~~~~~ ~~~~~~~~~~ ~~~~~~~~~~ ~~~~~~~~~~
BC040301  NQLPLIDTSN SEAISSSSIP QFPITSDSTI STLETPQDYI RLWQELSDSL

          701                                                  750
OX-TES-1  ~~~~~~~~~~ ~~~~~~~~~~ ~~~~~~~~~~ ~~~~~~~~~~ ~~~~~~~~~~
BC040301  GPVVQVNTWS CDEQGTLHGQ PTYHQVQVSE VGVEGPPDPQ AFQGPAAYQP

          751                   773
OX-TES-1  ~~~~~~~~~~ ~~~~~~~~~~ ~~~
BC040301  DQMRSAEQTR LMPAEQRDSN KPC
```

FIG. 6.

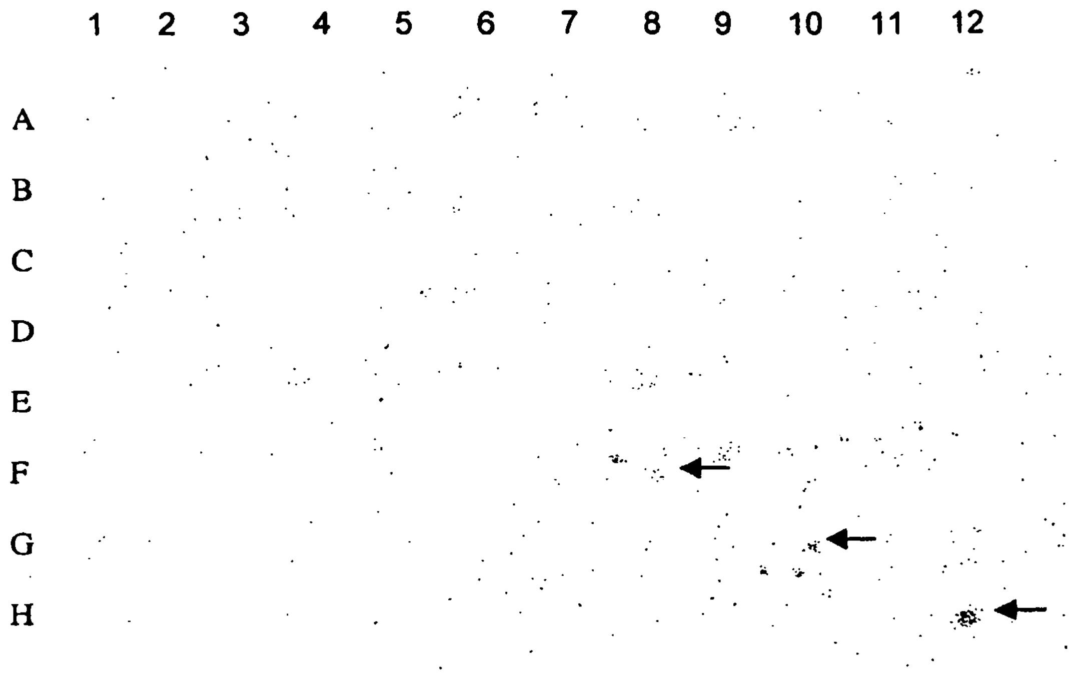


| | 1 | 2 | 3 | 4 | 5 | 6 | 7 | 8 | 9 | 10 | 11 | 12 |
|---|---|---|---|---|---|---|---|---|---|---|---|---|
| A | whole brain | cerebellum, left | substantia nigra | heart | esophagus | colon, transverse | kidney | lung | liver | leukemia, HL-60 | fetal brain | yeast total RNA |
| B | cerebral cortex | cerebellum, right | accumbens nucleus | aorta | stomach | colon, desending | skeletal muscle | placenta | pancreas | HeLa S3 | fetal heart | yeast tRNA |
| C | frontal lobe | corpus callosum | thalamus | atrium, left | duodenum | rectum | spleen | bladder | adrenal gland | leukemia, K-562 | fetal kidney | *E. coli* rRNA |
| D | parietal lobe | amygdala | pituitary gland | atrium, right | jejunum | | thymus | uterus | thyroid gland | leukemia, MOLT-4 | fetal liver | *E. coli* DNA |
| E | occipital lobe | caudate nucleus | spinal cord | ventricle, left | ileum | | peripheral blood leukocyte | prostate | salivary gland | Burkitt's lymphoma, Raji | fetal spleen | Poly r(A) |
| F | temporal lobe | hippo-campus | | ventricle, right | ilocecum | | lymph node | testis | mammary gland | Burkitt's lymphoma, Daudi | fetal thymus | human $C_0t$-1 DNA |
| G | p. g.* of cerebral cortex | medulla oblongata | | inter-ventricular septum | appendix | | bone marrow | ovary | | colorectal adeno-carcinoma, SW480 | fetal lung | human DNA 100 ng |
| H | pons | putamen | | apex of the heart | colon, ascending | | trachea | | | lung carcinoma, A549 | | human DNA 500 ng |

* paracentral gyrus

FIG. 7.

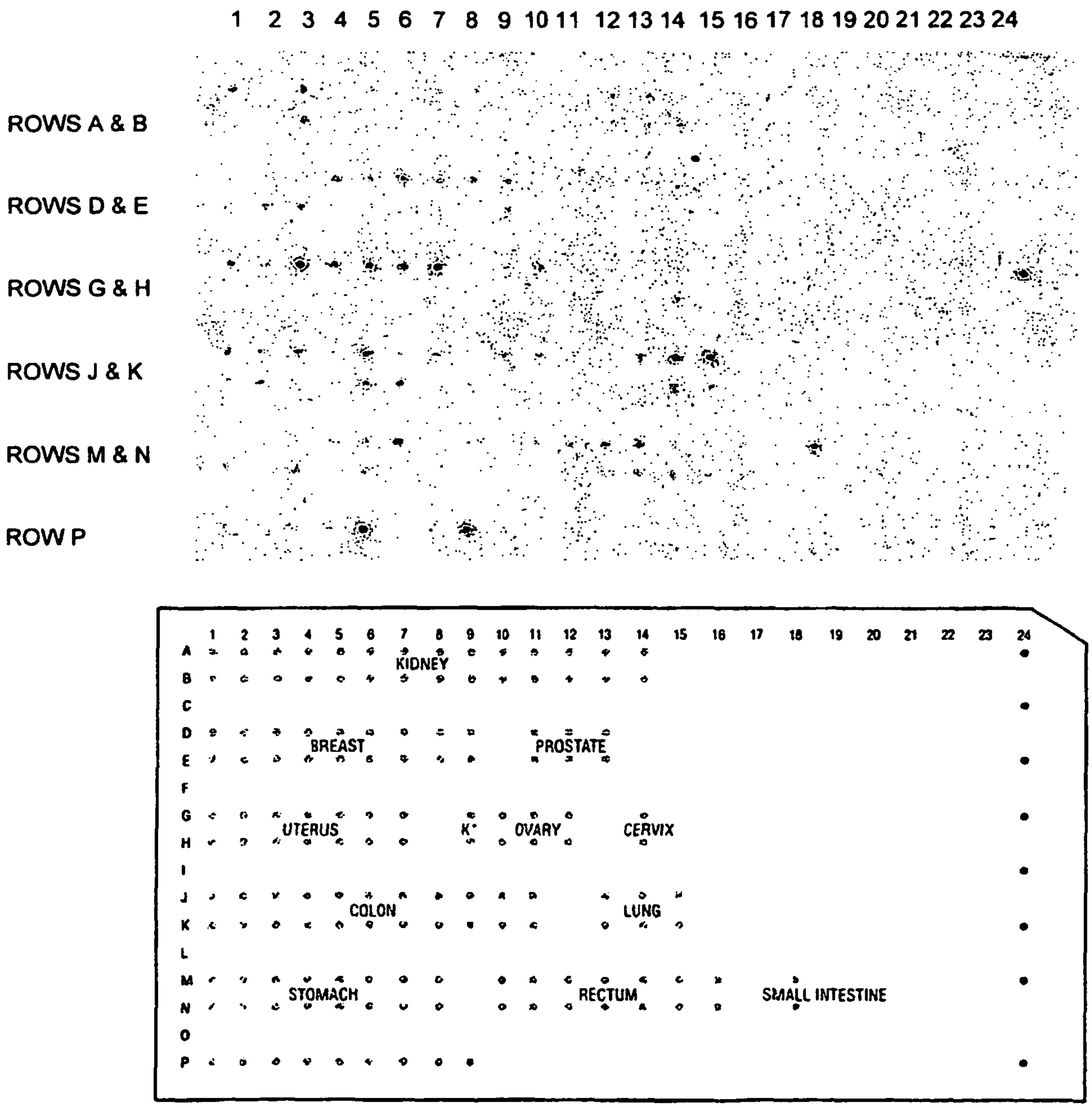
1 2 3 4 5 6 7 8 9 10 11 12 13 14 15 16 17 18 19 20 21 22 23 24
ROWS A & B
ROWS D & E
ROWS G & H
ROWS J & K
ROWS M & N
ROW P
KIDNEY
BREAST
PROSTATE
UTERUS
K
OVARY
CERVIX
COLON
LUNG
STOMACH
RECTUM
SMALL INTESTINE

FIG. 8.

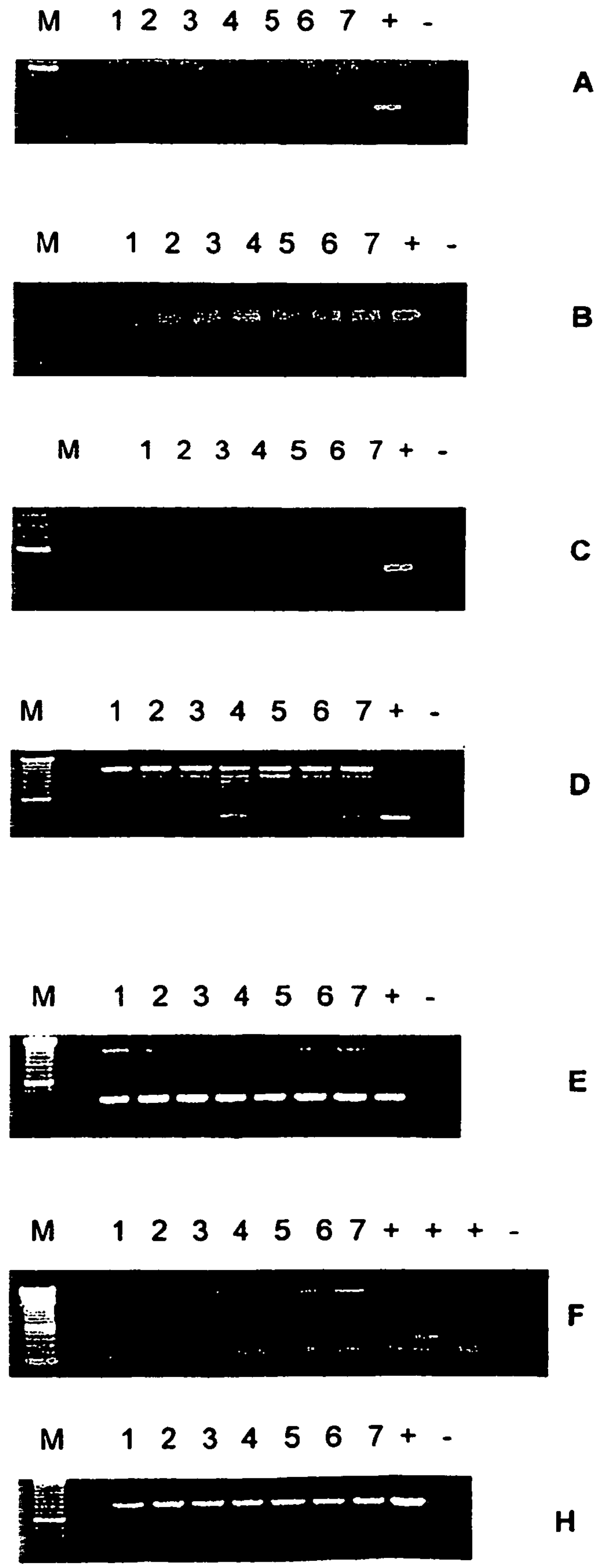
M 1 2 3 4 5 6 7 + -
A
M 1 2 3 4 5 6 7 + -
B
M 1 2 3 4 5 6 7 + -
C
M 1 2 3 4 5 6 7 + -
D
M 1 2 3 4 5 6 7 + -
E
M 1 2 3 4 5 6 7 + + + -
F
M 1 2 3 4 5 6 7 + -
H

## 2. BAC1

FIG. 9.

A) cDNA sequence

GCTAAATTGT GTTTCCCCCC ACCGAAATTC ATGCGTTGAA TCCTAATCCC
CAGTACCTCA AAATGTGACT GTATTTGGAG GTAGAAACTT TAAAAGGTGA
TTAAGTTAAA ATGAGGTCAT TAGGGTGGGC CCTAATCCAA TCTGACTGGT
GTCTTTATGA GAAGAGGAGA TTAGGCCACA TGGAGAAACC AGGGATGCCT
GCACACAGAG AAAAGACCTT TTGAGGATGT AGTGAGAAGA TACCATCTGC
AAGGCAAGGA AAAGGGCCTC AGAAGAAACC AAACTCACAA GAAAGCCAAC
ACCTTGATCT TAGACTTCCA GCCTCCAGAA TTGTGAGAAA ACACATTTCT
GTTTAAGCCA CCCAATCTGT GGTGTTTTGT GGTGGCAGCC CTAGCAAACT
AATACAGGTA GCTACTGTGA ATGGGAACTT CCCGTATTAT ATATTACATC
TCTAACCAGT TATTTGTTTT GTAGGAAATA TGATGATTTT GTAGTTTATT
TTCCTGGATT TTCTGGATGG AAAAATGTTT TTATCTGCAA ATGATCACAG
TTTTGCTGCC CACTTTCTAG TCATTTTGCC TTTTGTTTTT TTTTCTTTTC
TTTTTTTTTT TTTTTTTTGA GACAAGAGTC TCGCTCTGTC ACCCAGGCTG
GAGTGCAGTG GTGTGATCTC GGCTCACTGC AACCTCCATC TCCTGGGTTC
AAGCGATTCT TCTGCCTCAG CCTCCTGAGT AGCTGGGACT ACAGGCACAT
GCCACCATGC CCGGCTAATT TTTGTATTTT TAGTAGAGAT GGGGTTTCAC
CATGTTGGCC AGGATGGTCT CGATCTCCTG ACCTCGTGAT CCACATGCCT
CGGCCTCACA AAGTGCTGGG ATTACAGGCA TGAGCCACCA CTCCTGGCCT
TTTATTTCTT TTTAAATCCT TGCTCATATA TCTTTGAGTA TATCTGTGAA
TATTTCTATC AGACAAATTC ATAAAAGTTA TTACTAGTTT GTAATGTTCC
TTTTTCTAAA GAATGGGCAT GCAGTTTTAT TAAATATCTT TTAGTGTCCA
TTGAGATGAC CATTTTTTTC CCTTTATTCT GTTTTTTTAT ATGTATTACA
TTAGCCCATC TCCAATATTA AACTAGCTTT GTGTTTCTGG GATAAAAACT
CCTTTGACAT TTGACGTGTG CTGACACTGA CATTCTTTGA GTAAATAAAA
TAAAAAAACAG GAGTATACCA GTGTACACCA ACATACCCTG CTGCTTTATT
CGGGTTACTG ACTATACTTT CTGTCTGAAA TTGTCTGCTG TGTCCTCAGT
AGATGACTCT ATTTAGTAAC TACAAACCCT GAGTCAGGCT TTCAGAATGC
CCTTCTTTTT ATTTATTAGT AGAGATGAGA TCTTGCTATG TTGCCAGCGC
TGGTCTCGAA CTCCTGGGCT CAAGTAATCC TCTCTCCTCA GCCTCCCAAA
GTGCTGGGAT TACAGGCATG AGCCACTGTT CCTGGCCAGA GTGACCTTCT
AATGGTGCTT CCATGGACCT CTCCTTTGCT CCTATTTTCT TTCTCTTGCT
TTTGGAGCTG CTCCTTTAGA GGTGAGACCA CCCTTCAAGT GGGTGAAATC
AGCTGTGAGT CTCTCATTGC TTCTGTGTAC TTCCCACAGC CTAGTAAATT
TTCTCTTTGC TCCTCAAGCC CTGGAACATT TGGGCCTCCA CCCACCTGTC
TTACTCTCTG CGGAGAGTCT TGCTTCATAT TTAGAGTCAG ATTAAGACCA
GTGGAAATGA ACTGCCTTAC ATTTTCTCTG TTTCAGCATA TCTTCCTCTG
TCACCTATTT TCTCCAGGTT TGGATAAAAG GTAGTCTCTC CTGATAATTC
TCAACACCTG ATGGACACCT ATTCCAGGAG AACACTCTGC ATCCTGAAGC
TCATTCTGTC CTAAATGGAT CCCATATCTC CTCTCAACGC AGAGACAAAA
ACAAACATTC CTTAGGCCTA GGACCATCAC TTCCCTTGTG TCTGTTCATG
GACCACAATA CCTTGCCTGC TTCAATGGCT GCAAGTGTTT ATCATCTCTG

FIG. 9 (CONTINUED).

TCTTGTCTGG GCCCTGCATA CAAGTGACCT GCCCTCATAT CCAACACTCC
AGTTTAGACT CCAGTAACCT CTTACTGGTG GGTTCTAATA ACCTTTTAAT
AACTTCCCTC TATCTTGCCC CTTGCTTTAT ATTGAGATCA TATTCATTGT
TTCCCCTCCA TCTTGCTGCC TGGATCATGA ATGATGTCTA TACCACAGGG
ACCAGGATGC CATCTAGGGA CAATGGAGCA ATGAGCTGGG AGGAACCTGG
ATCCTGAACA TTGTGTGGAG TACAGCTGTC ATAAGGCTGA GGTTGCATTG
TGTGTGTGTG TGTGTGTGTG TGAGTGAGTG TGTGTGTGTG TGTGTTGTTT
ACTTTAACCT GAGCTTATGA GAGCTCTGGG TAATGTAAAT AGAATAGCTC
CTTTAGGAAG TGTCTTAGTC ATTTTGGGCT GCTATAACAA AAATGCTGTA
TACTGGTGGC TTAAATAACT ATTTATCATA ATTTTGGAGG CTGGGAAGTC
CAAGATCCAG ACACTGGCAG ATCTGGCATC AGGTGGGGGC CTGCTTCCTG
GCTTATAGAC AGTTGCCTTC TTGCTGTATC TTCACATAGC TGATGGAGAG
ATCATTTTGC TCTTATGTCT TCTTATAAGG GCACTCATCC CATTTGTGAA
GGCTCCACCC TCATGACCTG ATTACCTCCC CAAAGCACTA CCTGCTAGTA
CCATCACATT GGGGATTAGG CTTCAACCTA TACATTTTAT GAGGAAATGA
ACAATCAGTC CATATCAGGG AGAAATAAAT ATATGTAACT ATAATCACAA
GTTAAGTAGT TAAAATAAAA AATATCAATA TCTTATTCTG TGTGTGGCCC
TGCCAATTTA CTAAAAGCTA TCATAAAGGA CACTGATCAT AAAACTTAGC
CAATCATTTT AGCCTGCACA AATTCTATAC GGTATTTGAA ATAATCAACC
AAACAATAAT TCTTCAAACT TTGTTTCGTT TTGTTTTGTT TTGAGACAGT
TTCGCTCTGT CGCCCAGGCT GGAGTGCAGT GGCACAATCT CGGTTCACTG
CAACCTCTGC CTCCCGGGTT CAAGCAGTTC TCTTGCCTCA GCCTCCCAAG
TAACTGGTAC TACAGTTTGT CCTCGGCTGT TACAGAGAAT TGATACAAAA
AATGATCATG TGAAGAATTT GCATCTTCTG CAGAACTACC GGTATCAGTC
AGCAGTAGGA GATTGCCAGA CACGGTGGCT CACGCCTATA ATCCTAGCAC
TTTGGGAGGC TGAGGTGTGT GGATCACTTG AGCCCAGGAG TTCAAGACCA
GCCTGAGCAA CATGGTGAAA CCCCATCTCT GCAAAAAATA CAAAAATTAG
CCAGGCGTGG TGGTGTGTAC CTGTGGTCCC AGCTGCTGGG AAGGCTGAGG
TGGGAGGAAC ATTTGAGCCC AGAAGTTGAG CCGTGATCAC GCCACTGCAC
TCCAGCCTGG ATGACACAGC AAGACCTTGT CTCAAAAAAT AAAATAAAAT
AAATTTTAAA AAAAAAAAAA AAAAAAA

FIG. 10.

B) Longest predicted protein product

MVLPWTSPLL LFSFSCFWSC SFRGETTLQV GEISCESLIA SVYFPQPSKF
SLCSSSPGTF GPPPTCLTLC GESCFIFRVR LRPVEMNCLT FSLFQHIFLC
HLFSPGLDKR

FIG. 11.

A) TA195 cDNA sequence

CTCGTGCCGA ATTCGGCACG AGGAAGATGG CGCCTGTTGT GACAGGGGAA
GCTATGCGAA ATTATTTAAA AGAGCGAGGG GATCAAACAG TACTTATTCT
TCATGCAAAA GTTGCACAGA AGTCATATGG AAATGAAAAA AGGTTTTTTT
GCCCACCTCC TTGTGTATAT CTTATGGGCA GTGGATGGAA GAAAAAAAAA
GAACAAATGG AACGCGATGG TTGTTCTGAA CAAGAGTCTC AACCGTGTGC
ATTTATTGGG ATAGGAAATA GTGACCAAGA AATGCAGCAG CTAAACTTGG
AAGGAAAGAA CTATTGCACA GCCAAAACAT TGTATATATC TGACTCAGAC
AAGCGAAAGC ACTTCATGTT GTCTGTAAAG ATGTTCTATG GCAACAGTGA
TGACATTGGT GTGTTCCTCA GCAAGCGGAT AAAAGTCATC TCCAAACCTT
CCAAAAAGAA GCAGTCATTG AAAAATGCTG ACTTATGCAT TGCCTCAGGA
ACAAAGGTGG CTCTGTTTAA TCGACTACGA TCCCAGACAG TTAGTACCAG
ATACTTGCAT GTAGAAGGAG GTAATTTTCA TGCCAGTTCA CAGCAGTGGG
GAGCCTTTTT TATTCATCTC TTGGATGATG ATGAATCAGA AGGAGAAGAA
TTCACAGTCC GAGATGGCTA CATCCATTAT GGACAAACAG TCAAACTTGT
GTGCTCAGTT ACTGGCATGG CACTCC

FIG. 12.

B) TA195 protein sequence

RAEFGTRKMA PVVTGEAMRN YLKERGDQTV LILHAKVAQK SYGNEKRFFC
PPPCVYLMGS GWKKKKEQME RDGCSEQESQ PCAFIGIGNS DQEMQQLNLE
GKNYCTAKTL YISDSDKRKH FMLSVKMFYG NSDDIGVFLS KRIKVISKPS
KKKQSLKNAD LCIASGTKVA LFNRLRSQTV STRYLHVEGG NFHASSQQWG
AFFIHLLDDD ESEGEEFTVR DGYIHYGQTV KLVCSVTGMA L

C) Protein sequence predicted from BC020780

MGGCRKFGER PPPKRLTREA MRNYLKERGD QTVLILHAKV AQKSYGNEKR
FFCPPPCVYL MGSGWKKKKE QMERDGCSEQ ESQPCAFIGI GNSDQEMQQL
NLEGKNYCTA KTLYISDSDK RKHFMLSVKM FYGNSDDIGV FLSKRIKVIS
KPSKKKQSLK NADLCIASGT KVALFNRLRS QTVSTRYLHV EGGNFHASSQ
QWGAFFIHLL DDDESEGEEF TVRDGYIHYG QTVKLVCSVT GMALPRLIIR
KVDKQTALLD ADDPVSQLHK CAFYLKDTER MYLCLSQERI IQFQATPCPK
EPNKEMINDG ASWTIISTDK AEYTFYEGMG PVLAPVTPVP VVESLQLNGG
GDVAMLELTG QNFTPNLRVW FGDVEAETMY RCGESMLCVV PDISAFREGW
RWVRQPVQVP VTLVRNDGII YSTSLTFTYT PEPGPRPHCS AAGAILRANS
SQVPPNESNT NSEGSYTNAS TNSTSVTSST ATVVS

FIG. 13.

TA172 cDNA sequence

CGAGGACTGG TTCCAGTTCA CTCGGCAGCG GCGCCGGGCG GAGGGGGAGA
GCGCGGGCCG CGCGGGCGGG AAGCGAAGAG GCGGGCGGGC CAGCGAGGAG
CGCGGAGAGA AAAGGCGCGA GCGGCCAGGA GGGCTCAGGC CGAGACACCT
TGCAGCTGCC GCCGCCGCCA CCGAGCCGCC GCTGTGCTCA CTGATCCGCC
TCCAGGGCCA CCGCCATGTC GAGCCGCGGT GGGAAGAAGA AGTCCACCAA
GACGTCCAGG TCTGCCAAAG CAGGAGTCAT CTTTCCCGTG GGGCGGATGC
TGCGGTACAT CAAGAAAGGC CACCCCAAGT ACAGGATTGG AGTGGGGGCA
CCCGTGTACA TGGCCGCCGT CCTGGAATAC CTGACAGCGG AGATTCTGGA
GCTGGCTGGC AATGCAGCGA GAGACAACAA GAAGGGACGG GTCACACCCC
GGCACATCCT GCTGGCTGTG GCCAATGATG AAGAGCTGA

FIG. 14.

A) Protein sequence encoded by XM_016176 (not the protein sequence predicted in the Genbank reference)

FGHVDQENSP SFMFQGSSNT EFKSTKEGFS IAVSADGFKF GISEPGNQEK
KSEKPLENDT GFQAQDISGQ KNGRGVIFGQ TSSTFTFADV AKSTSGEGFQ
FGKKDPNFKG FSGAGEKLFS SQCGKMANKA NTSGDFEKDD DACKTEDSDD
IHFEPVVQMP EKVELVTGEE GEKVLYSQGV KLFRFDAEIS QWKERGLGNL
KILKNEVNGK PRMLMRRDQV LKVCANHWIT TTMNLKPLSG SDRAWMWLAS
DFSDGDAKLE RLAAQFKTPE LAEEFKQKFE ECQRLLLDIP LQTPHKLVDT
GRAAKLIQRA EEMKSGLKDF KTFLTNDQTK VTEEENKGSG TGAAGASDTT
IKPNPENTGP TLEWDNYDLR EDALDDNVSS SSVHDSPLAS SPVRKNIFRF
DESTTGFNFS FKSALSLSKS PAKLNQSGTS VGTDEESDVT QEEERDGQYF
EPVVPLPDLV EVSSGEENEQ VVFSHMAELY RYDKDVGQWK ERGIGDIKIL
QNYDNKQVRI VMRRDQVLKL CANHRITPDM SLQNMKGTER VWVWTACDFA
DGERKVEHLA VRFKLQDVAD SFKKIFDEAK TAQEKDSLIT PHVSRSSTPR
ESPCGKIAVA VLEETTRERT DVIQGDDVAD AASEVEVSST SETTTKAVVS
PPKFVFGSES VKRIFSSEKS NPFAFGNSSA TGSLFGFSFN APLKSNDSET
SSVAQSGSES KVEPKKCELS KNSDIEQSSD SKVKNLSASF PMEESSINYT
FKTPEKAKEK KKPEDSPSD

Translated sequence from 5' of TA204 is underlined

FIG. 15.

```
    CAGAGGCAGCCCGGGCAGTCGCCGGGTCGGTAGAGGTGCTGACAGGGCGGAGCCGCCGCT
  1 ---------+---------+---------+---------+---------+---------+ 60
    GTCTCCGTCGGGCCCGTCAGCGGCCCAGCCATCTCCACGACTGTCCCGCCTCGGCGGCGA

     E  A  A  R  A  V  A  G  S  V  E  V  L  T  G  R  S  R  R  L

    TGCCTCAGCCGGCCTCACCCCTCCCGCCCGGGACAGGTGGCTGGAGCGCGCTCCGCCTCC
 61 ---------+---------+---------+---------+---------+---------+ 120
    ACGGAGTCGGCCGGAGTGGGGAGGGCGGGCCCTGTCCACCGACCTCGCGCGAGGCGGAGG

     P  Q  P  A  S  P  L  P  P  G  T  G  G  W  S  A  L  R  L  R

    GCCAGCTGCGAGTGGGAGCGAGGGAGGGCCGGTCCCGGACGGTCGCCGCCAGCTGCGAGT
121 ---------+---------+---------+---------+---------+---------+ 180
    CGGTCGACGCTCACCCTCGCTCCCTCCCGGCCAGGGCCTGCCAGCGGCGGTCGACGCTCA

     Q  L  R  V  G  A  R  E  G  R  S  R  T  V  A  A  S  C  E  W

    GGAAGTGAGCGAGGGCTAGTCCCGGACCGCCGGGCCAGGGGTCCGGCGGCAGCAGACGGG
181 ---------+---------+---------+---------+---------+---------+ 240
    CCTTCACTCGCTCCCGATCAGGGCCTGGCGGCCCGGTCCCCAGGCCGCCGTCGTCTGCCC

     K  *  A  R  A  S  P  G  P  P  G  Q  G  S  G  G  S  R  R  V

    TACCGTGGCGGCCAAAAAAATGCTCCTGTACCGAGGGGCCCCCGCCGGCCCTGGCGCGCC
241 ---------+---------+---------+---------+---------+---------+ 300
    ATGGCACCGCCGGTTTTTTTACGAGGACATGGCTCCCCGGGGGCGGCCGGGACCGCGCGG

     P  W  R  P  K  K  C  S  C  T  E  G  P  P  P  A  L  A  R  R

    GGGCTGCGGGCTGGCCCGGCCCGGCGGCGGCCCGCAGGCCTTCGGGATCCGCCTGAGCAC
301 ---------+---------+---------+---------+---------+---------+ 360
    CCCGACGCCCGACCGGGCCGGGCCGCCGCCGGGCGTCCGGAAGCCCTAGGCGGACTCGTG

     A  A  G  W  P  G  P  A  A  A  R  R  P  S  G  S  A  *  A  R

    GATGAGCCCCCGCTACCTCCAGAGCAACTCCAGCAGCCACACGCGACCCTTCAGTGCCAT
361 ---------+---------+---------+---------+---------+---------+ 420
    CTACTCGGGGGCGATGGAGGTCTCGTTGAGGTCGTCGGTGTGCGCTGGGAAGTCACGGTA

     *  A  P  A  T  S  R  A  T  P  A  A  T  R  D  P  S  V  P  S

    CGCGGAGCTGCTAGATAATGCTGTAGATCCAGATGTATCTGCCAGGACGGTCTTTATAGA
421 ---------+---------+---------+---------+---------+---------+ 480
    GCGCCTCGACGATCTATTACGACATCTAGGTCTACATAGACGGTCCTGCCAGAAATATCT

     R  S  C  *  I  M  L  *  I  Q  M  Y  L  P  G  R  S  L  *  M
```

FIG. 15 (CONTINUED 1).

```
     TGTTGAGGAGGTCAAGAATAAATCTTGTTTGACCTTTACCGATGATGGATGTGGGATGAC
481  ---------+---------+---------+---------+---------+---------+  540
     ACAACTCCTCCAGTTCTTATTTAGAACAAACTGGAAATGGCTACTACCTACACCCTACTG

       L  R  R  S  R  I  N  L  V  *  P  L  P  M  M  D  V  G  *  H

     ACCTCATAAACTACACCGAATGCTCAGCAGATGAGGTTGATTACTCTCCCTGTGGCTTCC
541  ---------+---------+---------+---------+---------+---------+  600
     TGGAGTATTTGATGTGGCTTACGAGTCGTCTACTCCAACTAATGAGAGGGACACCGAAGG

       L  I  N  Y  T  E  C  S  A  D  E  V  D  Y  S  P  C  G  F  L

     TGAACTAGAGTGTGTGAAGCACGAGTAACAATGGTGGAATGCTGTGGAGGAGGGAAAGGG
601  ---------+---------+---------+---------+---------+---------+  660
     ACTTGATCTCACACACTTCGTGCTCATTGTTACCACCTTACGACACCTCCTCCCTTTCCC

       N  *  S  V  *  S  T  S  N  N  G  G  M  L  W  R  R  E  R  A

     CTCCCAACTATAAAAATGGAAAACAAATTCATGTGGTTGTAGTTACCCTTAAATAAAATG
661  ---------+---------+---------+---------+---------+---------+  720
     GAGGGTTGATATTTTTACCTTTTGTTTAAGTACACCAACATCAATGGGAATTTATTTTAC

       P  N  Y  K  N  G  K  Q  I  H  V  V  V  V  T  L  K  *  N  G

     GAAAATCTGAGTTGGACTTTGATACAGATCAATATGACATCCTGGTATCAGACTTTGACA
721  ---------+---------+---------+---------+---------+---------+  780
     CTTTTAGACTCAACCTGAAACTATGTCTAGTTATACTGTAGGACCATAGTCTGAAACTGT

       K  S  E  L  D  F  D  T  D  Q  Y  D  I  L  V  S  D  F  D  T

     CAGAAGAAAAAATGACTGGCGGTGTTACCTCTGAGCTACCAGAAACAGAATATTCTTTAA
781  ---------+---------+---------+---------+---------+---------+  840
     GTCTTCTTTTTTACTGACCGCCACAATGGAGACTCGATGGTCTTTGTCTTATAAGAAATT

       E  E  K  M  T  G  G  V  T  S  E  L  P  E  T  E  Y  S  L  R

     GGGCATTTTGTGGTATTCTATACATGAAGCCACGCATGAAAATTTTTCTGCGTCAAAAGA
841  ---------+---------+---------+---------+---------+---------+  900
     CCCGTAAAACACCATAAGATATGTACTTCGGTGCGTACTTTTAAAAAGACGCAGTTTTCT

       A  F  C  G  I  L  Y  M  K  P  R  M  K  I  F  L  R  Q  K  K

     AGGTGACTACCCAGATGATTGCCAAGAGCCTGGCCAATGTAGAATATGATACATATAAAC
901  ---------+---------+---------+---------+---------+---------+  960
     TCCACTGATGGGTCTACTAACGGTTCTCGGACCGGTTACATCTTATACTATGTATATTTG

       V  T  T  Q  M  I  A  K  S  L  A  N  V  E  Y  D  T  Y  K  P
```

FIG. 15 (CONTINUED 2).

```
     CTACCTTCACAAATAAGCAGGTGAGAATCACCTTTGGGTTCTCTTGCAAGAATAGTAACC
 961 ---------+---------+---------+---------+---------+---------+ 1020
     GATGGAAGTGTTTATTCGTCCACTCTTAGTGGAAACCCAAGAGAACGTTCTTATCATTGG

       T  F  T  N  K  Q  V  R  I  T  F  G  F  S  C  K  N  S  N  Q

     AGTTTGGAATAATGATGTATCATAACAACCGACTCATAAAATCTTTTGAGAAGGTGGGGT
1021 ---------+---------+---------+---------+---------+---------+ 1080
     TCAAACCTTATTACTACATAGTATTGTTGGCTGAGTATTTTAGAAAACTCTTCCACCCCA

       F  G  I  M  M  Y  H  N  N  R  L  I  K  S  F  E  K  V  G  C

     GCCAGGTGAAGCCAACTCGTGGAGAAGGTGTAGGAGTAATTGGAGTCATTGAGTGCAATT
1081 ---------+---------+---------+---------+---------+---------+ 1140
     CGGTCCACTTCGGTTGAGCACCTCTTCCACATCCTCATTAACCTCAGTAACTCACGTTAA

       Q  V  K  P  T  R  G  E  G  V  G  V  I  G  V  I  E  C  N  F

     TCCTAAAACCTGCCTACAACAAACAAGACTTTGAGTATACCAAGGAGTACCGGCTAACAA
1141 ---------+---------+---------+---------+---------+---------+ 1200
     AGGATTTTGGACGGATGTTGTTTGTTCTGAAACTCATATGGTTCCTCATGGCCGATTGTT

       L  K  P  A  Y  N  K  Q  D  F  E  Y  T  K  E  Y  R  L  T  I

     TAAATGCCCTTGCCCAGAAGCTCAATGCTTACTGGAAGGAAAAAACATCTCAAGATAATT
1201 ---------+---------+---------+---------+---------+---------+ 1260
     ATTTACGGGAACGGGTCTTCGAGTTACGAATGACCTTCCTTTTTTGTAGAGTTCTATTAA

       N  A  L  A  Q  K  L  N  A  Y  W  K  E  K  T  S  Q  D  N  F

     TTGAGACCTCAACTGTAGCCAGGCCAAT
1261 ---------+---------+-------- 1288
     AACTCTGGAGTTGACATCGGTCCGGTTA

       E  T  S  T  V  A  R  P
```

FIG. 16.

```
    AACCAGCAAAACAAAAAAATGATTATTACCGAGGATTCATTGCCCAGCCTAGAAGCCATC
  1 ---------+---------+---------+---------+---------+---------+ 60
    TTGGTCGTTTTGTTTTTTTACTAATAATGGCTCCTAAGTAACGGGTCGGATCTTCGGTAG

                      M  I  I  T  E  D  S  L  P  S  L  E  A  I
                                         1

    TTGAACTATTCCATTTTCAACCGTGAAAATGACCTGCTGGCCCAGTTTGATGCCATCCCA
 61 ---------+---------+---------+---------+---------+---------+ 120
    AACTTGATAAGGTAAAAGTTGGCACTTTTACTGGACGACCGGGTCAAACTACGGTAGGGT

    L  N  Y  S  I  F  N  R  E  N  D  L  L  A  Q  F  D  A  I  P

    GGCAAAAAAGGCACTCGTGTTCTCATTTGGAACATCCGCAGAAATAAAAATGGAAAATCT
121 ---------+---------+---------+---------+---------+---------+ 180
    CCGTTTTTTCCGTGAGCACAAGAGTAAACCTTGTAGGCGTCTTTATTTTTACCTTTTAGA

    G  K  K  G  T  R  V  L  I  W  N  I  R  R  N  K  N  G  K  S

    GAGTTGGACTTTGATACAGATCAATATGACATCCTGGTTTCAGACTTTGACACAGAAGAA
181 ---------+---------+---------+---------+---------+---------+ 240
    CTCAACCTGAAACTATGTCTAGTTATACTGTAGGACCAAAGTCTGAAACTGTGTCTTCTT

    E  L  D  F  D  T  D  Q  Y  D  I  L  V  S  D  F  D  T  E  E

    AAAATGACTGGCGGTGTTACCTCTGAGCTACCAGAAACAGAATATTCTTTAAGGGCATTT
241 ---------+---------+---------+---------+---------+---------+ 300
    TTTTACTGACCGCCACAATGGAGACTCGATGGTCTTTGTCTTATAAGAAATTCCCGTAAA

    K  M  T  G  G  V  T  S  E  L  P  E  T  E  Y  S  L  R  A  F

    TGTGGTATTCTATACATGAAGCCACGCATGAAAATTTTTCTGCGTCAAAAGAAGGTGACT
301 ---------+---------+---------+---------+---------+---------+ 360
    ACACCATAAGATATGTACTTCGGTGCGTACTTTTAAAAAGACGCAGTTTTCTTCCACTGA

    C  G  I  L  Y  M  K  P  R  M  K  I  F  L  R  Q  K  K  V  T
```

FIG. 16(CONTINUED 1).

```
    ACCCAGATGATTGCCAAGAGCCTGGCCAATGTAGAATATGATACACATAAACCTACCTTC
361 ---------+---------+---------+---------+---------+---------+ 420
    TGGGTCTACTAACGGTTCTCGGACCGGTTACATCTTATACTATGTGTATTTGGATGGAAG

    T  Q  M  I  A  K  S  L  A  N  V  E  Y  D  T  H  K  P  T  F

    ACAAATAAGCAGGTGAGAATCACCTTTGGGTTCTCTTGCAAGAATAGTAACCAGTTTGGA
421 ---------+---------+---------+---------+---------+---------+ 480
    TGTTTATTCGTCCACTCTTAGTGGAAACCCAAGAGAACGTTCTTATCATTGGTCAAACCT

    T  N  K  Q  V  R  I  T  F  G  F  S  C  K  N  S  N  Q  F  G

    ATAATGATGTATCATAACAACCGACTCATAAAATCTTTTGAGAAGGTGGGGTGCCAGGTG
481 ---------+---------+---------+---------+---------+---------+ 540
    TATTACTACATAGTATTGTTGGCTGAGTATTTTAGAAAACTCTTCCACCCCACGGTCCAC

    I  M  M  Y  H  N  N  R  L  I  K  S  F  E  K  V  G  C  Q  V

    AAGCCAACTCGTGGAGAAGGTGTAGGAGTAATTGGAGTCATTGAGTGCAATTTCCTAAAA
541 ---------+---------+---------+---------+---------+---------+ 600
    TTCGGTTGAGCACCTCTTCCACATCCTCATTAACCTCAGTAACTCACGTTAAAGGATTTT

    K  P  T  R  G  E  G  V  G  V  I  G  V  I  E  C  N  F  L  K

    CCTGCCTACAACAAACAAGACTTTGAGTATACCAAGGAGTACCGGCTAACAATAAATGCC
601 ---------+---------+---------+---------+---------+---------+ 660
    GGACGGATGTTGTTTGTTCTGAAACTCATATGGTTCCTCATGGCCGATTGTTATTTACGG

    P  A  Y  N  K  Q  D  F  E  Y  T  K  E  Y  R  L  T  I  N  A

    CTTGCCCAGAAGCTCAATGCTTACTGGAAGGAAAAAACATCTCAAGATAATTTTGAGACC
661 ---------+---------+---------+---------+---------+---------+ 720
    GAACGGGTCTTCGAGTTACGAATGACCTTCCTTTTTTGTAGAGTTCTATTAAAACTCTGG

    L  A  Q  K  L  N  A  Y  W  K  E  K  T  S  Q  D  N  F  E  T

    TCAACTGTAGCCAGGCCAATACCGAAGGTTCCTGACCAGACATGGGTTCAGTGTGATGAG
721 ---------+---------+---------+---------+---------+---------+ 780
    AGTTGACATCGGTCCGGTTATGGCTTCCAAGGACTGGTCTGTACCCAAGTCACACTACTC

    S  T  V  A  R  P  I  P  K  V  P  D  Q  T  W  V  Q  C  D  E
```

FIG. 16 (CONTINUED 2).

```
     TGTCTTAAATGGAGAAAGCTTCCTGGGAAGATTGATCCATCCATGTTACCTGCAAGATGG
781  ---------+---------+---------+---------+---------+---------+  840
     ACAGAATTTACCTCTTTCGAAGGACCCTTCTAACTAGGTAGGTACAATGGACGTTCTACC

     C  L  K  W  R  K  L  P  G  K  I  D  P  S  M  L  P  A  R  W

     TTTTGTTATTATAATTCCCATCCAAAGTACAGGAGATGCTCTGTTCCAGAGGAACAAGAA
841  ---------+---------+---------+---------+---------+---------+  900
     AAAACAATAATATTAAGGGTAGGTTTCATGTCCTCTACGAGACAAGGTCTCCTTGTTCTT

     F  C  Y  Y  N  S  H  P  K  Y  R  R  C  S  V  P  E  E  Q  E
                          ____________________
                                   2

     CTCATTGATGAAGACCTGTGCTTGAGCAAAGCTAAGAAACAAGAACAAACTGTTGAGGAG
901  ---------+---------+---------+---------+---------+---------+  960
     GAGTAACTACTTCTGGACACGAACTCGTTTCGATTCTTTGTTCTTGTTTGACAACTCCTC

     L  I  D  E  D  L  C  L  S  K  A  K  K  Q  E  Q  T  V  E  E

     AAGAAGAAGATGCCTATGGAAAATGAGAACCACCAGGTATTCAGTAATCCACCAAAGATC
961  ---------+---------+---------+---------+---------+---------+  1020
     TTCTTCTTCTACGGATACCTTTTACTCTTGGTGGTCCATAAGTCATTAGGTGGTTTCTAG

     K  K  K  M  P  M  E  N  E  N  H  Q  V  F  S  N  P  P  K  I

     CTCACTGTTCAAGAAATGGCTGGATTGAATAACAAGACAATTGGATATGAGGGAATTCAT
1021 ---------+---------+---------+---------+---------+---------+  1080
     GAGTGACAAGTTCTTTACCGACCTAACTTATTGTTCTGTTAACCTATACTCCCTTAAGTA

     L  T  V  Q  E  M  A  G  L  N  N  K  T  I  G  Y  E  G  I  H

     AGCCCTAGTGTGCTTCCTTCTGGTGGAGAAGAAAGCAGATCACCATCTCTTCAACTTAAG
1081 ---------+---------+---------+---------+---------+---------+  1140
     TCGGGATCACACGAAGGAAGACCACCTCTTCTTTCGTCTAGTGGTAGAGAAGTTGAATTC

     S  P  S  V  L  P  S  G  G  E  E  S  R  S  P  S  L  Q  L  K
```

FIG. 16 (CONTINUED 3).

```
     CCTCTGGATTCCAGTGTTTTACAGTTTTCCAGTAAGTACAAATGGATCCTAGGTGAAGAA
1141 ---------+---------+---------+---------+---------+---------+ 1200
     GGAGACCTAAGGTCACAAAATGTCAAAAGGTCATTCATGTTTACCTAGGATCCACTTCTT

     P  L  D  S  S  V  L  Q  F  S  S  K  Y  K  W  I  L  G  E  E

     CCGGTGGAGAAACGAAGAAGGCTCCAGAATGAGATGACAACACCTTCTCTAGATTATTCC
1201 ---------+---------+---------+---------+---------+---------+ 1260
     GGCCACCTCTTTGCTTCTTCCGAGGTCTTACTCTACTGTTGTGGAAGAGATCTAATAAGG

     P  V  E  K  R  R  R  L  Q  N  E  M  T  T  P  S  L  D  Y  S
     --------------------
              2

     ATGCCTGCTCCTTACAGGAGGGTAGAAGCACCTGTTGCCTACCCAGAAGGGGAGAACAGC
1261 ---------+---------+---------+---------+---------+---------+ 1320
     TACGGACGAGGAATGTCCTCCCATCTTCGTGGACAACGGATGGGTCTTCCCCTCTTGTCG

     M  P  A  P  Y  R  R  V  E  A  P  V  A  Y  P  E  G  E  N  S

     CATGATAAATCGAGTTCTGAGAGAAGTACACCACCATACCTTTTCCCAGAATACCCAGAA
1321 ---------+---------+---------+---------+---------+---------+ 1380
     GTACTATTTAGCTCAAGACTCTCTTCATGTGGTGGTATGGAAAAGGGTCTTATGGGTCTT

     H  D  K  S  S  S  E  R  S  T  P  P  Y  L  F  P  E  Y  P  E

     GCAAGCAAGAATACAGGTCAGAATAGGGAGGTTTCAATTCTGTATCCAGGGGCCAAAGAC
1381 ---------+---------+---------+---------+---------+---------+ 1440
     CGTTCGTTCTTATGTCCAGTCTTATCCCTCCAAAGTTAAGACATAGGTCCCCGGTTTCTG

     A  S  K  N  T  G  Q  N  R  E  V  S  I  L  Y  P  G  A  K  D

     CAACGCCAGGGGTCCCTGCTTCCTGAAGAATTAGAAGATCAGATGCCAAGATTGGTGGCA
1441 ---------+---------+---------+---------+---------+---------+ 1500
     GTTGCGGTCCCCAGGGACGAAGGACTTCTTAATCTTCTAGTCTACGGTTCTAACCACCGT

     Q  R  Q  G  S  L  L  P  E  E  L  E  D  Q  M  P  R  L  V  A

     GAAGAATCTAACAGAGGTAGCACAACCATAAACAAAGAAGAAGTCAACAAGGGACCTTTT
1501 ---------+---------+---------+---------+---------+---------+ 1560
     CTTCTTAGATTGTCTCCATCGTGTTGGTATTTGTTTCTTCTTCAGTTGTTCCCTGGAAAA

     E  E  S  N  R  G  S  T  T  I  N  K  E  E  V  N  K  G  P  F
```

FIG. 16 (CONTINUED 4).

```
     GTAGCTGTTGTGGGTGTTGCCAAAGGTGTTAGAGATTCAGGAGCTCCCATTCAGCTGATC
1561 ---------+---------+---------+---------+---------+---------+ 1620
     CATCGACAACACCCACAACGGTTTCCACAATCTCTAAGTCCTCGAGGGTAAGTCGACTAG

     V  A  V  V  G  V  A  K  G  V  R  D  S  G  A  P  I  Q  L  I

     CCTTTTAACAGAGAGGAGCTTGCTGAGAGACGAAAAGCAGTTGAATCCTGGAACCCAGTG
1621 ---------+---------+---------+---------+---------+---------+ 1680
     GGAAAATTGTCTCTCCTCGAACGACTCTCTGCTTTTCGTCAACTTAGGACCTTGGGTCAC

     P  F  N  R  E  E  L  A  E  R  R  K  A  V  E  S  W  N  P  V

     CCTTATTCTGTGGCCTCTGCTGCAATCCCTGCTGCAGCCATTGGGGAGAAAGCAAGAGGC
1681 ---------+---------+---------+---------+---------+---------+ 1740
     GGAATAAGACACCGGAGACGACGTTAGGGACGACGTCGGTAACCCCTCTTTCGTTCTCCG

     P  Y  S  V  A  S  A  A  I  P  A  A  A  I  G  E  K  A  R  G

     TATGAGGAGAGCGAAGGTCATAATACACCAAAGTTGAAGAACCAGAGAGAGCTGGAAGAA
1741 ---------+---------+---------+---------+---------+---------+ 1800
     ATACTCCTCTCGCTTCCAGTATTATGTGGTTTCAACTTCTTGGTCTCTCTCGACCTTCTT

     Y  E  E  S  E  G  H  N  T  P  K  L  K  N  Q  R  E  L  E  E
                                ________________________________

     TTGAAGAGAACCACAGAAAAATTGGAACGTGTTTTGGCTGAAAGGAATTTGTTCCAGCAA
1801 ---------+---------+---------+---------+---------+---------+ 1860
     AACTTCTCTTGGTGTCTTTTTAACCTTGCACAAAACCGACTTTCCTTAAACAAGGTCGTT

     L  K  R  T  T  E  K  L  E  R  V  L  A  E  R  N  L  F  Q  Q
     __________________________________________________________

     AAGGTGGAGGAGCTGGAACAGGAGAGGAATCACTGGCAGTCTGAATTCAAGAAAGTCCAA
1861 ---------+---------+---------+---------+---------+---------+ 1920
     TTCCACCTCCTCGACCTTGTCCTCTCCTTAGTGACCGTCAGACTTAAGTTCTTTCAGGTT

     K  V  E  E  L  E  Q  E  R  N  H  W  Q  S  E  F  K  K  V  Q
     __________________________________________________________
                                    3
```

FIG. 16 (CONTINUED 5).

```
     CATGAATTGGTGATCTACAGTACCCAGGAGGCGGAAGGCTTGTACTGGAGCAAGAAACAC
1921 ---------+---------+---------+---------+---------+---------+ 1980
     GTACTTAACCACTAGATGTCATGGGTCCTCCGCCTTCCGAACATGACCTCGTTCTTTGTG

     H  E  L  V  I  Y  S  T  Q  E  A  E  G  L  Y  W  S  K  K  H

     ATGGGTTATCGCCAAGCTGAATTCCAGATTCTGAAAGCTGAGCTGGAAAGAACCAAAGAG
1981 ---------+---------+---------+---------+---------+---------+ 2040
     TACCCAATAGCGGTTCGACTTAAGGTCTAAGACTTTCGACTCGACCTTTCTTGGTTTCTC

     M  G  Y  R  Q  A  E  F  Q  I  L  K  A  E  L  E  R  T  K  E

     GAAAAGCAAGAGTTAAAAGAGAAACTGAAGGAAACAGAGACACACCTGGAAATGCTGCAG
2041 ---------+---------+---------+---------+---------+---------+ 2100
     CTTTTCGTTCTCAATTTTCTCTTTGACTTCCTTTGTCTCTGTGTGGACCTTTACGACGTC
                                      4
     E  K  Q  E  L  K  E  K  L  K  E  T  E  T  H  L  E  M  L  Q

     AAGGCTCAGGTCTCCTACCGGACCCCAGAGGGAGATGACCTAGAAAGGGCTTTGGCAAAG
2101 ---------+---------+---------+---------+---------+---------+ 2160
     TTCCGAGTCCAGAGGATGGCCTGGGGTCTCCCTCTACTGGATCTTTCCCGAAACCGTTTC

     K  A  Q  V  S  Y  R  T  P  E  G  D  D  L  E  R  A  L  A  K

     CTTACGCGGCTACGTATCCACGTCAGCTATCTCCTTACTTCTGTCCTCCCTCACTTGGAG
2161 ---------+---------+---------+---------+---------+---------+ 2220
     GAATGCGCCGATGCATAGGTGCAGTCGATAGAGGAATGAAGACAGGAGGGAGTGAACCTC

     L  T  R  L  R  I  H  V  S  Y  L  L  T  S  V  L  P  H  L  E

     CTTCGTGAGATCGGGTATGACTCAGAACAAGTGGATGGGATCCTGTACACGGTGCTGGAG
2221 ---------+---------+---------+---------+---------+---------+ 2280
     GAAGCACTCTAGCCCATACTGAGTCTTGTTCACCTACCCTAGGACATGTGCCACGACCTC

     L  R  E  I  G  Y  D  S  E  Q  V  D  G  I  L  Y  T  V  L  E

     GCAAATCACATACTGGATTGAGCACCAGACTGTATACCCTTCTCTTCTCTTATCTTCTGT
2281 ---------+---------+---------+---------+---------+---------+ 2340
     CGTTTAGTGTATGACCTAACTCGTGGTCTGACATATGGGAAGAGAAGAGAATAGAAGACA

     A  N  H  I  L  D  *
```

FIG. 16 (CONTINUED 6).

```
     CTGTTCTCTTTTCTCTCCCTCCCTCACGTCTCTCTCTCTCTCTCTCTCTCTCTCTCTCTC
2341 ---------+---------+---------+---------+---------+---------+ 2400
     GACAAGAGAAAAGAGAGGGAGGGAGTGCAGAGAGAGAGAGAGAGAGAGAGAGAGAGAGAG

     TCTCACCCTCACCTTTATGCCTTATATAGAGAATCTCTGTGTAAATCCTGGCTCATAATC
2401 ---------+---------+---------+---------+---------+---------+ 2460
     AGAGTGGGAGTGGAAATACGGAATATATCTCTTAGAGACACATTTAGGACCGAGTATTAG

     AGTCTCCTTTTTATCAGTTTTGGTGTGGAGAAAGAGGCCAGTTTAAATAGGCTTTCAAGA
2461 ---------+---------+---------+---------+---------+---------+ 2520
     TCAGAGGAAAAATAGTCAAAACCACACCTCTTTCTCCGGTCAAATTTATCCGAAAGTTCT

     GTCTAGGGTCAGAAAAGCAATAGTCACTAAGCTAGGTGACCTGAAAGCTTTAATTTTCAT
2521 ---------+---------+---------+---------+---------+---------+ 2580
     CAGATCCCAGTCTTTTCGTTATCAGTGATTCGATCCACTGGACTTTCGAAATTAAAAGTA

     GACCTGGATATGTGGTCTATTGTATATCTTTTTCTGAAATGGTTTGTATTCATTTAGGTT
2581 ---------+---------+---------+---------+---------+---------+ 2640
     CTGGACCTATACACCAGATAACATATAGAAAAAGACTTTACCAAACATAAGTAAATCCAA

     AGATCAATCAGCAGATATTGGGTCCGGTATACCAGGTATTATTTTGGGGTAAGCTAACAA
2641 ---------+---------+---------+---------+---------+---------+ 2700
     TCTAGTTAGTCGTCTATAACCCAGGCCATATGGTCCATAATAAAACCCCATTCGATTGTT

     GTACAACTCATGTTTGCAGCCTTCGAAGATGTAACAATCTCGTTGGAAACATAAGACATA
2701 ---------+---------+---------+---------+---------+---------+ 2760
     CATGTTGAGTACAAACGTCGGAAGCTTCTACATTGTTAGAGCAACCTTTGTATTCTGTAT

     CATCACATTATACACAAAAGTGTATGATATGTACAACTGAGTGGTACAGATATGACATGG
2761 ---------+---------+---------+---------+---------+---------+ 2820
     GTAGTGTAATATGTGTTTTCACATACTATACATGTTGACTCACCATGTCTATACTGTACC
```

FIG. 16(CONTINUED 7).

```
     AAGATCTGGGGGAGGAAGTTCAAGGAAGGCACCACACCAGAAATGGGACCTAAATTAAGG
2821 ---------+---------+---------+---------+---------+---------+ 2880
     TTCTAGACCCCCTCCTTCAAGTTCCTTCCGTGGTGTGGTCTTTACCCTGGATTTAATTCC

     CTTAAAGAATGAGCATGGCCACACTGTCAGTGAGTGTTCTTTAGGTGGTAGGACTATGGT
2881 ---------+---------+---------+---------+---------+---------+ 2940
     GAATTTCTTACTCGTACCGGTGTGACAGTCACTCACAAGAAATCCACCATCCTGATACCA

     TGATATTTTTCTTCTTCCTATCTTCCTGCCTTTTTCAGATTTTCAATATTAAACTTGTTA
2941 ---------+---------+---------+---------+---------+---------+ 3000
     ACTATAAAAAGAAGAAGGATAGAAGGACGGAAAAAGTCTAAAAGTTATAATTTGAACAAT

     TTCTTACATTGGAAAAAAATAAATTGTTTTTAAAAAG
3001 ---------+---------+---------+------- 3037
     AAGAATGTAACCTTTTTTTATTTAACAAAAATTTTTC
```

1 Signal peptide

2 Nuclear localisation signal

3 Coiled-coil domain

4 Peroxisomal targeting signal

FIG. 17

```
             1                                                      51
OX-TES-4     AATTCGGCAC GAGCAGAGGC AGCCCGGGCA GTCGCCGGGT CGGTAGAGGT
FLJ31673     ~~~~~~~~~~ ~~~~~~~~~~ ~~~~~~~~~~ ~~~~~~~~~~ ~~~~~~~~~~

             51                                                    100
OX-TES-4     GCTGACAGGG CGGAGCCGCC GCTTGCCTCA GCCGGCCTCA CCCCTCCCGC
FLJ31673     ~~~~~~~~~~ ~~~~~~~~~~ ~~~~~~~~~~ ~~~~~~~~~~ ~~~~~~~~~~

             101                                                   150
OX-TES-4     CCGGGACAGG TGGCTGGAGC GCGCTCCGCC TCCGCCAGCT GCGAGTGGGA
FLJ31673     ~~~~~~~~~~ ~~~~~~~~~~ ~~~~~~~~~~ ~~~~~~~~~~ ~~~~~~~~~~

             151                                                   200
OX-TES-4     GCGAGGGAGG GCCGGTCCCG GACGGTCGCC GCCAGCTGCG AGTGGAAGTG
FLJ31673     ~~~~~~~~~~ ~~~~~~~~~~ ~~~~~~~~~~ ~~~~~~~~~~ ~~~~~~~~~~

             201                                                   250
OX-TES-4     AGCGAGGGCT AGTCCCGGAC CGCCGGGCCA GGGGTCCGGC GGCAGCAGAC
FLJ31673     ~~~~~~~~~~ ~~~~~~~~~~ ~~~~~~~~~~ ~~~~~~~~~~ ~~~~~~~~~~

             251                                                   300
OX-TES-4     GGGTACCGTG GCGGCCAAAA AAATGCTCCT GTACCGAGGG GCCCCCGCCG
FLJ31673     ~~~~~~~~~~ ~~~~~~~~~~ ~~~~~~~~~~ ~~~~~~~~~~ ~~~~~~~~~~

             301                                                   350
OX-TES-4     GCCCTGGCGC GCCGGGCTGC GGGCTGGCCC GGCCCGGCGG CGGCCCGCAG
FLJ31673     ~~~~~~~~~~ ~~~~~~~~~~ ~~~~~~~~~~ ~~~~~~~~~~ ~~~~~~~~~~

             351                                                   400
OX-TES-4     GCCTTCGGGA TCCGCCTGAG CACGATGAGC CCCCGCTACC TCCAGAGCAA
FLJ31673     ~~~~~~~~~~ ~~~~~~~~~~ ~~~~~~~~~~ ~~~~~~~~~~ ~~~~~~~~~~

             401                                                   450
OX-TES-4     CTCCAGCAGC CACACGCGAC CCTTCAGTGC CATCGCGGAG CTGCTAGATA
FLJ31673     ~~~~~~~~~~ ~~~~~~~~~~ ~~~~~~~~~~ ~~~~~~~~~~ ~~~~~~~~~~

             451                                                   500
OX-TES-4     ATGCTGTAGA TCCAGATGTA TCTGCCAGGA CGGTCTTTAT AGATGTTGAG
FLJ31673     ~~~~~~~~~~ ~~~~~~~~~~ ~~~~~~~~~~ ~~~~~~~~~~ ~~~~~~~~~~

             501                                                   550
OX-TES-4     GAGGTCAAGA ATAAATCTTG TTTGACCTTT ACCGATGATG GATGTGGGAT
FLJ31673     ~~~~~~~~~~ ~~~~~~~~~~ ~~~~~~~~~~ ~~~~~~~~~~ ~~~~~~~~~~
```

FIG. 17 (CONTINUED 1).

```
            551                                                    600
OX-TES-4    GACACCTCAT AAACTACACC GAATGCTCAG CAGATGAGGT TGATTACTCT
FLJ31673    ~~~~~~~~~~ ~AACCAGCAA AACAAAAAAA TGATTATTAC CGAGGATTCA

            601                                                    650
OX-TES-4    CCCTGTGCCT TCCTGAACTA GAGTGTGTGA AGCACGAGTA ACAATGGTGG
FLJ31673    TTGCCCAGCC TAGAAGCCAT CTTGAACTAT TCCATTTTCA ACCGTGAAAA

            651                                                    700
OX-TES-4    AATGCTGTGG AGGAGGGAAA GGGCTCCCAA CTATAAAAAT GGAAAACAAA
FLJ31673    TGACCTGCTG GCCCAGTTTG ATGCCATCCC AGGCAAAAAA GGCACTCGTG

            701                                                    750
OX-TES-4    TTCATGTGGT TGTAGTTACC CTTAAATAAA AATGGAAAATC TGAGTTGGAC
FLJ31673    TTCTCATTTG GAACATCCGC AGAAATAAA AATGGAAAATC TGAGTTGGAC

            751                                                    800
OX-TES-4    TTTGATACAG ATCAATATGA CATCCTGGTA TCAGACTTTG ACACAGAAGA
FLJ31673    TTTGATACAG ATCAATATGA CATCCTGGTT TCAGACTTTG ACACAGAAGA

            801                                                    850
OX-TES-4    AAAAATGACT GCCGGTGTTA CCTCTGAGCT ACCAGAAACA GAATATTCTT
FLJ31673    AAAAATGACT GCCGGTGTTA CCTCTGAGCT ACCAGAAACA GAATATTCTT

            851                                                    900
OX-TES-4    TAAGCGCATT TTGTGGTATT CTATACATGA AGCCACGCAT GAAAATTTTT
FLJ31673    TAAGCGCATT TTGTGGTATT CTATACATGA AGCCACGCAT GAAAATTTTT

            901                                                    950
OX-TES-4    CTGCCGTCAAA AGAAGGTGAC TACCCAGATG ATTGCCAAGA GCCTGGCCAA
FLJ31673    CTGCCGTCAAA AGAAGGTGAC TACCCAGATG ATTGCCAAGA GCCTCGCCAA

            1001                                                  1050
OX-TES-4    TGTAGAATAT GATACATATA AACCTACCTT CACAAATAAG CAGGTGAGAA
FLJ31673    TGTAGAATAT GATACACATA AACCTACCTT CACAAATAAG CAGGTGAGAA

            1051                                                  1100
OX-TES-4    TCACCTTTGG GTTCTCTTGC AAGAATAGTA ACCAGTTTGG AATAATGATG
FLJ31673    TCACCTTTGG GTTCTCTTGC AAGAATAGTA ACCAGTTTGG AATAATGATG

            1101                                                  1150
OX-TES-4    TATCATAACA ACCGACTCAT AAAATCTTTT GAGAAGCTGG GGTGCCAGGT
FLJ31673    TATCATAACA ACCGACTCAT AAAATCTTTT GAGAAGCTGG GGTGCCAGGT

            1151                                                  1200
OX-TES-4    GAAGCCAACT CGTGGAGAAG GTGTAGGAGT AATTGGAGTC ATTGAGTGCA
FLJ31673    GAAGCCAACT CGTGGAGAAG GTGTAGGAGT AATTGGAGTC ATTGAGTGCA
```

FIG. 17 (CONTINUED 2)

```
           1201                                                   1250
OX-TES-4   ATTTCCTAAA ACCTGCCTAC AACAAACAAG ACTTTGAGTA TACCAAGGAG
FLJ31673   ATTTCCTAAA ACCTGCCTAC AACAAACAAG ACTTTGAGTA TACCAAGGAG

           1251                                                   1300
OX-TES-4   TACCGGCTAA CAATAAATGC CCTTGCCCAG AAGCTCAATG CTTACTGGAA
FLJ31673   TACCGGCTAA CAATAAATGC CCTTGCCCAG AAGCTCAATG CTTACTGGAA

           1301                                                   1350
OX-TES-4   GGAAAAAACA TGTCAAGATA ATTTTGAGAC CTCAACTGTA GCCAGGCCAA
FLJ31673   GGAAAAAACA TGTCAAGATA ATTTTGAGAC CTCAACTGTA GCCAGGCCAA

           1351                                                   1400
OX-TES-4   T~~~~~~~~~ ~~~~~~~~~~ ~~~~~~~~~~ ~~~~~~~~~~ ~~~~~~~~~~
FLJ31673   TACCGAAGGT TCCTGACCAG ACATGGGTTC AGTGTGATGA GTGTCTTAAA

           1401                                                   1450
OX-TES-4   ~~~~~~~~~~ ~~~~~~~~~~ ~~~~~~~~~~ ~~~~~~~~~~ ~~~~~~~~~~
FLJ31673   TGGAGAAAGC TTCCTGGGAA GATTGATCCA TCCATGTTAC CTGCAAGATG

           1451                                                   1500
OX-TES-4   ~~~~~~~~~~ ~~~~~~~~~~ ~~~~~~~~~~ ~~~~~~~~~~ ~~~~~~~~~~
FLJ31673   GTTTTGTTAT TATAATTCCC ATCCAAAGTA CAGGAGATGC TCTGTTCCAG

           1501                                                   1550
OX-TES-4   ~~~~~~~~~~ ~~~~~~~~~~ ~~~~~~~~~~ ~~~~~~~~~~ ~~~~~~~~~~
FLJ31673   AGGAACAAGA ACTCATTGAT GAAGACCTGT GCTTGAGCAA AGCTAAGAAA

           1551                                                   1600
OX-TES-4   ~~~~~~~~~~ ~~~~~~~~~~ ~~~~~~~~~~ ~~~~~~~~~~ ~~~~~~~~~~
FLJ31673   CAAGAACAAA CTGTTGAGGA GAAGAAGAAG ATGCCTATGG AAAATGAGAA

           1601                                                   1650
OX-TES-4   ~~~~~~~~~~ ~~~~~~~~~~ ~~~~~~~~~~ ~~~~~~~~~~ ~~~~~~~~~~
FLJ31673   CCACCAGGTA TTCAGTAATC CACCAAAGAT CCTCACTGTT CAAGAAATGG

           1651                                                   1700
OX-TES-4   ~~~~~~~~~~ ~~~~~~~~~~ ~~~~~~~~~~ ~~~~~~~~~~ ~~~~~~~~~~
FLJ31673   CTGGATTGAA TAACAAGACA ATTGGATATG AGGGAATTCA TAGCCCTAGT

           1701                                                   1750
OX-TES-4   ~~~~~~~~~~ ~~~~~~~~~~ ~~~~~~~~~~ ~~~~~~~~~~ ~~~~~~~~~~
FLJ31673   GTGCTTCCTT CTGGTGGAGA AGAAAGCAGA TCACCATCTC TTCAACTTAA

           1751                                                   1800
OX-TES-4   ~~~~~~~~~~ ~~~~~~~~~~ ~~~~~~~~~~ ~~~~~~~~~~ ~~~~~~~~~~
FLJ31673   GCCTCTGGAT TCCAGTGTTT TACAGTTTTC CAGTAAGTAC AAATGGATCC
```

FIG. 18.

```
           1                                                     50
OX-TES-4   EAARAVAGSV EVLTGRSRRL PQPASPLPPG TGGWSALRLR QLRVGAREGR
FLJ31673   ~~~~~~~~~~ ~~~~~~~~~~ ~~~~~~~~~~ ~~~~~~~~~~ ~~~~~~~~~~

           51                                                   100
OX-TES-4   SRTVAASCEW K*ARASPGPP GQGSGGSRRV PWRPKKCSCT EGPPPALARR
FLJ31673   ~~~~~~~~~~ ~~~~~~~~~~ ~~~~~~~~~~ ~~~~~~~~~~ ~~~~~~~~~~

           101                                                  150
OX-TES-4   AAGWPGPAAA RRPSGSA*AR *APATSRATP AATRDPSVPS RSC*IML*IQ
FLJ31673   ~~~~~~~~~~ ~~~~~~~~~~ ~~~~~~~~~~ ~~~~~~~~~~ ~~~~~~~~~~

           151                                                  200
OX-TES-4   MYLPGRSL*M LRRSRINLV* PLPMMDVG*H LINYTECSAD EVDYSPCGFL
FLJ31673   ~~~~~~~~~~ ~~~~~~~~~~ ~~~~~~~~~~ ~MIITEDSLP SLE....AIL

           201                                                  250
OX-TES-4   N*SV*STSNN GGMLWRRERA PNYKNGKQIH VVVVTLK*NG KSELDFDTDQ
FLJ31673   NYSIFNREND ..LLAQFDAI PG.KKGTRVL IWNIRRNKNG KSELDFDTDQ

           251                                                  300
OX-TES-4   YDILVSDFDT EEKMTGGVTS ELPETEYSLR AFCGILYMKP RMKIFLRQKK
FLJ31673   YDILVSDFDT EEKMTGGVTS ELPETEYSLR AFCGILYMKP RMKIFLRQKK

           301                                                  350
OX-TES-4   VTTQMIAKSL ANVEYDTYKP TFTNKQVRIT FGFSCKNSNQ FGIMMYHNNR
FLJ31673   VTTQMIAKSL ANVEYDTHKP TFTNKQVRIT FGFSCKNSNQ FGIMMYHNNR

           351                                                  400
OX-TES-4   LIKSFEKVGC QVKPTRGEGV GVIGVIECNF LKPAYNKQDF EYTKEYRLTI
FLJ31673   LIKSFEKVGC QVKPTRGEGV GVIGVIECNF LKPAYNKQDF EYTKEYRLTI

           401                                                  450
OX-TES-4   NALAQKLNAY WKEKTSQDNF ETSTVARP~~ ~~~~~~~~~~ ~~~~~~~~~~
FLJ31673   NALAQKLNAY WKEKTSQDNF ETSTVARPIP KVPDQTWVQC DECLKWRKLP

           451                                                  500
OX-TES-4   ~~~~~~~~~~ ~~~~~~~~~~ ~~~~~~~~~~ ~~~~~~~~~~ ~~~~~~~~~~
FLJ31673   GKIDPSMLPA RWFCYYNSHP KYRRCSVPEE QELIDEDLCL SKAKKQEQTV

           501                                                  550
OX-TES-4   ~~~~~~~~~~ ~~~~~~~~~~ ~~~~~~~~~~ ~~~~~~~~~~ ~~~~~~~~~~
FLJ31673   EEKKKMPMEN ENHQVFSNPP KILTVQEMAG LNNKTIGYEG IHSPSVLPSG

           551                                                  600
OX-TES-4   ~~~~~~~~~~ ~~~~~~~~~~ ~~~~~~~~~~ ~~~~~~~~~~ ~~~~~~~~~~
FLJ31673   GEESRSPSLQ LKPLDSSVLQ FSSKYKWILG EEPVEKRRRL QNEMTTPSLD
```

FIG. 18 (CONTINUED)

```
          601                                                    650
OX-TES-4  ~~~~~~~~~~ ~~~~~~~~~~ ~~~~~~~~~~ ~~~~~~~~~~ ~~~~~~~~~~
FLJ31673  YSMPAPYRRV EAPVAYPEGE NSHDKSSSER STPPYLFPEY PEASKNTGQN

          651                                                    700
OX-TES-4  ~~~~~~~~~~ ~~~~~~~~~~ ~~~~~~~~~~ ~~~~~~~~~~ ~~~~~~~~~~
FLJ31673  REVSILYPGA KDQRQGSLLP EELEDQMPRL VAEESNRGST TINKEEVNKG

          701                                                    750
OX-TES-4  ~~~~~~~~~~ ~~~~~~~~~~ ~~~~~~~~~~ ~~~~~~~~~~ ~~~~~~~~~~
FLJ31673  PFVAVVGVAK GVRDSGAPIQ LIPFNREELA ERRKAVESWN PVPYSVASAA

          751                                                    800
OX-TES-4  ~~~~~~~~~~ ~~~~~~~~~~ ~~~~~~~~~~ ~~~~~~~~~~ ~~~~~~~~~~
FLJ31673  IPAAAIGEKA RGYEESEGHN TPKLKNQREL EELKRTTEKL ERVLAERNLF

          801                                                    850
OX-TES-4  ~~~~~~~~~~ ~~~~~~~~~~ ~~~~~~~~~~ ~~~~~~~~~~ ~~~~~~~~~~
FLJ31673  QQKVEELEQE RNHWQSEFKK VQHELVIYST QEAEGLYWSK KHMGYRQAEF

          851                                                    900
OX-TES-4  ~~~~~~~~~~ ~~~~~~~~~~ ~~~~~~~~~~ ~~~~~~~~~~ ~~~~~~~~~~
FLJ31673  QILKAELERT KEEKQELKEK LKETETHLEM LQKAQVSYRT PEGDDLERAL

          901                                                 948
OX-TES-4  ~~~~~~~~~~ ~~~~~~~~~~ ~~~~~~~~~~ ~~~~~~~~~~ ~~~~~~~~
FLJ31673  AKLTRLRIHV SYLLTSVLPH LELREIGYDS EQVDGILYTV LEANHILD
```

FIG. 19.

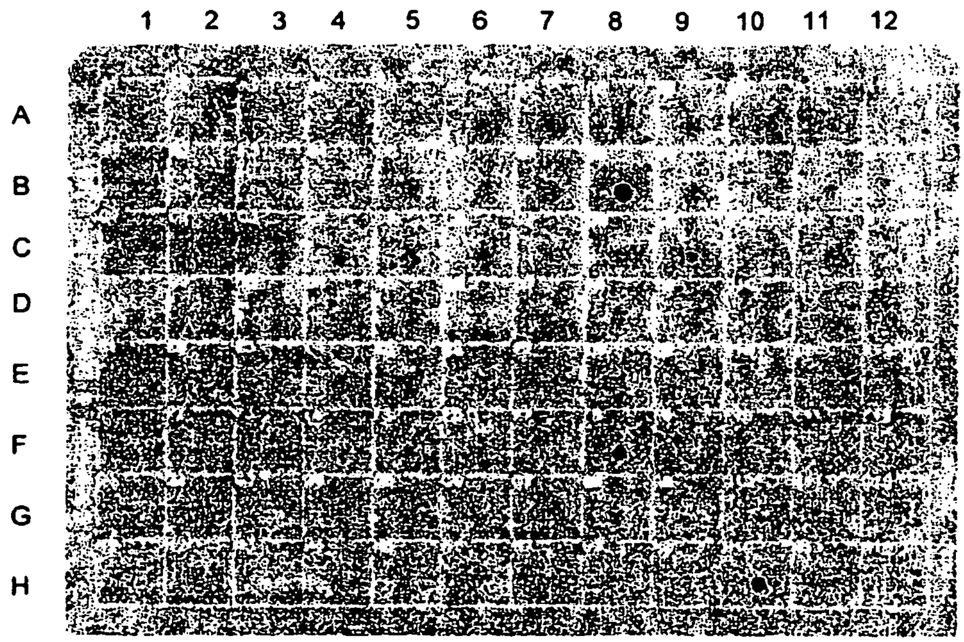


| | 1 | 2 | 3 | 4 | 5 | 6 | 7 | 8 | 9 | 10 | 11 | 12 |
|---|---|---|---|---|---|---|---|---|---|---|---|---|
| A | whole brain | cerebellum, left | substantia nigra | heart | esophagus | colon, transverse | kidney | lung | liver | leukemia, HL-60 | fetal brain | yeast total RNA |
| B | cerebral cortex | cerebellum, right | accumbens nucleus | aorta | stomach | colon, desending | skeletal muscle | placenta | pancreas | HeLa S3 | fetal heart | yeast tRNA |
| C | frontal lobe | corpus callosum | thalamus | atrium, left | duodenum | rectum | spleen | bladder | adrenal gland | leukemia, K-562 | fetal kidney | *E. coli* rRNA |
| D | parietal lobe | amygdala | pituitary gland | atrium, right | jejunum | | thymus | uterus | thyroid gland | leukemia, MOLT-4 | fetal liver | *E. coli* DNA |
| E | occipital lobe | caudate nucleus | spinal cord | ventricle, left | ileum | | peripheral blood leukocyte | prostate | salivary gland | Burkitt's lymphoma, Raji | fetal spleen | Poly r(A) |
| F | temporal lobe | hippo-campus | | ventricle, right | ileocecum | | lymph node | testis | mammary gland | Burkitt's lymphoma, Daudi | fetal thymus | human $C_{0t}$-1 DNA |
| G | p. g.* of cerebral cortex | medulla oblongata | | inter-ventricular septum | appendix | | bone marrow | ovary | | colorectal adeno-carcinoma, SW480 | fetal lung | human DNA 100 ng |
| H | pons | putamen | | apex of the heart | colon, ascending | | trachea | | | lung carcinoma, A549 | | human DNA 500 ng |

* paracentral gyrus

FIG. 20.

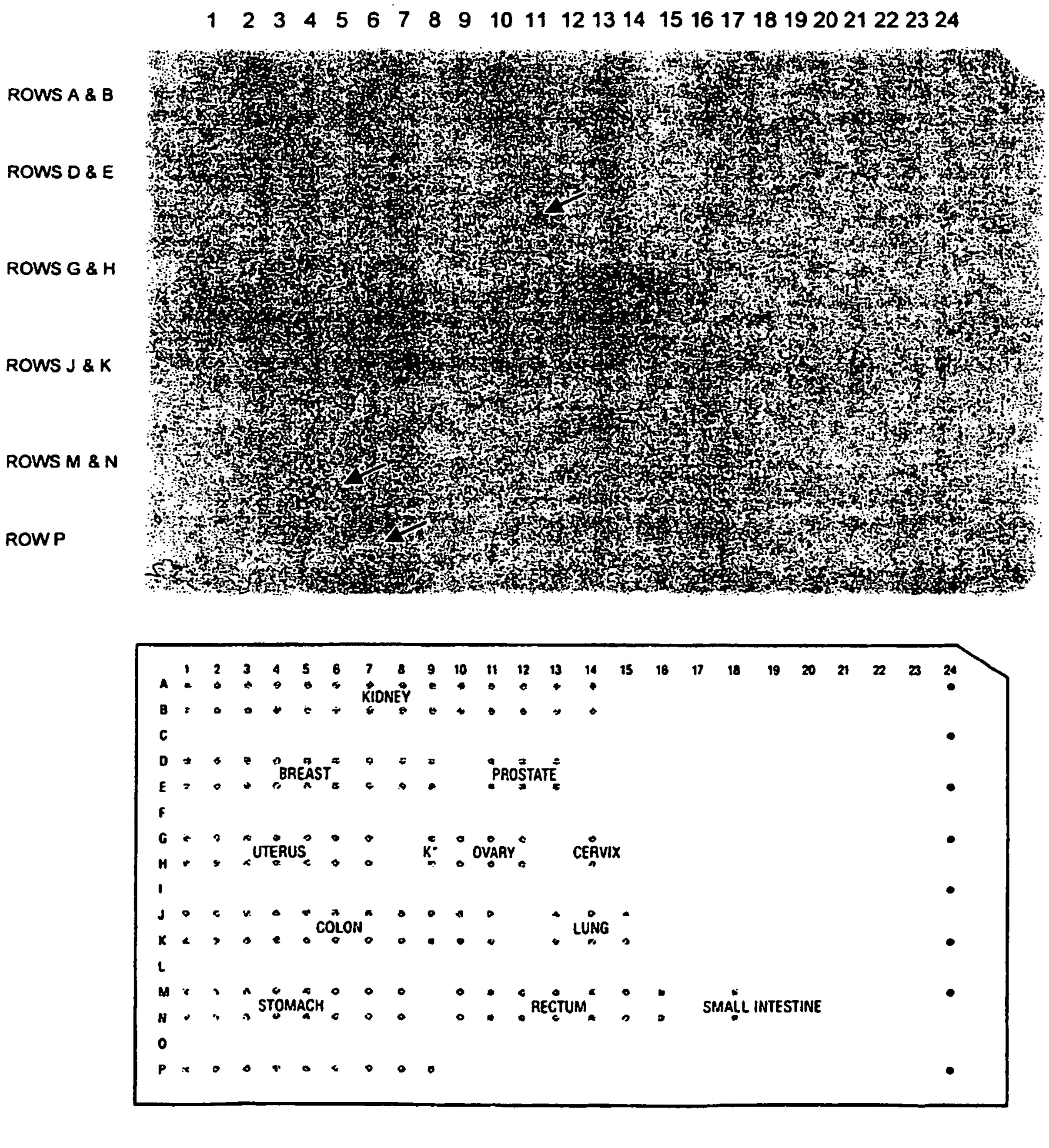
1 2 3 4 5 6 7 8 9 10 11 12 13 14 15 16 17 18 19 20 21 22 23 24
ROWS A & B
ROWS D & E
ROWS G & H
ROWS J & K
ROWS M & N
ROW P
A
B
C
D
E
F
G
H
I
J
K
L
M
N
O
P
KIDNEY
BREAST
PROSTATE
UTERUS
K
OVARY
CERVIX
COLON
LUNG
STOMACH
RECTUM
SMALL INTESTINE

FIG. 21a.

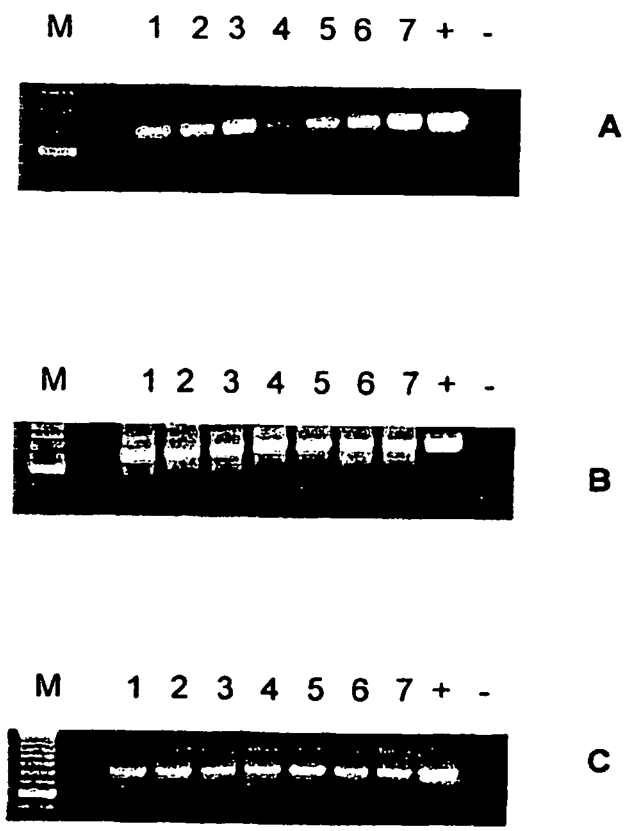

FIG. 21b.

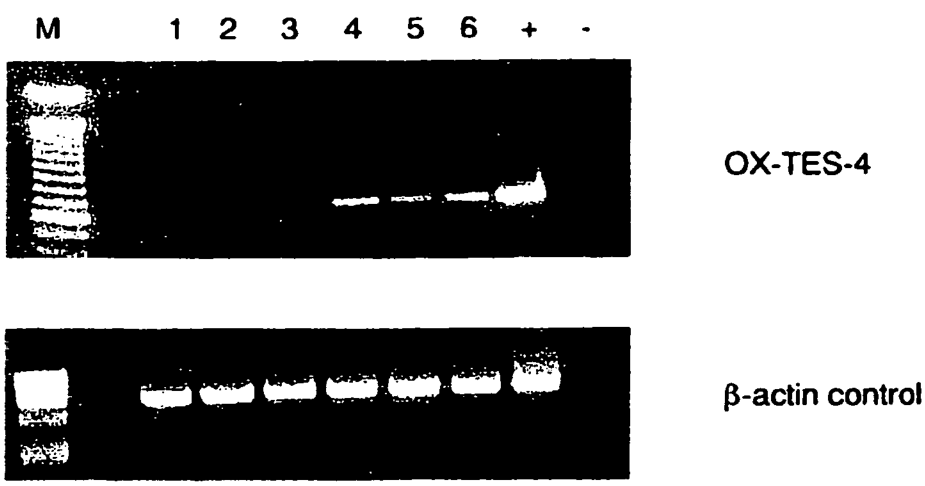


RT-PCR analysis of OX-TES-4 expression in germinal centre (1-3), activated (4-5) and subtype unknown (6) DLBCL cell lines. 1=SUDHL-6; 2=SUDHL-10; 3=DB; 4=OCI-Ly3; 5=OCI-Ly10; 6=HLY-1; + = positive control; - = negative control.

FIG. 22.

A) TA97 cDNA sequence

AATTCGGCAC GAGCTCGTGC CGACGGAAAT TTAAGAAAAC GACTTATGGA
TTATGTACTT CAGATCACAG CACAACTAGA AGATGTTGAT ACATTACATA
AAAAATCAGG AAACCTATGA AGAACATCAA AAGTGGCAAG TACTTTGTTT
TGAAAAGTTC AGATCAACTG ATAATACTAG TGCTGTGATG AGACTCAGCT
TGTACTCAAG AACAGATTTA TGACTAGCTT ATAGTTGGCT GCTTCACCAG
GACTCTACAA ATTGCTCAGA ACCCAAGGAG ACCTAGAGGA TCAAGACATA
ATGGGAGCAT TTTTAGACAA GCCAAAGATG GAAAAGCATA ATGCCCAGGG
GCAGGGTAAT GGGTTGCGAT ATGGGCTAAG CAGCATGCAA GGCTGGCGTG
TTGAAATGGA GGATGCACAT ACGGCTGTGA TCGGTTTGCC AAGTGGACTT
GAATCGTGGT CATTCTTTGC TGTGTATGAT GGGCATGCTG GTTCTCAGGT
TGCCAAATAC TGCTGTGAGC ATTTGTTAGA TCACATCACC AATAACCAAG
ATTTTAAAGG GTCTGCAGGA GCACCTTCTG TGGAAAATGT AAAGAATGGA
ATCAGAACAG GTTTTCTGGA GATTGATGAA CACATGAGAG TTAT

FIG. 23.

B) TA97 translated amino acid sequence

ARADGNLRKR LMDYVLQITA QLEDVDTLHK KSGNLORTSK VASTLFOKVQ
INOOYOCCDE TQLVLKNRFM TSLOLAASPG LYKLLRTQGD LEDQDIMGAF
LDKPKMEKHN AQGQGNGLRY GLSSMQGWRV EMEDAHTAVI GLPSGLESWS
FFAVYDGHAG SQVAKYCCEH LLDHITNNQD FKGSAGAPSV ENVKNGIRTG
FLEIDEHMRV

Accession numbers: NM_021003, AF070670, S87759

FIG. 24.

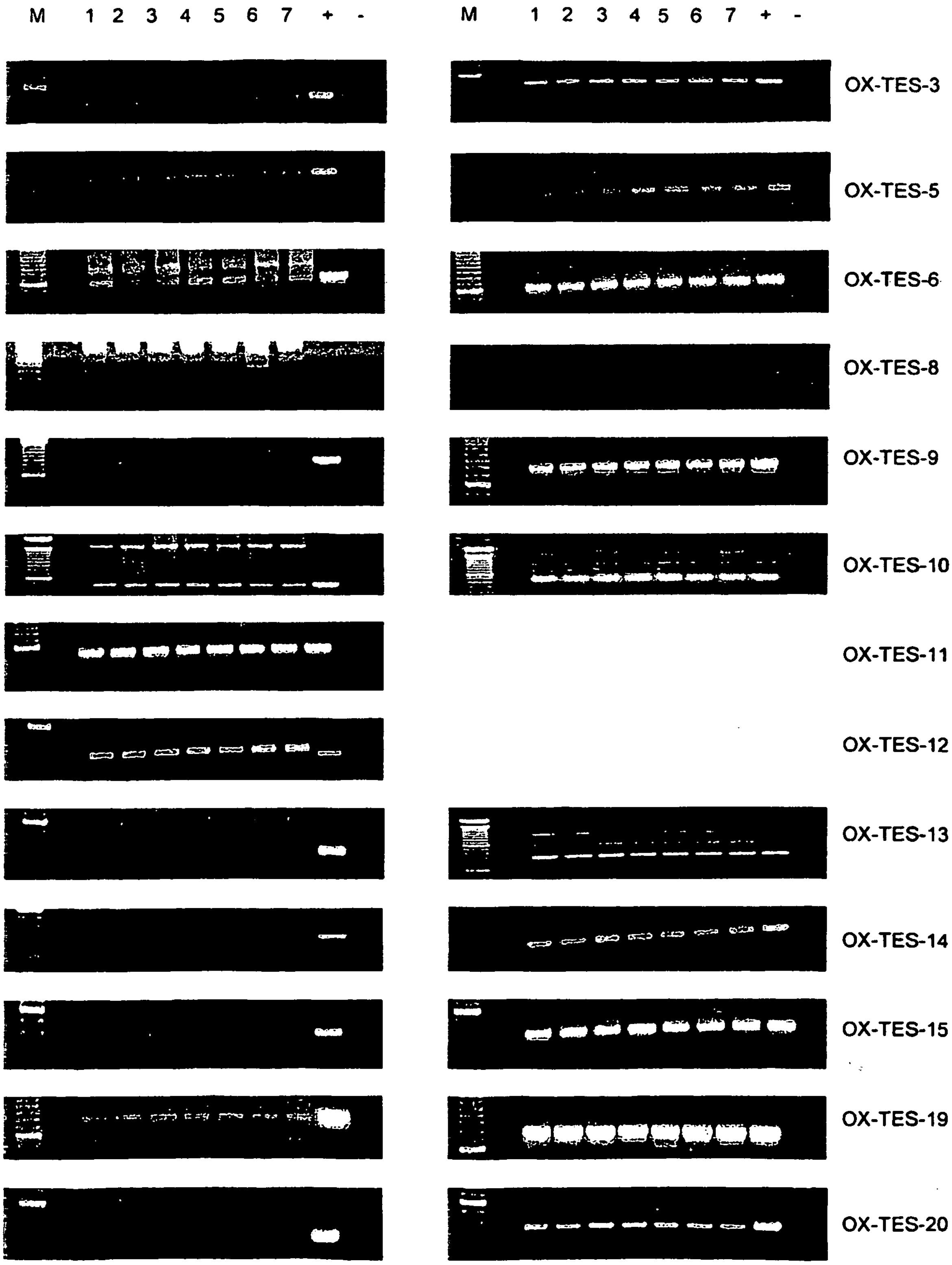
M 1 2 3 4 5 6 7 + -
M 1 2 3 4 5 6 7 + -
OX-TES-3
OX-TES-5
OX-TES-6
OX-TES-8
OX-TES-9
OX-TES-10
OX-TES-11
OX-TES-12
OX-TES-13
OX-TES-14
OX-TES-15
OX-TES-19
OX-TES-20

FIG. 24 (CONTINUED).

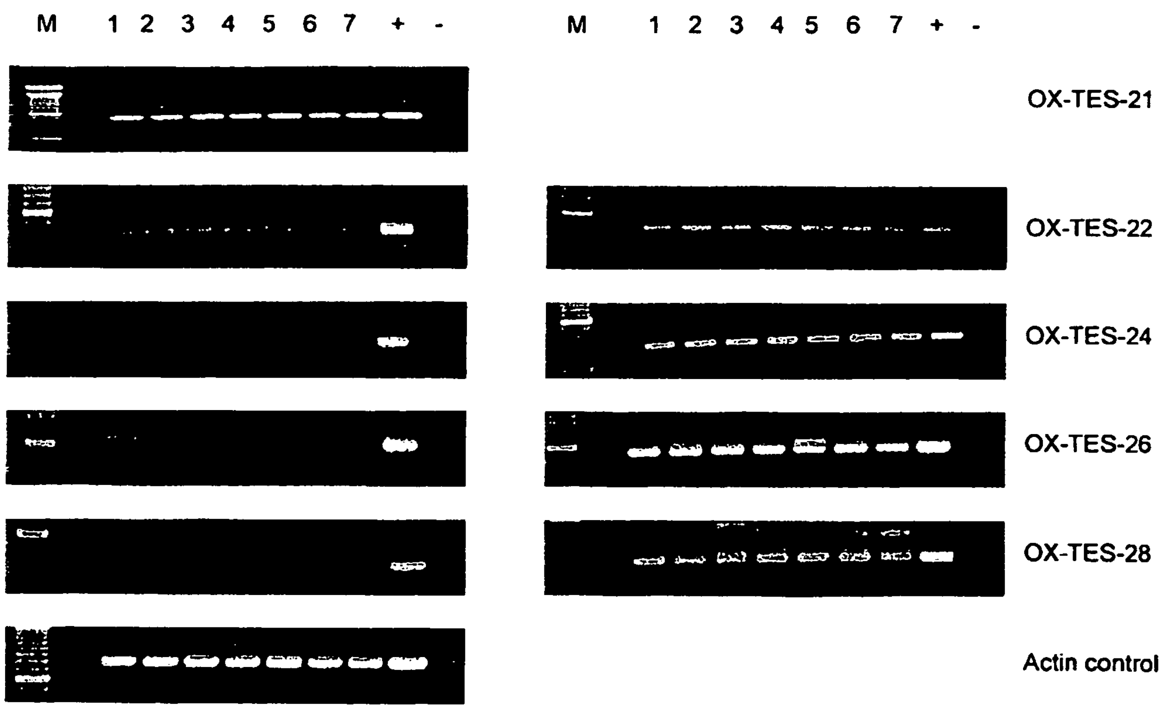

FIG. 25.

Immunostaining of DLBCL cell lines with the DLBCL patient's serum used to screen the testis cDNA library

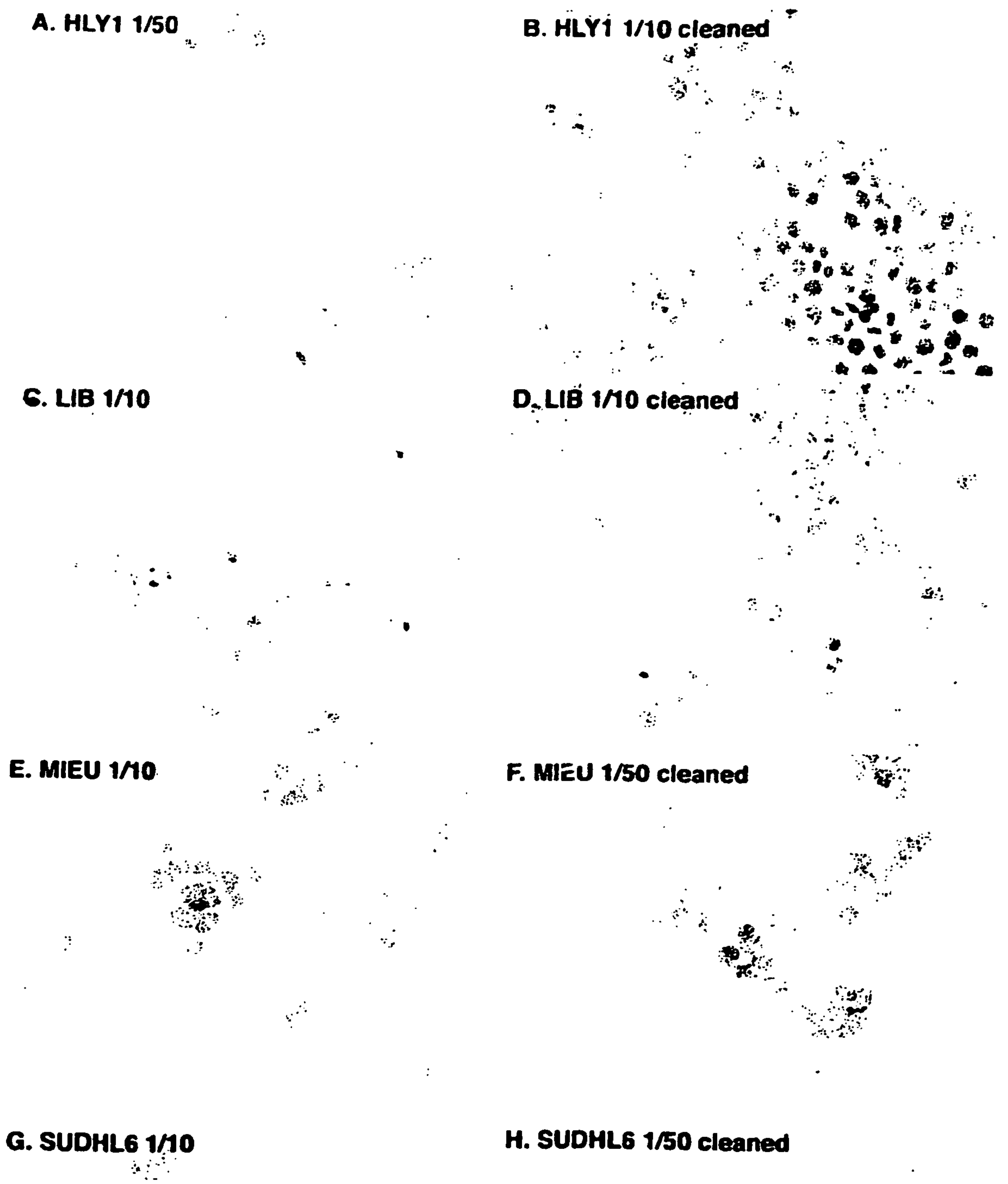

FIG. 26.

**Immunostaining of DLBCL cell lines with serum from a DLBCL patient that was subsequently used in tertiary screening of the DLBCL testis antigens**

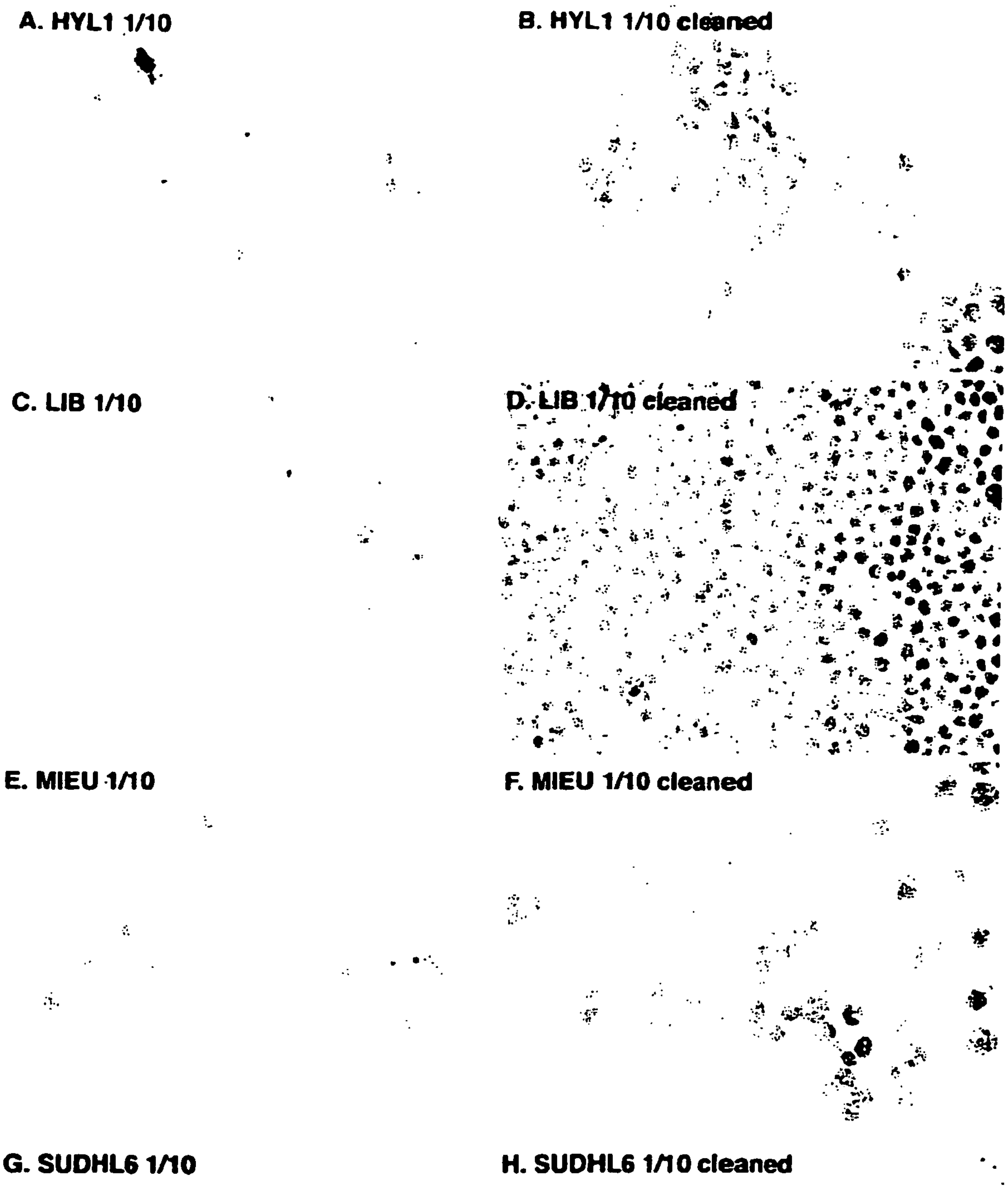

FIG. 27. Immunostained lytic membrane from second round screening.

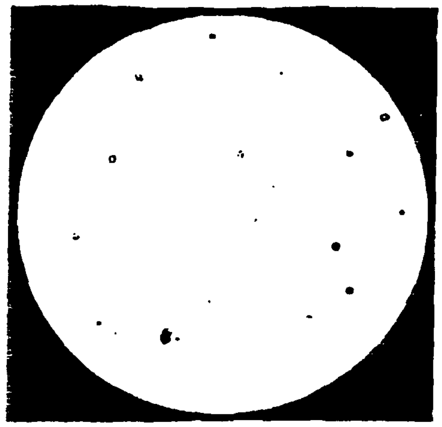

The figure above illustrates an immunostained lytic membrane from the secondary screening of cDNA clones to identify diffuse large B-cell lymphoma tumour antigens. The strongly stained plaques indicate a positive reaction between the patient's serum and a protein expressed from a testis cDNA clone. This can easily be distinguished from the weak background reactivity seen with the surrounding negative plaques.

FIG. 28. Immunostained lytic membrane from tertiary screening.

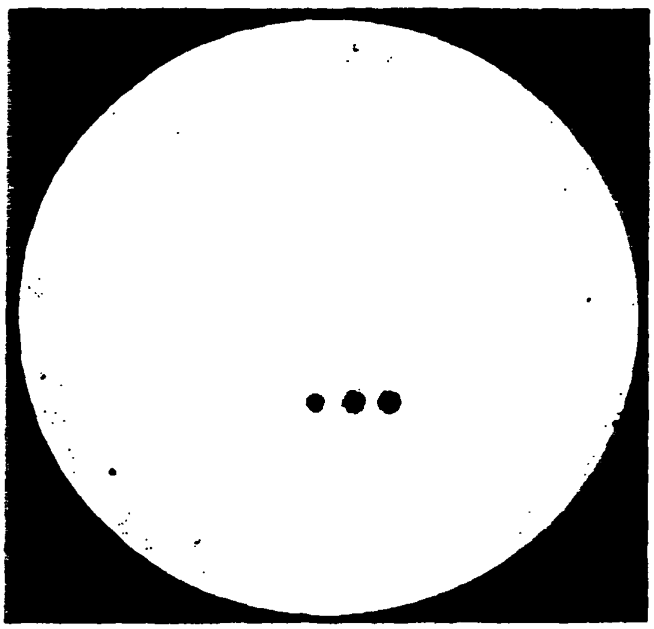

The figure above illustrates an immunostained lytic membrane from the tertiary screening used to assess the reactivity of positive clones with serum from a second patient with diffuse large B-cell lymphoma. Each plaque of interest was spotted three times in a row with two control spots from a phage clone without a cDNA insert spotted both above and below the clone of interest to form a rosette shape. A positive reaction in indicated when the triple spots in the centre have a stronger positivity than the flanking phage clones. This membrane shows clones that have either strong, weak or no reactivity with antibodies from a second lymphoma patient.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1580263 A
- EP 1347046 A
- WO 9211358 A
- WO 9724441 A
- US 5151254 A
- WO 9420078 A
- WO 9423701 A
- WO 9606638 A

### Non-patent literature cited in the description


- **Sambrook et al.** Molecular Cloning: a Laboratory Manual. 1989
- **Price.** *Blood Rev.,* 1993, vol. 7, 127-134
- **Trask.** *Trends Genet.,* 1991, vol. 7, 149-154
- **Verma.** Human Chromosomes: A Manual of Basic Techniques. Pergamon Press, 1988
- **Hudson.** *Science,* 1995, vol. 270, 1945-1954
- **Verma et al.** Human Chromosomes: a Manual of Basic Techniques. Pergamon Press, 1988
- **Okano.** *J. Neurochem.,* 1991, vol. 56, 560
- Oligodeoxynucleotides as Antisense Inhibitors of Gene Expression. CRC Press, 1988
- **Lee et al.** *Nucl. Acids Res.,* 1979, vol. 6, 3073
- **Cooney et al.** *Science,* 1988, vol. 241, 456
- **Dervan et al.** *Science,* 1991, vol. 251, 1360
- **Wagner et al.** *Nature Biotechnology,* 1996, vol. 14, 840-844
- **Robbins, J.** *GENE TARGETING. The Precise Manipulation of the Mammalian Genome Circ. Res.,* 1993, vol. 73, 3-9
- **Kohler ; Milstein.** *Nature,* 1975, vol. 256, 995-497
- **Breedveld F.C.** Therapeutic Monoclonal Antibodies. *Lancet,* 26 February 2000, vol. 335, 735-40
- **Clark, W.R.** The Experimental Foundations of Modern Immunology. Wiley & Sons, Inc, 1986
- **Roitt, I.** Essential Immunology. Blackwell Scientific Publications, 1991
- **Winter et al.** *Annu. Rev. Immunol.,* 1994, vol. 12, 433-55
- **Byrn et al.** *Nature,* 1990, vol. 344, 667-670
- **Mosmann ; Coffman.** *Ann. Rev. Immunol.,* 1989, vol. 7, 145-173
- **Banchereau ; Steinman.** *Nature,* 1998, vol. 392, 245-251
- **Timmerman ; Levy.** *Ann. Rev. Med.,* 1999, vol. 50, 507-529
- **Zitvogel et al.** *Nature Med.,* 1998, vol. 4, 594-600
- **Mahvi et al.** *Immunology and cell Biology,* 1997, vol. 75, 456-460
- **Aasland, R. ; T. J. Gibson ; A. F. Stewart.** The PHD finger: implications for chromatin-mediated transcriptional regulation. *Trends Biochem. Sci.,* 1995, vol. 20, 56-59
- **Aguiar, R. C. ; Y. Yakushijin ; S. Kharbanda et al.** BAL is a novel risk-related gene in diffuse large B-cell lymphomas that enhances cellular migration. *Blood,* 2000, vol. 96, 4328-4334
- **Alizadeh, A. A. ; M. B. Eisen ; R. E. Davis et al.** Distinct types of diffuse large B-cell lymphoma identified by gene expression profiling. *Nature,* 2000, vol. 403, 503-511
- **Amakawa, R. ; W. Jing ; K. Ozawa et al.** Human Jk recombination signal binding protein (IGKJRB): comparison with its mouse homologue. *Genomics,* 1993, vol. 17, 306-315
- **Angrand, P.O. ; F. Apiou ; A. F. Stewart et al.** NSD3, a new SET domain-containing gene, maps to 8p12 and is amplified in human breast cancer cell lines. *Genomics,* 2001, vol. 74, 79-88
- **Baars, J. W. ; D. de Jong ; E. M. Willemse et al.** Diffuse large B-cell non-Hodgkin lymphomas: the clinical relevance of histological subclassification. *Br. J. Cancer,* 1999, vol. 79, 1770-1776
- **Banerjee, D.** Technology evaluation: G-3139. *Curr Opin Mol Ther,* 1999, vol. 1, 404-8
- **Bea, S. ; M. Ribas ; J. M. Hernandez et al.** Increased number of chromosomal imbalances and high-level DNA amplifications in mantle cell lymphoma are associated with blastoid variants. *Blood,* 1999, vol. 93, 4365-4374
- **Bei, R. ; L. Masuelli ; E. Moriconi et al.** *Immune responses to all ErbB family receptors detectable in serum of cancer patients,* 1999, vol. 18, 1267-1275
- **Benharroch, D. ; Z. Meguerian-Bedoyan ; L. Lamant et al.** ALK- positive lymphoma: a single disease with a broad spectrum of morphology. *Blood,* 1998, vol. 91, 2076-2084

- **Biamonti, G. ; M. Ruggiu ; S. Saccone et al.** Two homologous genes, originated by duplication, encode the human hnRNP proteins A2 and A1. *Nucleic Acids Res.,* 1994, vol. 22, 1996-2002
- **Bochar, D. A. ; J. Savard ; W. Wang et al.** A family of chromatin remodeling factors related to Williams syndrome transcription factor. *Proc. Natl. Acad. Sci. U.S.A.,* 2000, vol. 97, 1038-1043
- **Boon, T. ; L. J. Old.** Cancer Tumor Antigens. *Curr Opin Immunol,* 1997, vol. 9, 681-3
- **Brass, N.** Translation initiation factor eIF-4g is encoded by an amplified gene and induces an immune response in squamous lung carcinoma. *Hum. Mol. Genet.,* 1997, vol. 6, 33-39
- **Brichard, V. ; A. Van Pel ; T. Wölfel et al.** The tyrosinase gene codes for an antigen recognized by autologous cytolytic T lymphocytes on HLA-A2 melanomas. *J. Exp. Med.,* 1993, vol. 178, 489-495
- **Capowski, E. E. ; S. Esnault ; S. Bharracharya et al.** Y box-binding factor promotes eosinophil survival by stabilizing granulocyte-macrophage colony-stimulating factor mRNA. *J. Immunol.,* 2001, vol. 167, 5970-5976
- **Caron de Fromental, C. ; F. May-Levin ; H. Mouriesse et al.** Presence of circulating antibodies against cellular protein p53 in a notable proportion of children with B cell lymphoma. *Int. J. Cancer,* 1987, vol. 39, 185-189
- **Castiglia, D. ; A. Cestelli ; M. Scaturro et al.** H1(0) and H3.3B mRNA levels in developing rat brain. *Neurochem. Res.,* 1994, vol. 19, 1531-1537
- **Changolkar, L. N. ; J. R. Pehrson.** Reconstitution of nucleosomes with Histone MacroH2A1.2. *Biochem.,* 2002, vol. 41, 179-184
- **Chen, C. Y. ; R. Gherzi ; J. S. Anderson et al.** Nucleolin and YB-1 are required for JNK-mediated interleukin-2 mRNA stabilization during T-cell activation. *Genes Dev.,* 2000, vol. 14, 1236-1248
- **Chen, Y. T. ; L. J. Old.** Cancer-testis antigens: targets for cancer immunotherapy. *Cancer J. Sci. Am.,* 1999, vol. 5, 16-17
- **Chen, Y. T.** Cancer vaccine: identification of human tumor antigens by SEREX. *Cancer J Sci Am,* 2000, vol. 6 (3), 208-17
- **Chen, Y. T. ; Gure, A. O. ; Tsang, S. ; Stockert, E. ; Jager, E. ; Knuth, A. ; Old, L. J.** Identification of multiple cancer/testis antigens by allogeneic antibody screening of a melanoma cell line library. *Proc Natl Acad Sci U S A,* 1998, vol. 95 (12), 6919-23
- **Cheng, A. ; P. Kaldis ; M. J. Solomon.** Dephosphorylation of human cyclin-dependent kinases by protein phosphatase type 2C alpha and beta 2 isoforms. *J. Biol. Chem.,* 2000, vol. 275, 34744-34749
- **Cheng, M. ; T. G. Bouton ; M. H. Cobb.** ERK3 is a constitutively nuclear protein kinase. *J. Biol. Chem.,* 1996, vol. 271, 8951-8958
- **Chiou, C. C. ; C. C. Chan ; D. L. Sheu et al.** Helicobacter pylori infection induced alteration of gene expression in human gastric cells. *Gut,* 2001, vol. 48, 598-604
- **Cigudosa, J. C. ; N. Z. Parsa ; D. C. Louie et al.** Cytogenetic analysis of 363 consecutively ascertained diffuse large B-cell lymphomas. *Genes Chrm. Cancer,* 1999, vol. 25, 123-133
- **Coles, L. S. ; P. Diamond ; F. Occhiodoro et al.** Cold shock domain proteins repress transcription from the GM-CSF promoter. *Nucl. Acids Res.,* 1996, vol. 24, 2311-2317
- **Cordell, J. L. ; K. A. F. Pulford ; B. Bigerna et al.** Detection of normal and chimeric nucleophosmin in human cells. *Blood,* 1999, vol. 93, 632-642
- **Crawford, L. V. ; D. C. Pim ; R. D. Bulbrook.** Detection of antibodies against the cellular protein p53 in sera from patients with breast cancer. *Int. J. Cancer,* 1982, vol. 30, 403-408
- **Dalla-Favera, R. ; B. H. Ye ; F. Le Coco et al.** Identification of genetic lesions associated with diffuse large-cell lymphoma. *Ann. Oncol.,* 1994, vol. 5, S55-S60
- **Daniel, J. M. ; A. B. Reynolds.** The catenin p120(ctn) interacts with Kaiso, a novel BTB/POZ domain zinc finger transcription factor. *Mol. Cell Biol.,* 1999, vol. 19, 3614-3623
- **Das, A. K. ; N. R. Helps ; P. T. W. Cohen et al.** Crystal structure of the protein serine/threonine phosphatase 2C at 2.0 angstrom resolution. *EMBO J.,* 1996, vol. 15, 6798-6809
- **Davis, R. J.** Transcriptional regulation by MAP kinases. *Mol. Reprod. Dev.,* 1995, vol. 42, 459-467
- **Deltour, S. ; C. Guerardel ; D. Leprince.** Recruitment of SMRT/N-CoR-mSin3A-HDAC-repressing complexes is not a general mechanism for BTB/POZ transcriptional repressors: the case of HIC-1 and gammaFBP-B. *Proc. Natl. Acad. Sci. U.S.A.,* 1999, vol. 96, 14831-14836
- **Didier, D. K. ; J. Schiffenbauer ; S. L. Woulfe et al.** Characterization of the cDNA encoding a protein binding to the major histocompatibility complex class II Y box. *Proc. Natl. Acad. Sci. U.S.A.,* 1988, vol. 85, 7322-7326
- **Disis, M. L. ; M. A. Cheever.** Oncogenic proteins as tumour antigens. *Curr. Opin. Immunol.,* 1996, vol. 8, 637-642
- **Disis, M. L. ; Calenoff, E. ; McLaughlin, G. ; Murphy, A. E. ; Chen, W. ; Groner, B. ; Jeschke, M. ; Lydon, N. ; McGlynn, E. ; Livingston, R. B. et al.** Existent T-cell and antibody immunity to HER-2/neu protein in patients with breast cancer. *Cancer Res,* 1994, vol. 54 (1), 16-20
- **Donoghue, S. ; H. S. Baden ; I. Lauder et al.** Immunohistochemical localization of caspase-3 correlates with clinical outcome in B-cell diffuse large-cell lymphoma. *Cancer Res.,* 1999, vol. 59, 5386-5391

- **Dropcho, E. J. ; Y.-T. Chen ; J. B. Posner et al.** Cloning of a brain protein identified by autoantibodies from a patient with paraneoplastic cerebellar degeneration. *Proc. Natl. Acad. Sci. USA,* 1987, vol. 84, 4552-4556
- **Dunbar, P. ; G. Ogg ; J. Chen et al.** Direct isolation, phenotyping and cloning of low-frequency antigen specific cytotoxic T lymphocytes from peripheral blood. *Curr. Biol.,* 1998, vol. 8, 413-6
- **Evdokimova, V. ; P. Ruzanov ; H. Imataka et al.** The major mRNA-associated protein YB-1 is a potent 5' cap-dependent mRNA stabilizer. *EMBO J.,* 2001, vol. 20, 2491-5502
- **Falini, B. ; Pileri, S. ; Zinzani, P.L. ; Carbone, A. ; Zagonel, V. ; Wolf-Peeters, C. ; Verhoef, G. ; Menestrina, F. ; Todeschini, G. ; Paulli, M.** ALK+ lymphoma: clinico-pathological findings and outcome. *Blood,* 1999, vol. 93, 2697-706
- **Favreau, C. ; H. J. Worman ; R. W. Wozniak et al.** Cell cycle-dependent phosphorylation of nucleoporins and nuclear pore membrane protein Gp210. *Biochem.,* 1996, vol. 35, 8035-8044
- **Ferrando-May, E. ; V. Cordes ; I. Biller-Ckovric et al.** Caspases mediate nucleoporin cleavage, but not early redistribution of nuclear transport factors and modulation of nuclear permeability in apoptosis. *Cell Death Differ,* 2001, vol. 8, 495-505
- **Ferreira, P. A. ; C. Yunfei ; D. Schick et al.** The cyclophilin-like domain mediates the association of Ran-binding protein 2 with subunits of the 19 S regulatory complex of the proteasome. *J. Biol. Chem.,* 1998, vol. 273, 24676-24682
- **Fleischhacker, M. ; T. Beinert ; M. Ermitsch et al.** Detection of ampifiable messenger RNA in the serum of patients with lung cancer. *Ann. N.Y. Acad. Sci.,* 2001, vol. 945, 179-188
- **Fox, A.H. ; Lam, Y.W. ; Leung, A.K. ; Lyon, C.E. ; Andersen, J. ; Mann, M. ; Lamond, A.I.** Paraspeckles. A novel nuclear domain. *Curr Biol.,* 2002, vol. 12, 13-25
- **Fowler, L. ; J. Everitt ; J. L. Stevens et al.** Redistribution and enhanced protein kinase C-mediated phosphorylation of alpha- and gamma-adducin during renal tumor progression. *Cell Growth Differ,* 1998, vol. 9, 405-413
- **Freemont, P. S.** RING for destruction?. *Curr. Biol.,* 2000, vol. 10, R84-87
- **Friedman, L. S. ; E. A. Ostermeyer ; E. D. Lynch et al.** 22 genes from chromosome 17q21: cloning, sequencing, and characterization of mutations in breast cancer families and tumors. *Genomics,* 1995, vol. 25, 256-263
- **Fukata, Y. ; N. Oshiro ; N. Kinoshita et al.** Phosphorylation of adducin by Rho-kinase plays a crucial role in cell motility. *J. Cell Biol.,* 1999, vol. 145, 347-361
- **Gascoyne, R.D. ; Aoun, P. ; Wu, D. ; Chhanabhai, M. ; Skinnider, B.F. ; Greiner, T.C. ; Morris, S.W. ; Connors, J.M. ; Vose, J.M. ; Viswanatha, D.S.** Prognostic significance of anaplastic lymphoma kinase (ALK) protein expression in adults with anaplastic large cell lymphoma. *Blood,* 1999, vol. 93, 3913-21
- **Gilboa, E.** The risk of autoimmunity associated with tumor immunotherapy. *Nature Immunol.,* 2001, vol. 2, 789-792
- **Goyns, M. H. ; D. W. Hammond ; C. J. Harrison et al.** Structural abnormalities of the X-chromosome in non-Hodgkins-lymphoma. *Leukemia,* 1993, vol. 7, 848-852
- **Grillo-Lopez, A. J. ; B. K. Dallaire ; A. McClure et al.** Monoclonal antibodies: a new era in treatment of Non-Hodgkin's lymphoma. *Curr Pharm Biotechnol,* 2001, vol. 2, 301-311
- **Gu, C. ; T. Oyama ; T. Osaki et al.** Expression of Y box-binding protein-1 correlates with DNA topoisomerase IIalpha and proliferating cell nuclear antigen expression in lung cancer. *Anticancer Res.,* 2001, vol. 21, 2357-2362
- **Gu, Z. ; G. Kuntz-Simon ; J. Rommelaere et al.** Oncogenic transformation-dependent expression of a transcription factor NF-Y subunit. *Mol. Carcinog.,* 1999, vol. 24, 294-299
- **Gui, J. F. ; W. S. Lane ; X. D. Fu.** A serine kinase regulates intracellular localization of spicing factors in the cell cycle. *Nature,* 1994, vol. 369, 678-682
- **Halbach, T. ; N. Scheer ; W. Werr.** Transcriptional activation by the PHD finger is inhibited through an adjacent leucine zipper that binds 14-3-3 proteins. *Nuc. Acids Res.,* 2000, vol. 28, 3542-3550
- **Hanada, M. ; T. Kobayashi ; M. Ohnishi et al.** Selective suppression of stress-activated protein kinase pathway by protein phosphatase 2C in mammalian cells. *FEBS Lett.,* 1998, vol. 437, 172-176
- **Harris, N. L. ; E. S. Jaffe ; H. Stein et al.** A revised European-American classification of lymphoid neoplasms - a proposal from the International Lymphoma Study-Group. *Blood,* 1994, vol. 84, 1361-1392
- The World Health Organization classification of neoplastic diseases of the hematopoietic and lymphoid tissues. **Harris, N. L. ; E. S. Jaffe ; J. Diebold et al.** Report of the Clinical Advisory Committee meeting. Airlie House, November 1997, vol. 10, 1419-1432
- **Hassfeld, W. ; G. Steiner ; A. Studnicka-Benke et al.** Autoimmune response to the spliceosome. An immunologic link between rheumatoid arthritis, mixed connective tissue disease, and systemic lupus erythematosus. *Arthritis Rheum.,* 1995, vol. 38, 777-785

- **Hayess, K. ; R. Kraft ; J. Sachsinger et al.** Mammalian protein homologous to VAT-1 of Torpedo californica: isolation from Ehrlich ascites tumor cells, biochemical characterization, and organization of its gene. *J. Cell Biochem.,* 1998, vol. 69, 304-315
- **Hofer, T. ; P. Spielmann ; P. Stengel et al.** Mammalian PASKIN, a PAS-serine/threonine kinase related to bacterial oxygen sensors. *Biochem. Biophys. Res. Commun.,* 2001, vol. 288, 757-764
- **Hraba-Renevey, S. ; M. Kress.** Expression of a mouse replacement histone H3.3 gene with a highly conserved 3' noncoding region during SV40- and polyoma-induced Go to S-phase transition. *Nucleic Acids Res.,* 1989, vol. 17, 2449-2461
- **Hsieh, J. J.-D. ; T. Henkel ; P. Salmon et al.** Truncated mammalian Notch1 activates CBF1/RBPJk-repressed genes by a mechanism resembling that of Epstein=Barr virus EBNA2. *Mol. Cell. Biol.,* 1996, vol. 16, 952-959
- **Hsu, F. J. ; C. B. Caspar ; C. Czerwinski et al.** Tumor-specific idiotype vaccines in the treatment of patients with B-cell lymphoma: long term results of a clinical trial. *Blood,* 1997, vol. 89, 3129-3135
- **Hu, Z. ; S. Jin ; K. W. Scott.** Transcriptional activation of the MDR1 gene by UV irradiation. Role of NF-Y and Sp1. *J. Biol. Chem.,* 2000, vol. 275, 2979-2985
- **Huang, N. ; E. vom Baur ; J. M. Garnier et al.** Two distinct nuclear receptor interaction domains in NSD1, a novel SET protein that exhibits characteristics of both corepressors and coactivators. *EMBO J.,* 1998, vol. 17, 3398-3412
- **Ise, T. ; G. Nagatani ; T. Imamura et al.** Transcription factor Y-box binding protein 1 binds preferentially to cisplatin-modified DNA and interacts with proliferating cell nuclear antigen. *Cancer Res.,* 1999, vol. 59, 342-346
- **Ise, T. ; Nagatani, G. ; Imamura, T. ; Kato, K. ; Takano, H. ; Nomoto, M. ; Izumi, H. ; Ohmori, H. ; Okamoto, T. ; Ohga, T.** Transcription Factor Y-Box Binding Protein 1 Binds Preferentially to Cisplatin-modified DNA and Interacts with Proliferating Cell Nuclear Antigen. *Cancer Res,* 1999, vol. 59 (2), 342-346
- **Itoh, M. ; M. Watanabe ; Y. Yamada et al.** HUB1 is an autoantigen frequently eliciting humoral immune response in patients with adult T cell leukemia. *Int. J. Oncol.,* 1999, vol. 14, 703-708
- **Jager, E. ; Jager, D. ; Knuth, A.** Peptide Vaccination in Clinical Oncology. *Onkologie,* 2000, vol. 23 (5), 410-415
- **Jäger, E. ; D. Jäger ; A. Knuth.** Peptide vaccination in clinical oncology. *Onkologie,* 2000, vol. 23, 410-415
- **Jager, E. ; Chen, Y. T. ; Drijfhout, J. W. ; Karbach, J. ; Ringhoffer, M. ; Jager, D. ; Arand, M. ; Wada, H. ; Noguchi, Y. ; Stockert, E.** Simultaneous humoral and cellular immune response against cancer-testis antigen NY-ESO-1: definition of human histocompatibility leukocyte antigen (HLA)-A2-binding peptide epitopes. *J Exp Med,* 1998, vol. 187 (2), 265-70
- **Jäger, E. ; Y.-T. Chen ; J. Drijfhout et al.** Simultaneous humoral and cellular immune response against cancer-testis antigen NY-ESO-1: Definition of human histocompatibility leucocyte antigen (HLA)-A2-binding peptide epitopes. *J. Exp. Med.,* 1998, vol. 187, 265-270
- **Jäger, E. ; Y. Nagata ; S. Gnjatic et al.** Monitoring CD8 T cell responses to NY-ESO-1: Correlation of humoral and cellular immune responses. *Proc. Natl. Acad. Sci. U.S.A.,* 2000, vol. 97, 4760-4765
- **Jaju, R. J. ; C. Fidler ; O. A. Haas et al.** A novel gene, NSD1, is fused to NUP98 in the t(5;11)(q35;p15.5) in de novo childhood acute myeloid leukemia. *Blood,* 2001, vol. 98, 1264-1267
- **Janz, M. ; N. Harbeck ; P. Dettmar et al.** Y-box factor YB-1 predicts drug resistance and patient outcome in breast cancer independently of clinically relevant tumor biologic factors HER2, uPA and PAI-1. *Int. J. Cancer,* 2002, vol. 97, 278-282
- **Jarrett, R. F. ; J. MacKenzie.** Epstein-Barr virus and other candidate viruses in the pathogenesis of Hodgkin's disease. *Sem. in Haematol.,* 1999, vol. 36, 260-269
- **Jensen, D. E. ; M. Proctor ; S. T. Marquis et al.** BAP1: a novel ubiquitin hydrolase which binds to the BRCA1 RING finger and enhances BRCA1-mediated cell growth suppression. *Oncogene,* 1998, vol. 16, 1097-1112
- **Jung, M. S. ; J. Yun ; H. D. Chae et al.** p53 and its homologues, p63 and p73, induce a replicative senescence through inactivation of NF-Y transcription factor. *Oncogene,* 2001, vol. 20, 5818-5825
- **Kamihira, S. ; K. Toriya ; T. Amagasaki et al.** Antibodies against p40tax gene product of human T-lymphotropic virus type-1 (HTLV-1) under various conditions of HTLV-1 infection. *Jpn. J. Cancer Res.,* 1989, vol. 80, 1066-1071
- **Kamiyama, J. ; T. Inoue ; N. Ohtani-Fujita et al.** The ubiquitous transcription factor NF-Y positively regulates the transcription of human p27Kip1 through a CCAAT box located in the 5-upstream region of the p27Kip1 gene. *FEBS Lett.,* 1999, vol. 455, 281-285
- **Kamma, H. ; M. Fujimoto ; M. Fujiwara et al.** Interaction of hnRNP A2/B1 isoforms with telomeric ssDNA and the in vitro function. *Biochem. Biophys. Res. Commun.,* 2001, vol. 280, 625-630

- **Kamma, H. ; H. Satoh ; M. Matusi et al.** Characterization of hnRNP A2 and B1 using monoclonal antibodies: intracellular distribution and metabolism through cell cycle. *Immmunol. Lett.,* 2001, vol. 76, 49-54
- **Karhumaa, P. ; S. Parkkila ; A. Waheed et al.** Nuclear NonO/p54(nrb) protein is a nonclassical carbonic anhydrase. *J. Biol. Chem.,* 2000, vol. 275, 16044-16049
- **Karuman, P. ; O. Gozani ; R. D. Odze et al.** The Peutz-Jegher gene product LKB1 is a mediator of p53-dependent cell death. *Mol. Cell,* 2001, vol. 7, 1307-1319
- **Kiley, S. C. ; K. J. Clark ; S. K. Duddy et al.** Increased protein kinase C delta in mammary tumor cells: relationship to transformtion and metastatic progression. *Oncogene,* 1999, vol. 18, 6748-6757
- **King, C. A. ; M. B. Spellerberg ; D. Zhu et al.** DNA vaccines with single-chain Fv fused to fragment C of tetanus toxin induce induce protective immunity against lymphoma and myeloma. *Nature Med.,* 1998, vol. 4, 1281-1286
- **Kozu, T. ; B. Henrich ; K. P. Schafer.** Structure and expression of the gene (HNRPA2B1) encoding the human hnRNP protein A2/B1. *Genomics,* 1995, vol. 25, 365-371
- **Krackhardt, A. M. ; Witzens, M. ; Harig, S. ; Hodi, F. S. ; Zauls, A. J. ; Chessia, M. ; Barrett, P. ; Gribben, J. G.** Identification of tumor-associated antigens in chronic lymphocytic leukemia by SEREX. *Blood,* 2002, vol. 100 (6), 2123-31
- **Krimer, D. B. ; G. Cheng ; A. I. Skoultchi.** Induction of H3.3 replacement histone mRNAs during the precommitment period of murine erythroleukemia cell differentiation. *Nucleic Acids Res.,* 1993, vol. 21, 2873-2879
- **Kurotaki, N. ; N. Harada ; K. Yoshiura et al.** Molecular characterization of NSD1, a human homologue of the mouse Nsd1 gene. *Gene,* 2001, vol. 279, 197-204
- **Kuroyanagi, N. ; H. Onogi ; T. Wakabayashi et al.** Novel SR-protein-specific kinase, SRPK2, disassembles nuclear speckles. *Biochem. Biophys. Res. Commun.,* vol. 242, 357-364
- **Kwak, L. ; M. Wilson ; L. M. Weiss et al.** Clinical significance of morphological subdivision in diffuse large B cell lymphoma. *Cancer,* 1991, vol. 68, 1988-1993
- **Kwak, L. W. ; D. D. Taub ; P. L. Duffey et al.** Transfer of myeloma idiotype-specific immunity from an actively immunized marrow donor. *Lancet,* 1995, vol. 345, 1016-1020
- **Kwak, L. W. ; M. J. Campbell ; D. K. Czerwinski et al.** Induction of immune responses in patients with B-cell lymphoma against the surface-immunoglobulin idiotype expressed by their tumors. *New Engl. J. Med.,* 1993, vol. 327, 1209-1215
- **LaFond, R. E. ; R. B. Eaton ; R. A. Watt et al.** Autoantibodies to c-myc protein: elevated levels in patients with African Burkitt's lymphoma and normal Ghanians. *Autoimmunity,* 1992, vol. 13, 215-224
- **Lee, S. H. ; X. Wang ; J. DeJpng.** Functional interactions between an atypical NF-kappaB site from the rat CYP2B1 promoter and the transcriptional repressor RBP-Jkappa/CBF1. *Nucleic. Acids Res.,* 2000, vol. 28, 2091-2098
- **Lennert, K. ; N. Mohri ; H. Stein et al.** The histopathology of malignant lymphoma. *Br. J. Haematol.,* 1975, vol. 31, 193-203
- **Lifschitz-Mercer, B. ; Y. Sheinin ; D. Ben-Meir et al.** Protein phosphatase 2Calpha expression in normal human tissues: an immunohistochemical study. *Histochem. Cel Biol.,* 2001, vol. 116, 31-39
- **Ling, M. ; Y.-J. Wen ; S. H. Lim.** Prevalence of antibodies against proteins derived from leukemia cells in patients with chronic myeloid leukemia. *Blood,* 1998, vol. 92, 4764-4770
- **Ling, P. D. ; J. J.-D. Hsieh ; I. K. Ruf et al.** EBNA-2 upregulation of Epstein-Barr virus latency promoters and the cellular CD23 promoter utilizes a common targeting intermediate, CBF1. *J. Virol.,* 1994, vol. 68, 5375-5383
- **Linial, M. ; O. Levius.** The protein VAT-1 from Torpedo electric organ exhibits an ATPase activity. *Neirosci Lett.,* 1993, vol. 152, 155-157
- **Linial, M. ; K. Miller ; R. H. Scheller.** VAT-1: an abundant membrane protein from Torpedo cholinergic synaptic vesicles. *Neuron,* vol. 2, 1265-1273
- **Liu, J. ; S. Akoulitchev ; A. Weber et al.** Defective interplay of activators and repressors with TFHIIH in xeroderma pigmentosum. *Cell,* 2001, vol. 104, 353-363
- **Loewith, R. ; M. Meijer ; S. P. Lees-Miller et al.** Three yeast proteins related to the human candidate tumor suppressor p33(ING1) are associated with histone acetyltransferase activities. *Mol. Cell. Biol.,* 2000, vol. 20, 3807-3816
- **Lubin, R. ; B. Schlichtholz ; J. L. Teillaud et al.** p53 antibodies in patients with various types of cancer: Assay, identification, and characterization. *Clin. Can. Res.,* 1995, vol. 1, 1463-1469
- **Luo, J. ; A. Y. Nikolaev ; S. Imai et al.** Negative control of p53 by SIR2alpha promotes cell survival under stress. *Cell,* 2001, vol. 107, 137-148
- **Makino, Y. ; R. Cao ; K. Svensson et al.** Inhibitory PAS domain protein is a negative regulator of hypoxia-inducible gene expression. *Nature,* 2001, vol. 414, 550-554
- **Makino, Y. ; T. Ohga ; S. Toh et al.** Structural and functional analysis of the human Y-box binding protein (YB-1) gene promoter. *Nucleic Acids Res.,* 1996, vol. 24, 1873-1878

- **Mann, D. J. ; D. G. Campbell ; C. H. McGown et al.** Mammalian protein serine/threonine phosphatase 2C: cDNA cloning and comparative analysis of amino acid sequences. *Biochim. Biophys. Acta,* 1992, vol. 1130, 100-104
- **Marchand, M. ; N. van Baren ; P. Weynants et al.** Tumor regressions observed in patients with metastatic melanoma treated with an antigenic peptide encoded by gene MAGE-3 and presented by HLA-A1. *Int. J. Cancer,* 1999, vol. 80, 912
- **Marx, J.** *Science,* 2001, vol. 292, 426-429
- **Matsuoka, Y. ; X. Li ; V. Bennett.** Adducin: structure, function and regulation. *Cell. Mol.,* 2000, vol. 57, 884-895
- **Matsuoka, Y. ; X. Li ; V. Bennett.** Adducin is an in vivo substrate for protein kinase C: phosphorylation in the MARCKS-related domain inhibits activity in promoting spectrin-actin complexes and occurs in many cells, including dendritic spines of neurons. *J. Cell. Biol.,* 1998, vol. 142, 485-497
- **Mermoud, J. E. ; A. M. Tassin ; J. R. Pehrson et al.** Centrosomal association of histone macroH2A1.2 in embryonic stem cells and somatic cells. *Exp. Cell. Res.,* 2001, vol. 268, 245-251
- **Monni, O. ; H. Joensuu ; K. Franssila et al.** DNA copy number changes in diffuse large B-cell lymphoma comparative genomic hybridization study. *Blood,* 1996, vol. 87, 5269-5278
- **Mosolits, S. ; U. Harmenberg ; U. Ruden et al.** Autoantibodies against the tumour-associated antigen GA733-2 in patients with colorectal carcinoma. *Cancer Immunol. Immunother,* 1999, vol. 47, 315-320
- **Mushegian, A. R. ; D. E. J. Bassett ; M. S. Boguski et al.** Positionally cloned human disease genes: patterns of evolutionary conservation and functional motifs. *Proc. Natl. Acad. Sci. U.S.A.,* 1997, vol. 20, 5831-5836
- **Nestle, F. O. ; S. Alijagic ; M. Gilliet et al.** Vaccination of melanoma patients with peptide- or tumor lysate pulsed dendritic cells. *Nat. Med.,* 1998, vol. 4, 328
- **Nishikawa, H. ; Tanida, K. ; Ikeda, H. ; Sakakura, M. ; Miyahara, Y. ; Aota, T. ; Mukai, K. ; Watanabe, M. ; Kuribayashi, K. ; Old, L. J.** Role of SEREX-defined immunogenic wild-type cellular molecules in the development of tumor-specific immunity. *Proc Natl Acad Sci U S A,* 2001, vol. 98 (25), 14571-6
- **Ohga, T. ; Koike, K. ; Ono, M. ; Makino, Y. ; Itagaki, Y. ; Tanimoto, M. ; Kuwano, M. ; Kohno, K.** Role of the human Y box-binding protein YB-1 in cellular sensitivity to the DNA-damaging agents cisplatin, mitomycin C, and ultraviolet light. *Cancer Res,* 1996, vol. 56 (18), 4224-8
- **Offit, K. ; S. C. Jhanwar ; M. Ladanyi et al.** Cytogenetic analysis of 434 consecutively ascertained specimens of non-Hodgkin's lymphoma: correlations between recurrent aberations, histology and exposure of cytotoxic treatment. *Genes Chrom. Cancer,* 1991, vol. 3, 189-201
- **Okabe, S. ; T. Fukuda ; K. Ishibashi et al.** BAZF, a novel Bcl6 homolog, functions as a transcriptional repressor. *Mol. Cell Biol.,* 1998, vol. 18, 4235-4244
- **Okamoto, T. ; H. Izumi ; T. Imamura et al.** Direct interaction of p53 with the Y-box binding protein, YB-1: a mechanism for regulation of human gene expression. *Oncogene,* 2000, vol. 19, 6194-6202
- **Old, L. J. ; Chen, Y.-T.** New paths in human cancer serology. *Journal of Experimental Medicine,* 1998, vol. 187 (8), 1163-1167
- **Olesen, C. ; C. Hansen ; E. Bendsen et al.** Identification of human candidate genes for male infertility by digital differential display. *Mol. Hum. Reprod.,* 2001, vol. 7, 11-20
- **Ono, T. ; Sato, S. ; Kimura, N. ; Tanaka, M. ; Shibuya, A. ; Old, L. J. ; Nakayama, E.** Serological analysis of BALB/C methylcholanthrene sarcoma Meth A by SEREX: identification of a cancer/testis antigen. *Int J Cancer,* 2000, vol. 88 (6), 845-51
- **Pasqualucci, L. ; P. Neumeister ; T. Goossens et al.** Hypermutation of multiple proto-oncogenes in B-cell diffuse large-cell lymphomas. *Nature,* 2001, vol. 412, 341-346
- **Pichler, A. ; A. gast ; J. S. Seeler et al.** The nucleoporin RanBP2 has SUMO1 E3 ligase activity. *Cell,* 2002, vol. 108, 109-120
- **Ponting, C. P.** Tudor domains in proteins that interact with RNA. *Trends Biochem. Sci.,* vol. 22, 51-52
- **Preuss, K. D. ; Zwick, C. ; Bormann, C. ; Neumann, F. ; Pfreundschuh, M.** Analysis of the B-cell repertoire against antigens expressed by human neoplasms. *Immunol Rev,* 2002, vol. 188 (1), 43-50
- **Project., T. N.-H. L. C.** A clinical evaluation of the International Lymphoma Study Group classification of non-Hodgkin's lymphoma. *Blood,* 1997, vol. 89, 3909-3918
- **Pulford, K. ; B. Falini ; A. H. Banham et al.** Immune response to the ALK oncogenic tyrosine kinase in patients with anaplastic large-cell lymphoma. *Blood,* 2000, vol. 96, 1605-1607
- **Pulford, K. ; H. Roberton ; A. H. Banham et al.** Immunochemical studies of antigenic lymphoma-associated proteins. *Brit. J. Haematol.,* 2001
- **Pulford, K. ; L. Lamant ; S. W. Morris et al.** Detection of anaplastic lymphoma kinase (ALK) and nucleolar protein nucleophosmin (NPM)-ALK proteins in normal and neoplastic cells with the monoclonal antibody ALK1. *Blood,* 1997, vol. 89, 1394-1404

- **Rao, P. H. ; J. Houldsworth ; K. Dyomina et al.** Chromosomal and gene amplification in diffuse large B-cell lymphoma. *Blood,* 1998, vol. 92, 234
- **Rasmussen, T. P. ; T. Huang ; M. A. Mastrangelo et al.** Messenger RNAs encoding mouse histone macroH2A1 isoforms are expressed at similar levels in male and female cells and result from alternative splicing. *Nucleic Acids Res.,* 1999, vol. 27, 3685-3689
- **Reeves, R.** Structure and function of the HMGI(Y) family of architectural transcription factors. *Environ. Health Perspect.,* 2000, vol. 108, 803-809
- **Rifkind, R. A. ; Richon, V. M. ; Marks, P. A.** Induced differentiation, the cell cycle, and the treatment of cancer. *Pharmacol Ther,* 1996, vol. 69 (2), 97-102
- **Robles, A. I. ; C. C. Harris.** p53-mediated apoptosis and genomic instability diseases. *Acta Oncol.,* 2001, vol. 40, 696-701
- **Romero, P. ; P. Dunbar ; D. Valmori et al.** Ex vivo staining of metastatic lymph nodes by class I major histocompatibility complex tetramer labelling reveals high numbers of antigen-experienced tumour-specific cytolytic T lymphocytes. *J. Exp. Med.,* vol. 188, 1641-50
- **Rosenberg, S. A.** The immunotherapy of solid cancers based on cloning the genes encoding tumour-rejection antigens. *Annu. Rev. Med. J. Natl. Can. Inst.,* vol. 88, 481-91
- **Rutter, J. ; C. H. Michnoff ; S. M. Harper et al.** PAS kinase: an evolutionarily conserved PAS domain-regulated serine/threonine kinase. *Proc. Natl. Acad. Sci. U.S.A.,* 2001, vol. 98, 8991-8996
- **Sahin, U. ; Ö. Türeci ; H. Schmitt et al.** Human neoplasms elicit multiple specific immune responses in the autologous host. *Proc. Natl. Acad. Sci. USA,* 1995, vol. 92, 11810-11813
- **Sahin, U. ; Tureci, O. ; Schmitt, H. ; Cochlovius, B. ; Johannes, T. ; Schmits, R. ; Stenner, F. ; Luo, G. ; Schobert, I. ; Pfreundschuh, M.** Human neoplasms elicit multiple specific immune responses in the autologous host. *PNAS USA,* 1995, vol. 92, 11810-11813
- **Saitoh, H. ; M. Delli Pizzi ; J. Wang.** Perturbation of SUMOlation enzyme Ubc9 by distinct domain within nucleoporin RanBP2/Nup358. *J. Biol. Chem.,* 2001
- **Sampson, E. R. ; S. Y. Yeh ; H. C. Chang et al.** Identification and characterization of androgen receptor associated coregulators in prostate cancer cells. *J. Biol. Regul. Homeost. Agents,* 2001, vol. 15, 123-129
- **Sato, M. ; T. Tanaka ; T. Maeno et al.** Inducible Expression of Endothelial PAS Domain Protein-1 by Hypoxia in Human Lung Adenocarcinoma A549 Cells. Role of src family kinases-dependent pathway. *Am. J. Respir. Cell. Mol. Biol.,* 2002, vol. 26, 127-134
- **Satoh, H. ; H. Kamma ; H. Ishikawa et al.** Expression of hnRNP A2/B1 proteins in human cancer cell lines. *Int. J. Oncol.,* 2000, vol. 16, 763-767
- **Scanlan, M. J. ; Jager, D.** Challenges to the development of antigen-specific breast cancer vaccines. *Breast Cancer Res,* 2001, vol. 3 (2), 95-8
- **Scanlan, M. J. ; Y.-T. Chen ; B. Williamson et al.** Charaterization of human colon cancer antigens recognized by autologous antibodies. *Int. J. Cancer,* 1998, vol. 76, 652-658
- **Scaturro, M. ; A. Cestelli ; D. Castiglia et al.** Posttranscriptional regulation of H1 zero and H3.3B histone genes in differentiating rat cortical neurons. *Neurochem. Res.,* 1995, vol. 20, 969-976
- **Schaeffer, L. ; R. Roy ; S. Humbert et al.** DNA repair helicase: a component of BTF2 (THFIIH) basic transcription factor. *Science,* 1993, vol. 260, 58-63
- **Schenk, P. W. ; A. W. Boersma ; J. A. Brandsma et al.** SKY1 is involved in cisplatin-induced cell kill in Saccharomyces cerevisiae, and inactivation of its human homologue, SRPK1, induces cisplatin resistance in a human ovarian carcinoma cell line. *Cancer Res.,* 2001, vol. 61, 6982-6986
- **Schenk, P. W. ; Boersma, A. W. ; Brandsma, J. A. ; den Dulk, H. ; Burger, H. ; Stoter, G. ; Brouwer, J. ; Nooter, K.** SKY1 is involved in cisplatin-induced cell kill in Saccharomyces cerevisiae, and inactivation of its human homologue, SRPK1, induces cisplatin resistance in a human ovarian carcinoma cell line. *Cancer Res,* 2001, vol. 61 (19), 6982-6
- **Schmits, R. ; Cochlovius, B. ; Treitz, G. ; Regitz, E. ; Ketter, R. ; Preuss, K. D. ; Romeike, B. F. ; Pfreundschuh, M.** Analysis of the antibody repertoire of astrocytoma patients against antigens expressed by gliomas. *Int J Cancer,* 2002, vol. 98 (1), 73-7
- **Schillace, R. V. ; S. F. Andrews ; G. A. Liberty et al.** Identification and characterization of myeloid translocation gene 16b as a novel kinase anchoring protein in T lymphocytes. *J. Immunol.,* 2002, vol. 168, 1590-1599
- **Schultze, J. L.** Vaccination as immunotherapy for B cell lymphoma. *Hematol. Oncol.,* 1997, vol. 15, 129-139
- **Schwab, M. ; L. C. Amler.** Amplification of cellular oncogenes: A predictor of clinical outcome in human cancer. *Genes Chrm. Cancer,* 1990, vol. 1, 81
- **Schweisguth, F. ; J. W. Posakony.** Suppressor of hairless, the drosophila homolog of the mouse recombination signal-binding protein gene, controls sensory organ cell fates. *Cell,* vol. 69, 1199-1212
- **Selenko, P. ; R. Sprangers ; G. Stier et al.** SMN tudor domain structure and its interaction with the Sm proteins. *Nat. Struct. Biol.,* 2001, vol. 8, 27-31

- **Shibahara, K. ; K. Sugio ; T. Osaki et al.** Nuclear expression of the Y-box binding protein, YB-1, as a novel marker of disease progression in non-small cell lung cancer. *Clin. Cancer Res.,* 2001, vol. 7, 3151-3155
- **Shipp, M. A. ; K. N. Ross ; P. Tamayo et al.** Diffuse large B-cell lymphoma outcome prediction by gene-expression profiling and supervised machine learning. *Nature Med.,* 2002, vol. 8, 68-74
- **Siegel, D. S. ; X. Zhang ; R. Feinman et al.** Hexamethylene bisacetamide induces programmed cell death (apoptosis) and down-regulates BCL-2 expression in human myeloma cells. *Proc. Natl. Acad. Sci. U.S.A.,* 1998, vol. 95, 162-166
- **Skotheim, R. I. ; S. M. Kraggerud ; S. D. Fossa et al.** Familial/bilateral and sporadic testicular germ line tumors show frequent genetic changes at loci with suggestive linkage evidence. *Neoplasia,* 2001, vol. 3, 196-203
- **Smith, C. ; V. Cerundolo.** Immunotherapy of melanoma. *Immunology,* 2001, vol. 104, 1-7
- **Smith, T. M. ; M. K. Lee ; C. I. Szabo et al.** Complete genomic sequence and analysis of 117 kb of human DNA containing the gene BRCA1. *Genome Res.,* 1996, vol. 6, 1029-1049
- **Spitkovsky, D. D. ; B. Royer-Pokora ; H. Delius et al.** Tissue restricted expression and chromosomal localzation of the YB-1 gene encoding a 42 kD nuclear CCAAT binding protein. *Nucl. Acids Res.,* 1992, vol. 20, 797-803
- **Staudt, L. M. ; A. L. Dent ; A. L. Shaffer et al.** Regulation of lymphocyte cell fate decisions and lymphomagenesis by BCL-6. *Int. Rev. Immunol.,* 1999, vol. 18, 381-403
- Diffuse large cell lymphomas of B and T cell type. **Stein, H. ; F. Dallenbach.** Neoplastic Hematopathology. Williams & Wilkins, 1992, 675-714
- **Stenina, O. I. ; K. M. Shaneyfelt ; P. E. DiCorleto.** Thrombin induces the release of the Y-box protein dbpB from mRNA: a mechnism of trancriptional activation. *Proc. Natl. Acad. Sci. U.S.A.,* 2001, vol. 98, 7277-7282
- **Stevenson, F. K.** DNA vaccines against cancer: from genes to therapy. *Ann Oncol,* 1999, vol. 10, 1913-8
- **Stevenson, F. K. ; D. Zhu ; J. Rice.** New strategies for vaccination and immunomodulation in NHL. *Ann Hematol,* 2001, vol. 80 (3), B132-4
- **Suhasini, M. ; R. B. Pilz.** Transcriptional elongation of c-myb is regulated by NF-kappaB (p50/RelB). *Oncogene,* 1999, vol. 18, 7360-7369
- **Sun, W. ; F. Hou ; M. P. Panchenko et al.** A member of the Y-box family interacts with an upstream element in the alphal(I) collagen gene. *Matrix Biol.,* 2001, vol. 20, 527-541
- **Syrengelas, A. D. ; T. T. Chen ; R. Levy.** DNA immunization induces protective immunity against B-cell lymphoma. *Nat. Med.,* 1996, vol. 2, 1038-1041
- **Takahashi, K. ; S. Shichijo ; M. Noguchi et al.** Identification of MAGE-1 and MAGE-4 proteins in spermatogonia and primary spermatocytes of testis. *Cancer Res.,* 1995, vol. 55, 3478-3482
- **Takayama, T. ; A. E. Morelli ; P. D. Robbins et al.** Feasibility of CTL4AIg gene delivery and expression in vivo usibg retrovirally transduced myeloid dendritic cells that induce alloantigen-specific T cell anergy in vitro. *Gene Ther,* 2000, vol. 7, 1265-73
- **Takeda, N. ; M. Shibuya ; Y. Maru.** The BCR-ABL oncoprotein potentially interacts with the xeroderma pigmentosum group B protein. *Proc. Natl. Acad. Sci. U.S.A,* 1999, vol. 96, 203-207
- **Takekawa, M. ; T. Maeda ; H. Saito.** Protein phosphatase 2C-alpha inhibits the human stress-responsive p38 and JNK MAPK pathways. *EMBO J.,* 1998, vol. 17, 4744-4752
- **Tarte, K. ; Z. G. Zhang ; E. Legouffe et al.** Induced expresion of B7-1 on myeloma cells following retroviral gene transfer results in tumor-specific recognition by cytotoxic T cells. *J Immunol,* 1999, vol. 163, 514-24
- **Travis, S. M. ; H. A. Berger ; M. J. Welsh.** Protein phosphatase 2C dephosphorylates and inactivates cystic fibrosis transmembrane conductance regulator. *Proc. Natl. Acad. Sci. U.S.A.,* 1997, vol. 94, 11055-11060
- **Tureci, O. ; Sahin, U. ; Pfreundschuh, M.** Serological analysis of human tumor antigens: molecular definition and implications. *Mol Med Today,* 1997, vol. 3 (8), 342-9
- **Türeci, O. ; U. Sahin ; M. Pfreundschuh.** Serological analysis of human tumor antigens: molecular definition and implications. *Mol. Med. Today,* 1997, vol. 3, 342-349
- **Türeci, Ö. ; U. Sahin ; C. Zwick et al.** Identification of a meiosis-specific protein as a member of the class of cancer/testis antigens. *Proc. Natl. Acad. Sci. U.S.A.,* 1998, vol. 95, 5211-5216
- **Türeci, Ö. ; U. Sahin ; C. Zwick et al.** Exploitation of the antibody repertoire of cancer patients for the identification of human tumor antigens. *Hybridoma,* 1999, vol. 18, 23-28
- **van der Bruggen, P. ; C. Traversari ; P. Chomez et al.** A gene encoding an antigen recognized by cytolytic T lymphocytes on a human melanoma. *Science,* 1991, vol. 254, 1643-1647
- **van der Bruggen, P. ; Szikora, J. P. ; Boel, P. ; Wildmann, C. ; Somville, M. ; Sensi, M. ; Boon, T.** Autologous cytolytic T lymphocytes recognize a MAGE-1 nonapeptide on melanomas expressing HLA-Cw*1601. *Eur J Immunol,* 1994, vol. 24 (9), 2134-40

- **van de Water, B. ; I. B. Tijens ; A. Verbrugge et al.** Cleavage of the actin-capping protein alpha -adducin at Asp-Asp-Ser-Asp633-Ala by caspase-3 is preceded by its phosphorylation on serine 726 in cisplatin-induced apoptosis of renal epithelial cells. *J. Biol. Chem.,* 2000, vol. 275, 25805-25813
- **Vaziri, H. ; S. K. Dessain ; E. N. Eaton et al.** hSIR2 (SIRT1) functions as an NAD-dependent p53 deacetylase. *Cell,* 2001, vol. 107, 149-159
- **Venkitaraman, A. R.** Functions of BRCA1 and BRCA2 in the biological response to DNA damage. *J. Cell Sci.,* 2001, vol. 114, 3591-3598
- **Vineis, P. ; G. Masala ; A. S. Costantini.** Does a gene in the Xq28 region increase the risk of non-Hodgkin's lymphomas? Working group for the Epidemiology of Hematolymphopoietic Malignancies in Italy. *Ann. Oncol.,* 1999, vol. 10, 471-473
- **Wang, H. Y. ; W. Lin ; J. A. Dyck et al.** SRPK2: a differentially expressed SR protein-specific kinase involved in mediating the interaction and localization of pre-mRNA splicing factors in mammalian cells. *J. Cell Biol.,* 1998, vol. 140, 737-750
- **Wang, X. ; S. Yeh ; G. Wu et al.** Identification and characterization of a novel androgen receptor coregulator ARA267-alpha in prostate cancer cells. *J. Biol. Chem.,* 2001, vol. 276, 40417-40423
- **Witt, O. ; W. Albig ; D. Doenecke.** Transcriptional regulation of the human replacement hitsone gene H3.3B. *FEBS Lett.,* 1997, vol. 408, 255-260
- **Witt, O. ; W. Albig ; D. Doenecke.** cAMP/phorbol ester response element is involved in transcriptional regulation of the human replacement histone gene H3.3B. *Biochem J.,* 1998, vol. 329, 609-613
- **Wu, J. ; D. Kraemer et al.** Nup358, a cytoplasmically exposed nucleoporin with peptide repeats, Ran-GTP binding sites, zinc fingers, a cyclophilin A homologous domain, and a leucine-rich region. *J. Biol. Chem.,* 1995, vol. 270, 14209-14213
- **Xia, G. ; Y. Kageyama ; T. Hayashi et al.** Regulation of vascular endothelial growth factor transcription by endothelial PAS domain protein 1 (EPAS1) and possible involvement of EPAS1 in the angiogenesis of renal cell carcinoma. *Cancer,* vol. 91, 1429-1436
- **Xie, X. ; Wacker, H.-H. ; Huang, S. ; Regitz, E. ; Preuss, K.-D. ; Romeike, B. ; Parwaresch, R. ; Tiemann, M. ; Pfreundschuh, M.** Differential Expression of Cancer Testis Genes in Histological Subtypes of Non-Hodgkin's Lymphomas. *Clin Cancer Res,* 2003, vol. 9 (1), 167-173
- **Yahata, H. ; Kobayashi, H. ; Kamura, T. ; Amada, S. ; Hirakawa, T. ; Kohno, K. ; Kuwano, M. ; Nakano, H.** Increased nuclear localization of transcription factor YB-1 in acquired cisplatin-resistant ovarian cancer. *J Cancer Res Clin Oncol,* 2002, vol. 128 (11), 621-6
- **Yamada, K. ; R. L. Printz ; H. Osawa et al.** Human ZHX1: cloning, chromosomal location, and interaction with transcription factor NF-Y. *Biochem. Biophys. Res. Commun.,* 1999, vol. 261, 614-621
- **Ylikorkala, A. ; D. J. Rossi ; N. Korsisaari et al.** Vascular abnormalities and deregulation of VEGF in Lkb1-deficient mice. *Science,* 2001, vol. 293, 1323-1326
- **Yuasa, K. ; K. Omori ; N. Yanaka.** Binding and phosphorylation of a novel male germ cell-specific cGMP-dependent protein kinse-anchoring protein by cGMP-dependent protein kinase Ia. *J. Biol. Chem.,* 2000, vol. 275, 4897-4905
- **Yun, J. ; H. D. Chae ; H. E. Choy et al.** p53 negatively regulates cdc2 transcription via the CCAAT-binding NF-Y transcription factor. *J. Biol. Chem.,* 1999, vol. 274, 29677-29682
- **Zendman, A. J. ; Ruiter, D. J. ; Van Muijen, G. N.** Cancer/testis-associated genes: Identification, expression profile, and putative function. *J Cell Physiol,* 2003, vol. 194 (3), 272-88
- **Zhang, A. ; K. Ohshima ; K. Sato et al.** Prognostic clinicopathologic factors, including immunologic expression in diffuse large B-cell lymphomas. *Pathol. Int.,* 1999, vol. 49, 1043-1052
- **Zhang, W. ; J. Mi ; N. Li et al.** Identification and characterisation of DPZF, a novel human BTB/POZ zinc finger protein sharing homology to BCL-6. *Biochem. Biophys. Res. Commun.,* 2001, vol. 282, 1067-1073
- **Zhou, J. ; D. C. Allred ; I. Avis et al.** Differential expression of the early lung cancer detection marker, heterogeneous nuclear ribonucleoprotein-A2/B1 (hnRNP-A2/B1) in normal breast and neoplastic breast cancer. *Breast Cancer Res. Treat.,* 2001, vol. 66, 217-224
- **Zhou, J. ; L. Nong ; M. Wloch et al.** Expression of early lung cancer detection marker: hnRNP-A2/B1 and its relation to microsatellite alteration in non-small cell lung cancer. *Lung Cancer,* 2001, vol. 34, 341-350